# EUROPEAN PATENT APPLICATION

(11) **EP 4 180 432 A1**
(43) Date of publication of application: **17.05.2023**
(21) Application number: 21837179.7
(22) Date of filing: 07.07.2021
(51) Int. Cl.: C07D 487/04, C07D 403/00, A61K 31/395, A61P 35/00, A61P 29/00, A61P 37/06, A61P 19/02, A61P 25/00

(54) **COMPOUND HAVING BTK KINASE DEGRADING ACTIVITY, AND PREPARATION METHOD AND PHARMACEUTICAL USE THEREFOR**

(30) Priority: 07.07.2020 CN 202010644112; 10.11.2020 CN 202011237752
(71) Applicant: Sichuan Haisco Pharmaceutical Co., Ltd., Sichuan Province 611130 (CN)
(72) Inventor: ZHANG, Chen, Chengdu City, Sichuan 611130 (CN); LIAO, Yuting, Chengdu City, Sichuan 611130 (CN); WANG, Jianmin, Chengdu City, Sichuan 611130 (CN); CHENG, Xinfan, Chengdu City, Sichuan 611130 (CN); CHEN, Xiaogang, Chengdu City, Sichuan 611130 (CN); ZOU, Sijia, Chengdu City, Sichuan 611130 (CN); YUAN, Shuai, Chengdu City, Sichuan 611130 (CN); YE, Fei, Chengdu City, Sichuan 611130 (CN); TANG, Pingming, Chengdu City, Sichuan 611130 (CN); ZHU, Guozhi, Chengdu City, Sichuan 611130 (CN); HUANG, Zhenggang, Chengdu City, Sichuan 611130 (CN); WU, Shoutao, Chengdu City, Sichuan 611130 (CN); LI, Yao, Chengdu City, Sichuan 611130 (CN); NI, Jia, Chengdu City, Sichuan 611130 (CN); YAN, Pangke, Chengdu City, Sichuan 611130 (CN)
(74) Representative: Hoffmann Eitle
(86) International application number: PCT/CN2021/104905
(87) International publication number: WO 2022/007824

(57) **Abstract**

A compound as shown in general formula (I) or stereoisomers, deuterated compounds, solvates, prodrugs, metabolites, pharmaceutically acceptable salts or co-crystals thereof, and intermediates thereof, a preparation method therefor, and a use thereof for treating BTK-related diseases, such as cancer or autoimmune diseases.

B-L-K (I)

## Description

### Technical Field

The present invention relates to a compound represented by general formula (I) or a stereoisomer, a solvate, a prodrug, a metabolite, a pharmaceutically acceptable salt or a co-crystal thereof, and intermediates thereof and preparation methods therefor, as well as the use thereof in BTK-related diseases such as tumors or autoimmune system diseases.

### Background Art

Bruton's tyrosine kinase (BTK), a member of the Tec family of non-receptor protein tyrosine kinases, is a key regulator in the B cell antigen receptor (BCR) signaling pathway, and is distributed in the lymphatic system, hematopoietic system and blood system. BTK mutations may activate downstream signaling pathways in tumor cell proliferation, differentiation, angiogenesis, etc., which may lead to X-linked agammaglobulinemia, non-Hodgkin's lymphoma (NHL) and many B-cell malignancies, including chronic lymphocytic leukemia (CLL), mantle cell lymphoma, and diffuse large B-cell lymphoma. As mainly expressed in B cells and myeloid cells, BTK is a target with relatively high targeting ability and safety.

PROTAC (proteolysis targeting chimera) molecules are a class of dual function compounds which are capable of binding to both targeted proteins and E3 ubiquitin ligases. This class of compounds can be recognized by proteasomes in a cell to cause the degradation of the targeted proteins, which can effectively reduce the contents of the targeted proteins in the cell. By introducing a ligand capable of binding to various targeted proteins into the PROTAC molecules, it is possible to apply the PROTAC technology to the treatment of various diseases, and this technology has attracted extensive attention in recent years.

### Summary of the Invention

The present invention develops a BTK inhibitor with a novel structure, good efficacy, high bioavailability and higher safety, for use in the treatment of BTK-related diseases such as tumors or autoimmune system diseases.

The present invention develops a PROTAC compound of BTK inhibitors and E3 ubiquitin ligases, which has a novel structure, good efficacy, high bioavailability and higher safety, and can inhibit or degrade BTKs, for use in the treatment of BTK-related diseases such as tumors or autoimmune system diseases.

The present invention relates to a compound represented by general formula (I) or a stereoisomer, a solvate, a prodrug, a metabolite, a pharmaceutically acceptable salt or a co-crystal thereof, wherein

B-L-K (I);

In some embodiments, L is selected from -Ak1-Cy1-Ak2-Cy2-Ak3-Cy3-Ak4-Cy4-Ak5- or a bond;
in some embodiments, L is selected from -Cy1-CH₂-Cy2-;
in some embodiments, Ak1, Ak2, Ak3, Ak4 and Ak5 are each independently selected from CH₂, O or a bond;
in some embodiments, Cy1, Cy2, Cy3 and Cy4 are each independently selected from 3- to 12-membered heterocyclic ring, 3- to 12-membered cycloalkyl, 6- to 10-membered aryl or a bond, wherein the heterocyclic ring, cycloalkyl or aryl is optionally further substituted with 0 to 4 substituents selected from H, F, Cl, Br, I, OH, NH₂, oxo (O=), CF₃, COOH, CN, C₁₋₄ alkyl, hydroxyl-substituted C₁₋₄ alkyl, halogen-substituted C₁₋₄ alkyl or C₁₋₄ alkoxy, and the heterocyclic ring contains 1 to 4 heteroatoms selected from O, S or N;
in some embodiments, Cy1, Cy2, Cy3 and Cy4 are each independently selected from a bond, 4- to 7-membered mono-heterocyclic ring, 5- to 10-membered fused heterocyclic ring, 6- to 12-membered spiro-heterocyclic ring, 7- to 10-membered bridged-heterocyclic ring, 4- to 7-membered monocycloalkyl, 5- to 10-membered fused cycloalkyl, 6- to 12-membered spiro cycloalkyl, 7- to 10-membered bridged cycloalkyl or 6- to 10-membered aryl, wherein the aryl, cycloalkyl (monocycloalkyl, fused cycloalkyl, spiro cycloalkyl and bridged cycloalkyl), mono-heterocyclic ring, fused heterocyclic ring, spiro-heterocyclic ring or bridged-heterocyclic ring optionally is further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy, and the mono-heterocyclic ring, fused heterocyclic ring, spiro-heterocyclic ring or bridged-heterocyclic ring contains 1 to 4 heteroatoms selected from O, S or N;
in some embodiments, Cy1, Cy2, Cy3 and Cy4 are each independently selected from a bond or one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 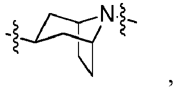 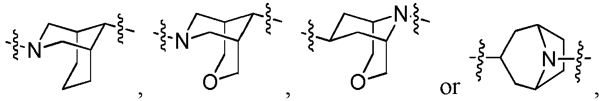 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
in some embodiments, Cy1, Cy2, Cy3 and Cy4 are each independently selected from a bond or one of the following substituted or unsubstituted groups: 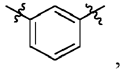 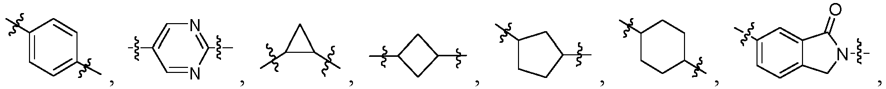 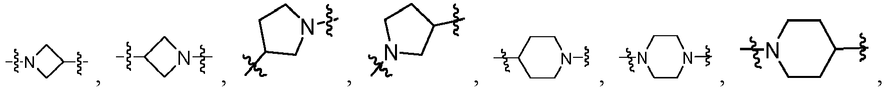 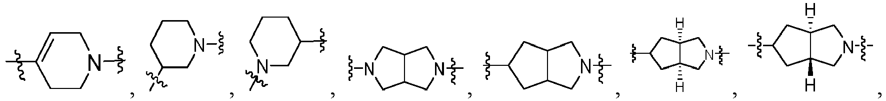 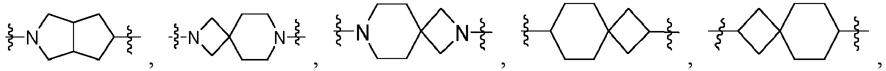 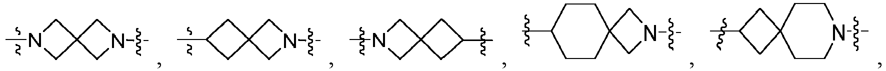 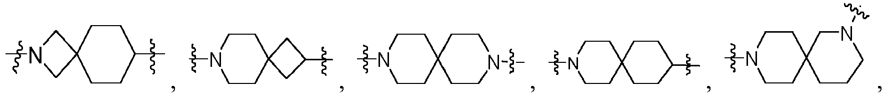 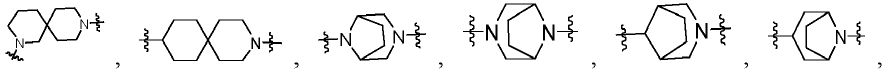 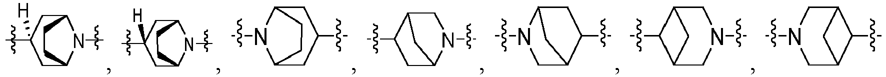 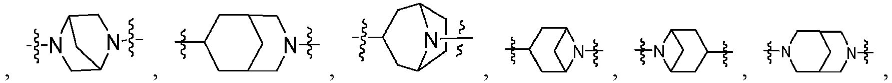 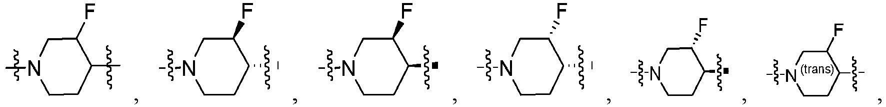 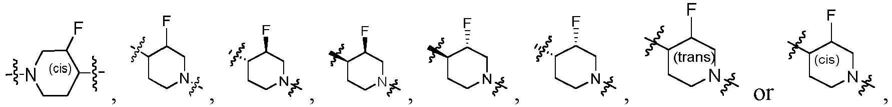 which, when substituted, is optionally further substituted with 0 to 4 substituents selected from H, F, CF₃, methyl, =O, hydroxymethyl, COOH, CN or NH₂;
Cy1, Cy2, Cy3 and Cy4 cannot all be a bond;
when Ak1, Ak2, Ak3, Ak4 or Ak5 is O, they cannot be directly connected to B;
when Ak1, Ak2, Ak3, Ak4 or Ak5 is not a bond, they cannot be directly connected to one another;
when 4 or more of Ak1, Cy1, Ak2, Cy2, Ak3, Cy3, Ak4, Cy4 and Ak5 are not a bond, at least one of Cy1, Cy2, Cy3 and Cy4 is not piperidine, piperazine, pyrimidine or pyridine;
in some embodiments, B is selected from B1-W1-B2-B3-B4-;
in some embodiments, B1 is selected from 6-membered heteroaryl ring or phenyl, wherein the heteroaryl ring or phenyl is optionally further substituted with 0 to 4 R^{b1}, and the heteroaryl ring contains 1 to 4 heteroatoms selected from O, S or N;
in some embodiments, B1 is selected from phenyl or pyridyl, wherein the phenyl or pyridyl is optionally further substituted with 0 to 4 R^{b1},
in some embodiments, W1 is selected from -O-, -S-, -NH-, -NHCO- or -CONH-;
in some embodiments, W1 is selected from -O-, -NHCO- or -CONH-;
in some embodiments, B2 is selected from 6-membered heteroaryl ring or phenyl, wherein the heteroaryl ring or phenyl is optionally further optionally substituted with 0 to 4 R^{b2}, and the heteroaryl ring contains 1 to 4 heteroatoms selected from O, S or N;
in some embodiments, B3 is selected from 8- to 10-membered fused heterocyclic ring, wherein the fused heterocyclic ring is optionally further optionally substituted with 0 to 4 R^{b3}, and the fused heterocyclic ring contains 1 to 4 heteroatoms selected from O, S or N;
in some embodiments, B4 is selected from 4- to 10-membered saturated heterocyclic ring, wherein the saturated heterocyclic ring is selected from monocyclic ring, fused ring or spiro ring, the saturated heterocyclic ring, monocyclic ring, fused ring or spiro ring is optionally further optionally substituted with 0 to 4 R^{b4}, and the saturated heterocyclic ring contains 1 to 2 heteroatoms selected from O, S or N;
in some embodiments, B2 is selected from phenyl or pyridyl, wherein the phenyl or pyridyl is optionally further substituted with 0 to 4 R^{b2};
in some embodiments, B3 is selected from one of the following substituted or unsubstituted groups: imidazopyrimidine, pyrazolopyrimidine, imidazopyrazine, pyrazolopyrazine, imidazotetrahydropyrimidine or pyrazolotetrahydropyrimidine which, when substituted, is optionally further optionally substituted with 0 to 4 R^{b3};
in some embodiments, B4 is selected from one of the following substituted or unsubstituted groups: azetidinyl, azacyclopentyl, piperidine, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, cyclobutyl-spiro-piperidine, cyclopentyl-spiro-piperidine, azetidinyl-spiro-piperidine, azacyclopentyl-spiro-piperidine, 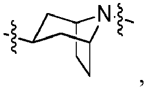 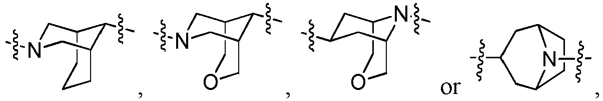 which, when substituted, is optionally further optionally substituted with 0 to 4 R^{b4}; and
in some embodiments, B is selected from 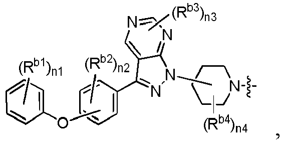 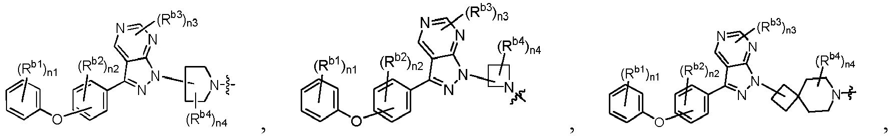 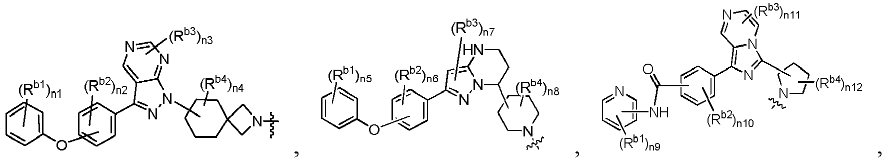 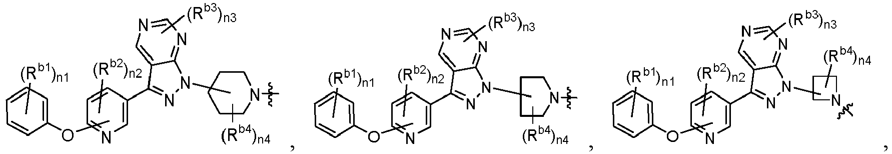 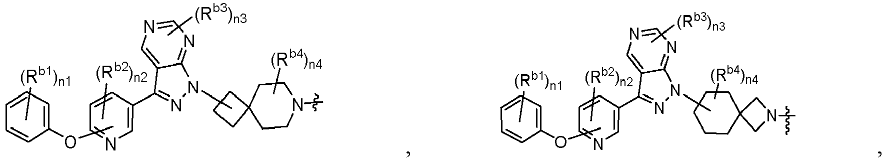 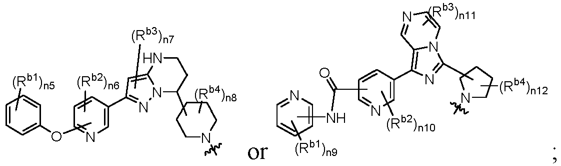
in some embodiments, B is selected from 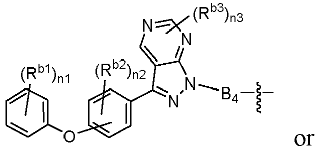 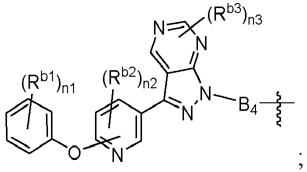
in some embodiments, B is selected from 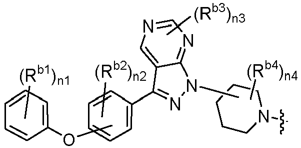 or 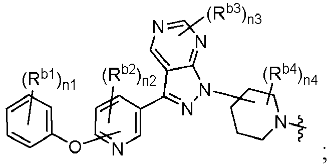
in some embodiments, B is selected from 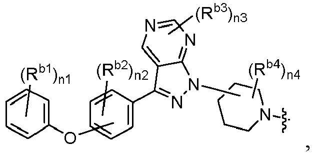 and at least one of Cy1, Cy2, Cy3 and Cy4 is substituted with 1 to 4 substituents selected from CF₃, COOH, CN or hydroxyl-substituted C₁₋₄ alkyl;
in some embodiments, R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy, and n1, n2, n3, and n4 is each independently selected from 0, 1, 2, 3 or 4;
in some embodiments, R^{b1}, R^{b2}, R^{b3} or R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I, and n1, n2, n3, and n4 is each independently selected from 0, 1, 2, 3 or 4;
when 3 of Cy1, Cy2, Cy3 and Cy4 are a bond, B is not 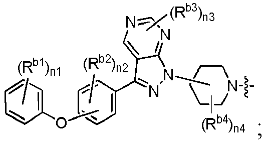
in some embodiments, R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
in some embodiments, B is selected from 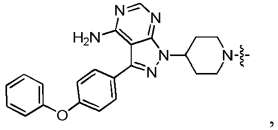 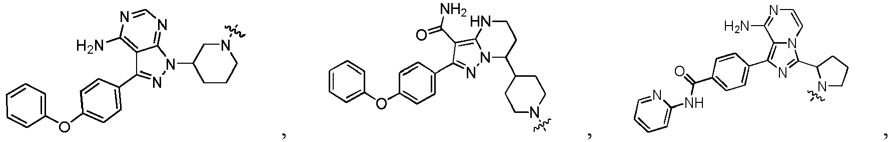 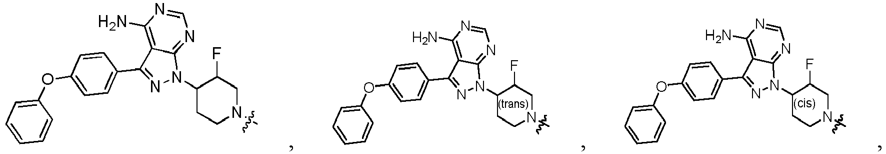 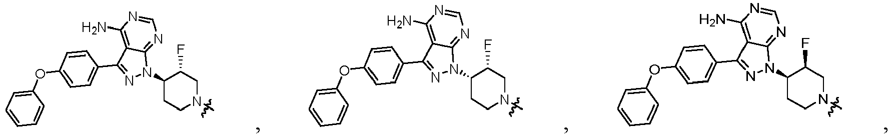 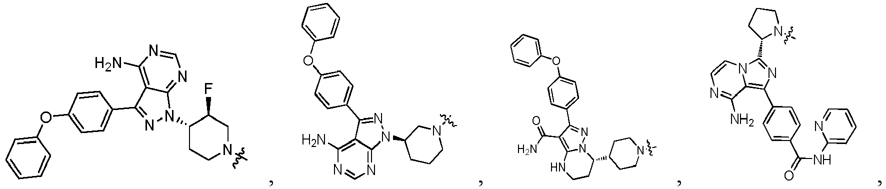 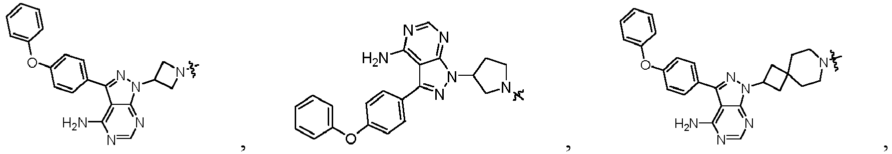 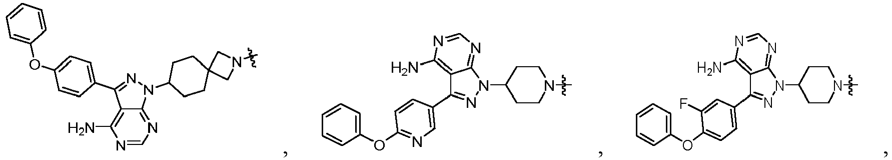 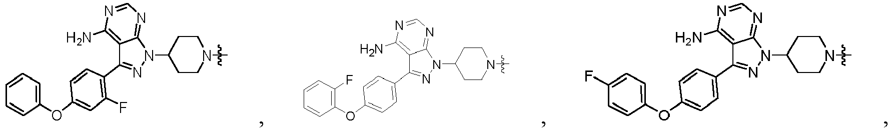 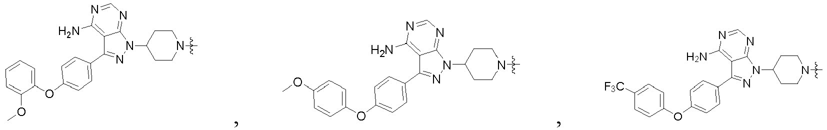 or 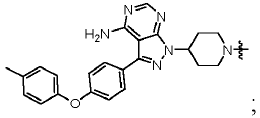
in some embodiments, B is selected from 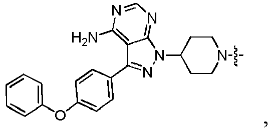 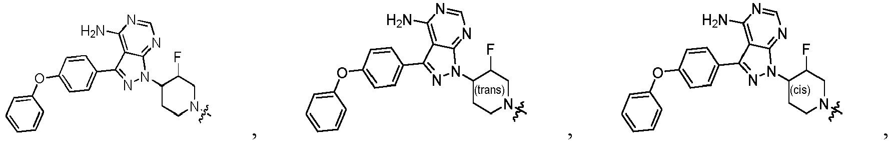 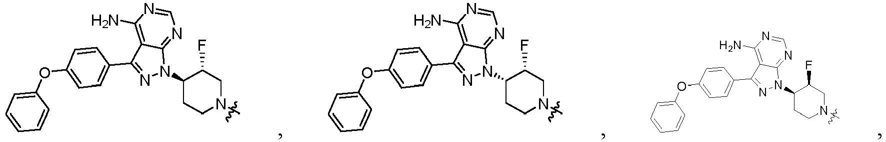 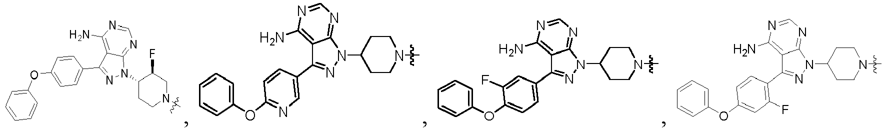 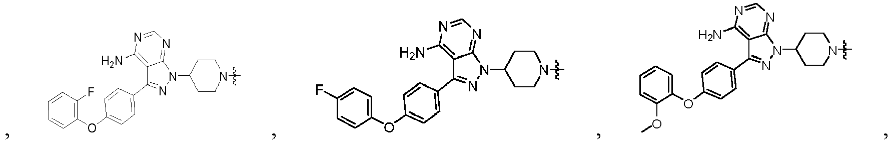 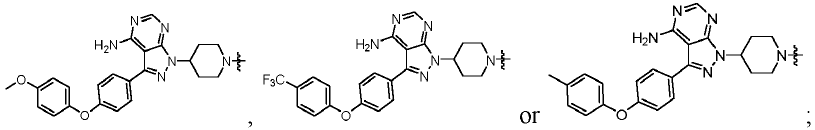
in some embodiments, B is selected from 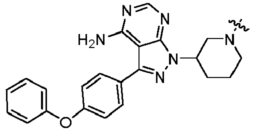 or 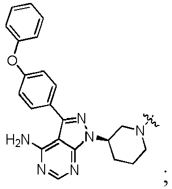
in some embodiments, B is selected from 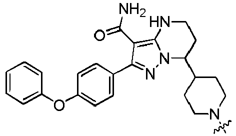 or 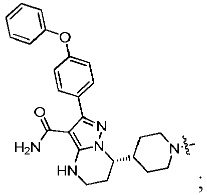
in some embodiments, B is selected from 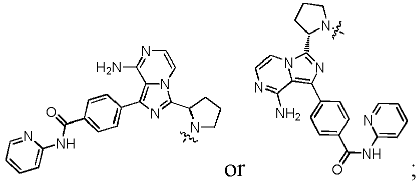
in some embodiments, B is selected from 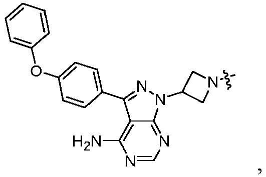 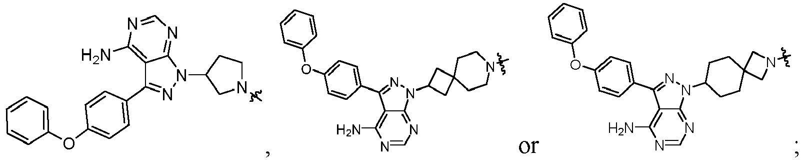
in some embodiments, B-L is selected from 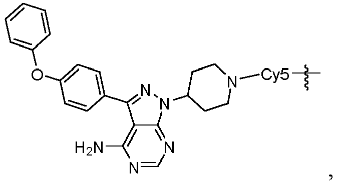 wherein Cy5 is selected from one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 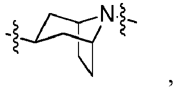 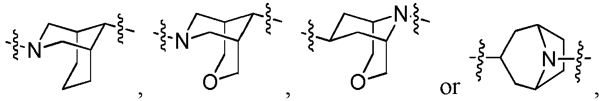 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
in some embodiments, Cy5 is selected from one of the following substituted or unsubstituted groups: 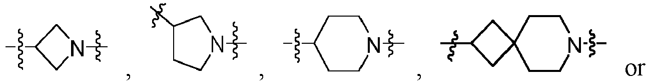 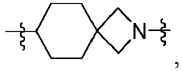 which, when substituted, is optionally further substituted with 0 to 4 substituents selected from H, F, Cl, Br, I, OH, NH₂, oxo, CF₃, COOH, CN, methyl, hydroxymethyl or methoxy;
in some embodiments, B is selected from 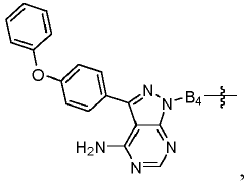 wherein B4 is selected from one of the following substituted or unsubstituted groups: azetidinyl, azacyclopentyl, 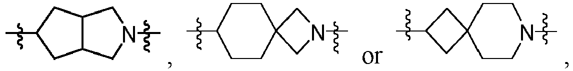 which, when substituted, is optionally further optionally substituted with 0 to 4 R^{b4};
R^{b4} is each independently selected from H, F, Cl, Br, I, OH, NH₂, CN, CF₃, CONH₂, COOH, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
in some embodiments, B is selected from 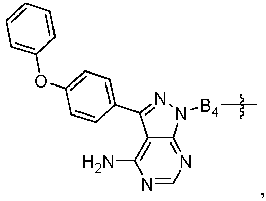 wherein B4 is selected from one of the following substituted or unsubstituted groups: 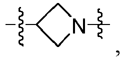 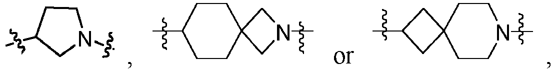 which, when substituted, is optionally further optionally substituted with 0 to 4 substituents selected from R^{b4};
R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I;
in some embodiments, L is selected from a bond, 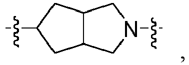 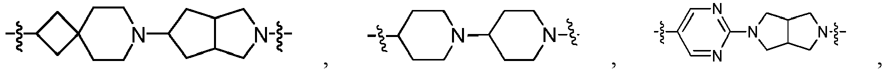 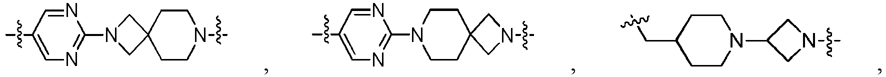 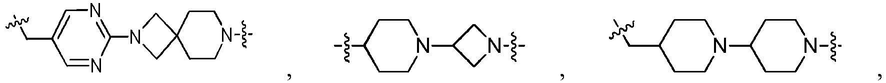 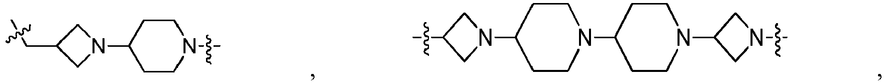 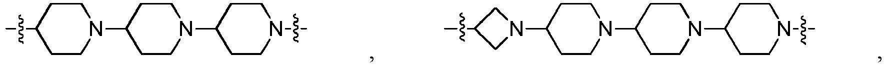 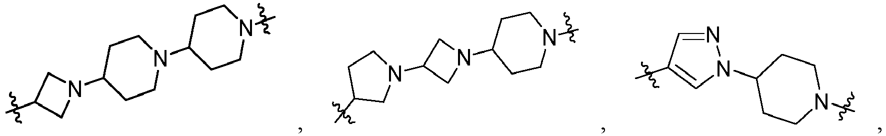 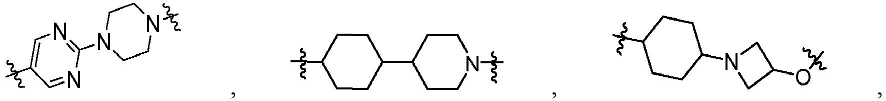 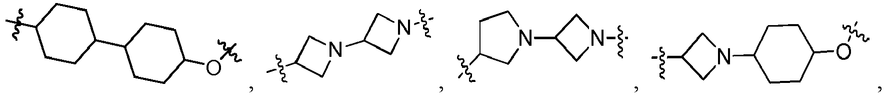 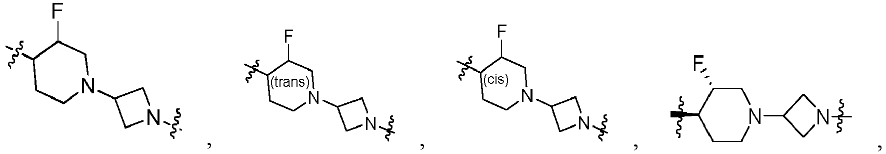 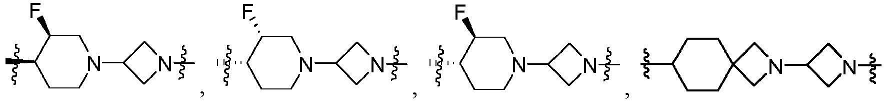 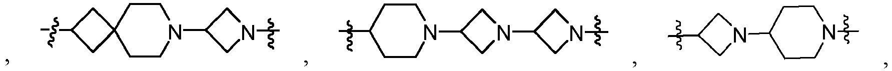 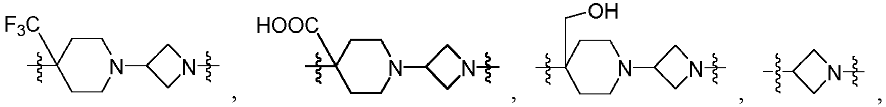 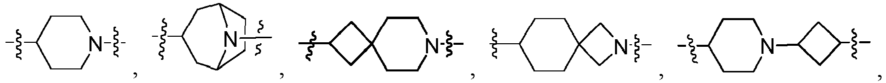 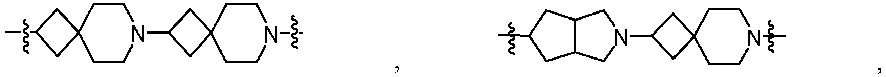 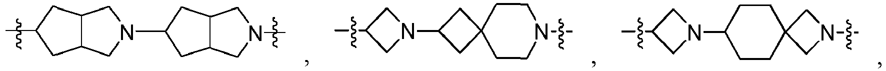 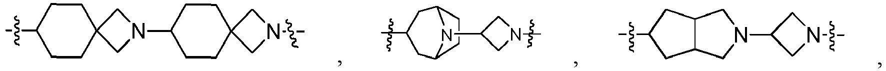 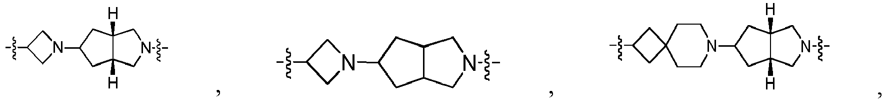 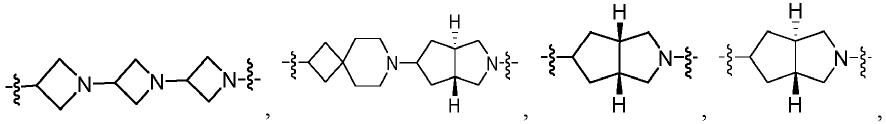 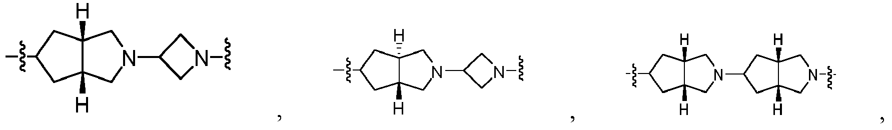 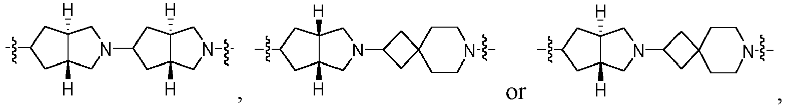 and L is connected to B on the left side, and is connected to K on the right side;
in some embodiments, L is selected from 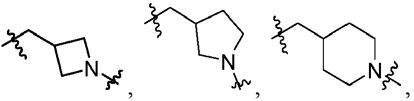 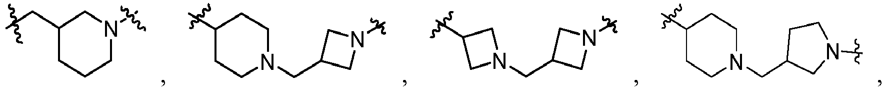 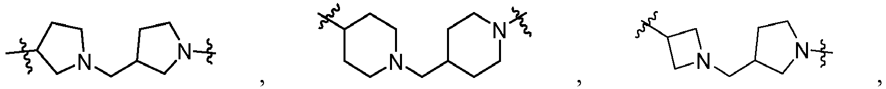 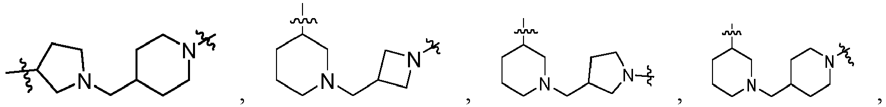 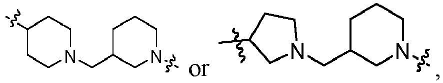 and L is connected to B on the left side, and is connected to K on the right side;
in some embodiments, L is selected from 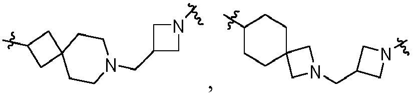 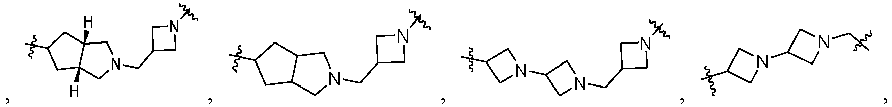 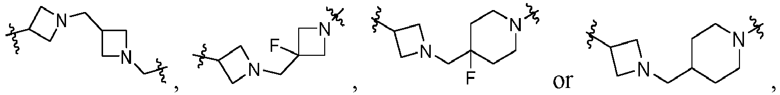 and L is connected to B on the left side, and is connected to K on the right side;
in some embodiments, L is selected from 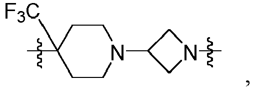 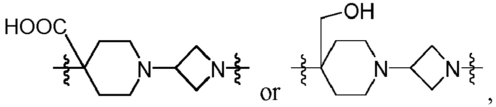 and L is connected to B on the left side, and is connected to K on the right side;
in some embodiments, K is selected from 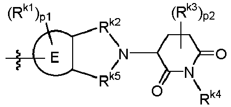 or 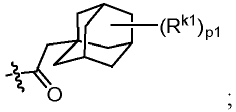
in some embodiments, K is selected from 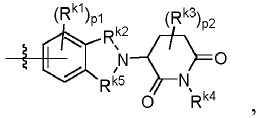 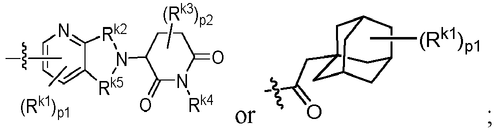
in some embodiments, K is selected from 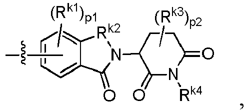 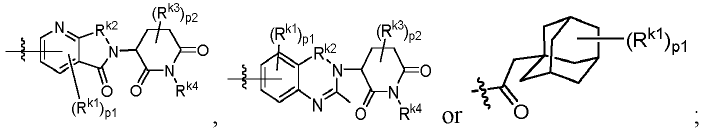
in some embodiments, K is selected from 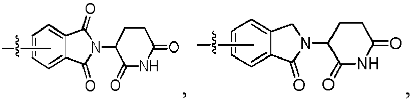 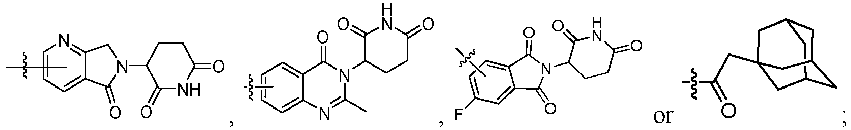
in some embodiments, K is selected from 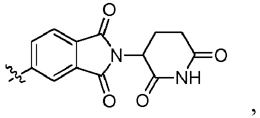 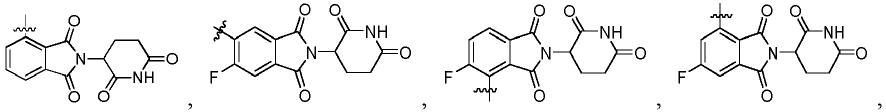 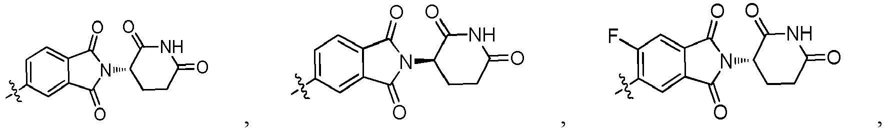 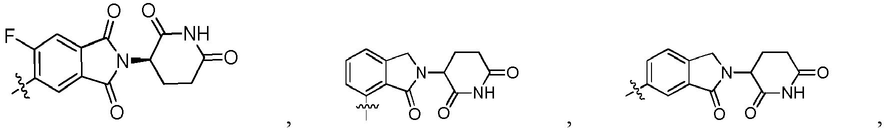 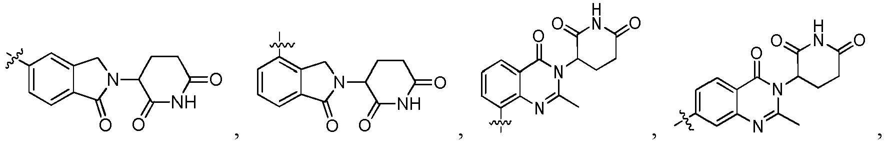 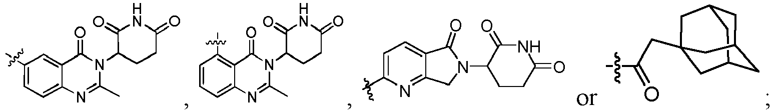
in some embodiments, K is selected from 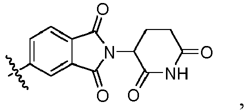 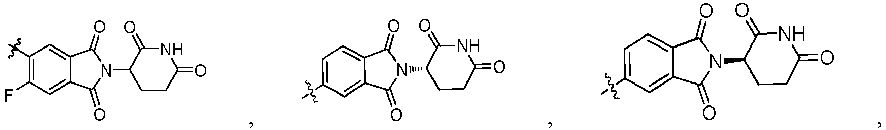 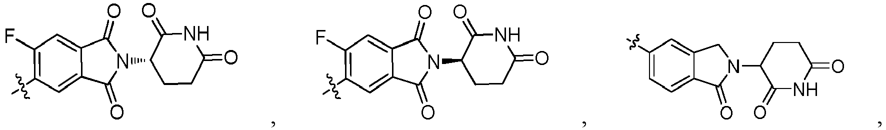 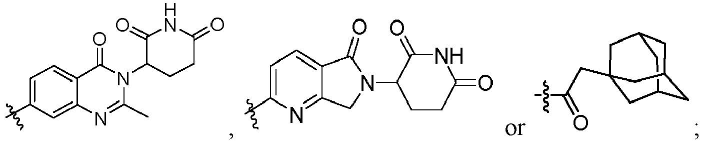
in some embodiments, ring E is selected from benzene ring or 5- to 6-membered heteroaryl ring, wherein the heteroaryl ring contains 1 to 2 heteroatoms selected from O, S or N;
in some embodiments, ring E is selected from benzene ring, pyridine, thiophene, furan, pyrrole, thiazole, oxazole, imidazole or pyrazole;
in some embodiments, R^{k2} is each independently selected from CH₂, C=O, S=O or SO₂;
in some embodiments, R^{k1}, R^{k3} or R^{k4} is each independently selected from H, F, Cl, Br, I, OH, NH₂, CF₃, CN, COOH, C₁₋₄ alkyl or C₁₋₄ alkoxy;
in some embodiments, R^{k5} is selected from C=O or 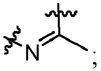 p1 or p2 is each independently selected from 0, 1, 2, 3 or 4;
n1, n2, n3, and n4 are each independently selected from 0, 1, 2, 3 or 4; and
in some embodiments, R^{k2} is each independently selected from CH₂ or C=O, R^{k1}, R^{k3} or R^{k4} is each independently selected from H, F, Cl, Br, I, OH or NH₂, and p1 or p2 is each independently selected from 0, 1 or 2.

As a first embodiment of the present invention, provided is a compound represented by general formula (I) or a stereoisomer, a solvate, a prodrug, a metabolite, a pharmaceutically acceptable salt or a co-crystal thereof, wherein L is selected from - Ak1-Cy1-Ak2-Cy2-Ak3-Cy3-Ak4-Cy4-Ak5- or a bond;
Ak1, Ak2, Ak3, Ak4 and Ak5 are each independently selected from CH₂, O or a bond;
Cy1, Cy2, Cy3 and Cy4 are each independently selected from 3-membered heterocyclic ring, 4-membered heterocyclic ring, 5-membered heterocyclic ring, 6-membered heterocyclic ring, 7-membered heterocyclic ring, 8-membered heterocyclic ring, 9-membered heterocyclic ring, 10-membered heterocyclic ring, 11-membered heterocyclic ring, 12-membered heterocyclic ring, 3-membered cycloalkyl, 4-membered cycloalkyl, 5-membered cycloalkyl, 6-memberedcycloalkyl, 7-membered cycloalkyl, 8-membered cycloalkyl, 9-membered cycloalkyl, 10-membered cycloalkyl, 11-membered cycloalkyl, 12-membered cycloalkyl, 6- to 10-membered aryl or a bond, wherein the heterocyclic ring, cycloalkyl or aryl is optionally further substituted with 0 to 4 substituents selected from H, F, Cl, Br, I, OH, NH₂, oxo, CF₃, COOH, CN, C₁₋₄ alkyl , hydroxyl-substituted C₁₋₄ alkyl, halogen-substituted C₁₋₄ alkyl or C₁₋₄ alkoxy, and the heterocyclic ring contains 1 to 4 heteroatoms selected from O, S or N;
Cy1, Cy2, Cy3 and Cy4 cannot all be a bond;
when Ak1, Ak2, Ak3, Ak4 or Ak5 is O, they cannot be directly connected to B;
when Ak1, Ak2, Ak3, Ak4 or Ak5 is not a bond, they cannot be directly connected to one another;
when 4 or more of Ak1, Cy1, Ak2, Cy2, Ak3, Cy3, Ak4, Cy4 and Ak5 are not a bond, at least one of Cy1, Cy2, Cy3 and Cy4 is not piperidine, piperazine, pyrimidine or pyridine;
B is selected from B1-W1-B2-B3-B4-;
B1 is selected from 6-membered heteroaryl ring or phenyl, wherein the heteroaryl ring or phenyl is optionally further substituted with 0 to 4 R^{b1}, and the heteroaryl ring contains 1 to 4 heteroatoms selected from O, S or N;
W1 is selected from -O-, -S-, -NH-, -NHCO- or -CONH-;
B2 is selected from 6-membered heteroaryl ring or phenyl, wherein the heteroaryl ring or phenyl is optionally further substituted with 0 to 4 R^{b2}, and the heteroaryl ring contains 1 to 4 heteroatoms selected from O, S or N;
B3 is selected from 8- to 10-membered fused heterocyclic ring, wherein the fused heterocyclic ring is optionally further substituted with 0 to 4 R^{b3}, and the fused heterocyclic ring contains 1 to 4 heteroatoms selected from O, S or N;
B4 is selected from 4- to 10-membered saturated heterocyclic ring, wherein the saturated heterocyclic ring is selected from monocyclic ring, fused ring or spiro ring, the saturated heterocyclic ring, monocyclic ring, fused ring or spiro ring is optionally further substituted with 0 to 4 R^{b4}, and the saturated heterocyclic ring contains 1 to 2 heteroatoms selected from O, S or N;
when 3 of Cy1, Cy2, Cy3 and Cy4 are a bond, B is not 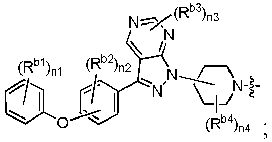 R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
K is selected from 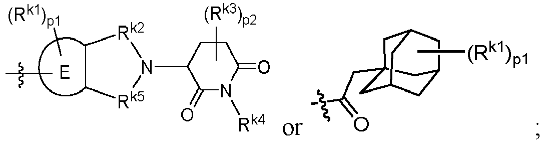
ring E is selected from benzene ring or 5- to 6-membered heteroaryl ring, wherein the heteroaryl ring contains 1 to 2 heteroatoms selected from O, S or N;
R^{k2} is each independently selected from CH₂, C=O, S=O or SO₂;
R^{k1}, R^{k3} or R^{k4} is each independently selected from H, F, Cl, Br, I, OH, NH₂, CF₃, CN, COOH, C₁₋₄ alkyl or C₁₋₄ alkoxy;
R^{k5} is selected from C=O or 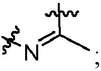 p1 or p2 is each independently selected from 0, 1, 2, 3 or 4;
n1, n2, n3, and n4 are each independently selected from 0, 1, 2, 3 or 4; and optionally, the compound is not a compound shown in Table A.

As a second embodiment of the present invention, provided is a compound represented by general formula (I) or a stereoisomer, a solvate, a prodrug, a metabolite, a pharmaceutically acceptable salt or a co-crystal thereof, wherein
L is selected from -Ak1-Cy1-Ak2-Cy2-Ak3-Cy3-Ak4-Cy4-Ak5- or a bond;
Ak1, Ak2, Ak3, Ak4 and Ak5 are each independently selected from CH₂, O or a bond;
Cy1, Cy2, Cy3 and Cy4 are each independently selected froma bond, 4-membered mono-heterocyclic ring, 5-membered mono-heterocyclic ring, 6-membered mono-heterocyclic ring, 7-membered mono-heterocyclic ring, 5-membered fused heterocyclic ring, 6-membered fused heterocyclic ring, 7-membered fused heterocyclic ring, 8-membered fused heterocyclic ring, 9-membered fused heterocyclic ring, 10-membered fused heterocyclic ring, 6-membered spiro-heterocyclic ring, 7-membered spiro-heterocyclic ring, 8-membered spiro-heterocyclic ring, 9-membered spiro-heterocyclic ring, 10-membered spiro-heterocyclic ring, 11-membered spiro-heterocyclic ring, 12-membered spiro-heterocyclic ring, 7-membered bridged-heterocyclic ring, 8-membered bridged-heterocyclic ring, 9-membered bridged-heterocyclic ring, 10-membered bridged-heterocyclic ring, 4-membered monocycloalkyl, 5-membered monocycloalkyl, 6-membered monocycloalkyl, 7-membered monocycloalkyl, 5-membered fused cycloalkyl, 6-membered fused cycloalkyl, 7-membered fused cycloalkyl, 8-membered fused cycloalkyl, 9-membered fused cycloalkyl, 10-membered fused cycloalkyl, 6-membered spiro cycloalkyl, 7-membered spiro cycloalkyl, 8-membered spiro cycloalkyl, 9-membered spiro cycloalkyl, 10-membered spiro cycloalkyl, 11-membered spiro cycloalkyl, 12-membered spiro cycloalkyl, 7-membered bridged cycloalkyl, 8-membered bridged cycloalkyl, 9-membered bridged cycloalkyl, 10-membered bridged cycloalkyl or 6- to 10-membered aryl, wherein the aryl, cycloalkyl, mono-heterocyclic ring, fused heterocyclic ring, spiro-heterocyclic ring or bridged-heterocyclic ring is optionally further substituted with 0, 1, 2, 3 or 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy, and the mono-heterocyclic ring, fused heterocyclic ring, spiro-heterocyclic ring or bridged-heterocyclic ring contains 1, 2, 3 or 4 heteroatoms selected from O, S or N;
B1 is selected from phenyl or pyridyl, wherein the phenyl or pyridyl is optionally further substituted with 0 to 4 R^{b1};
W1 is selected from -O-, -NHCO- or -CONH-;
B2 is selected from phenyl or pyridyl, wherein the phenyl or pyridyl is optionally further substituted with 0 to 4 R^{b2};
B3 is selected from one of the following substituted or unsubstituted groups: imidazopyrimidine, pyrazolopyrimidine, imidazopyrazine, pyrazolopyrazine, imidazotetrahydropyrimidine or pyrazolotetrahydropyrimidine which, when substituted, is optionally further optionally substituted with 0 to 4 R^{b3};
B4 is selected from one of the following substituted or unsubstituted groups: azetidinyl, azacyclopentyl, piperidine, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, cyclobutyl-spiro-piperidine, cyclopentyl-spiro-piperidine, azetidinyl-spiro-piperidine, azacyclopentyl-spiro-piperidine, 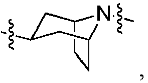 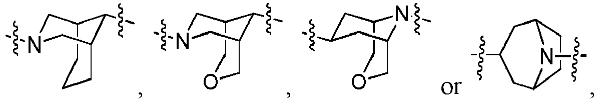 which, when substituted, is optionally further optionally substituted with 0 to 4 R^{b4}; and
ring E is selected from benzene ring, pyridine, thiophene, furan, pyrrole, thiazole, oxazole, imidazole or pyrazole; and
the definitions of other groups are the same as those in the first embodiment.

As a third embodiment of the present invention, provided is a compound represented by general formula (I) or a stereoisomer, a solvate, a prodrug, a metabolite, a pharmaceutically acceptable salt or a co-crystal thereof, wherein
K is selected from 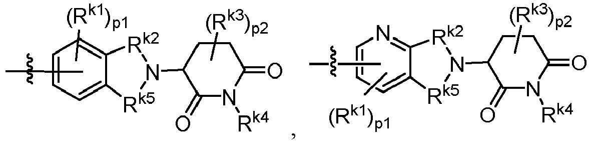 or 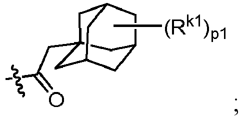 and
the definitions of other groups are the same as those in the second embodiment.

As a fourth embodiment of the present invention, provided is a compound represented by general formula (I) or a stereoisomer, a solvate, a prodrug, a metabolite, a pharmaceutically acceptable salt or a co-crystal thereof, wherein
Cy1, Cy2, Cy3 and Cy4 are each independently selected from a bond or one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 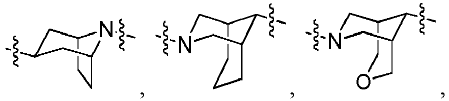 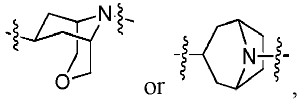 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
B is selected from 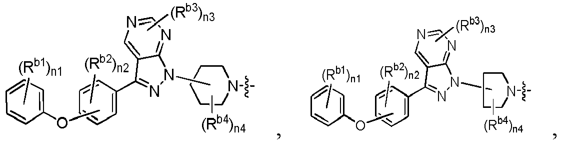 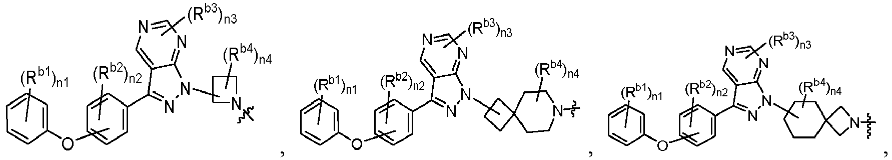 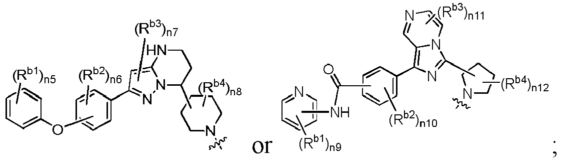
or B is selected from 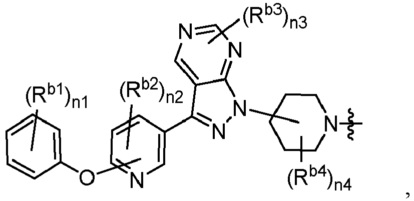 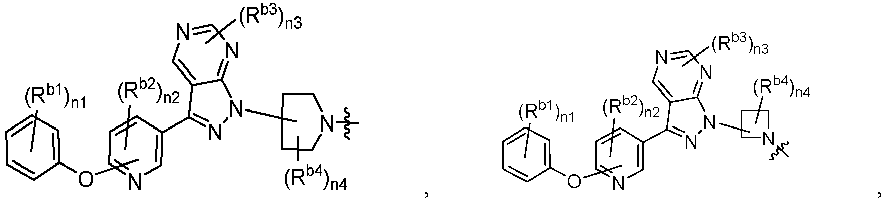 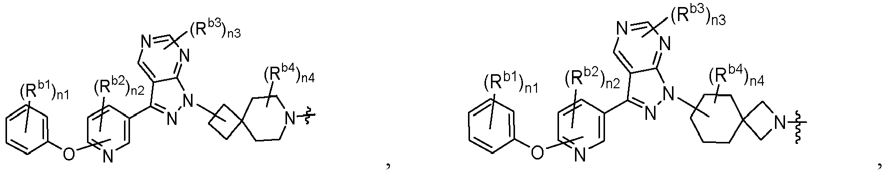 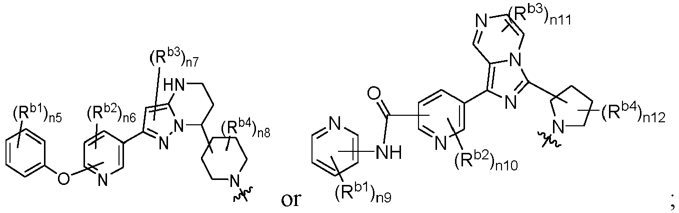
R^{b1}, R^{b2}, R^{b3} or R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I;
n1, n2, n3, n4, n5, n6, n7, n8, n9, n10, n11, and n12 are each independently selected from 0, 1, 2, 3 or 4;
K is selected from 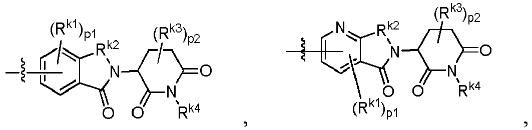 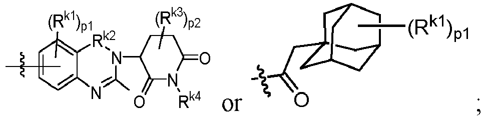
R^{k2} is each independently selected from CH₂ or C=O;
R^{k1}, R^{k3} or R^{k4} is each independently selected from H, F, Cl, Br, I, OH or NH₂; and
p1 or p2 is each independently selected from 0, 1 or 2; and
the definitions of other groups are the same as those in the third embodiment of the present invention.

As a fifth embodiment of the present invention, provided is a compound represented by general formula (Ia) or a stereoisomer, a solvate, a prodrug, a metabolite, a pharmaceutically acceptable salt or a co-crystal thereof, 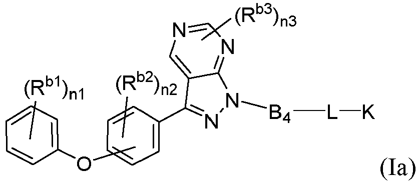
B4 is selected from one of the following substituted or unsubstituted groups: azetidinyl, azacyclopentyl, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, cyclobutyl-spiro-piperidine, cyclopentyl-spiro-piperidine, azetidinyl-spiro-piperidine, azacyclopentyl-spiro-piperidine, 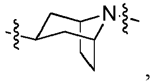 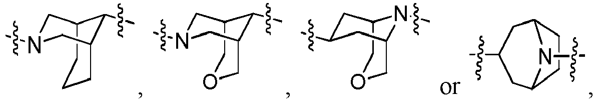 which, when substituted, is optionally further optionally substituted with 0 to 4 R^{b4};
R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
n1, n2 and n3 are each independently selected from 0, 1, 2, 3 or 4; and
the definitions of other groups are the same as those in any one of the first, second, third or fourth embodiment of the present invention.

As a sixth embodiment of the present invention, provided is a compound represented by general formula (Ib) or a stereoisomer, a solvate, a prodrug, a metabolite, a pharmaceutically acceptable salt or a co-crystal thereof, 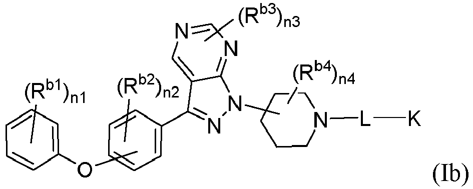
Cy1, Cy2, Cy3 and Cy4 are each independently selected from a bond or one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 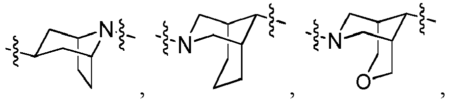 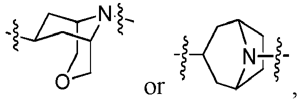 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
provided that at least one of Cy1, Cy2, Cy3 and Cy4 is substituted with 1 to 4 substituents selected from CF₃, COOH, CN or hydroxyl-substituted C₁₋₄ alkyl;
R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
n1, n2, n3, and n4 are each independently selected from 0, 1, 2, 3 or 4; and
the definitions of other groups are the same as those in any one of the first, second, third or fourth embodiment of the present invention.

As a seventh embodiment of the present invention, provided is a compound represented by general formula (I) or a stereoisomer, a solvate, a prodrug, a metabolite, a pharmaceutically acceptable salt or a co-crystal thereof, wherein
L is selected from a bond, 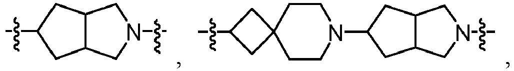 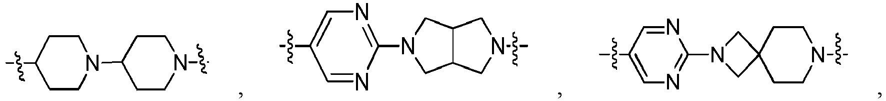 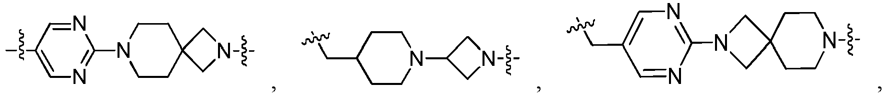 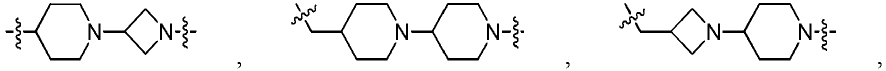 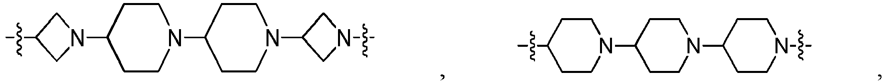 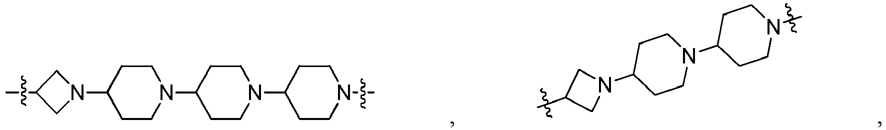 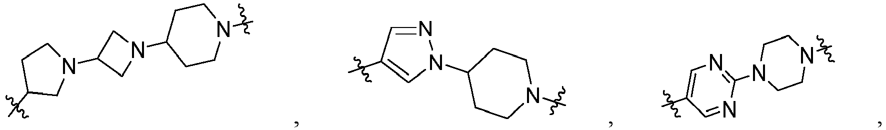 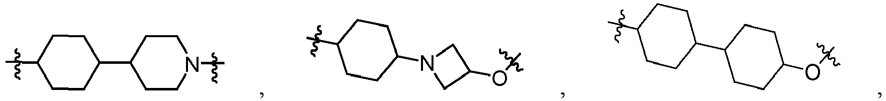 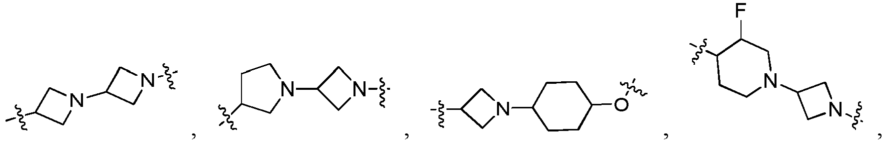 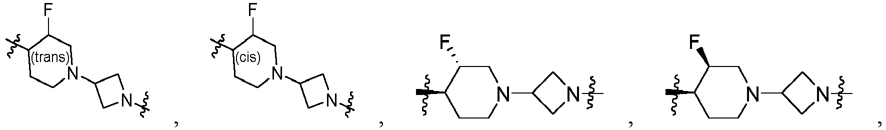 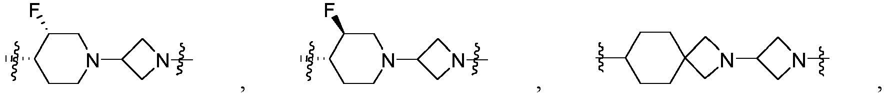 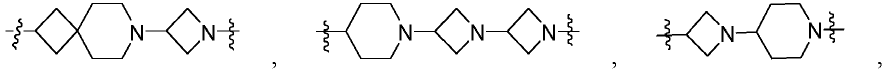 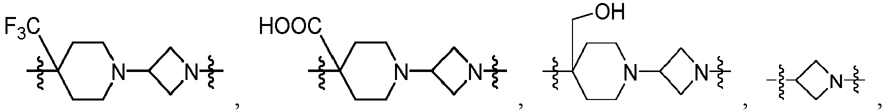 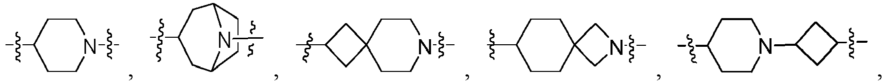 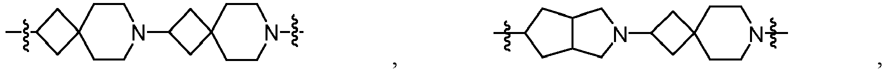 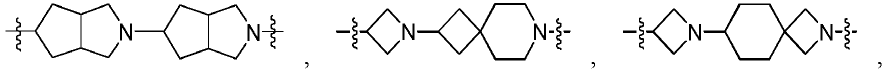 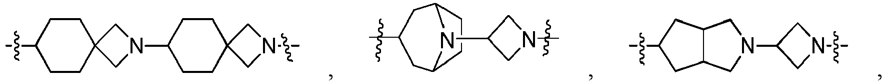 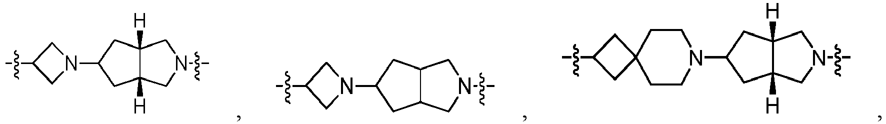 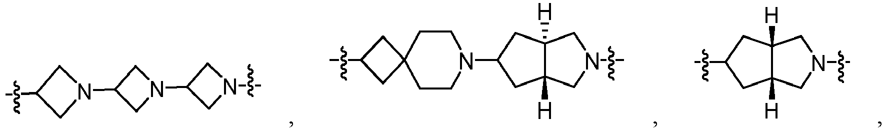 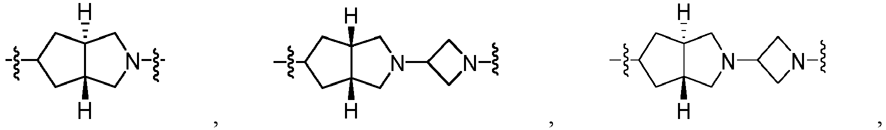 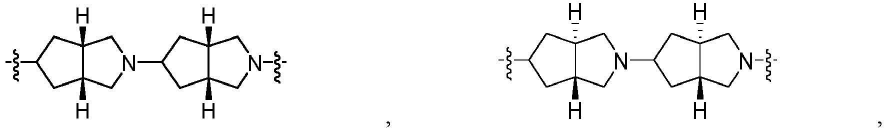 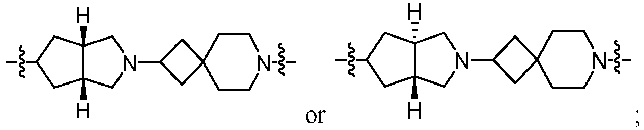
or L is selected from 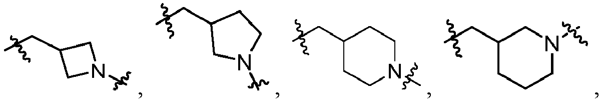 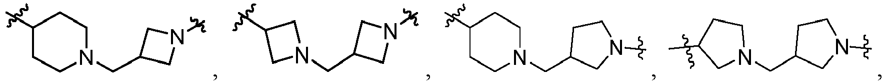 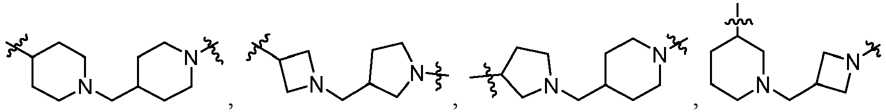 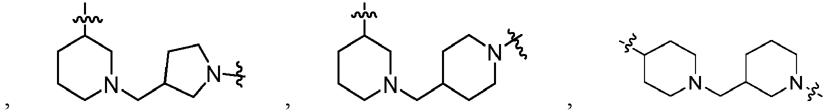 or 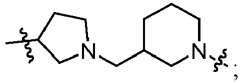
or L is selected from 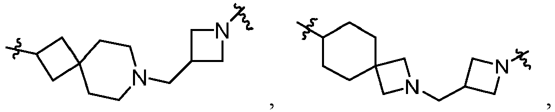 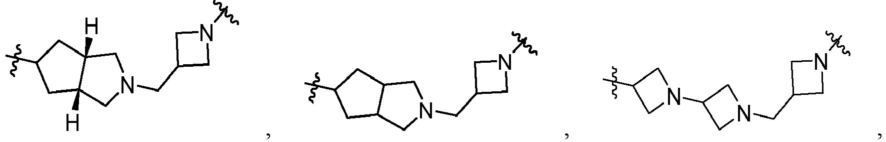 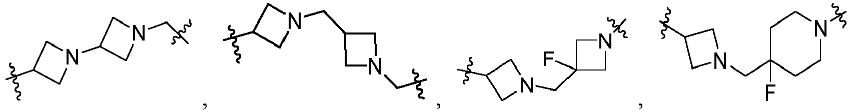 or 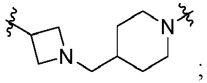
L is connected to B on the left side, and is connected to K on the right side;
B is selected from 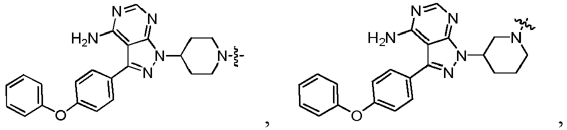 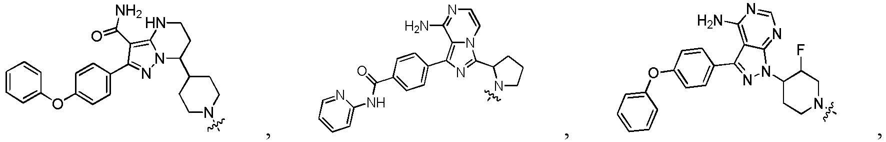 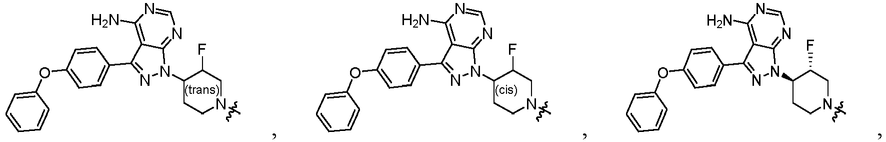 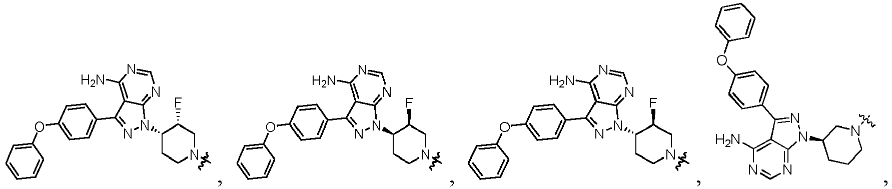 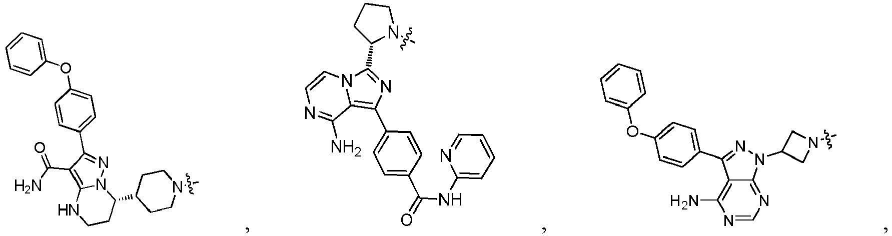 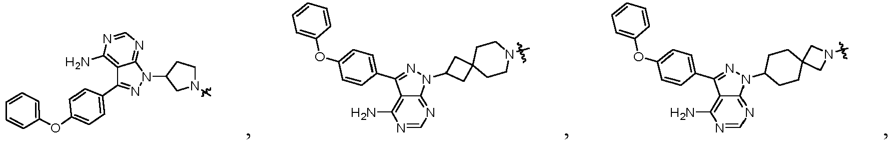 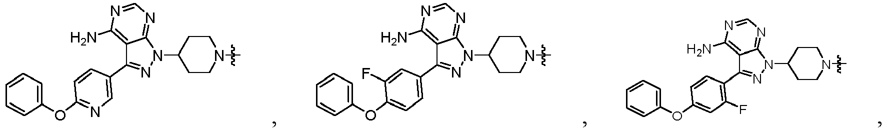 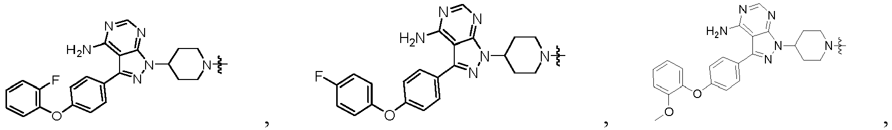 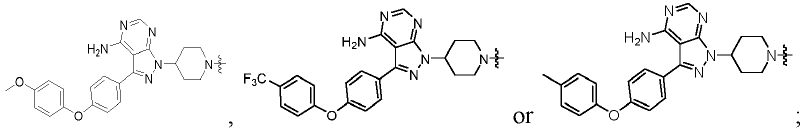 and
K is selected from

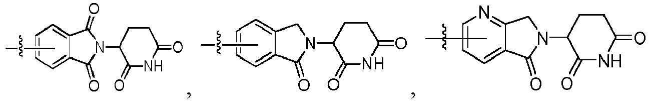

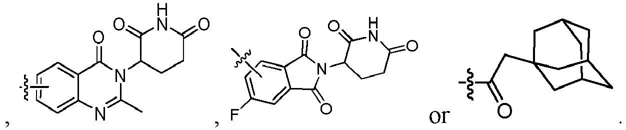

As an eighth embodiment of the present invention, provided is a compound represented by general formula (Ic) or a stereoisomer, a solvate, a prodrug, a metabolite, a pharmaceutically acceptable salt or a co-crystal thereof, 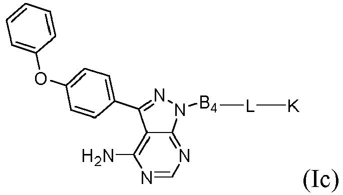
B4 is selected from one of the following substituted or unsubstituted groups: azetidinyl, azacyclopentyl, 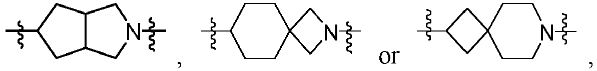 which, when substituted, is optionally further optionally substituted with 0 to 4 R^{b4}; and
R^{b4} is each independently selected from H, F, Cl, Br, I, OH, NH₂, CN, CF₃, CONH₂, COOH, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
the definitions of other groups are the same as those in the seventh embodiment of the present invention.

As a ninth embodiment of the present invention, provided is a compound represented by general formula (Ib) or a stereoisomer, a solvate, a prodrug, a metabolite, a pharmaceutically acceptable salt or a co-crystal thereof, wherein
L is selected from 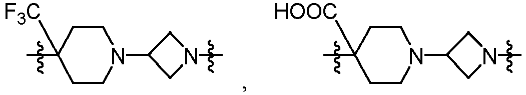 or 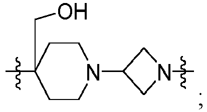
L is connected to B on the left side, and is connected to K on the right side;
the definitions of other groups are the same as those in the seventh embodiment of the present invention.

As a tenth embodiment of the present invention, provided is a compound represented by general formula (Ic) or a stereoisomer, a solvate, a prodrug, a metabolite, a pharmaceutically acceptable salt or a co-crystal thereof, wherein
B4 is selected from one of the following substituted or unsubstituted groups: 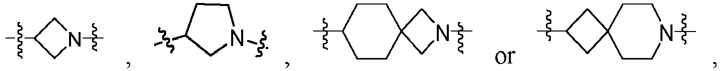 which, when substituted, is optionally further optionally substituted with 0 to 4 substituents selected from R^{b4}; and
R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I;
the definitions of other groups are the same as those in the eighth embodiment of the present invention.

As an eleventh embodiment of the present invention, provided is a compound represented by general formula (I), (Ia), (Ib) or (Ic) or a stereoisomer, a solvate, a prodrug, a metabolite, a pharmaceutically acceptable salt or a co-crystal thereof, wherein
K is selected from 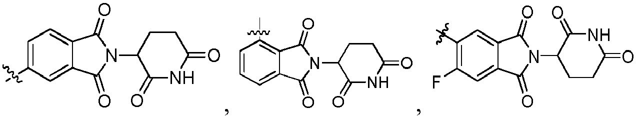 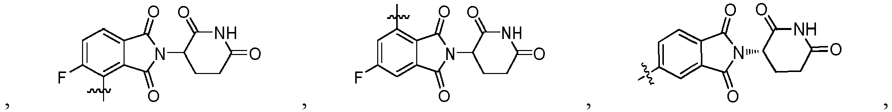 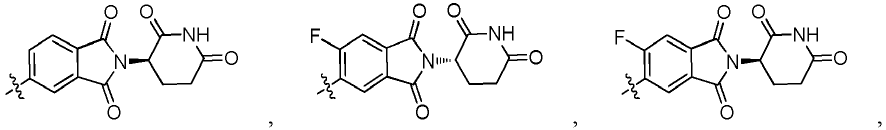 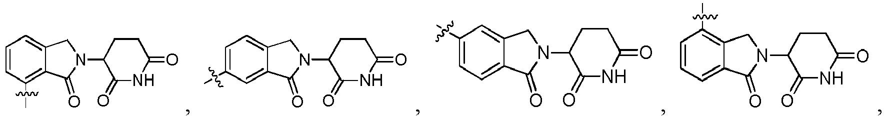 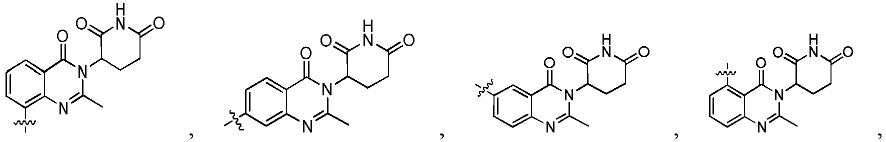 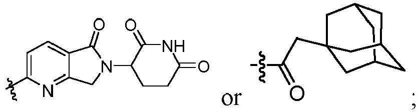
the definitions of other groups of general formula (I) are the same as those in the seventh embodiment;
the definitions of other groups of general formula (Ic) are the same as those in any one of the eighth or tenth embodiment; and
the definitions of other groups of general formula (Ib) are the same as those in the ninth embodiment.

As a twelfth embodiment of the present invention, provided is a compound represented by general formula (I), (Ia), (Ib) or (Ic) or a stereoisomer, a solvate, a prodrug, a metabolite, a pharmaceutically acceptable salt or a co-crystal thereof, wherein
K is selected from 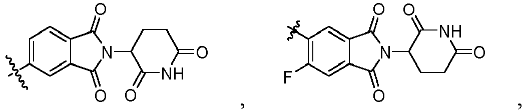 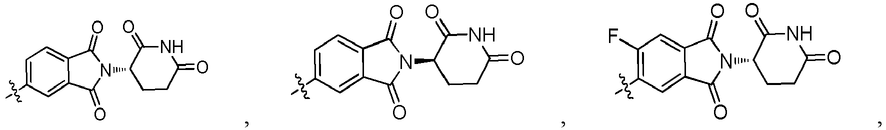 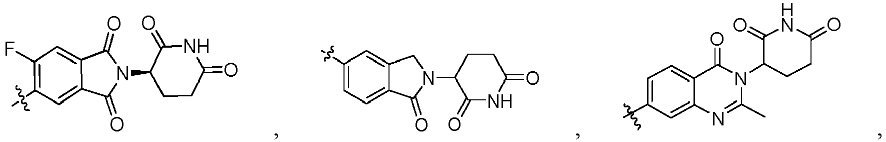 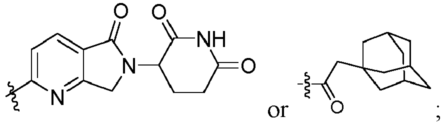 and
the definitions of other groups are the same as those in the eleventh embodiment.

As a thirteenth embodiment of the present invention, provided is a compound represented by general formula (I) or a stereoisomer, a solvate, a prodrug, a metabolite, a pharmaceutically acceptable salt or a co-crystal thereof, wherein
B is selected from 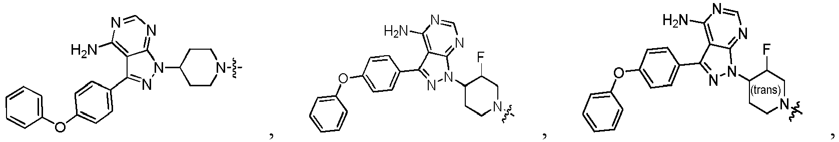 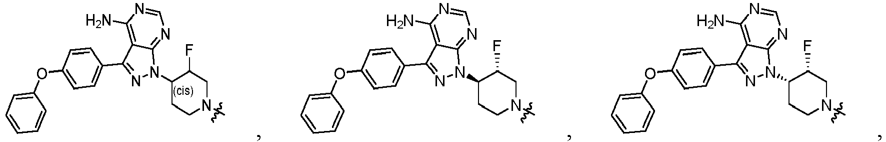 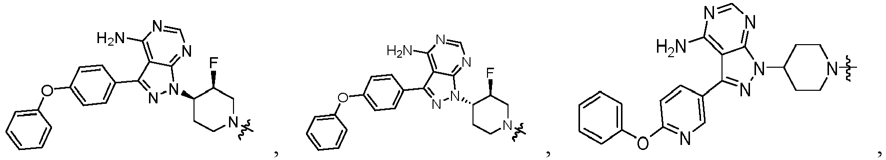 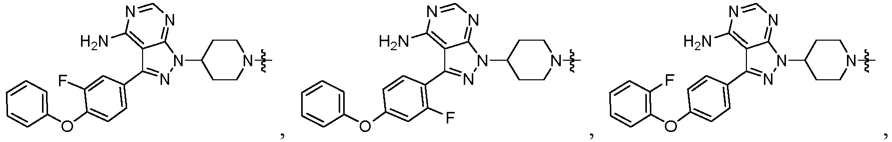 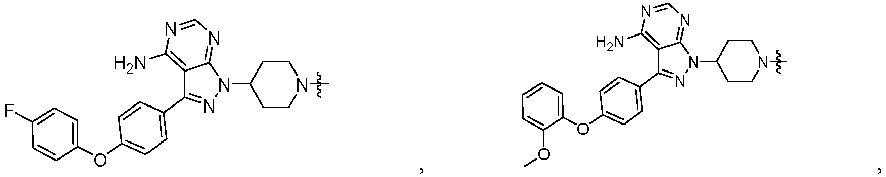 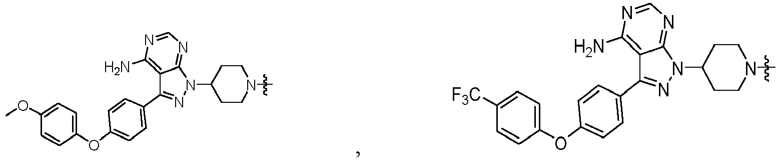 or 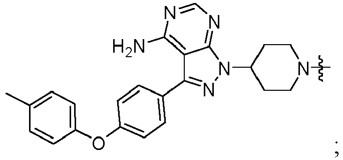 and
the definitions of other groups are the same as those in the twelfth embodiment.

As a fourteenth embodiment of the present invention, provided is a compound represented by general formula (I) or a stereoisomer, a solvate, a prodrug, a metabolite, a pharmaceutically acceptable salt or a co-crystal thereof, wherein
B is selected from 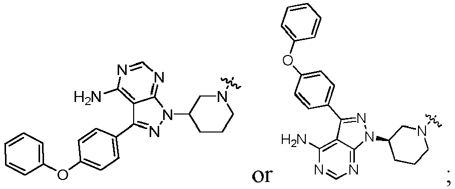 and
the definitions of other groups are the same as those in the twelfth embodiment.

As a fifteenth embodiment of the present invention, provided is a compound represented by general formula (I) or a stereoisomer, a solvate, a prodrug, a metabolite, a pharmaceutically acceptable salt or a co-crystal thereof, wherein
B is selected from 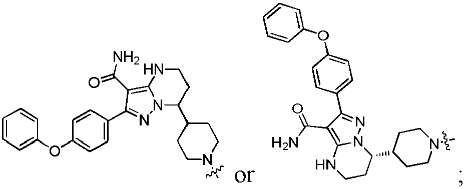 and
the definitions of other groups are the same as those in the twelfth embodiment.

As a sixteenth embodiment of the present invention, provided is a compound represented by general formula (I) or a stereoisomer, a solvate, a prodrug, a metabolite, a pharmaceutically acceptable salt or a co-crystal thereof, wherein
B is selected from 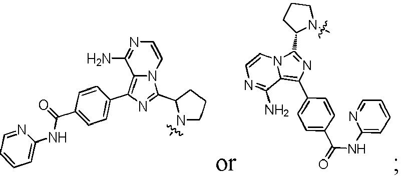 and
the definitions of other groups are the same as those in the twelfth embodiment.

As a seventeenth embodiment of the present invention, provided is a compound represented by general formula (I) or a stereoisomer, a solvate, a prodrug, a metabolite, a pharmaceutically acceptable salt or a co-crystal thereof, wherein
B is selected from 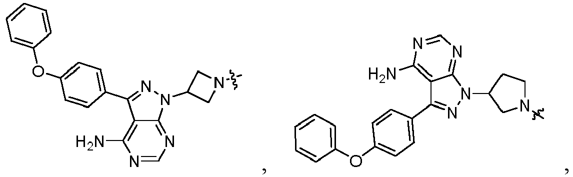 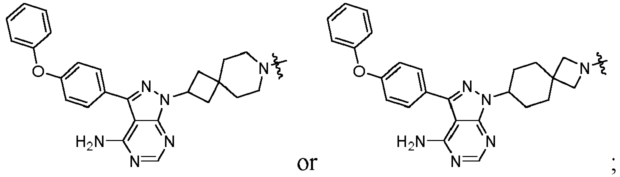 and
the definitions of other groups are the same as those in the twelfth embodiment.

As an eighteenth embodiment of the present invention, provided is a compound represented by general formula (II) or a stereoisomer, a solvate, a prodrug, a metabolite, a pharmaceutically acceptable salt or a co-crystal thereof, 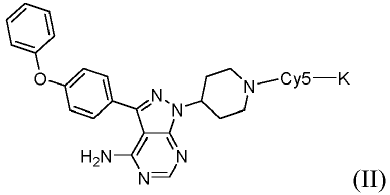
Cy5 is selected from one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 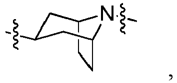 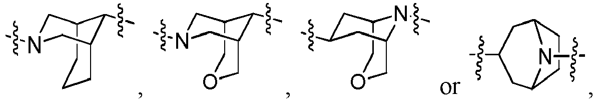 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
the definitions of other groups are the same as those in any one of the second, third, fourth, seventh, eleventh and twelfth embodiment of the present invention.

As a nineteenth embodiment of the present invention, provided is a compound represented by general formula (II) or a stereoisomer, a solvate, a prodrug, a metabolite, a pharmaceutically acceptable salt or a co-crystal thereof,
Cy5 is selected from one of the following substituted or unsubstituted groups: 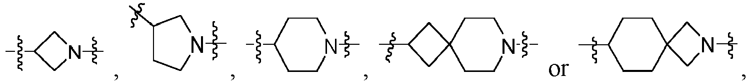 which, when substituted, is optionally further substituted with 0 to 4 substituents selected from H, F, Cl, Br, I, OH, NH₂, oxo, CF₃, COOH, CN, methyl, hydroxymethyl or methoxy;
the definitions of other groups are the same as those in any one of the first, second, third, fourth, seventh, eleventh and twelfth embodiment of the present invention.

Some embodiments of the present invention relate to a compound as described below or a stereoisomer, a solvate, a prodrug, a metabolite, a pharmaceutically acceptable salt or a co-crystal thereof, wherein the compound is of the structure selected from one of:

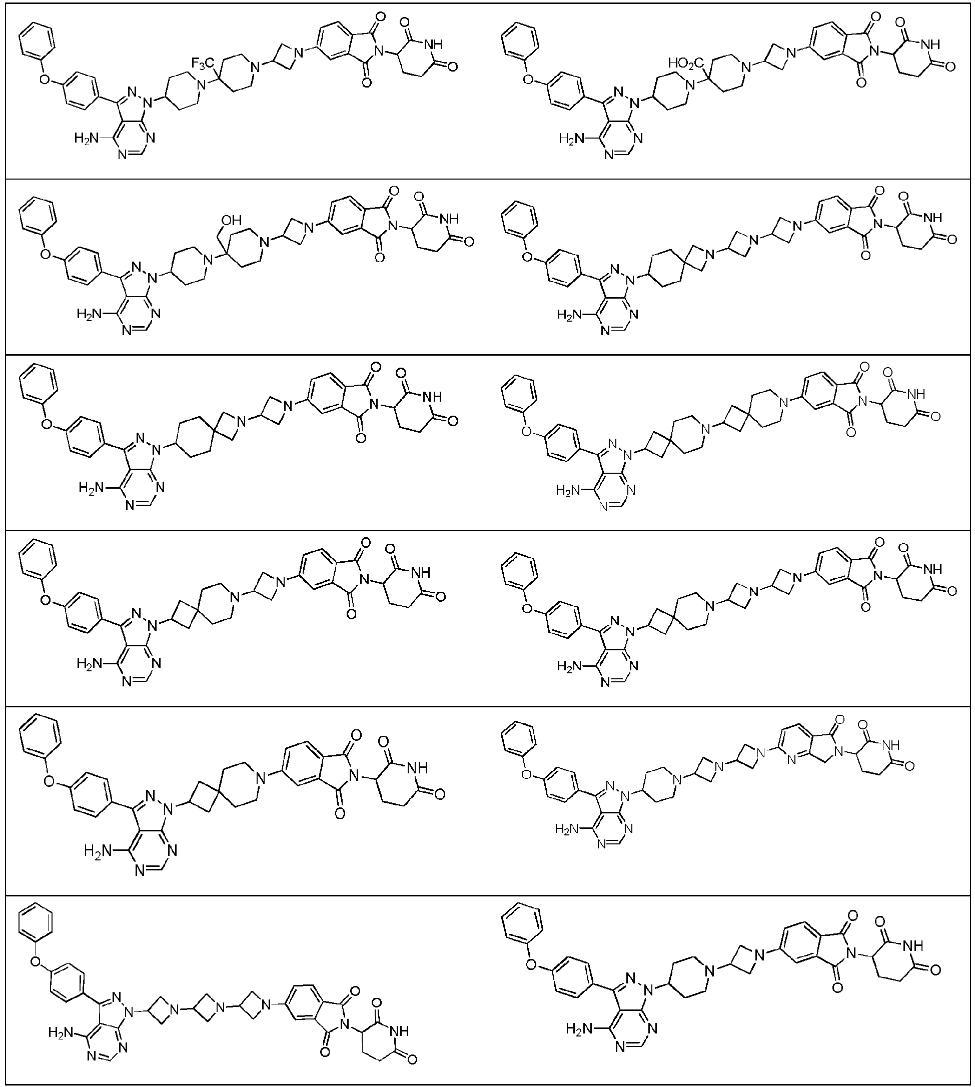

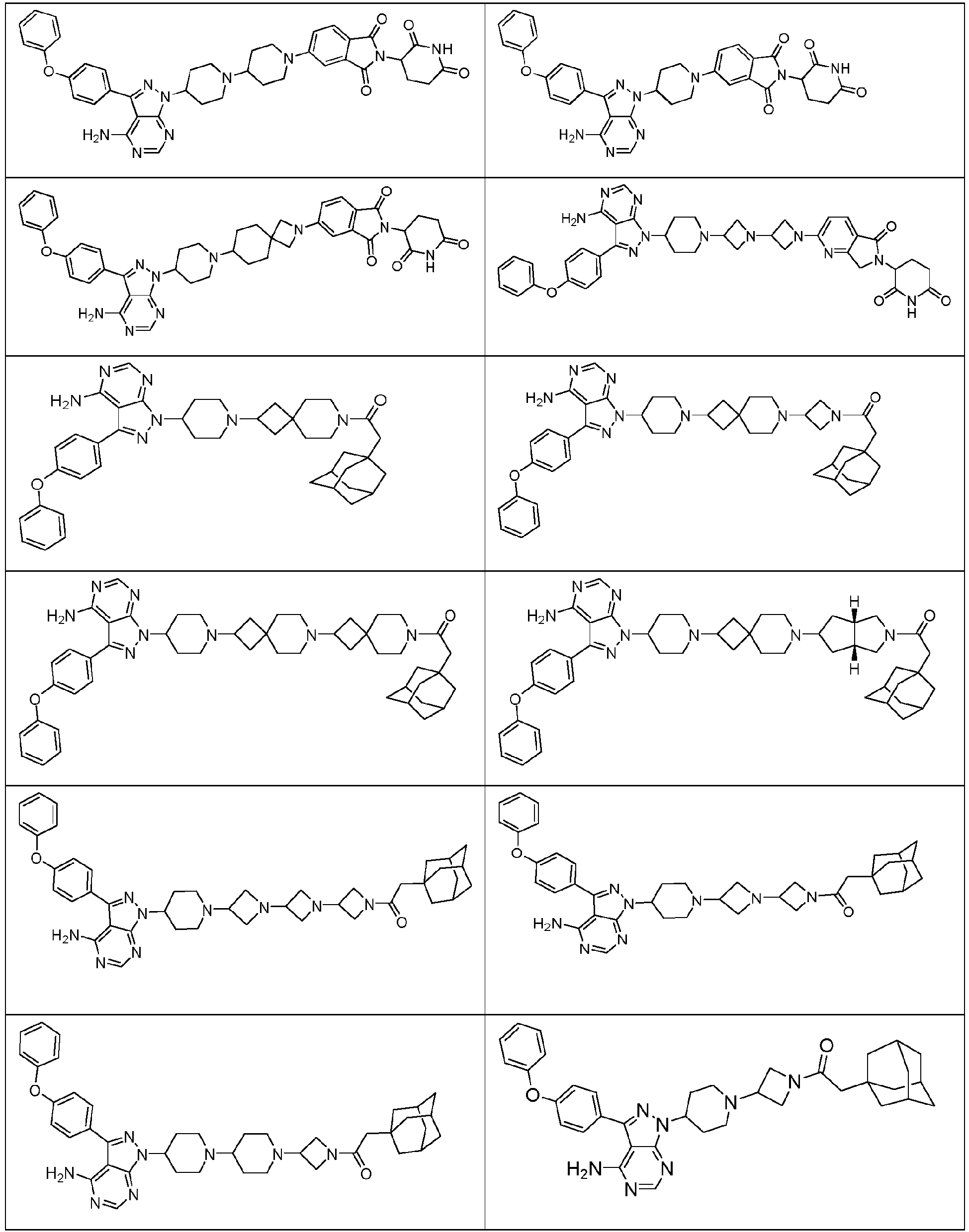

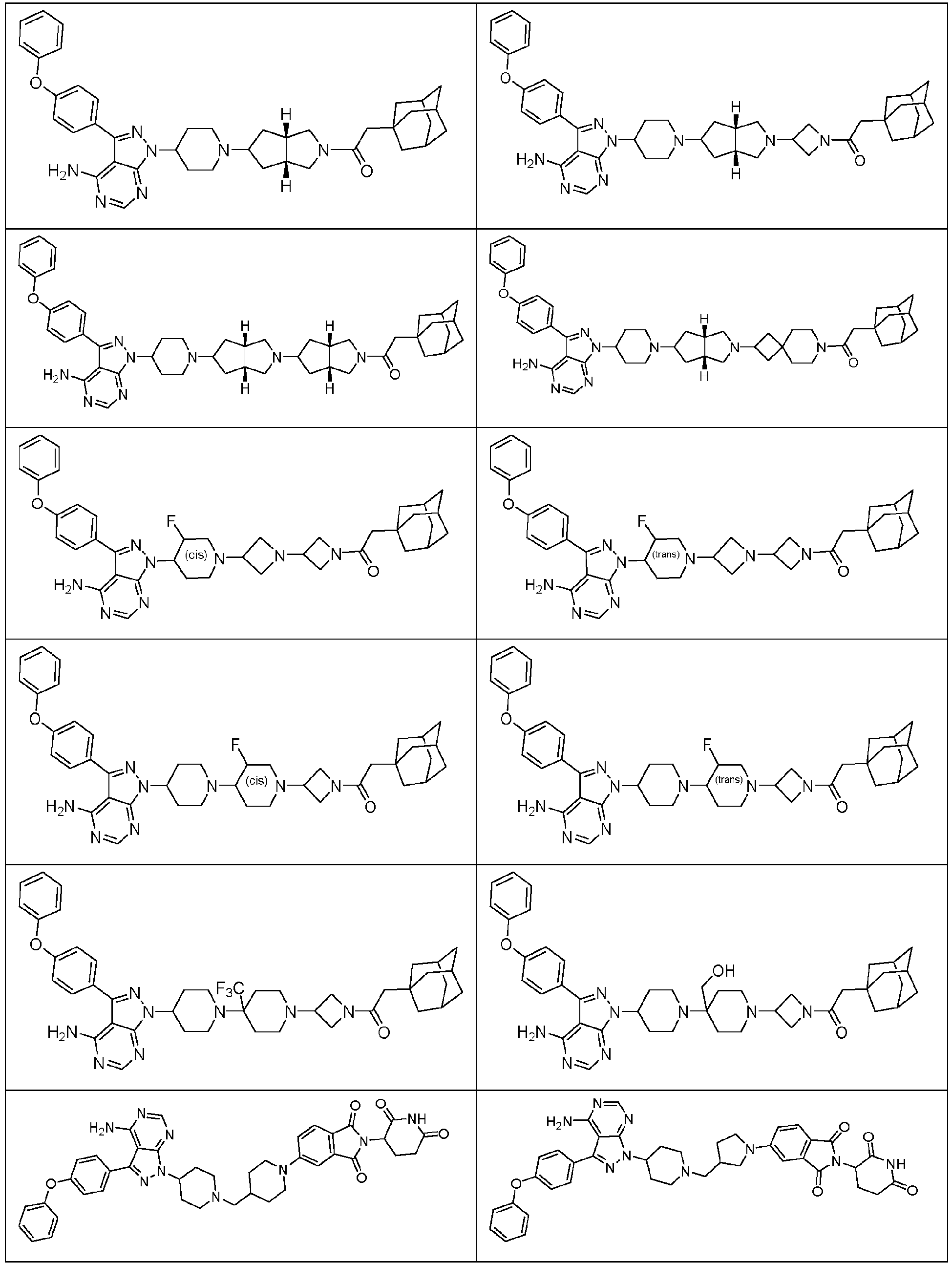

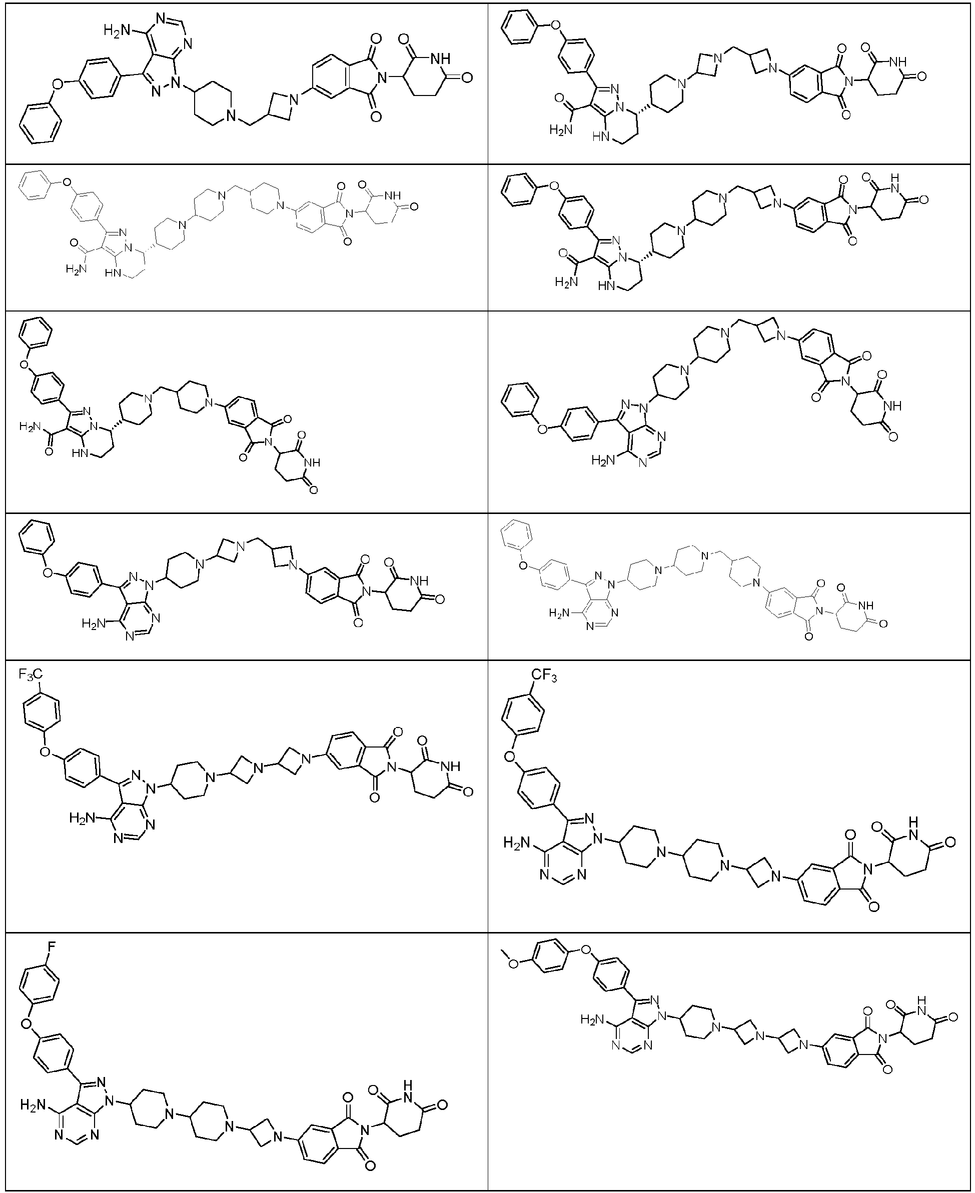

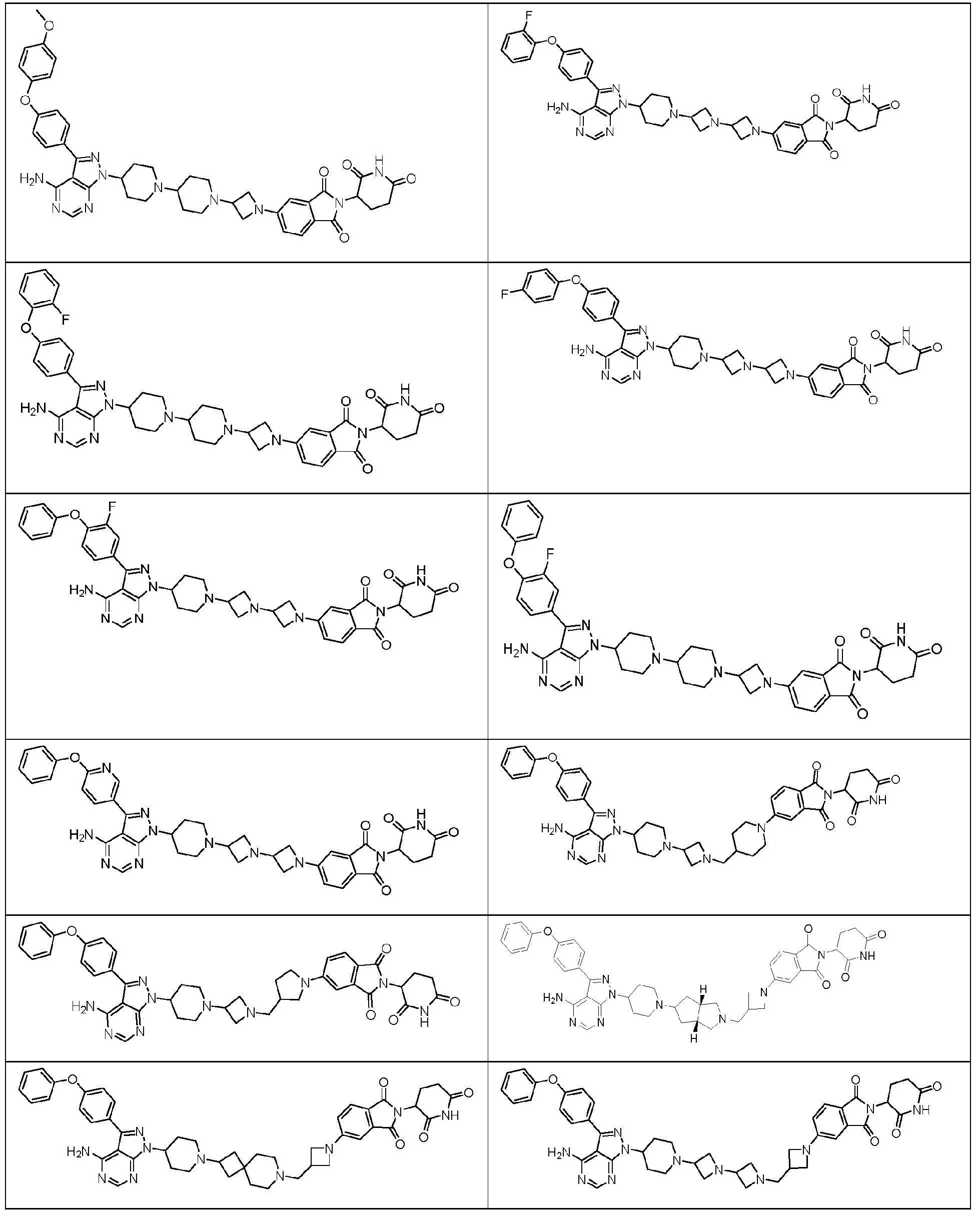

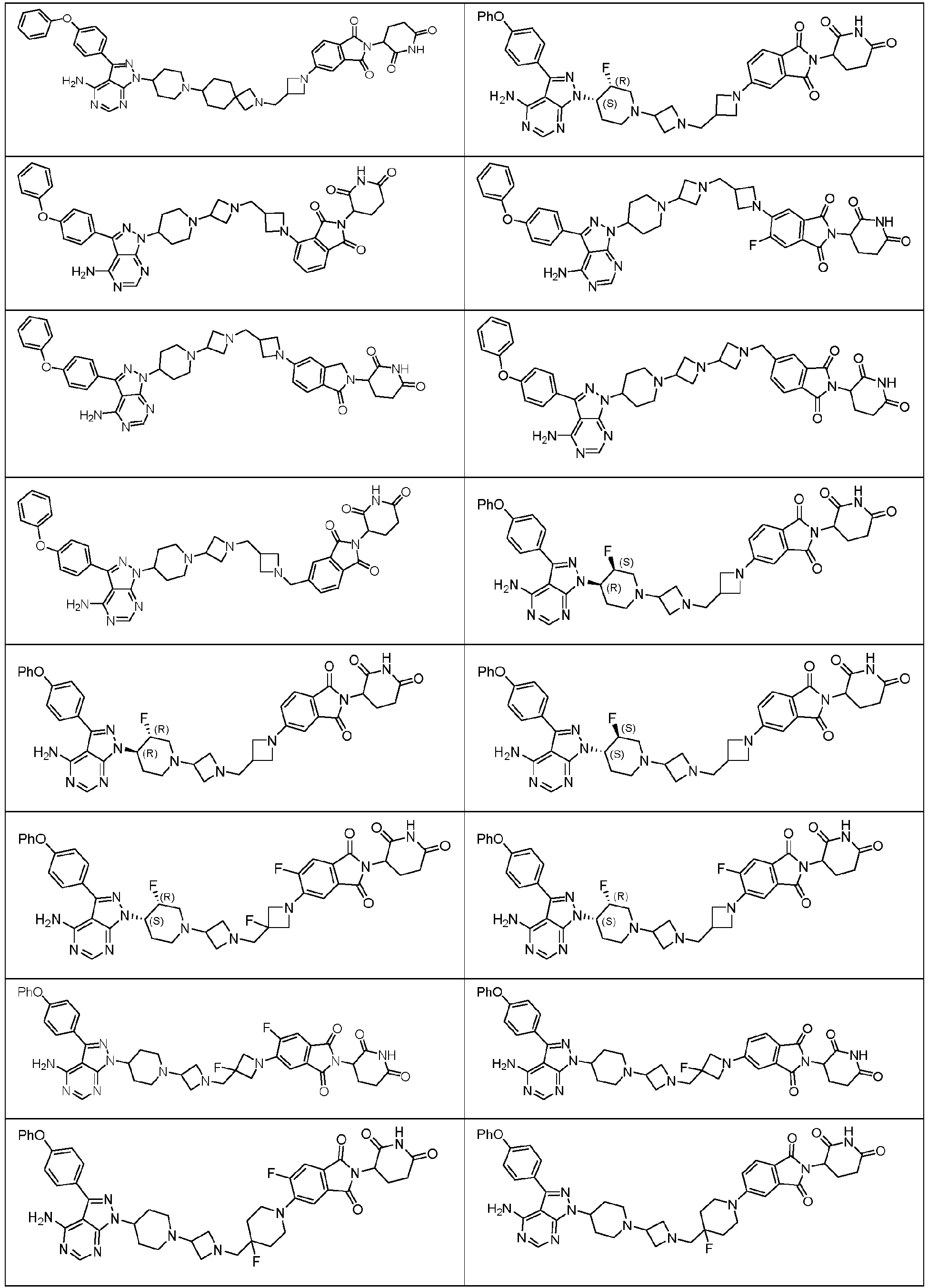

Some embodiments of the present invention relate to a compound represented by general formula (I) or a stereoisomer, a solvate, a prodrug, a metabolite, a pharmaceutically acceptable salt or a co-crystal thereof, optionally, the compound is not a compound shown in Table A,

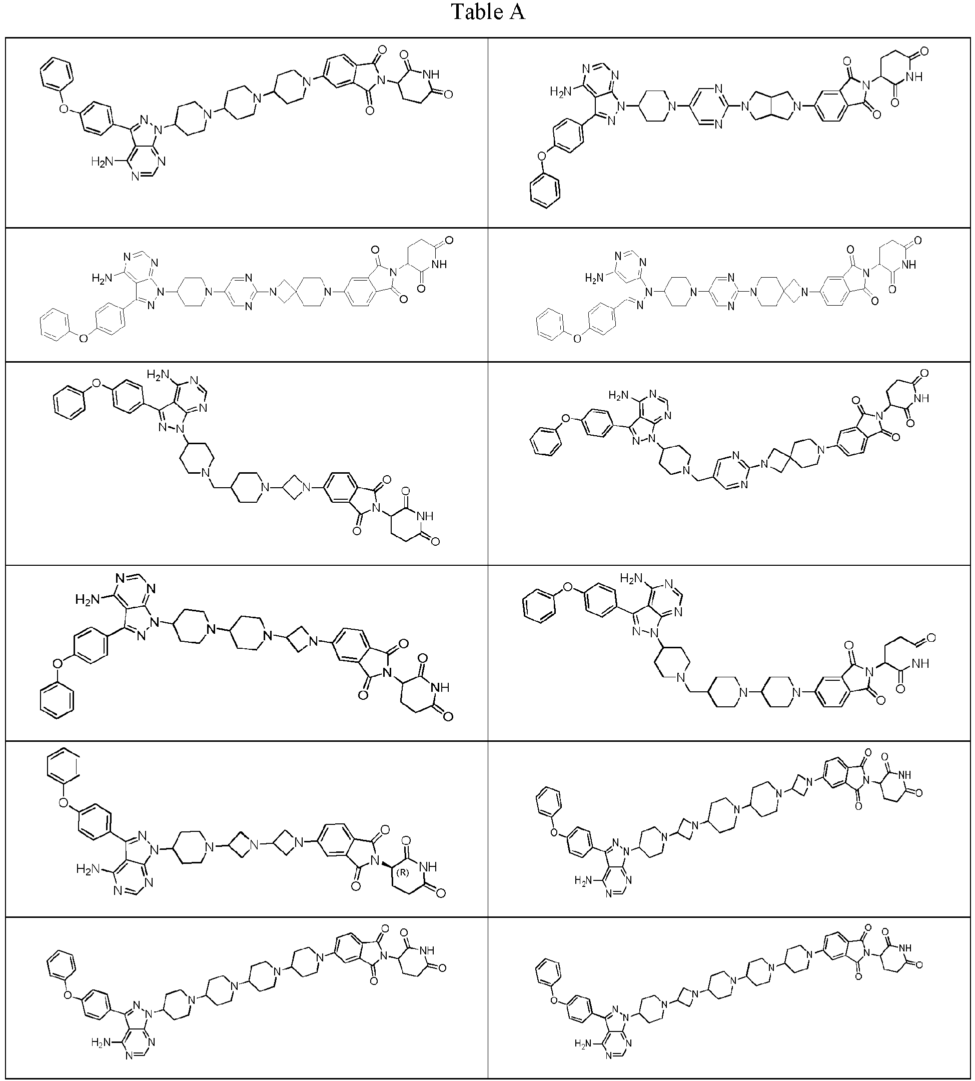

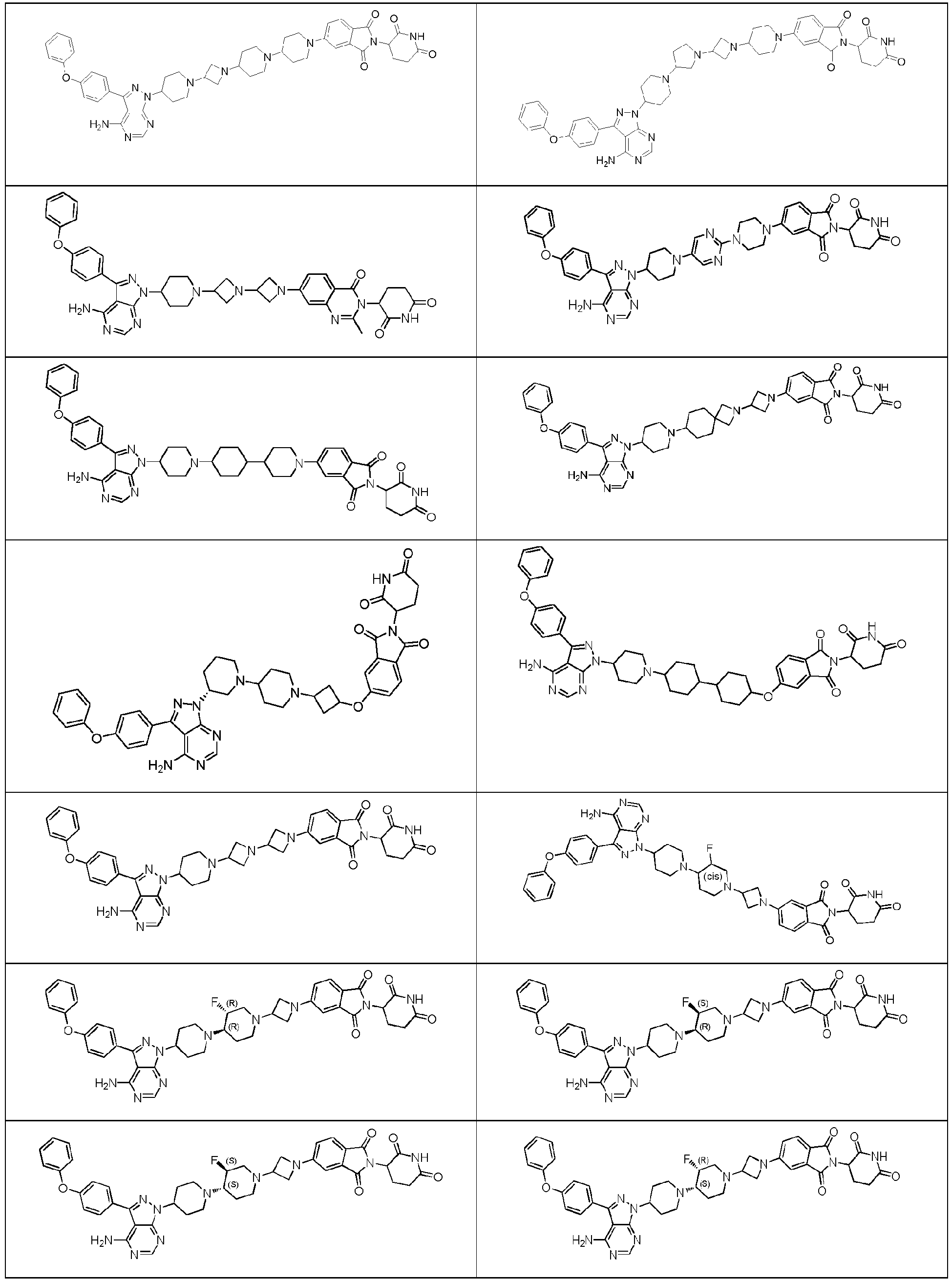

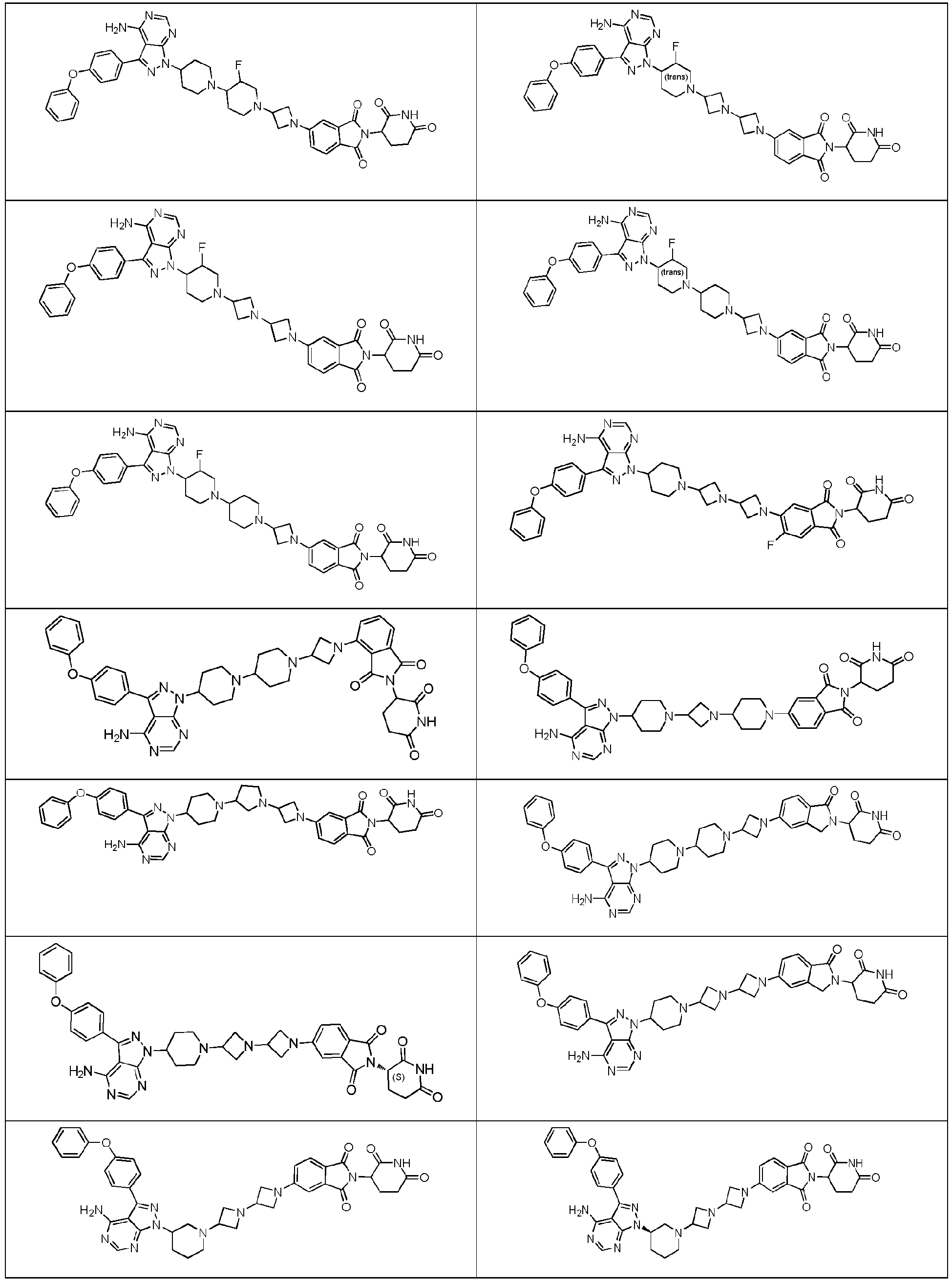

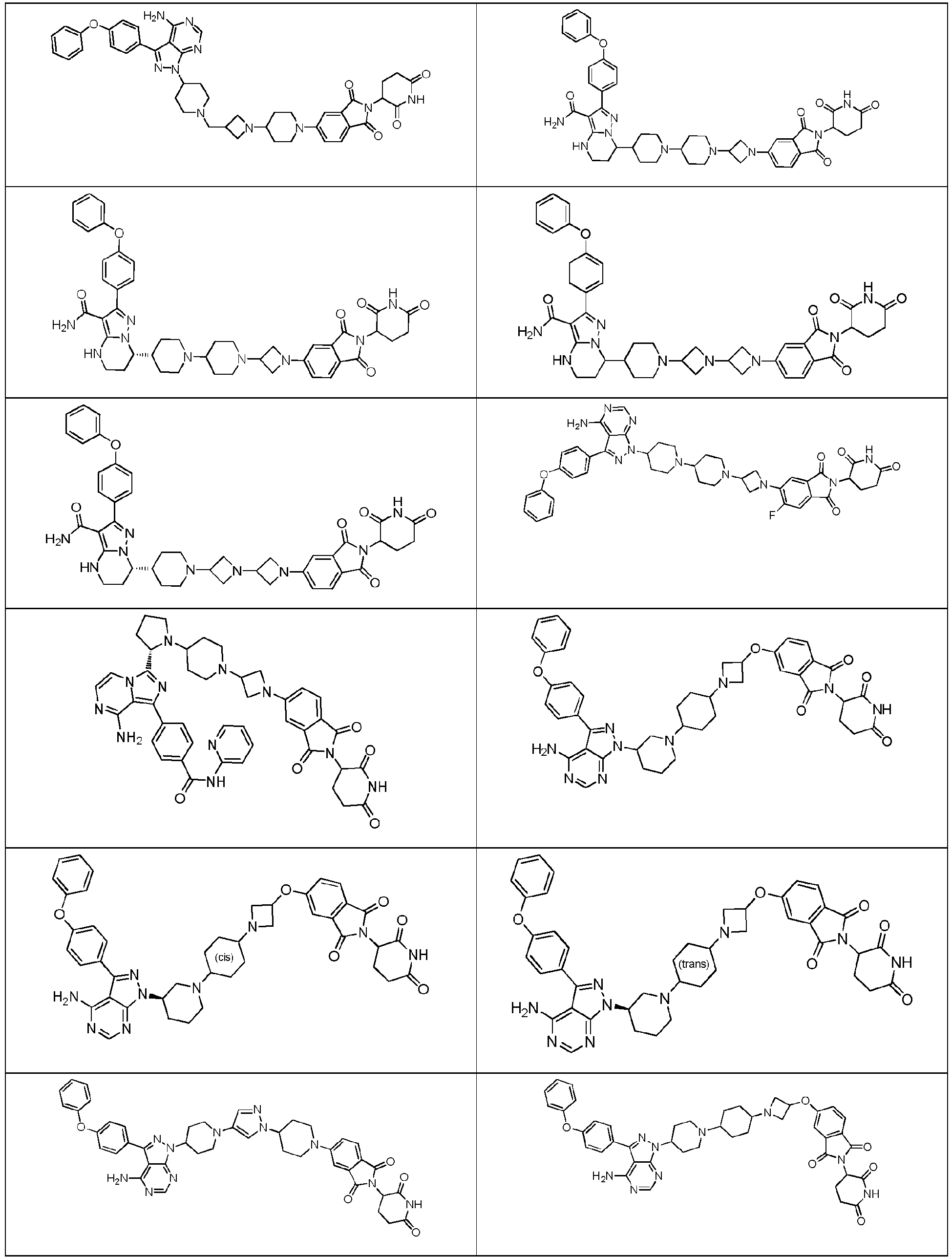

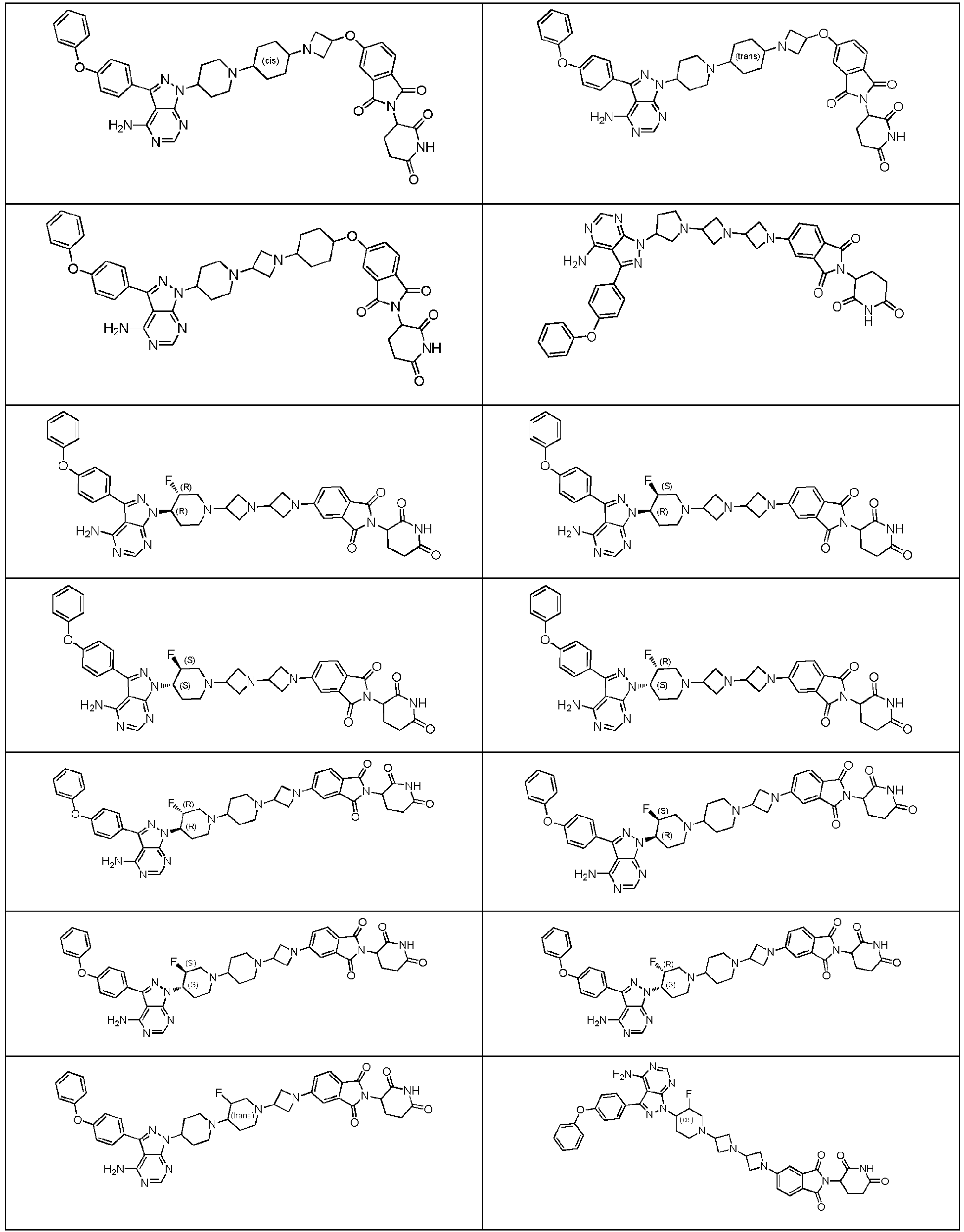

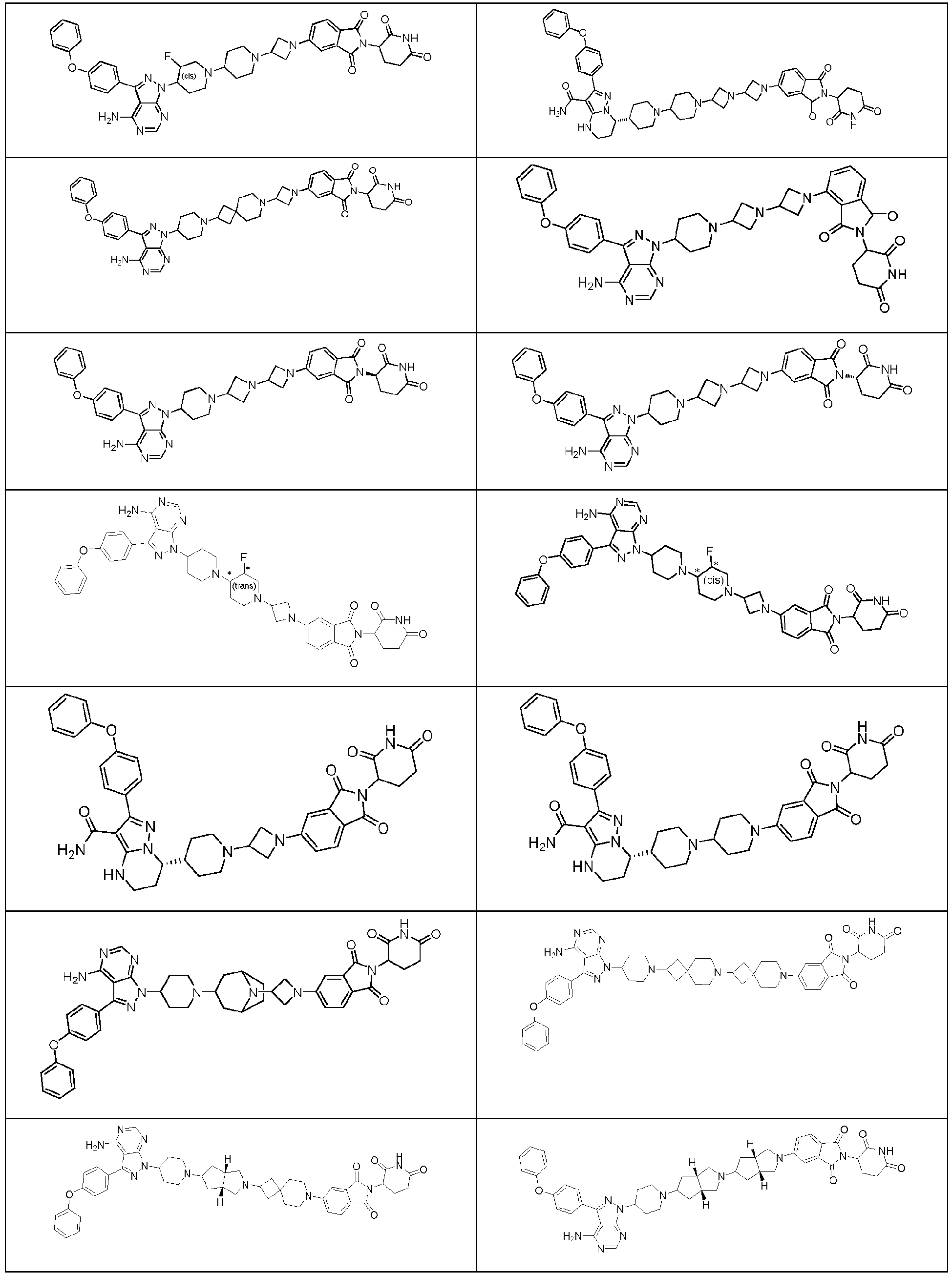

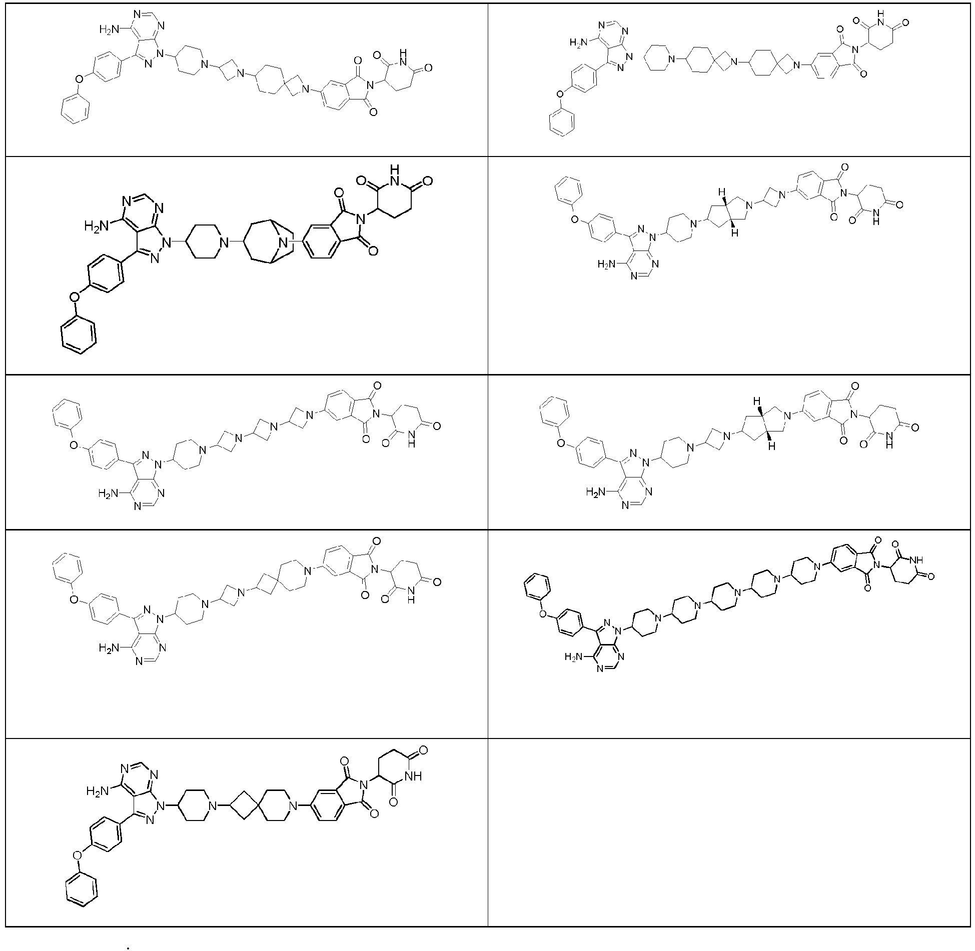

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein
K is selected from 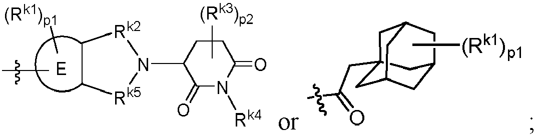
ring E is selected from benzene ring or 5- to 6-membered heteroaryl ring, wherein the heteroaryl ring contains 1 to 2 heteroatoms selected from O, S or N;
R^{k2} is each independently selected from CH₂, C=O, S=O or SO₂;
R^{k1}, R^{k3} or R^{k4} is each independently selected from H, F, Cl, Br, I, OH, NH₂, CF₃, CN, COOH, C₁₋₄ alkyl or C₁₋₄ alkoxy;
R^{k5} is selected from C=O or 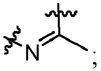 p1 or p2 is each independently selected from 0, 1, 2, 3 or 4.

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein
K is selected from 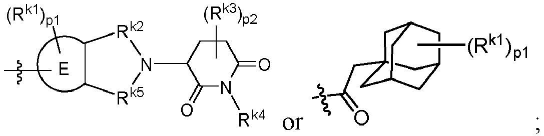
ring E is selected from benzene ring, pyridine, thiophene, furan, pyrrole, thiazole, oxazole, imidazole or pyrazole; and
R^{k2} is each independently selected from CH₂, C=O, S=O or SO₂;
R^{k1}, R^{k3} or R^{k4} is each independently selected from H, F, Cl, Br, I, OH, NH₂, CF₃, CN, COOH, C₁₋₄ alkyl or C₁₋₄ alkoxy;
R^{k5} is selected from C=O or 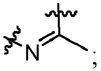 p1 or p2 is each independently selected from 0, 1, 2, 3 or 4.

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein
K is selected from 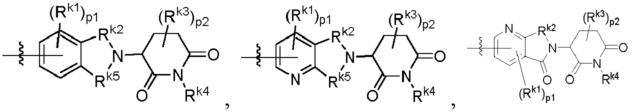 or 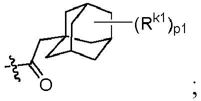
R^{k2} is each independently selected from CH₂, C=O, S=O or SO₂;
R^{k1}, R^{k3} or R^{k4} is each independently selected from H, F, Cl, Br, I, OH, NH₂, CF₃, CN, COOH, C₁₋₄ alkyl or C₁₋₄ alkoxy;
R^{k5} is selected from C=O or 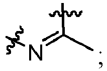 p1 or p2 is each independently selected from 0, 1, 2, 3 or 4.

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein K is selected from

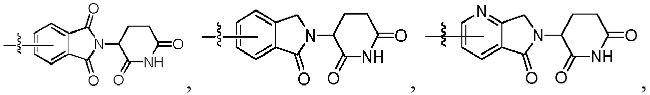

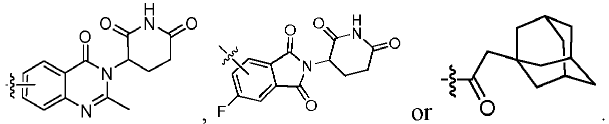

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein K is selected from

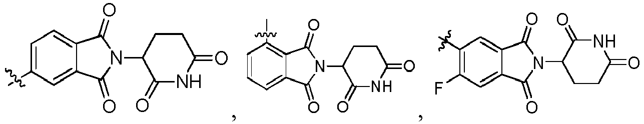

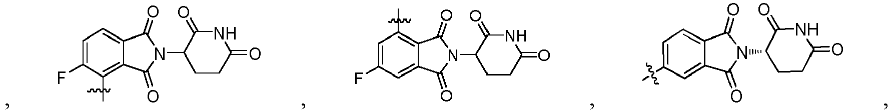

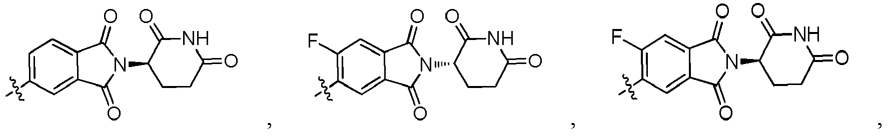

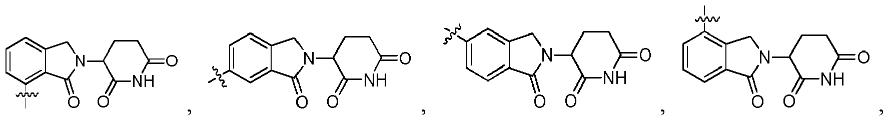

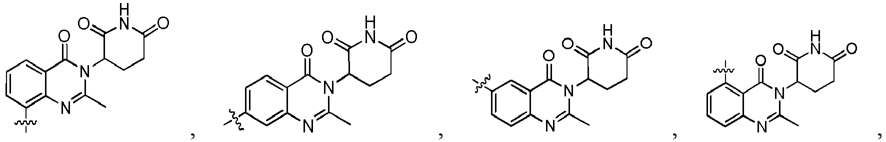

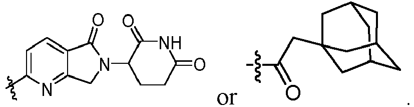

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein
K is selected from

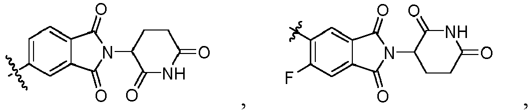

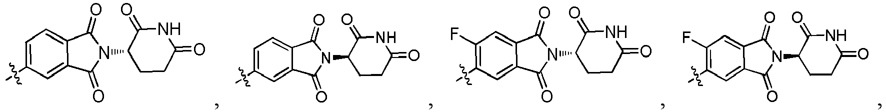

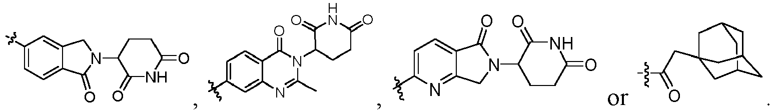

Some embodiments of the present invention relate to a compound represented by general formula (I) or a stereoisomer, a solvate, a prodrug, a metabolite, a pharmaceutically acceptable salt or a co-crystal thereof, wherein
B is selected from 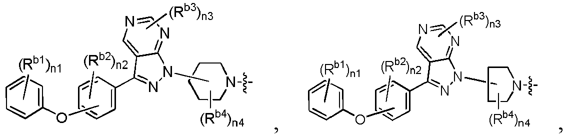 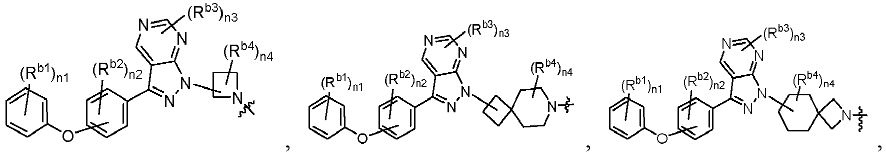 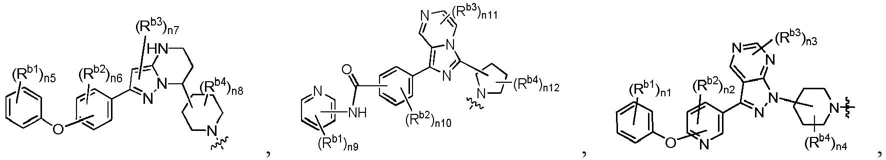 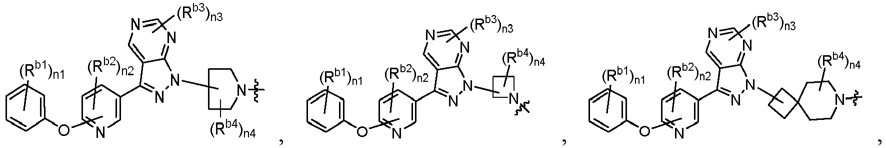 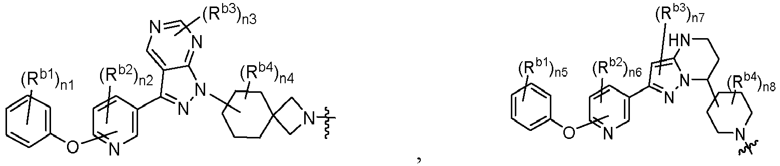 or 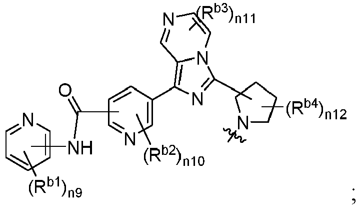
R^{b1}, R^{b2}, R^{b3} or R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I;
n1, n2, n3, n4, n5, n6, n7, n8, n9, n10, n11, and n12 are each independently selected from 0, 1, 2, 3 or 4.

Some embodiments of the present invention relate to a compound represented by general formula (I) or a stereoisomer, a solvate, a prodrug, a metabolite, a pharmaceutically acceptable salt or a co-crystal thereof, wherein
B is selected from

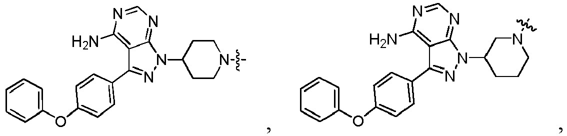

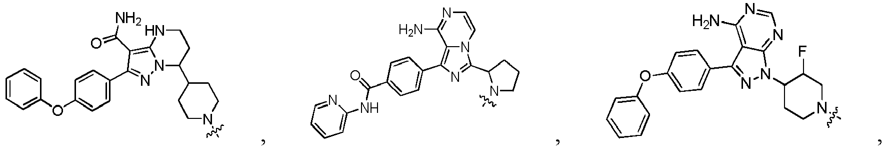

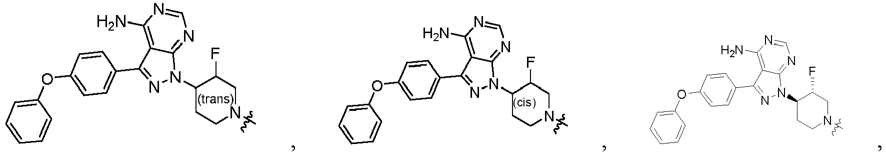

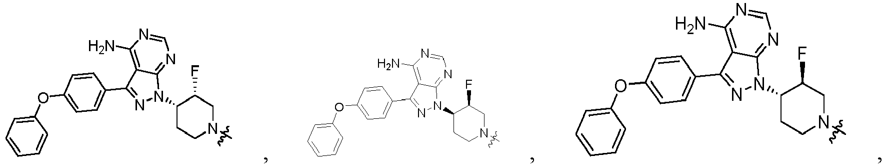

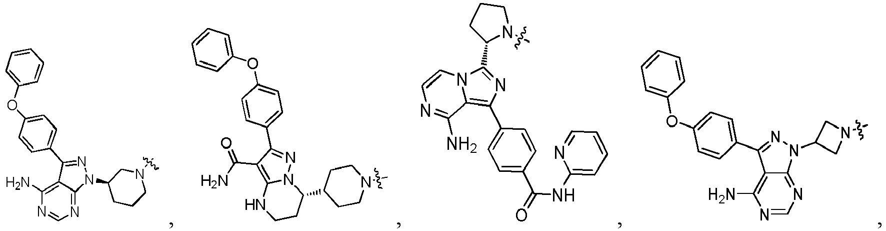

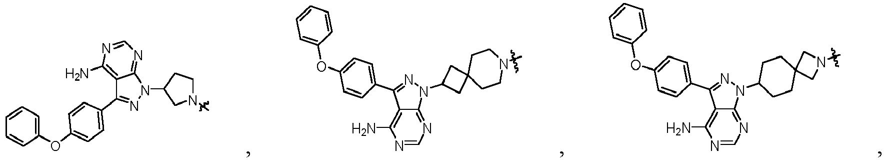

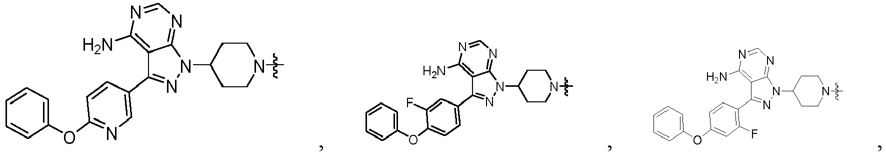

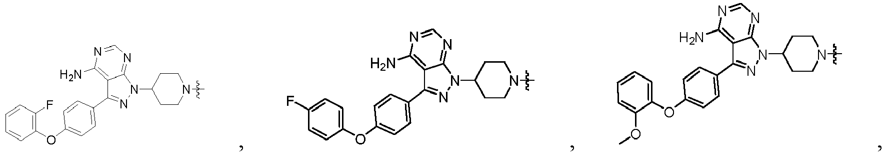

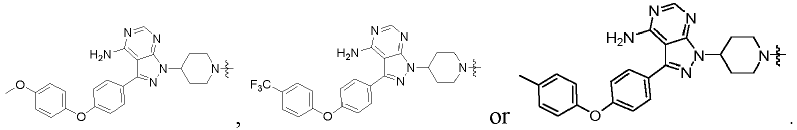

Some embodiments of the present invention relate to a compound represented by general formula (I) or a stereoisomer, a solvate, a prodrug, a metabolite, a pharmaceutically acceptable salt or a co-crystal thereof, wherein B is selected from 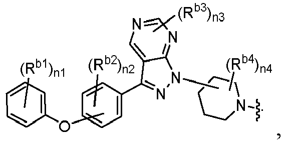 and at least one of Cy1, Cy2, Cy3 and Cy4 is substituted with 1 to 4 substituents selected from CF₃, COOH, CN or hydroxyl-substituted C₁₋₄ alkyl.

Some embodiments of the present invention relate to a compound represented by general formula (I) or a stereoisomer, a solvate, a prodrug, a metabolite, a pharmaceutically acceptable salt or a co-crystal thereof, wherein B is selected from

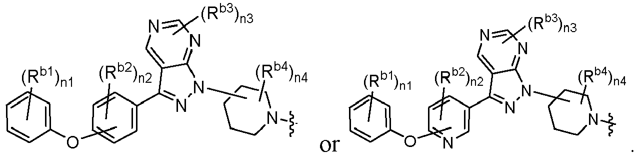

Some embodiments of the present invention relate to a compound represented by general formula (I) or a stereoisomer, a solvate, a prodrug, a metabolite, a pharmaceutically acceptable salt or a co-crystal thereof, wherein
B-L is selected from 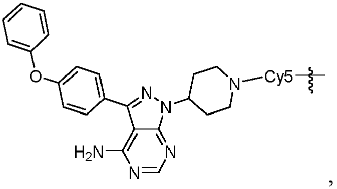 and Cy5 is selected from one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 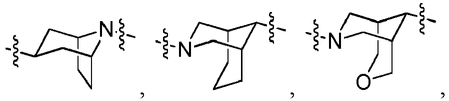 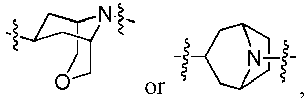 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy.

Some embodiments of the present invention relate to a compound represented by general formula (I) or a stereoisomer, a solvate, a prodrug, a metabolite, a pharmaceutically acceptable salt or a co-crystal thereof, wherein
Cy5 is selected from one of the following substituted or unsubstituted groups: 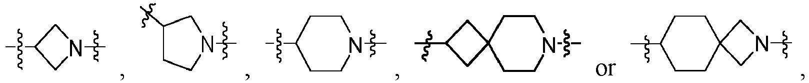 which, when substituted, is optionally further substituted with 0 to 4 substituents selected from H, F, Cl, Br, I, OH, NH₂, oxo, CF₃, COOH, CN, methyl, hydroxymethyl or methoxy.

Some embodiments of the present invention relate to a compound represented by general formula (I) or a stereoisomer, a solvate, a prodrug, a metabolite, a pharmaceutically acceptable salt or a co-crystal thereof, wherein B is selected from 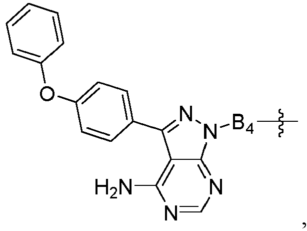 and B4 is selected from one of the following substituted or unsubstituted groups: azetidinyl, azacyclopentyl, 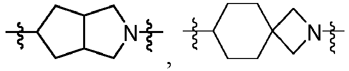 or 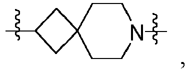 which, when substituted, is optionally further optionally substituted with 0 to 4 R^{b4}; and
R^{b4} is each independently selected from H, F, Cl, Br, I, OH, NH₂, CN, CF₃, CONH₂, COOH, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy.

Some embodiments of the present invention relate to a compound represented by general formula (I) or a stereoisomer, a solvate, a prodrug, a metabolite, a pharmaceutically acceptable salt or a co-crystal thereof, wherein B is selected from 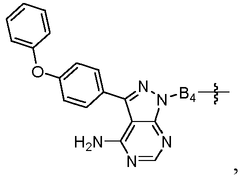 and B4 is selected from one of the following substituted or unsubstituted groups: 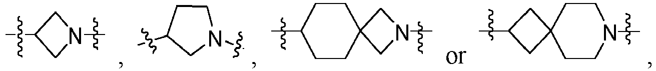 which, when substituted, is optionally further optionally substituted with 0 to 4 substituents selected from R^{b4}; and
R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I.

Some embodiments of the present invention relate to a compound represented by general formula (I) or a stereoisomer, a solvate, a prodrug, a metabolite, a pharmaceutically acceptable salt or a co-crystal thereof, wherein
B is selected from

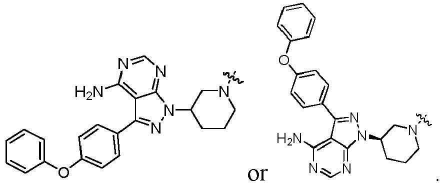

Some embodiments of the present invention relate to a compound represented by general formula (I) or a stereoisomer, a solvate, a prodrug, a metabolite, a pharmaceutically acceptable salt or a co-crystal thereof, wherein
B is selected from

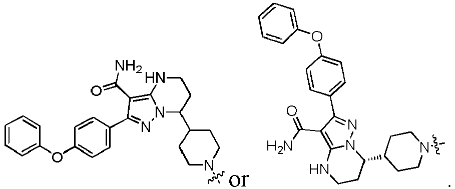

Some embodiments of the present invention relate to a compound represented by general formula (I) or a stereoisomer, a solvate, a prodrug, a metabolite, a pharmaceutically acceptable salt or a co-crystal thereof, wherein
B is selected from

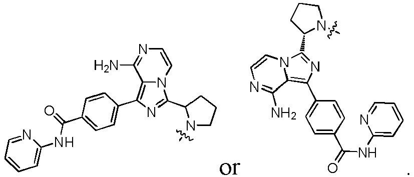

Some embodiments of the present invention relate to a compound represented by general formula (I) or a stereoisomer, a solvate, a prodrug, a metabolite, a pharmaceutically acceptable salt or a co-crystal thereof, wherein
B is selected from 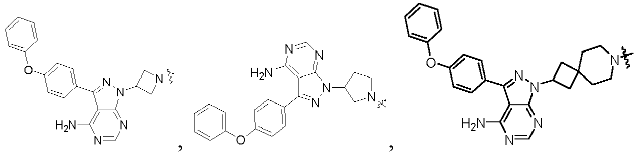 or

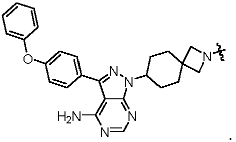

Some embodiments of the present invention relate to a compound represented by general formulas (I), (Ia) and (Ic) or a stereoisomer, a solvate, a prodrug, a metabolite, a pharmaceutically acceptable salt or a co-crystal thereof, wherein
Cy1, Cy2, Cy3 and Cy4 are each independently selected from 3-membered heterocyclic ring, 4-membered heterocyclic ring, 5-membered heterocyclic ring, 6-membered heterocyclic ring, 7-membered heterocyclic ring, 8-membered heterocyclic ring, 9-membered heterocyclic ring, 10-membered heterocyclic ring, 11-membered heterocyclic ring, 12-membered heterocyclic ring, 3-membered cycloalkyl, 4-membered cycloalkyl, 5-membered cycloalkyl, 6-memberedcycloalkyl, 7-membered cycloalkyl, 8-membered cycloalkyl, 9-membered cycloalkyl, 10-membered cycloalkyl, 11-membered cycloalkyl, 12-membered cycloalkyl, 6- to 10-membered aryl or a bond, preferably a bond, 4-membered mono-heterocyclic ring, 5-membered mono-heterocyclic ring, 6-membered mono-heterocyclic ring, 7-membered mono-heterocyclic ring, 5-membered fused heterocyclic ring, 6-membered fused heterocyclic ring, 7-membered fused heterocyclic ring, 8-membered fused heterocyclic ring, 9-membered fused heterocyclic ring, 10-membered fused heterocyclic ring, 6-membered spiro-heterocyclic ring, 7-membered spiro-heterocyclic ring, 8-membered spiro-heterocyclic ring, 9-membered spiro-heterocyclic ring, 10-membered spiro-heterocyclic ring, 11-membered spiro-heterocyclic ring, 12-membered spiro-heterocyclic ring, 7-membered bridged-heterocyclic ring, 8-membered bridged-heterocyclic ring, 9-membered bridged-heterocyclic ring, 10-membered bridged-heterocyclic ring, 4-membered monocycloalkyl, 5-membered monocycloalkyl, 6-membered monocycloalkyl, 7-membered monocycloalkyl, 5-membered fused cycloalkyl, 6-membered fused cycloalkyl, 7-membered fused cycloalkyl, 8-membered fused cycloalkyl, 9-membered fused cycloalkyl, 10-membered fused cycloalkyl, 6-membered spiro cycloalkyl, 7-membered spiro cycloalkyl, 8-membered spiro cycloalkyl, 9-membered spiro cycloalkyl, 10-membered spiro cycloalkyl, 11-membered spiro cycloalkyl, 12-membered spiro cycloalkyl, 7-membered bridged cycloalkyl, 8-membered bridged cycloalkyl, 9-membered bridged cycloalkyl, 10-membered bridged cycloalkyl or 6- to 10-membered aryl, wherein the aryl, cycloalkyl, mono-heterocyclic ring, fused heterocyclic ring, spiro-heterocyclic ring or bridged-heterocyclic ring is optionally further substituted with 0, 1, 2, 3 or 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy, and the mono-heterocyclic ring, fused heterocyclic ring, spiro-heterocyclic ring or bridged-heterocyclic ring contains 1, 2, 3 or 4 heteroatoms selected from O, S or N.

Some embodiments of the present invention relate to a compound represented by general formulas (I), (Ia) and (Ic) or a stereoisomer, a solvate, a prodrug, a metabolite, a pharmaceutically acceptable salt or a co-crystal thereof, wherein
Cy1, Cy2, Cy3 and Cy4 are each independently selected from one of the following substituted or unsubstituted groups: a bond, phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 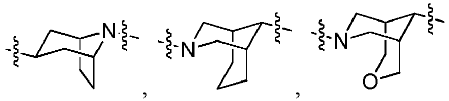 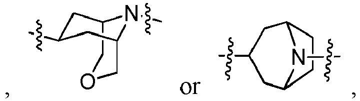 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy.

Some embodiments of the present invention relate to a compound represented by general formulas (I), (Ia) and (Ic) or a stereoisomer, a solvate, a prodrug, a metabolite, a pharmaceutically acceptable salt or a co-crystal thereof, wherein
L is selected from a bond, 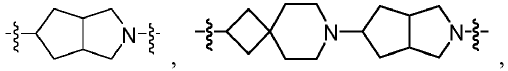 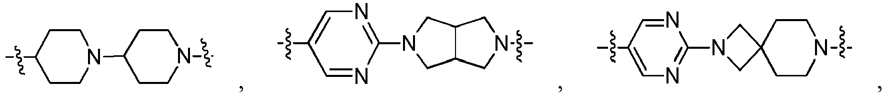 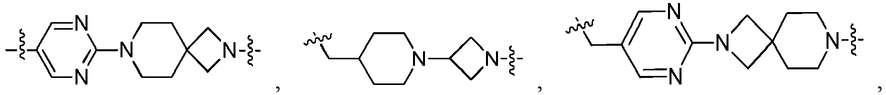 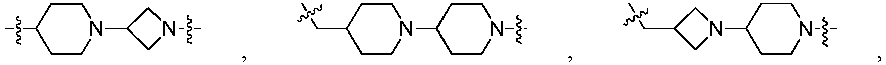 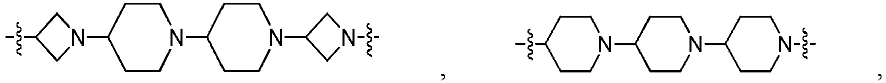 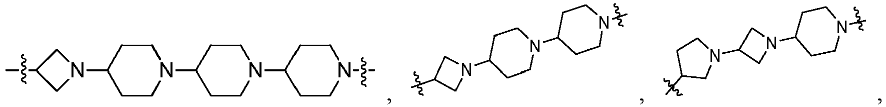 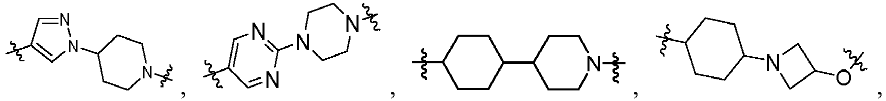 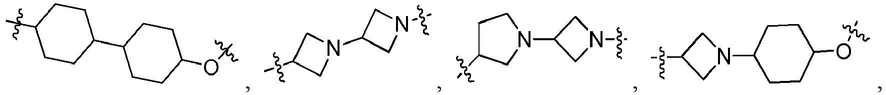 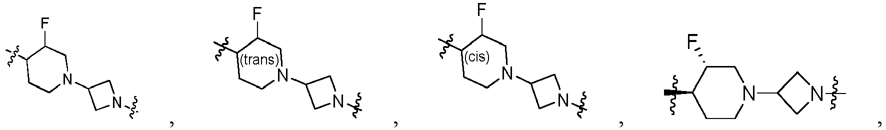 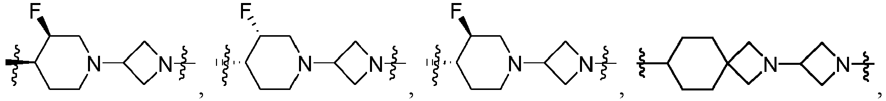 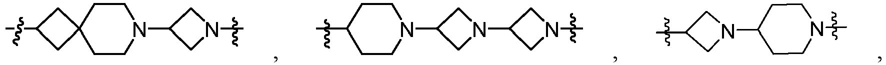 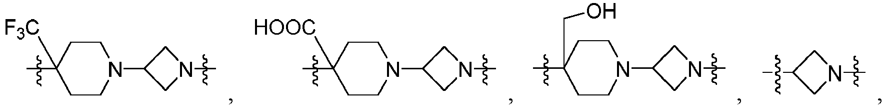 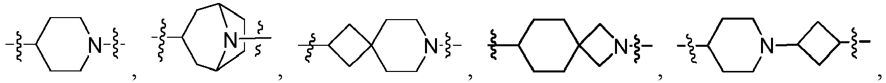 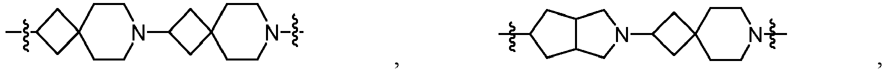 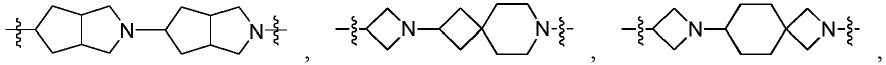 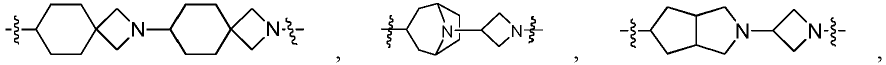 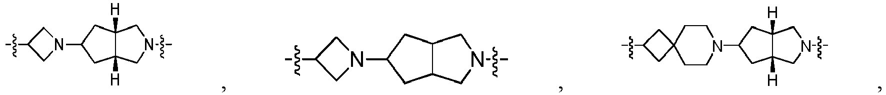 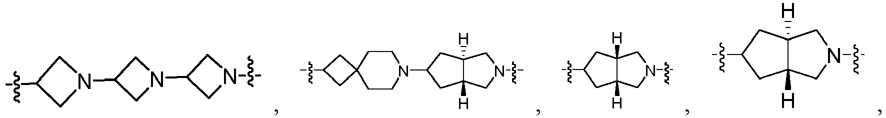 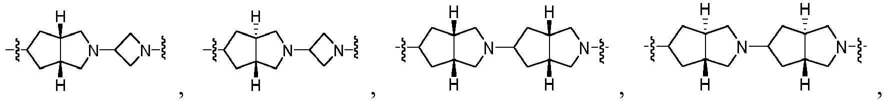 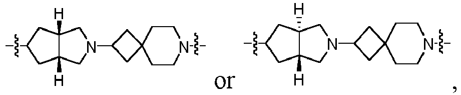
or L is selected from 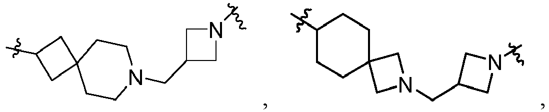 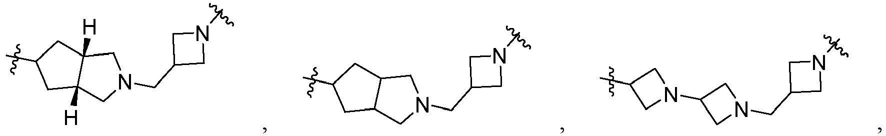 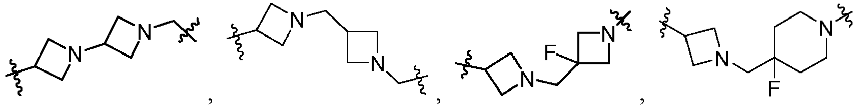 or

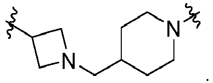

Some embodiments of the present invention relate to a compound represented by general formulas (Ia) and (Ic) or a stereoisomer, a solvate, a prodrug, a metabolite, a pharmaceutically acceptable salt or a co-crystal thereof, wherein
B4 is selected from one of the following substituted or unsubstituted groups: azetidinyl, azacyclopentyl, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, cyclobutyl-spiro-piperidine, cyclopentyl-spiro-piperidine, azetidinyl-spiro-piperidine, azacyclopentyl-spiro-piperidine, 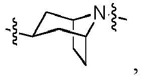 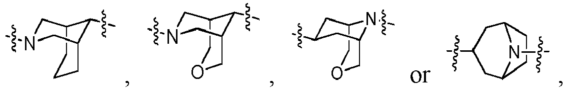 which, when substituted, is optionally further optionally substituted with 0 to 4 R^{b4}; and
R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy.

Some embodiments of the present invention relate to a compound represented by general formulas (Ia) and (Ic) or a stereoisomer, a solvate, a prodrug, a metabolite, a pharmaceutically acceptable salt or a co-crystal thereof, wherein
B4 is selected from one of the following substituted or unsubstituted groups: azetidinyl, azacyclopentyl, 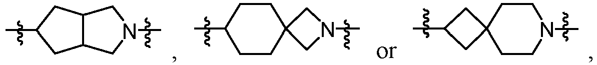 which, when substituted, is optionally further optionally substituted with 0 to 4 R^{b4}; and
R^{b4} is each independently selected from H, F, Cl, Br, I, OH, NH₂, CN, CF₃, CONH₂, COOH, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy.

Some embodiments of the present invention relate to a compound represented by general formulas (Ia) and (Ic) or a stereoisomer, a solvate, a prodrug, a metabolite, a pharmaceutically acceptable salt or a co-crystal thereof, wherein
B4 is selected from one of the following substituted or unsubstituted groups: 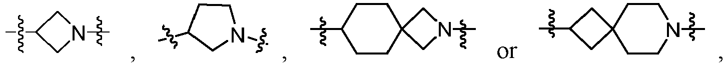 which, when substituted, is optionally further optionally substituted with 0 to 4 substituents selected from R^{b4}; and
R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I.

Some embodiments of the present invention relate to a compound represented by general formula (Ib) or a stereoisomer, a solvate, a prodrug, a metabolite, a pharmaceutically acceptable salt or a co-crystal thereof, wherein
L is selected from 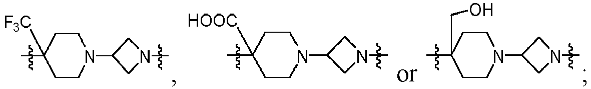
L is connected to B on the left side, and is connected to K on the right side.

Some embodiments of the present invention relate to a compound represented by general formulas (I) and (Ia) or a stereoisomer, a solvate, a prodrug, a metabolite, a pharmaceutically acceptable salt or a co-crystal thereof, wherein L is selected from -Cy1-CH₂-Cy2-, and Cy1 and Cy2 are each independently selected from 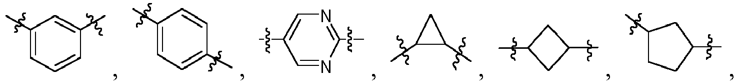 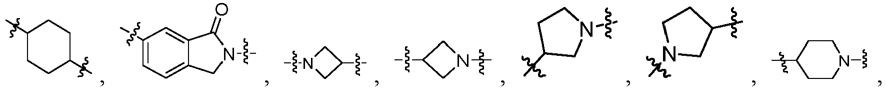 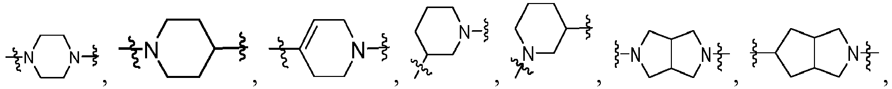 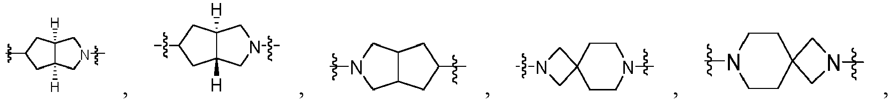 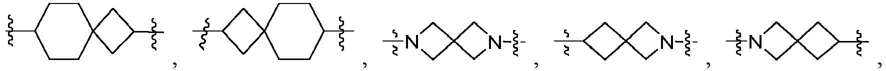 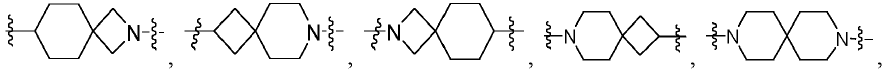 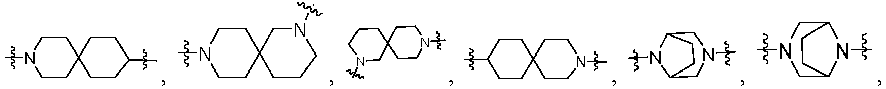 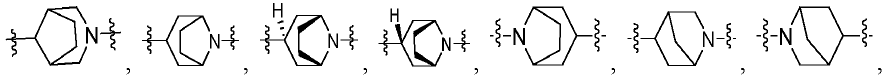 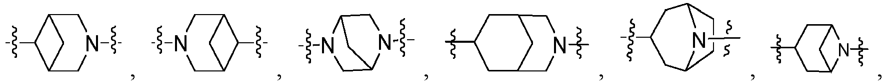 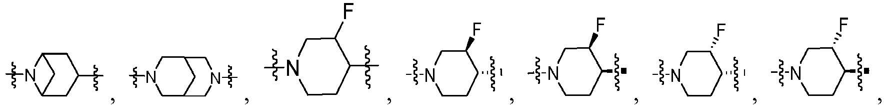 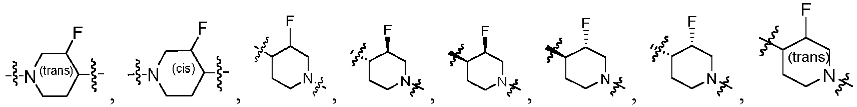 or 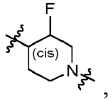 which, when substituted, is optionally further substituted with 0 to 4 substituents selected from H, F, CF₃, methyl, =O, hydroxymethyl, COOH, CN or NH₂.

Some embodiments of the present invention relate to a compound represented by general formulas (I), (Ia), (Ib) and (Ic) or a stereoisomer, a solvate, a prodrug, a metabolite, a pharmaceutically acceptable salt or a co-crystal thereof, wherein the salt is selected from trifluoroacetate.

The present invention relates to a pharmaceutical composition, comprising the compound described in the present invention or a stereoisomer, a solvate, a prodrug, a metabolite, a pharmaceutically acceptable salt or a co-crystal thereof, and a pharmaceutically acceptable carrier.

The present invention relates to the use of the compound described in the present invention or a stereoisomer, a solvate, a prodrug, a metabolite, a pharmaceutically acceptable salt or a co-crystal thereof in the preparation of a drug for treating diseases related to BTK activity or expression level.

The present invention relates to the use of the compound described in the present invention or a stereoisomer, a solvate, a prodrug, a metabolite, a pharmaceutically acceptable salt or a co-crystal thereof in the preparation of a drug for treating diseases related to the inhibition or degradation of BTK.

The present invention relates to the use of the compound described in the present invention or a stereoisomer, a solvate, a prodrug, a metabolite, a pharmaceutically acceptable salt or a co-crystal thereof, and the disease is selected from tumors or autoimmune diseases.

The present invention relates to the use of the compound described in the present invention or a stereoisomer, a solvate, a prodrug, a metabolite, a pharmaceutically acceptable salt or a co-crystal thereof, the tumor is selected from non-Hodgkin's lymphoma, chronic lymphocytic leukemia, mantle cell lymphoma, B cell lymphoma, and the autoimmune disease is selected from rheumatoid arthritis or psoriasis.

### Synthetic method I:

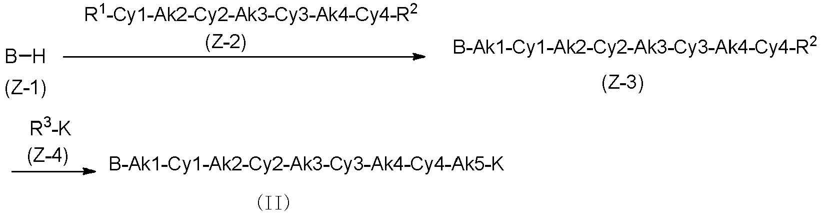

General formula (Z-1) and general formula (Z-2) are subjected to reductive amination, nucleophilic substitution reaction or coupling reaction to obtain the corresponding general formula (Z-3), and if the general formula (Z-3) has an amino protecting group at the reaction site, the amino protecting group is removed first, and then it is subjected to nucleophilic substitution reaction with general formula (Z-4) to obtain the corresponding general formula (II), namely, general formula (I), and the preparation of longer chains can be achieved by repeating the process in the first step above and removing the amino protecting group; 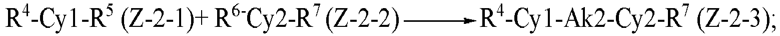
general formula (Z-2-1) and general formula (Z-2-2) can be subjected to nucleophilic substitution reaction, coupling reaction or reductive amination to obtain general formula (Z-2-3), and the preparation of longer chains can be achieved by repeating the process above;
if (Z-2-1) has an amino protecting group at the reaction site, the protecting group is removed, and then it can be subjected to nucleophilic substitution reaction or coupling reaction or reductive amination with general formula (Z-2-2) to obtain general formula (Z-2-3), and the preparation of longer chains can be achieved by repeating the process above;
or general formula (Z-1) and general formula (Z-2-1) are subjected to nucleophilic substitution reaction, coupling reaction or reductive amination reaction (the chain length can be increased by means of the preparation method for general formula (Z-2-3)) to obtain the corresponding general formula (II), namely, general formula (I), wherein the length of L chain can be prepared by means of the preparation method for general formula (Z-2-3).

### Synthetic method II:

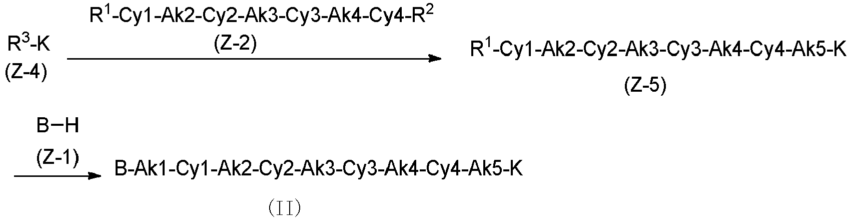

If general formula (Z-2) has an amino protecting group at the reaction site, the amino protecting group is removed first, and then it is subjected to nucleophilic substitution reaction or coupling reaction with general formula (Z-4) to obtain the corresponding general formula (Z-5), and general formula (Z-5) and general formula (Z-1) are subjected to nucleophilic substitution reaction or coupling reaction to obtain the corresponding general formula (II), namely, general formula (I).

### Synthetic method III:

A part of the chain L can be subjected to nucleophilic substitution reaction or coupling reaction with general formula (Z-1) first, and then subjected to nucleophilic substitution reaction or coupling reaction with other parts of the chain L (see the preparation of general formula (Z-2-3) for the synthetic method), by such analogy, to obtain general formula (Z-3), and general formula (Z-3) and general formula (Z-4) are subjected to nucleophilic substitution reaction or coupling reaction to obtain the corresponding general formula (II), namely, general formula (I).

### Synthetic method IV:

Alternatively, a part of the chain L can be subjected to nucleophilic substitution reaction or coupling reaction with general formula (Z-4) first, and then subjected to nucleophilic substitution reaction or coupling reaction with other parts of the chain L (see the preparation of general formula (Z-2-3) for the synthetic method), by such analogy, to obtain general formula (Z-5), and general formula (Z-5) and general formula (Z-1) are subjected to nucleophilic substitution reaction or coupling reaction to obtain the corresponding general formula (II), namely, general formula (I).

### Synthetic method V:

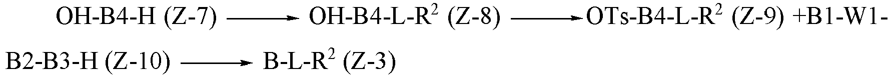

General formula (Z-7) and general formula (Z-2) are subjected to nucleophilic substitution reaction or coupling reaction to obtain general formula (Z-8)
or general formula (Z-7) is subjected to nucleophilic substitution reaction or coupling reaction with a part of the chain L, and then is subjected to nucleophilic substitution reaction or coupling reaction with other parts of the chain L (see the preparation of general formula (Z-2-3) for the synthetic method), to obtain general formula (Z-8), and general formula (Z-8) is reacted with p-toluenesulfonyl chloride to obtain general formula (Z-9), and general formula (Z-9) is subjected to nucleophilic substitution reaction or coupling reaction with general formula (Z-10) to obtain general formula (Z-3).

### Synthetic method VI:

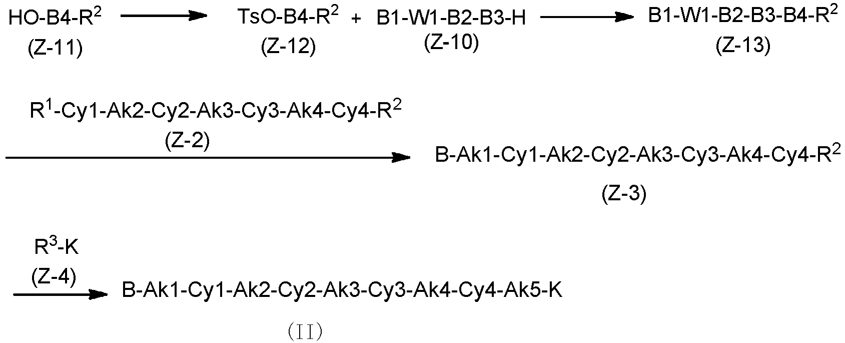

General formula (Z-11) is reacted with p-toluenesulfonyl chloride to obtain general formula (Z-12), and general formula (Z-12) is subjected to nucleophilic substitution reaction with general formula (Z-10) to obtain general formula (Z-13), and if general formula (Z-13) has an amino protecting group at reaction site, the amino protecting group is removed first, and then it is subjected to reductive amination, nucleophilic substitution reaction or coupling reaction with general formula (Z-2) to obtain the corresponding general formula (Z-3), and if general formula (Z-3) has an amino protecting group at the reaction site, the amino protecting group is removed first, and then it is subjected to nucleophilic substitution reaction with general formula (Z-4) to obtain the corresponding general formula (II), namely, general formula (I), and the preparation of longer chains can be achieved by repeating the process in the first step above and removing the amino protecting group; 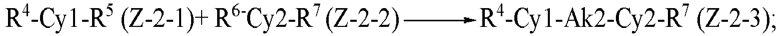
general formula (Z-2-1) and general formula (Z-2-2) can be subjected to nucleophilic substitution reaction, coupling reaction or reductive amination to obtain general formula (Z-2-3), and the preparation of longer chains can be achieved by repeating the process above;
if (Z-2-1) has an amino protecting group at the reaction site, the protecting group is removed, and then it can be subjected to nucleophilic substitution reaction or coupling reaction or reductive amination with general formula (Z-2-2) to obtain general formula (Z-2-3), and the preparation of longer chains can be achieved by repeating the process above;
or general formula (Z-13) and general formula (Z-2-1) are subjected to nucleophilic substitution reaction, coupling reaction or reductive amination reaction (the chain length can be increased by means of the preparation method for general formula (Z-2-3)) to obtain the corresponding general formula (II), namely, general formula (I), wherein the length of L chain can be prepared by means of the preparation method for general formula (Z-2-3).

### Synthetic method VII:

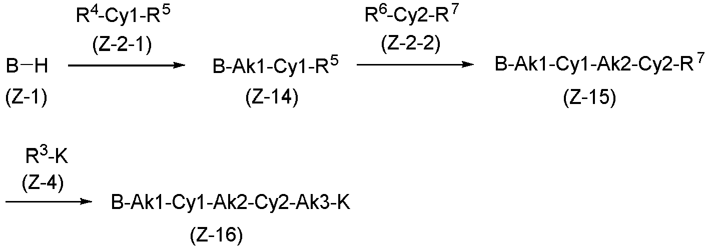

General formula (Z-1) and general formula (Z-2-1) are subjected to reductive amination reaction to obtain the corresponding general formula (Z-14), and if general formula (Z-14) has an amino protecting group at the reaction site, the amino protecting group is removed first, and then it is subjected to reductive amination reaction with general formula (Z-2-2) to obtain general formula (Z-15), and if general formula (Z-15) has an amino protecting group at the reaction site, the amino protecting group is removed first, and then it is subjected to nucleophilic substitution reaction with general formula (Z-4) to obtain general formula (Z-16), namely, general formula (I).

### Synthetic method VIII:

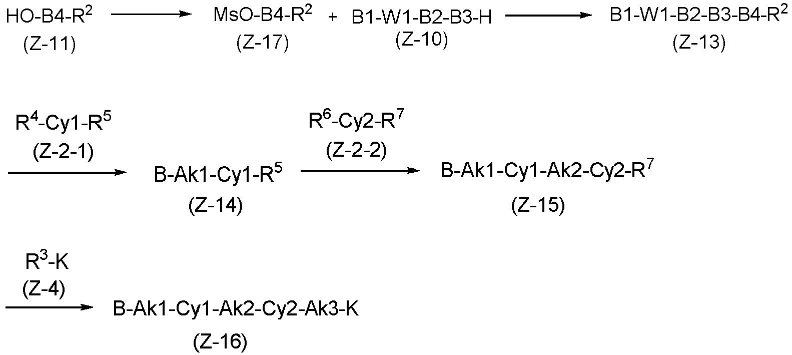

General formula (Z-11) is reacted with methanesulfonyl chloride to obtain general formula (Z-17), and general formula (Z-17) and general formula (Z-10) are subjected to nucleophilic substitution reaction to obtain general formula (Z-13), and if general formula (Z-13) has an amino protecting group at the reaction site, the amino protecting group is removed first, and then it is subjected to reductive amination with general formula (Z-2-1) to obtain the corresponding general formula (Z-14), and if general formula (Z-14) has an amino protecting group at the reaction site, the amino protecting group is removed first, and then it is subjected to reductive amination reaction with general formula (Z-2-2) to obtain general formula (Z-15), and if general formula (Z-15) has an amino protecting group at the reaction site, the amino protecting group is removed first, and then it is subjected to nucleophilic substitution reaction with general formula (Z-4) to obtain general formula (Z-16), namely, general formula (I).

See J. Med. Chem. 2015, 58, 9625-9638 for the synthetic method of general formula (Z-1); and
see WO 2017197056 for the synthetic method of general formula (Z-4);
R¹ is selected from (=O), -CHO, F, Cl, Br, I, or OTf;
R² is selected from H, (=O), -CHO, F, Cl, Br, I or an amino protecting group, preferably Boc;
R³ is selected from NH₂, F, Cl, Br, I, OTf, or OH; and
R⁴, R⁵, R⁶ and R⁷ are each independently selected from H, (=O), -CHO, H, F, Cl, Br, I, OTf or an amino protecting group.

Unless stated to the contrary, the terms used in the description and claims have the following meanings.

The carbon, hydrogen, oxygen, sulfur, nitrogen or F, Cl, Br, I involved in the groups and compounds of the present invention all comprises their isotopes, and the carbon, hydrogen, oxygen, sulfur or nitrogen involved in the groups and compounds of the present invention is optionally further substituted by one or more of their corresponding isotopes, wherein the isotopes of carbon comprise ¹²C, ¹³C and ¹⁴C, the isotopes of hydrogen comprise protium (H), deuterium (D, also known as heavy hydrogen), tritium (T, also known as superheavy hydrogen), the isotopes of oxygen comprise ¹⁶O, ¹⁷O and ¹⁸O, the isotopes of sulfur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"Alkyl" refers to a straight or branched saturated aliphatic hydrocarbon group containing 1 to 20 carbon atoms, preferably alkyl containing 1 to 8 carbon atoms, more preferably alkyl containing 1 to 6 carbon atoms, further preferably alkyl containing 1 to 4 carbon atoms. Non-limiting examples include methyl, ethyl, n-propyl, isopropyl, n-butyl, sec-butyl, isobutyl, tert-butyl, n-pentyl, isoamyl, neopentyl, n-hexyl and various branched isomers thereof; the alkyl is optionally further substituted with 0 to 6 substituents selected from F, Cl, Br, I, hydroxyl, sulfhydryl, nitro, cyano, amino, alkylamino, amido, alkenyl, alkynyl, C₁₋₆ alkyl, C₁₋₆ hydroxyalkyl, C₁₋₆ alkoxy, 3- to 8-membered carbocyclyl, 3- to 8-membered heterocyclic group, 3- to 8-membered carbocyclyloxy, 3- to 8-membered heterocyclyloxy, carboxyl or a carboxylate group, and the definition of the alkyl described herein is consistent with this definition.

"Alkoxy" refers to -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy. The alkyl can be optionally further substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, heterocyclic group, carbocyclyloxy, heterocyclyloxy, carboxyl or a carboxylate group. The definition of the alkoxy described herein is consistent with this definition.

"Heterocyclic group" or "heterocyclic ring" refers to a substituted or unsubstituted saturated or unsaturated aromatic ring or non-aromatic ring, and the aromatic ring or non-aromatic ring can be 3- to 8-membered monocyclic ring, 4- to 12-membered bicyclic ring or 10- to 15-membered tricyclic ring system, and contains 1 to 3 heteroatoms selected from N, O or S, preferably 3- to 8-membered heterocyclic group, and the optionally substituted N, S in the heterocyclic group can be oxidized into various oxidation states. Heterocyclic group can be connected to a heteroatom or carbon atom, and heterocyclic group can be connected to a bridged ring or spiro ring. Non-limiting examples include oxiranyl, azacyclopropyl, oxetanyl, azetidinyl, 1,3-dioxolane, 1,4-dioxolane, 1,3-dioxane, azacycloheptyl, pyridyl, furanyl, thienyl, pyranyl, N-alkylpyrrolyl, pyrimidinyl, pyrazinyl, pyridazinyl, imidazoly, piperidyl, morpholinyl, thiomorpholinyl, 1,3-dithianyl, dihydrofuranyl, dihydropyranyl, dithiolanyl, tetrahydrofuranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuranyl, azabicyclo[3.2.1]octyl, azabicyclo[5.2.0]nonyl, oxatricyclo[5.3.1.1]dodecyl, azaadamantyl and oxaspiro[3.3]heptyl. The heterocyclic group can be optionally further substituted with 0 to 5 substituents selected from F, Cl, Br, I, =O, hydroxyl, sulfhydryl, nitro, cyano, amino, alkylamino, amido, alkenyl, alkynyl, alkyl, hydroxyalkyl, alkoxy, carbocyclyl, heterocyclic group, carbocyclyloxy, heterocyclyloxy, carboxyl or a carboxylate group. The definition of the heterocyclic group described herein is consistent with this definition.

"Spiro ring" refers to a 5- to 20-membered polycyclic group sharing one carbon atom (referred to as a spiro atom) between substituted or unsubstituted monocyclic rings, which may contain 0 to 5 double bonds, and may contain 0 to 5 heteroatoms selected from N, O or S(=O)ₙ. The spiro ring is preferably 6- to 14-membered, further preferably 6- to 12-membered, and more preferably 6- to 10-membered. Its non-limiting examples include: 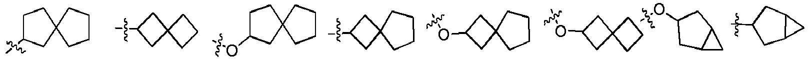 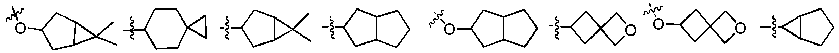 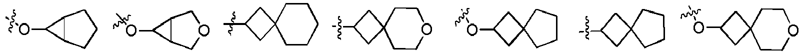 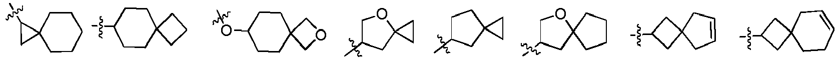 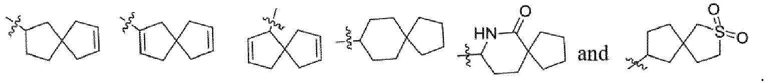 When the heteroaryl ring is substituted, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, heterocyclic group, bridged ring group, spiro ring group, fused ring group, hydroxyalkyl, =O, carbonyl, aldehyde, carboxylic acid, formate, -(CH₂)ₘ-C(=O)-R^{a}, -O-(CH₂)ₘ-C(=O)-R^{a}, -(CH₂)ₘ-C(=O)-NR^{b}R^{c}, - (CH₂)ₘS(=O)ₙR^{a}, -(CH₂)ₘ-alkenyl-R^{a}, OR^{d} or -(CH₂)ₘ-alkynyl-R^{a} (wherein m and n are 0, 1 or 2), arylthio, thiocarbonyl, silyl or -NR^{b}R^{c} and the like, wherein R^{b} and R^{c} are independently selected from H, hydroxyl, amino, carbonyl, alkyl, alkoxy, cycloalkyl, heterocyclic group, aryl, heteroaryl, sulfonyl, trifluoromethylsulfonyl. Alternatively, R^{b} and R^{c} may form a five- or six-membered cycloalkyl or heterocyclic group. R^{a} and R^{d} are each independently selected from aryl, heteroaryl, alkyl, alkoxy, cycloalkyl, heterocyclic group, carbonyl, ester group, bridged ring group, spiro ring group or fused ring group. The definition of the heterocyclic group described herein is consistent with this definition.

"Fused ring" refers to a polycyclic group in which each ring in the system shares an adjacent pair of carbon atoms with other rings in the system, wherein one or more of the rings may contain 0 or more double bonds, which may be substituted or unsubstituted, and each ring in the fused ring system may contain 0 to 5 heteroatoms selected from N, S(=O)ₙ or O. The fused ring is preferably 5- to 20-membered, further preferably 5- to 14-membered, more preferably 5- to 12-membered, and still further preferably 5- to 10-membered. Non-limiting examples include: 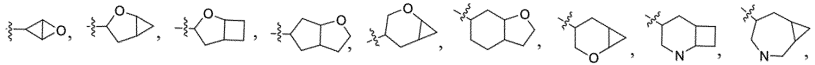 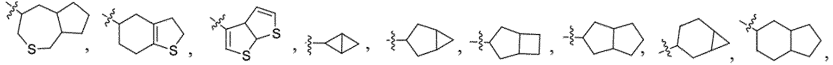 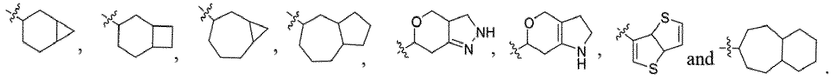 When the heteroaryl ring is substituted, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, heterocyclic group, bridged ring group, spiro ring group, fused ring group, hydroxyalkyl, =O, carbonyl, aldehyde, carboxylic acid, formate, -(CH₂)ₘ-C(=O)-R^{a}, -O-(CH₂)ₘ-C(=O)-R^{a}, -(CH₂)ₘ-C(=O)-NR^{b}R^{c}, - (CH₂)ₘS(=O)ₙR^{a}, -(CH₂)ₘ-alkenyl-R^{a}, OR^{d} or -(CH₂)ₘ-alkynyl-R^{a} (wherein m and n are 0, 1 or 2), arylthio, thiocarbonyl, silyl or -NR^{b}R^{c} and the like, wherein R^{b} and R^{c} are independently selected from H, hydroxyl, amino, carbonyl, alkyl, alkoxy, cycloalkyl, heterocyclic group, aryl, heteroaryl, sulfonyl, trifluoromethylsulfonyl. Alternatively, R^{b} and R^{c} may form a five- or six-membered cycloalkyl or heterocyclic group. R^{a} and R^{d} are each independently selected from aryl, heteroaryl, alkyl, alkoxy, cycloalkyl, heterocyclic group, carbonyl, ester group, bridged ring group, spiro ring group or fused ring group. The definition of the heterocyclic group described herein is consistent with this definition.

"Bridged ring" refers to a polycyclic group containing any two carbon atoms that are not directly connected, which group may contain 0 or more double bonds and can be substituted or unsubstituted, and any ring in the fused ring system may contain 0 to 5 heteroatoms or groups selected from N, S(=O)ₙ or O (wherein n is 1, 1, or 2). The ring atoms contain 5 to 20 atoms, preferably 5 to 14 atoms, further preferably 5 to 12 atoms, and still further preferably 5 to 10 atoms. Non-limiting examples include 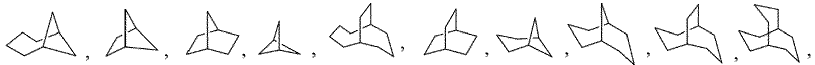 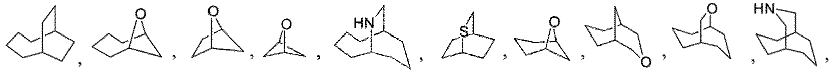 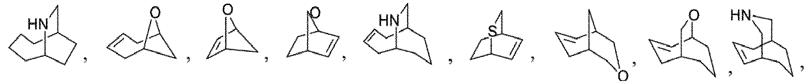 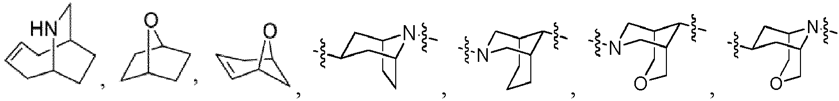 and adamantane. When the heteroaryl ring is substituted, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, heterocyclic group, bridged ring group, spiro ring group, fused ring group, hydroxyalkyl, =O, carbonyl, aldehyde, carboxylic acid, formate, -(CH₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d} or -(CH₂)ₘ-alkynyl-R^{a} (wherein m and n are 0, 1 or 2), arylthio, thiocarbonyl, silyl or -NR^{b}R^{c} and the like, wherein R^{b} and R^{c} are independently selected from H, hydroxyl, amino, carbonyl, alkyl, alkoxy, cycloalkyl, heterocyclic group, aryl, heteroaryl, sulfonyl, trifluoromethylsulfonyl. Alternatively, R^{b} and R^{c} may form a five- or six-membered cycloalkyl or heterocyclic group. R^{a} and R^{d} are each independently selected from aryl, heteroaryl, alkyl, alkoxy, cycloalkyl, heterocyclic group, carbonyl, ester group, bridged ring group, spiro ring group or fused ring group. The definition of the heterocyclic group described herein is consistent with this definition.

"Mono-heterocyclic ring" refers to "heterocyclic group" or "heterocyclic ring" in a monocyclic ring system, and the definition of the heterocyclic group described herein is consistent with this definition.

"Fused heterocyclic ring" refers to a "fused ring" containing heteroatom(s). The definition of the heterocyclic group described herein is consistent with this definition.

"Spiro-heterocyclic ring" refers to a "spiro ring" containing heteroatom(s). The definition of the heterocyclic group described herein is consistent with this definition.

"Bridged-heterocyclic ring" refers to a "bridged ring" containing heteroatom(s). The definition of the heterocyclic group described herein is consistent with this definition.

"Heteroaryl" or "heteroaryl ring" refers to a substituted or unsubstituted 5-to 14-membered aromatic ring, and contains 1 to 5 heteroatoms or groups selected from N, O or S(=O)ₙ, preferably 5- to 10-membered aromatic ring, further preferably 5- to 6-membered. Non-limiting examples of heteroaryl include, but are not limited to pyridyl, furanyl, thienyl, pyridyl, pyranyl, N-alkylpyrrolyl, pyrimidinyl, pyrazinyl, pyridazinyl, imidazolyl, piperidyl, morpholine, thiomorpholine, 1,3-dithiane, benzimidazole, benzimidazole, benzopyridine, pyrrolopyridine and the like. The heteroaryl ring can be fused to an aryl, heterocyclic group or cycloalkyl ring, wherein the ring connected to the parent structure is a heteroaryl ring, and non-limiting examples include 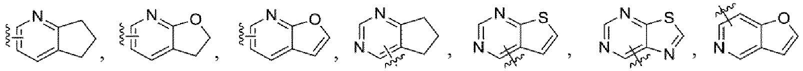 and

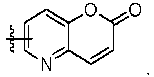

When the heteroaryl ring is substituted, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, heterocyclic group, bridged ring group, spiro ring group, fused ring group, hydroxyalkyl, =O, carbonyl, aldehyde, carboxylic acid, formate, -(CH₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d} or -(CH₂)ₘ-alkynyl-R^{a} (wherein m and n are 0, 1 or 2), arylthio, thiocarbonyl, silyl or -NR^{b}R^{c} and the like, wherein R^{b} and R^{c} are independently selected from H, hydroxyl, amino, carbonyl, alkyl, alkoxy, cycloalkyl, heterocyclic group, aryl, heteroaryl, sulfonyl, trifluoromethylsulfonyl. Alternatively, R^{b} and R^{c} may form a five- or six-membered cycloalkyl or heterocyclic group. R^{a} and R^{d} are each independently selected from aryl, heteroaryl, alkyl, alkoxy, cycloalkyl, heterocyclic group, carbonyl, ester group, bridged ring group, spiro ring group or fused ring group. The definition of the heterocyclic group described herein is consistent with this definition.

"Containing 1 to 4 heteroatoms selected from O, S or N" refers to contains 1, 2, 3 or 4 heteroatoms selected from O, S or N.

"Substituted with 0 to X substituents" refers to substituted with 0, 1, 2, 3, ..., or X substituents, wherein X is selected from any integer between 1 and 10. For example, "substituted with 0 to 4 substituents" refers to substituted with 0, 1, 2, 3 or 4 substituents. For example, "substituted with 0 to 5 substituents" refers to substituted with 0, 1, 2, 3, 4 or 5 substituents. For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is selected from an integer less than Y and greater than or equal to 3, and Y is selected from any integer between 4 and 12) includes X+1-, X+2-, X+3-, X+4-, ..., Y-membered rings. Rings include a heterocyclic ring, a carbocyclic ring, an aromatic ring, aryl, heteroaryl, cycloalkyl, a mono-heterocyclic ring, a fused heterocyclic ring, a spiro-heterocyclic ring or a bridged-heterocyclic ring. For example, "a 4- to 7-membered mono-heterocyclic ring" refers to a 4-membered, 5-membered, 6-membered or 7-membered mono-heterocyclic ring, and "a 5- to 10-membered fused heterocyclic ring" refers to a 5-membered, 6-membered, 7-membered, 8-membered, 9-membered or 10-membered fused heterocyclic ring.

The term "optional" or "optionally" refers to that the event or circumstance subsequently described may but not necessarily occur, and the description includes the occasions where the events or circumstances occur or do not occur. For example, "alkyl optionally substituted with F" means that an alkyl may but not necessarily be substituted by F, and the description includes the case where the alkyl is substituted with F and the case where the alkyl is not substituted with F.

"Pharmaceutically acceptable salt" or "pharmaceutically acceptable salt thereof' refers to a salt of the compound of the present invention maintaining the biological effectiveness and characteristics of the free acid or free base, and obtained by reacting the free acid with a non- toxic inorganic base or organic base, reacting the free base with a non- toxic inorganic acid or organic acid.

"Pharmaceutical composition" refers to a mixture of one or more of the compounds of the present invention, a pharmaceutically acceptable salt or a prodrug thereof, and other chemical components, wherein "other chemical components" refer to pharmaceutically acceptable carriers, excipients and/or one or more other therapeutic agents.

"Carrier" refers to a material that does not cause significant irritation to the organism and does not eliminate the biological activity and characteristics of the administered compound.

"Excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatin, vegetable oils, polyethylene glycols, diluents, granulating agents, lubricants, adhesives and disintegrants.

"Prodrug" refers to a compound that can be converted into a compound of the present invention with biological activity through metabolism *in vivo.* The prodrug of the present invention is prepared by modifying the amino or carboxyl group in the compound of the present invention, and the modification can be removed by conventional operations or *in vivo* to obtain the parent compound. When the prodrug of the present invention is administered to a mammalian individual, the prodrug is split to form free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

IC₅₀ (half-inhibitory concentration of a measured inhibitor): is obtained by testing the concentration of test compounds required to inhibit 50% binding of gabapentin to calcium ion channels.

### Detailed Description of Embodiments

The technical solutions of the present invention will be described in detail below in conjunction with examples, but the protection scope of the present invention includes but is not limited thereto.

The structures of the compounds are determined by nuclear magnetic resonance (NMR) or (and) mass spectrometry (MS). The NMR shift (δ) is given in the unit of 10⁻⁶ (ppm). NMR is measured with (Bruker Avance III 400 and Bruker Avance 300) NMR instrument, and the solvent for determination is deuterated dimethyl sulfoxide (DMSO-d₆), deuterated chloroform (CDCl₃), deuterated methanol (CD₃OD), and the internal standard is tetramethylsilane (TMS);
MS is measured with (Agilent 6120B(ESI) and Agilent 6120B(APCI));
HPLC is determined with Agilent 1260DAD high pressure liquid chromatograph (Zorbax SB-C18 100 × 4.6 mm, 3.5 µM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.

For the column chromatography, Yantai Huanghai silica gel of 200-300 mesh silica gel is generally used as a carrier;
the known starting materials of the present invention can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies;
P13I: 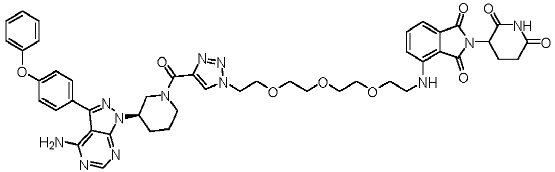 (References for synthesis: Y. Sun, X. Zhao, N. Ding, H. Gao, Y. Wu, Y. Yang, M. Zhao, J. Hwang, Y. Song, W. Liu, Y. Rao, Cell Res. 2018, 28, 779-781.)
Tf: Trifluoromethylsulfonyl;
Boc: Tert-butoxycarbonyl;
Ts: P- toluenesulfonyl;
*Cbz:* Benzyloxycarbonyl;
TMS: Trimethylsilane;

### Example 63:

### 5-(2-(4-amino-3-(4-phenoxyphenyl)-1H-pyrazolo[3,4-d]pyrimidin-1-yl)-7-azaspiro [3.5]nonan-7-yl)-2-(2,6-dioxopiperidin-3-yl)isoindoline-1,3-dione (compound 63)

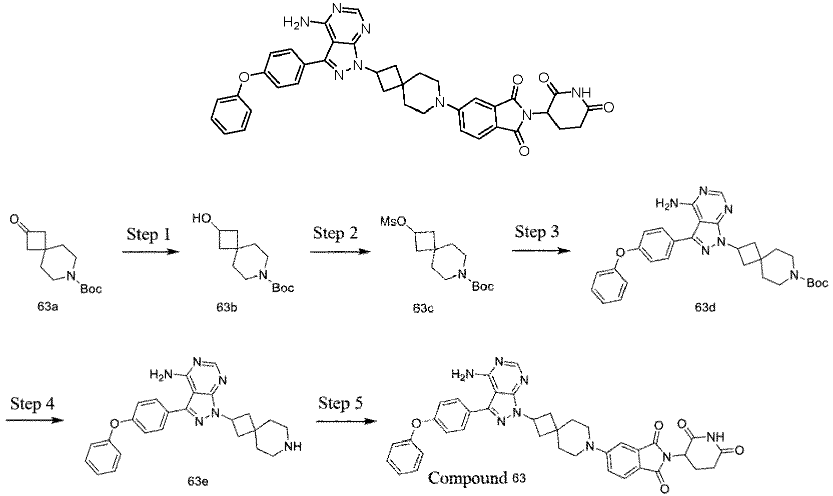

### Step 1:

### tert-butyl 2-hydroxyl-7-azaspiro[3.5]nonane-7-carboxylate (63b)

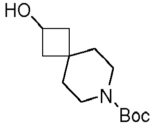

Tert-butyl 2-oxo-7-azaspiro[3.5]nonane-7-carboxylate (18 g, 75.22 mmol) was dissolved in 200 ml of anhydrous methanol, and sodium borohydride (5.7 g, 150.0 mmol) was slowly added in portions. Upon completion of the addition, the mixture was stirred at room temperature for 30 min. To the reaction solution was added 250 ml of saturated sodium bicarbonate aqueous solution, and the resulted solution was extracted with 100 mL of DCM. The organic phase was washed with 100 mL of water, dried over anhydrous sodium sulfate, and concentrated under reduced pressure. The crude product was separated and purified by silica gel column chromatography (petroleum ether /ethyl acetate (v/v) = 2 : 1), to obtain tert-butyl 2-hydroxyl-7-azaspiro[3.5]nonane-7-carboxylate (63b) (12 g, yield: 66%).
LCMS m/z = 242.3 [M+1] ⁺.

### Step 2:

### tert-butyl 2-((methylsulfonyl)oxy)-7-azaspiro[3.5]nonane-7-carboxylate (63c)

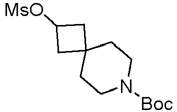

Tert-butyl 2-hydroxyl-7-azaspiro[3.5]nonane-7-carboxylate (63b) (3.0 g, 12.43 mmol) was dissolved in 80 mL of dichloromethane, DIPEA (4.82 g, 42.10 mmol) was added, then MsCl (1.71 g, 14.93 mmol) was slowly added dropwise. Upon completion of the addition, the mixture was stirred at room temperature for 0.5 h. The reaction solution was quenched with 150 mL of water, and extracted with 100 mL of DCM. The organic phase was washed with 100 mL of water, dried over anhydrous sodium sulfate, and concentrated under reduced pressure. The crude product was separated and purified by silica gel column chromatography (petroleum ether/ethyl acetate (v/v) = 5 : 1), to obtain tert-butyl 2-((methylsulfonyl)oxy)-7-azaspiro[3.5]nonane-7-carboxylate (63c) (3.5 g, yield: 88.5%).
LCMS m/z = 320.3 [M+1]⁺.

### Step 3:

### tert-butyl 2-(4-amino-3-(4-phenoxyphenyl)-1H-pyrazolo[3,4-d]pyrimidin-1-yl)-7-azaspiro[3.5] nonane-7-carboxylate (63d)

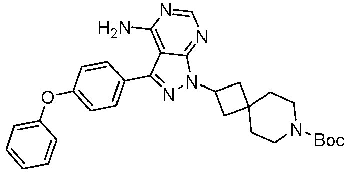

Tert-butyl 2-((methylsulfonyl)oxy)-7-azaspiro[3.5]nonane-7-carboxylate (63c) (2.5 g, 7.83 mmol) and 3-(4-phenoxyphenyl)-1H-pyrazolo[3,4-d]pyrimidin-4-amine (2.0 g, 6.60 mmol) were dissolved in 40 mL of DMF, cesium carbonate (6.45 g, 19.80 mmol) was added, and the reaction was stirred at 100°C for 6 h. The reaction solution was cooled to room temperature, 80 mL of water was added, and the resulted solution was extracted with 100 mL of ethyl acetate. The organic phase was washed with 100 mL of water, dried over anhydrous sodium sulfate, and concentrated under reduced pressure. The crude product was separated and purified by silica gel column chromatography (dichloromethane/methanol (v/v) = 15 : 1), to obtain tert-butyl 2-(4-amino-3-(4-phenoxyphenyl)-1H-pyrazolo[3,4-d]pyrimidin-1-yl)-7-azaspiro[3.5]nonane-7-carboxylate (63d) (3.25 g, yield: 78.6%).
LCMS m/z = 527.6 [M+1]⁺.

### Step 4:

### 3-(4-phenoxyphenyl)-1-(7-azaspiro[3.5]nonan-2-yl)-1H-pyrazolo[3,4-d]pyrimidin-4-amine (63e)

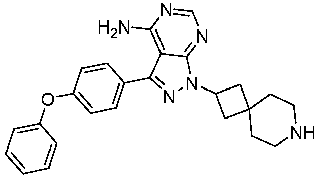

Tert-butyl 2-(4-amino-3-(4-phenoxyphenyl)-1H-pyrazolo[3,4-d]pyrimidin-1-yl)-7-azaspiro[3.5]nonane-7-carboxylate (63d) (1.0 g, 1.90 mmol) was dissolved in 20 mL of DCM, 8 mL of trifluoroacetic acid was added, and the mixture was stirred at room temperature for 4 hours. The system was subjected to rotary evaporation, and the residue was dissolved with 5 N sodium hydroxide aqueous solution, extracted with 100 mL of dichloromethane. The organic phase was washed with 100 mL of water, dried over anhydrous sodium sulfate, and concentrated under reduced pressure, to obtain 3-(4-phenoxyphenyl)-1-(7-azaspiro[3.5]nonan-2-yl)-1H-pyrazolo[3,4-d]pyrimidin-4-amine (63e) (780 mg, yield: 96.2%).
LCMS m/z = 427.6 [M+1]⁺.

### Step 5:

### 5-(2-(4-amino-3-(4-phenoxyphenyl)-1H-pyrazolo[3,4-d]pyrimidin-1-yl)-7-azaspiro [3.5]nonan-7-yl)-2-(2,6-dioxopiperidin-3-yl)isoindoline-1,3-dione (compound 63)

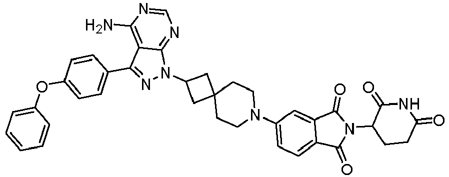

3-(4-phenoxyphenyl)-1-(7-azaspiro[3.5]nonan-2-yl)-1H-pyrazolo[3,4-d]pyrimidin-4-amine (63e) (260 mg, 0.61 mmol) was dissolved in 25 mL of DMSO, and 1.5 mL of DIPEA and 2-(2,6-dioxopiperidin-3-yl)-5-fluoroisoindoline-1,3-dione (see WO 2017197056 for the synthetic method) (252 mg, 0.91 mmol) were added, and the reaction was stirred at 80°C for 5 h. The reaction solution was cooled to room temperature, 50 mL of water was added, and the resulted solution was extracted with 100 mL of ethyl acetate. The organic phase was washed with 100 mL of water, dried over anhydrous sodium sulfate, and concentrated under reduced pressure. The crude product was separated and purified by silica gel column chromatography (dichloromethane/methanol (v/v) = 15 : 1), to obtain 5-(2-(4-amino-3-(4-phenoxyphenyl)-1H-pyrazolo[3,4-d]pyrimidin-1-yl)-7-azaspiro[3.5]nonan-7-yl)-2-(2,6-dioxopiperidin-3-yl)isoindoline-1,3-dione (compound 63) (150 mg, yield: 36.0%).
¹H NMR (400 MHz, CDCl₃) δ 8.92 (s, 1H), 8.38 (s, 1H), 7.72 - 7.63 (m, 3H), 7.44 - 7.36 (m, 2H), 7.30 (d, 1H), 7.22 - 7.13 (m, 3H), 7.12 - 7.04 (m, 3H), 5.89 (br.s, 2H), 5.55 - 5.43 (m, 1H), 4.94 (dd, 1H), 3.56 - 3.36 (m, 4H), 2.94 - 2.62 (m, 5H), 2.56 - 2.46 (m, 2H), 2.20 - 2.08 (m, 1H), 1.96 - 1.85 (m, 4H).
LCMS m/z = 683.3 [M+1]⁺.

### Example 64:

### 5-(3-(2-(4-amino-3-(4-phenoxyphenyl)-1H-pyrazolo[3,4-d]pyrimidin-1-yl)-7-azaspiro[3. 5]nonan-7-yl)azetidin-1-yl)-2-(2,6-dioxopiperidin-3-yl)isoindoline-1,3-dione (compound 64)

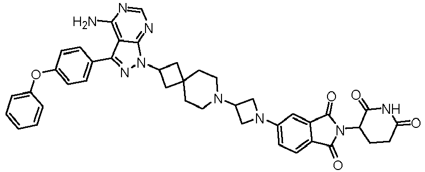

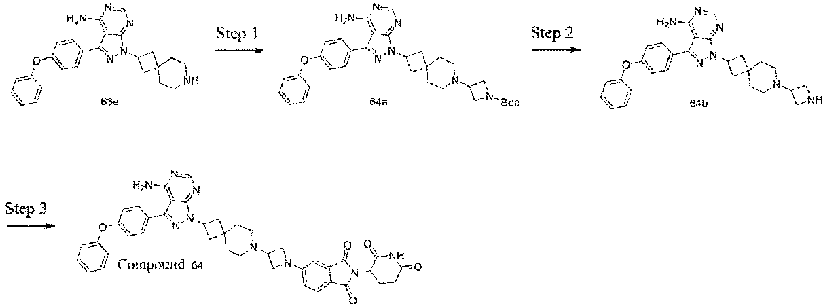

### Step 1:

### tert-butyl 3-(2-(4-amino-3-(4-phenoxyphenyl)-1H-pyrazolo[3,4-d]pyrimidin-1-yl)-7-azaspiro [3.5]nonan-7-yl)azetidine-1-carboxylate (64a)

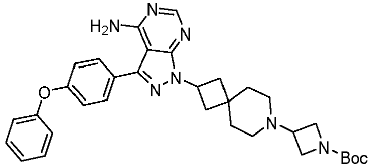

3-(4-phenoxyphenyl)-1-(7-azaspiro[3.5]nonan-2-yl)-1H-pyrazolo[3,4-d]pyrimidin-4-amine (63e) (520 mg, 1.22 mmol) was dissolved in 30 mL of DCE (1,2-dichloroethane), and tert-butyl 3-oxoazetidine-1-carboxylate (416 mg, 2.43 mmol) was added. The mixture was stirred at room temperature for 10 minutes, then sodium triacetoxyborohydride (1.03 g, 4.86 mmol) was added, and the mixture was stirred at room temperature overnight. To the reaction system was slowly added 100 mL of sodium bicarbonate aqueous solution, and the mixed solution was extracted with DCM (30 mL × 3). The organic phase was washed with 50 mL of water, dried over anhydrous sodium sulfate, and concentrated under reduced pressure. The crude product was separated and purified by silica gel column chromatography (dichloromethane/methanol (v/v) = 20 : 1), to obtain tert-butyl 3-(2-(4-amino-3-(4-phenoxyphenyl)-1H-pyrazolo[3,4-d]pyrimidin-1-yl)-7-azaspiro[3.5]nonan-7-yl)azetidine-1-carboxylate (64a) (630 mg, yield: 88.8%).
LCMS m/z = 582.4 [M+1]⁺.

### Step 2:

### 1-(7-(azetidin-3-yl)-7-azaspiro[3.5]nonan-2-yl)-3-(4-phenoxyphenyl)-1H-pyrazolo[3,4-d]pyrimidin-4-amine (64b)

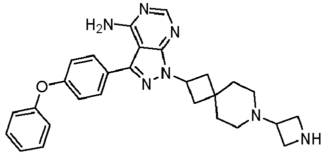

Tert-butyl 3-(2-(4-amino-3-(4-phenoxyphenyl)-1H-pyrazolo[3,4-d]pyrimidin-1-yl)-7-azaspiro[3.5]nonan-7-yl)azetidine-1-carboxylate (64a) (610 mg, 1.05 mmol) was dissolved in 20 mL of DCM, 4 mL of trifluoroacetic acid was added, and the mixture was stirred at room temperature for 4 hours. The reaction system was subjected to rotary evaporation, and the residue was dissolved with 30 mL of 5 N sodium hydroxide aqueous solution, extracted with DCM (30 mL × 4). The organic phase was combined, washed with 1 N sodium hydroxide aqueous solution (30 mL × 2), dried over anhydrous sodium sulfate, and concentrated under reduced pressure, to obtain 1-(7-(azetidine-3-yl)-7-azaspiro[3.5]nonan-2-yl)-3-(4-phenoxyphenyl)-1H-pyrazolo[3,4-d]pyrimidin-4-amine (64b) (0.43 g, yield: 85.0%).
LCMS m/z = 482.5 [M+1]⁺.

### Step 3:

### 5-(3-(2-(4-amino-3-(4-phenoxyphenyl)-1H-pyrazolo[3,4-d]pyrimidin-1-yl)-7-azaspiro[3. 5]nonan-7-yl)azetidin-1-yl)-2-(2,6-dioxopiperidin-3-yl)isoindoline-1,3-dione (compound 64)

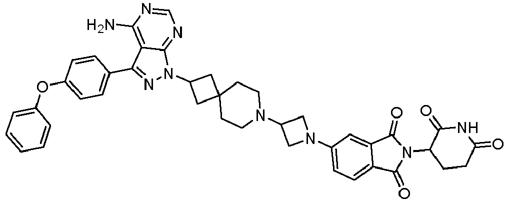

1-(7-(azetidine-3 -yl)-7-azaspiro[3 .5]nonan-2-yl)-3 -(4-phenoxyphenyl)-1H-pyrazolo[3,4-d]pyrimidin-4-amine (64b) (150 mg, 0.31 mmol) was dissolved in 15 mL of DMSO, and 1.5 mL of DIPEA and 2-(2,6-dioxopiperidin-3-yl)-5-fluoroisoindoline-1,3-dione (see WO 2017197056 for the synthetic method) (130 mg, 0.47 mmol) were added, and the reaction was stirred at 80°C for 5 hours. The reaction solution was cooled, 50 mL of water was added, and the resulted solution was extracted with 100 mL of ethyl acetate. The organic phase was washed with 100 mL of water, dried over anhydrous sodium sulfate, and concentrated under reduced pressure, and then the crude product was separated and purified by silica gel column chromatography (dichloromethane/methanol (v/v) = 15 : 1), to obtain 5-(3-(2-(4-amino-3-(4-phenoxyphenyl)-1H-pyrazolo[3,4-d]pyrimidin-1-yl)-7-azaspiro[3.5]nonan-7-yl)azetidine-1-yl)-2-(2,6-dioxopiperidin-3-yl)isoindoline-1,3-dione (compound 64) (95 mg, yield: 41.5%).
¹H NMR (400 MHz, CDCl₃) δ 9.09 (s, 1H), 8.39 (s, 1H), 7.70 - 7.61 (m, 3H), 7.43 - 7.35 (m, 2H), 7.22 - 7.12 (m, 3H), 7.12 - 7.05 (m, 2H), 6.79 (d, 1H), 6.53 (dd, 1H), 5.67 (br.s, 2H), 5.49 - 5.37 (m, 1H), 4.92 (dd, 1H), 4.15 - 4.07 (m, 2H), 4.00 - 3.89 (m, 2H), 3.43 - 3.31 (m, 1H), 2.93 - 2.66 (m, 3H), 2.66 - 2.57 (m, 2H), 2.55 - 2.25 (m, 6H), 2.16 - 2.08 (m, 1H), 1.93 - 1.81 (m, 4H).
LCMS m/z = 738.3 [M+1]⁺.

### Example 65:

### 5-(3-(2-(4-amino-3-(4-phenoxyphenyl)-1H-pyrazolo[3,4-d]pyrimidin-1-yl)-7-azaspiro[3.5]nonan-7-yl)-[1,3'-biazetidin]-1'-yl)-2-(2,6-dioxopiperidin-3-yl) isoindoline-1,3-dione (compound 65)

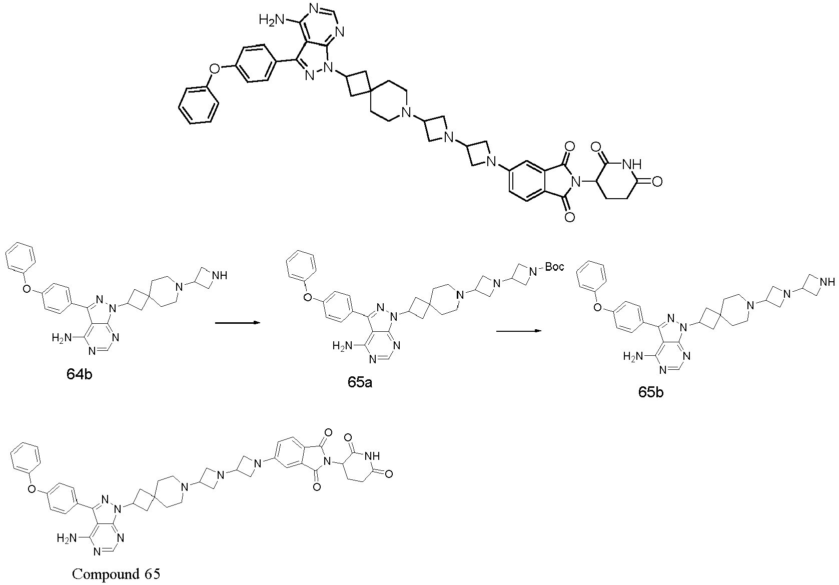

### Step 1:

### tert-butyl 3-(2-(4-amino-3-(4-phenoxyphenyl)-1H-pyrazolo[3,4-d]pyrimidin-1-yl)-7-azaspiro[3.5]nonan-7-yl)-[1,3'-biazetidine]-1'-carboxylate (65a)

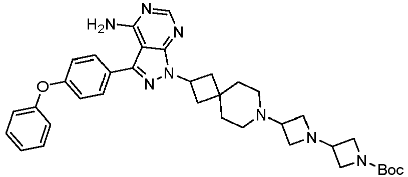

1-(7-(azetidine-3 -yl)-7-azaspiro[3 .5]nonan-2-yl)-3 -(4-phenoxyphenyl)-1H-pyrazolo[3,4-d]pyrimidin-4-amine (64b) (330 mg, 0.69 mmol) was dissolved in 30 mL of DCE, and tert-butyl 3-oxoazetidine-1-carboxylate (234 mg, 1.37 mmol) was added. The mixture was stirred at room temperature for 10 minutes, then sodium triacetoxyborohydride (581 mg, 2.74 mmol) was added, and the mixture was stirred at room temperature overnight. To the reaction system was slowly added 100 mL of saturated sodium bicarbonate aqueous solution, and the mixed solution was extracted with DCM (30 mL × 3). The organic phase was washed with 50 mL of water, dried over anhydrous sodium sulfate, and concentrated under reduced pressure. The crude product was separated and purified by silica gel column chromatography (dichloromethane/methanol (v/v) = 20 : 1), to obtain tert-butyl 3-(2-(4-amino-3-(4-phenoxyphenyl)-1H-pyrazolo[3,4-d]pyrimidin-1-yl)-7-azaspiro[3.5]nonan-7-yl)-[1,3'-diazetidinyl]-1'-carboxylate (65a) (350 mg, yield: 79.6%).
LCMS m/z = 637.5 [M+1]⁺.

### Step 2:

### 1-(7-([1,3'-biazetidin]-3-yl)-7-azaspiro[3.5]nonan-2-yl)-3-(4-phenoxyphenyl)-1H-pyrazolo[3,4-d]pyrimidin-4-amine (65b)

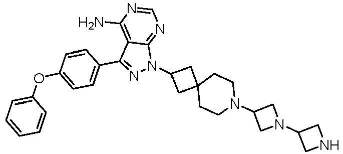

Tert-butyl 3-(2-(4-amino-3-(4-phenoxyphenyl)-1H-pyrazolo[3,4-d]pyrimidin-1-yl)-7-azaspiro[3.5]nonan-7-yl)-[1,3'-diazetidinyl]-1'-carboxylate (65a) (340 mg, 0.53 mmol) was dissolved in 20 mL of DCM, 8 mL of trifluoroacetic acid was added, and the mixture was stirred at room temperature for 4 hours. The reaction system was subjected to rotary evaporation, and the residue was dissolved with 30 mL of 5 N sodium hydroxide aqueous solution, extracted with DCM (30 mL × 4). The organic phase was washed with 1 N sodium hydroxide aqueous solution (30 mL × 2), dried over anhydrous sodium sulfate, and concentrated under reduced pressure, to obtain 1-(7-([1,3'-biazetidin]-3-yl)-7-azaspiro[3.5]nonan-2-yl)-3-(4-phenoxyphenyl)-1H-pyrazolo[3,4-d]pyrimidin-4-amine (65b) (260 mg, yield: 91.3%).
LCMS m/z = 537.5 [M+1]⁺.

### Step 3:

### 5-(3-(2-(4-amino-3-(4-phenoxyphenyl)-1H-pyrazolo[3,4-d]pyrimidin-1-yl)-7-azaspiro[3.5]nonan-7-yl)-[1,3'-biazetidin]-1'-yl)-2-(2,6-dioxopiperidin-3-yl) isoindoline-1,3-dione (compound 65)

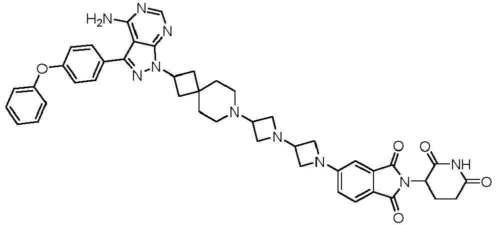

1-(7-([1,3'-biazetidin]-3-yl)-7-azaspiro[3.5]nonan-2-yl)-3-(4-phenoxyphenyl)-lH-pyrazolo[3,4-d]pyrimidin-4-amine (65b) (250 mg, 0.47 mmol) was dissolved in 15 mL of DMSO, and 1.5 mL of DIPEA and 2-(2,6-dioxopiperidin-3-yl)-5-fluoroisoindoline-1,3-dione (see WO 2017197056 for the synthetic method) (130 mg, 0.47 mmol) were added, and the reaction was stirred at 80°C for 5 hours. The reaction solution was cooled to room temperature, 50 mL of water was added, and the resulted solution was extracted with 100 mL of ethyl acetate. The organic phase was washed with 100 mL of water, dried over anhydrous sodium sulfate, and concentrated under reduced pressure, and then the crude product was separated and purified by silica gel column chromatography (dichloromethane/methanol (v/v) = 15 : 1), to obtain 5-(3-(2-(4-amino-3-(4-phenoxyphenyl)-1H-pyrazolo[3,4-d]pyrimidin-1-yl)-7-azaspiro[3.5]nonan-7-yl)-[1,3'-biazetidin]-1'-yl)-2-(2,6-dioxopiperidin-3-yl)isoindoline-1,3-dione (compound 65) (130 mg, yield: 34.9%).

¹H NMR (400 MHz, CDCl₃) δ 9.61 (s, 1H), 8.38 (s, 1H), 7.70 - 7.59 (m, 3H), 7.43 - 7.35 (m, 2H), 7.21 - 7.11 (m, 3H), 7.11 - 7.05 (m, 2H), 6.77 (d, 1H), 6.50 (dd, 1H), 5.70 (br.s, 2H), 5.46 - 5.35 (m, 1H), 4.92 (dd, 1H), 4.07 - 3.97 (m, 2H), 3.90 - 3.81 (m, 2H), 3.73 - 3.63 (m, 1H), 3.58 - 3.50 (m, 2H), 3.20 - 2.92 (m, 3H), 2.92 - 2.65 (m, 3H), 2.63 - 2.53 (m, 2H), 2.48 - 2.06 (m, 7H), 1.90 - 1.74 (m, 4H).
LCMS m/z = 793.3 [M+1]⁺.

### Example 66:

### 5-(2'-(4-amino-3-(4-phenoxyphenyl)-1H-pyrazolo[3,4-d]pyrimidin-1-yl)-7,7'-diaza[2,7'-bispiro[3.5]nonan]-7-yl)-2-(2,6-dioxopiperidin-3-yl)isoindoline-1,3-dione (compound 66)

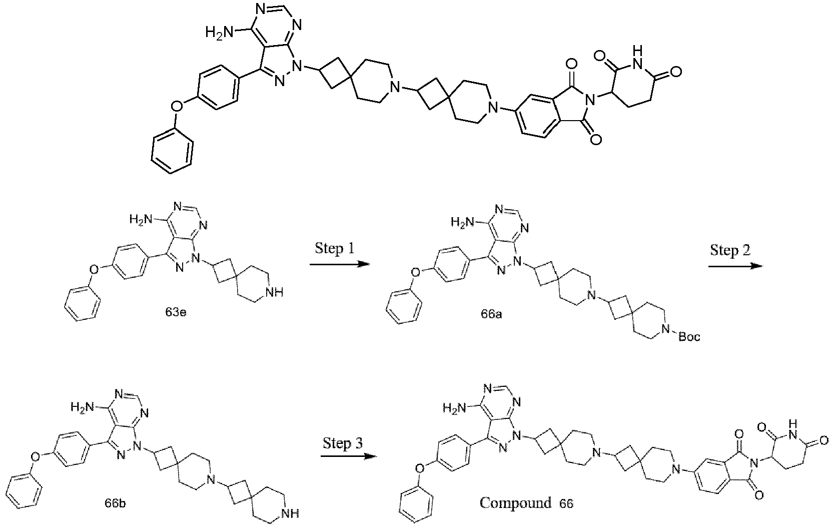

### Step 1:

### tert-butyl 2'-(4-amino-3-(4-phenoxyphenyl)-1H-pyrazolo[3,4-d]pyrimidin-1-yl)-7,7'-diaza [2,7'-bispiro[3.5]nonane]-7-carboxylate (66a)

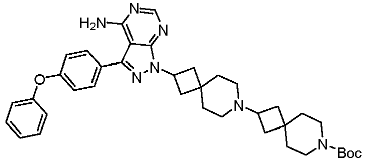

3-(4-phenoxyphenyl)-1-(7-azaspiro[3.5]nonan-2-yl)-1H-pyrazolo[3,4-d]pyrimidin-4-amine (63e) (250 mg, 0.59 mmol) was dissolved in 20 mL of DCE, and tert-butyl 2-oxo-7-azaspiro[3.5]nonane-7-carboxylate (280 mg, 1.17 mmol) was added. The mixture was stirred at room temperature for 10 minutes, then sodium triacetoxyborohydride (496 mg, 2.34 mmol) was added, and the mixture was stirred at room temperature overnight. To the reaction system was slowly added 100 ml of saturated sodium bicarbonate aqueous solution, and the mixed solution was extracted with DCM (30 ml × 3). The organic phase was washed with 50 mL of water, dried over anhydrous sodium sulfate, and concentrated under reduced pressure. The crude product was separated and purified by silica gel column chromatography (dichloromethane/methanol (v/v) = 20 : 1), to obtain tert-butyl 2'-(4-amino-3-(4-phenoxyphenyl)-1H-pyrazolo[3,4-d]pyrimidin-1-yl)-7,7'-diaza[2,7'-bispiro[3.5]nonane]-7-carboxylate (66a) (310 mg, yield: 80.8%).
LCMS m/z = 650.3 [M+1]⁺.

### Step 2:

### 1-(7,7'-diaza[2,7'-bispiro[3.5]nonan]-2'-yl)-3-(4-phenoxyphenyl)-1H-pyrazolo[3,4-d]pyrimidin-4-amine (66b)

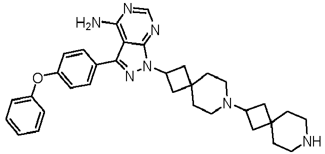

Tert-butyl 2'-(4-amino-3-(4-phenoxyphenyl)-1H-pyrazolo[3,4-d]pyrimidin-1-yl)-7,7'-diaza[2,7'-bispiro[3.5]nonane]-7-carboxylate (66a) (300 mg, 0.46 mmol) was dissolved in 20 mL of DCM, 8 mL of trifluoroacetic acid was added, and the mixture was stirred at room temperature for 4 hours. The reaction system was subjected to rotary evaporation, and the residue was dissolved with 30 mL of 5 N sodium hydroxide aqueous solution, extracted with DCM (30 mL × 4). The organic phase was washed with 1 N sodium hydroxide aqueous solution (30 mL × 4), dried over anhydrous sodium sulfate, and concentrated under reduced pressure, to obtain 1-(7,7'-diaza[2,7'-bispiro[3.5]nonan]-2'-yl)-3-(4-phenoxyphenyl)-1H-pyrazolo[3,4-d]pyrimidin-4-amine (66b) (240 mg, yield: 94.9%).
LCMS m/z = 550.3 [M+1]⁺.

### Step 3:

### 5-(2'-(4-amino-3-(4-phenoxyphenyl)-1H-pyrazolo[3,4-d]pyrimidin-1-yl)-7,7'-diaza[2,7'-bispiro[3.5]nonan]-7-yl)-2-(2,6-dioxopiperidin-3-yl)isoindoline-1,3-dione (compound 66)

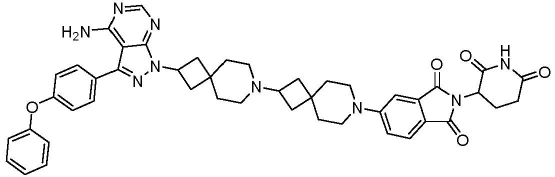

1-(7,7'-diaza[2,7'-bispiro[3.5]nonan]-2'-yl)-3-(4-phenoxyphenyl)-1H-pyrazolo[3,4-d]pyrimidin-4-amine (66b) (230 mg, 0.42 mmol) was dissolved in 15 mL of DMSO, and 1.5 mL of DIPEA and 2-(2,6-dioxopiperidin-3-yl)-5-fluoroisoindoline-1,3-dione (see WO 2017197056 for the synthetic method) (196 mg, 0.71 mmol) were added, and the reaction was stirred at 80°C for 5 hours. The reaction solution was cooled to room temperature, 50 mL of water was added, and the resulted solution was extracted with 100 mL of ethyl acetate. The organic phase was washed with 100 mL of water, dried over anhydrous sodium sulfate, and concentrated under reduced pressure, and then the crude product was separated and purified by silica gel column chromatography (dichloromethane/methanol (v/v) = 15 : 1), to obtain 5-(2'-(4-amino-3-(4-phenoxyphenyl)-1H-pyrazolo[3,4-d]pyrimidin-1-yl)-7,7'-diaza[2,7'-bispiro[3.5]nonan]-7-yl)-2-(2,6-dioxopiperidin-3-yl)isoindoline-1,3-dione (compound 66) (160 mg, yield: 47.3%).
¹H NMR (400 MHz, CDCl₃) δ 9.48 (br.s, 1H), 8.38 (s, 1H), 7.71 - 7.62 (m, 3H), 7.43 - 7.35 (m, 2H), 7.29 - 7.24 (m, 1H), 7.20 - 7.12 (m, 3H), 7.11 - 7.06 (m, 2H), 7.03 (dd, 1H), 5.71 (br.s, 2H), 5.48 - 5.35 (m, 1H), 4.93 (dd, 1H), 3.48 - 3.28 (m, 4H), 2.93 - 2.66 (m, 4H), 2.65 - 2.54 (m, 2H), 2.53 - 2.18 (m, 5H), 2.17 - 2.02 (m, 4H), 2.00 - 1.65 (m, 10H).
LCMS m/z = 806.3 [M+1]⁺.

### Example 67:

### 5-(3-(4-(4-amino-3-(4-phenoxyphenyl)-1H-pyrazolo[3,4-d]pyrimidin-1-yl)piperidin-1-yl)azetidin-1-yl)-2-(2,6-dioxopiperidin-3-yl)isoindoline-1,3-dione (compound 67)

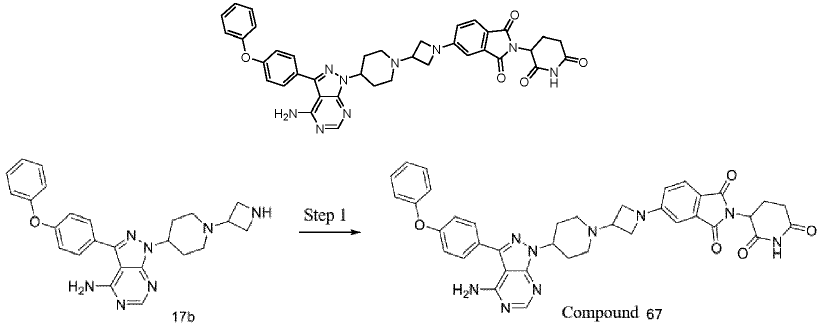

1-[1-(azetidin-3-yl)-4-piperidyl]-3-(4-phenoxyphenyl)pyrazolo[3,4-d]pyrimidin-4-amine (17b) (see WO 2020239103 for the synthetic method) (0.45 g, 1.02 mmol) was dissolved in 8 mL of DMSO, 2-(2,6-dioxopiperidin-3-yl)-5-fluoroisoindoline-1,3-dione (see WO 2017197056 for the synthetic method) (0.28 g, 1.01 mmol) and diisopropylethylamine (0.66 g, 5.10 mmol) were added at room temperature, and the mixture was warmed to 80°C and reacted for 3 h. The reaction solution was poured into 20 mL of water, and the aqueous phase was extracted with a mixed solvent of dichloromethane/methanol (v/v) =10 : 1 (30 mL × 3). The organic phase was combined, washed with 50 mL of water, dried over anhydrous sodium sulfate, and concentrated under reduced pressure. The crude product was separated and purified by silica gel column chromatography (dichloromethane/methanol (v/v) = 10 : 1-8 : 1), to obtain 5-(3-(4-(4-amino-3-(4-phenoxyphenyl)-1H-pyrazolo[3,4-d]pyrimidin-1-yl)piperidine-1-yl)azetidin-1-yl)-2-(2,6-dioxopiperidin-3-yl)isoindoline-1,3-dione (compound 67) (554 mg, yield: 78%).
¹H NMR (400 MHz, DMSO-*d₆*) δ 11.04 (br.s, 1H), 8.24 (s, 1H), 7.72 - 7.61 (m, 3H), 7.50 - 7.36 (m, 2H), 7.23 - 7.07 (m, 5H), 6.81 (d, 1H), 6.67 (dd, 1H), 5.06 (dd, 1H), 4.80 - 4.66 (m, 1H), 4.19 - 4.11 (m, 2H), 3.93 - 3.84 (m, 2H), 3.48 - 3.37 (m, 1H), 3.05 - 2.80 (m, 3H), 2.64 - 2.52 (m, 2H), 2.30 - 2.07 (m, 4H), 2.07 - 1.88 (m, 3H).
LCMS m/z = 698.3 [M+1]⁺.

### Example 68:

### 5-(4-(4-amino-3-(4-phenoxyphenyl)-1H-pyrazolo[3,4-d]pyrimidin-1-yl)-[1,4'-bipiperidin]-1'-yl)-2-(2,6-dioxopiperidin-3-yl)isoindoline-1,3-dione (compound 68)

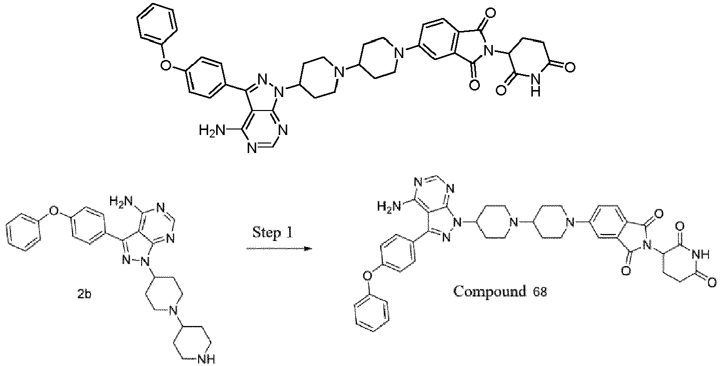

3-(4-phenoxyphenyl)-1-[1-(4-piperidyl)-4-piperidyl]pyrazolo[3,4-d]pyrimidin-4-amine (2b) (see WO 2020239103 for the synthetic method) (400 mg, 0.85 mmol) was dissolved in 4 mL of DMSO, 2-(2,6-dioxopiperidin-3-yl)-5-fluoroisoindoline-1,3-dione (see WO 2017197056 for the synthetic method) (0.23 g, 0.84 mmol) and diisopropylethylamine (0.44 g, 3.40 mmol) were added at room temperature, and the mixture was warmed to 80°C and reacted for 4 hours. The reaction solution was poured into 10 mL of water, and the aqueous phase was extracted with a mixed solvent of dichloromethane/methanol (v/v) =10 : 1 (30 mL × 3). The organic phase was combined, washed with 20 mL of water, dried over anhydrous sodium sulfate, and concentrated under reduced pressure. The crude product was separated and purified by silica gel column chromatography (dichloromethane/methanol (v/v) = 20 : 1-8 : 1), to obtain 5-(4-(4-amino-3-(4-phenoxyphenyl)-1H-pyrazolo[3,4-d]pyrimidin-1-yl)-[1,4'-bipiperidin]-1'-yl)-2-(2,6-dioxopiperidin-3-yl)isoindoline-1,3-dione (compound 68) (469 mg, yield: 65%).
¹H NMR (400 MHz, DMSO-*d₆*) δ 11.05 (s, 1H), 8.26 - 8.20 (m, 1H), 7.97 (dd, 1H), 7.66 (d, 1H), 7.57 - 7.49 (m, 2H), 7.36 - 7.29 (m, 1H), 7.29 - 7.17 (m, 3H), 7.14 (d, 1H), 6.30 (s, 2H), 5.06 (dd, 1H), 4.18 - 4.03 (m, 3H), 3.10 - 2.81 (m, 5H), 2.70 - 2.46 (m, 3H), 2.37 - 2.20 (m, 2H), 2.10 - 1.74 (m, 7H), 1.61 - 1.44 (m, 2H).
LCMS m/z = 363.8 [M/2 +1]⁺.

### Example 69:

### 5-(7-(4-(4-amino-3-(4-phenoxyphenyl)-1H-pyrazolo[3,4-d]pyrimidin-1-yl)piperidin-1-yl)-2-azaspiro[3.5]nonan-2-yl)-2-(2,6-dioxopiperidin-3-yl)isoindoline-1,3-dione (compound 69)

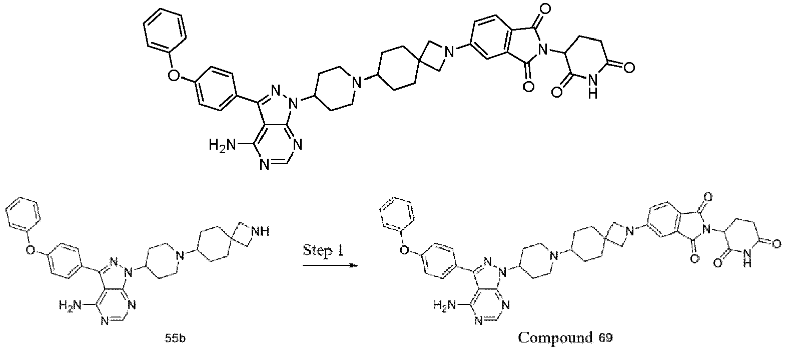

The above crude product 1-(1-(2-azaspiro[3.5]nonan-7-yl)piperidine-4-yl)-3-(4-phenoxyphenyl)-1H-pyrazolo[3,4-d]pyrimidin-4-amine hydrochloride (55b) (see WO 2020239103 for the synthetic method) (100 mg) was dissolved in 3 mL of DMSO, 2-(2,6-dioxopiperidin-3-yl)-5-fluoroisoindoline-1,3-dione (see WO 2017197056 for the synthetic method) (58 mg, 0.21 mmol) and diisopropylethylamine (129 mg, 1.00 mmol) were added at room temperature, and the mixture was warmed to 80°C and reacted for 5 hours. The reaction solution was poured into 9 mL of water, and the aqueous phase was extracted with a mixed solvent of dichloromethane/methanol (v/v) =10 : 1 (30 mL × 3). The organic phase was combined, washed with 50 mL of water, dried over anhydrous sodium sulfate, and concentrated under reduced pressure. The crude product was separated and purified by silica gel column chromatography (dichloromethane/methanol (v/v) = 20 : 1-8 : 1), to obtain 5-(7-(4-(4-amino-3-(4-phenoxyphenyl)-1H-pyrazolo[3,4-d]pyrimidin-1-yl)piperidine-1-yl)-2-azaspiro[3.5]nonan-2-yl)-2-(2,6-dioxopiperidin-3-yl)isoindoline-1,3-dione (compound 69) (92 mg).
¹H NMR (400 MHz, CDCl₃) δ 8.38 (s, 1H), 8.27 (br.s, 1H), 7.74 - 7.50 (m, 3H), 7.43 - 7.33 (m, 2H), 7.22 - 7.11 (m, 3H), 7.11 - 7.04 (m, 2H), 6.77 (d, 1H), 6.50 (dd, 1H), 5.50 (br.s, 2H), 4.93 (dd, 1H), 4.85 - 4.70 (m, 1H), 3.77 - 3.68 (m, 4H), 3.68 - 3.64 (m, 4H), 3.63 - 3.59 (m, 1H), 3.23 - 3.04 (m, 2H), 2.93 - 2.65 (m, 3H), 2.54 - 2.34 (m, 4H), 2.13 - 1.97 (m, 5H), 1.97 - 1.84 (m, 2H),
LCMS m/z = 766.3 [M+1]⁺.

### Example 70:

### 5-(3-(4-(4-amino-3-(4-phenoxyphenyl)-1H-pyrazolo[3,4-d]pyrimidin-1-yl)-4'-(trifluoromethyl)-[1,4'-bipiperidin]-1'-yl)azetidin-1-yl)-2-(2,6-dioxopiperidin-3-yl)isoindoline-1,3-dione (compound 70)

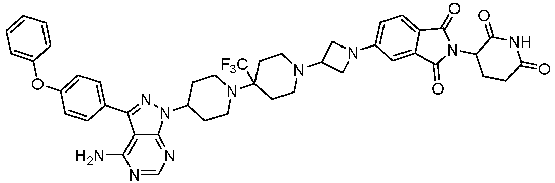

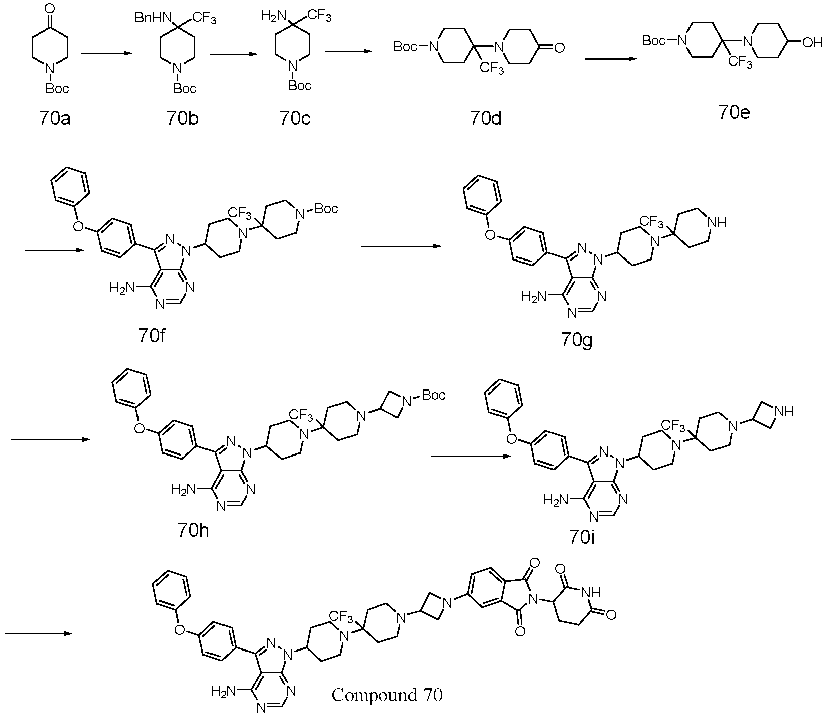

### Step 1:

### tert-butyl 4-(benzylamino)-4-(trifluoromethyl)piperidine-1-carboxylate (70b)

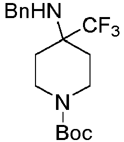

Tert-butyl 4-oxopiperidine-1-carboxylate (70a) (40 g, 0.2 mol) was dissolved in 500 mL of toluene, phenylmethanamine (21.4 g, 0.2 mol) was added, and the mixture was refluxed and reacted for 6 h. The reaction solution was cooled to room temperature, concentrated under reduced pressure, then 500 mL of acetonitrile and DMF (46 mL) were added, KHF₂ (1.2 g, 0.15 mol) was added, and the mixture was cooled to 0°C, and trifluoroacetic acid (20 mL, 0.25 mol) was continuously added dropwise under such condition. Upon completion of the dropwise addition, the mixture was reacted for another 5 min, TMSCF₃ (44 mL, 0.3 mol) was added dropwise under the condition of 0°C, and the mixture was warmed to room temperature and continuously stirred for 16 h. To the reaction solution was slowly added 100 mL of saturated sodium carbonate solution, and the reaction solution was diluted with 800 mL of ethyl acetate. The liquid separation was conducted. The organic phase was washed with 1000 mL of water, dried over anhydrous sodium sulfate, and concentrated under reduced pressure, and then the crude product was separated and purified by silica gel column chromatography (petroleum ether/ethyl acetate (v/v) = 10 : 1), to obtain tert-butyl 4-(benzylamino)-4-(trifluoromethyl)piperidine-1-carboxylate (70b) (40 g, yield: 56%).

### Step 2:

### tert-butyl 4-amino-4-(trifluoromethyl)piperidine-1-carboxylate (70c)

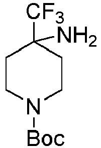

Tert-butyl 4-(benzylamino)-4-(trifluoromethyl)piperidine-1-carboxylate (70b) (40 g, 0.112 mol) was dissolved in 1000 mL of methanol, formic acid (25 mL, 0.66 mol) and 10% palladium/carbon (10 g) were added, and reaction was stirred at 45°C for 16 h. The reaction solution was cooled to room temperature, filtered, and the filtrate was concentrated under reduced pressure, to obtain a white solid. The white solid was dissolved with 900 mL of dichloromethane and 100 mL of methanol, 500 mL of saturated sodium carbonate solution was added, and the mixture was stirred for 30 min. The organic phase was separated, dried over anhydrous sodium sulfate, and concentrated under reduced pressure, to obtain tert-butyl 4-amino-4-(trifluoromethyl)piperidine-1-carboxylate (70c) (27 g, yield: 89%).

### Step 3:

### tert-butyl 4-oxo-4'-(trifluoromethyl)-[1,4'-bipiperidin]-1'-carboxylate (70d)

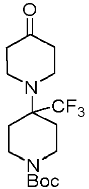

To tert-butyl 4-amino-4-(trifluoromethyl)piperidine-1-carboxylate (70c) (2.7 g, 0.01 mol) was added 15 mL of DMF, potassium carbonate (5.5 g, 0.05 mol) and 1,5-dichloro-3-pentanone (3.1 g, 0.02 mol) were added respectively, and the mixture was warmed to 90°C and reacted for 16 h. The reaction solution was cooled to room temperature, diluted with 200 mL of ethyl acetate, and washed with 200 mL of purified water. The organic phase was dried over anhydrous sodium sulfate, and concentrated under reduced pressure. The crude product was separated and purified by silica gel column chromatography (petroleum ether/ethyl acetate (v/v) = 5 : 1), to obtain 4-oxo-4'-(trifluoromethyl)-[1,4'-bipiperidin]-1'-carboxylate tert-butyl (70d) (1.0 g, yield: 28.6%).

### Step 4:

### tert-butyl 4-hydroxyl-4'-(trifluoromethyl)-[1,4'-bipiperidin]-1'-carboxylate (70e)

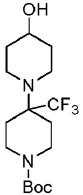

To tert-butyl 4-oxo-4'-(trifluoromethyl)-[1,4'-bipiperidin]-1'-carboxylate (70d) (1.0 g, 2.86 mmol) was added 20 mL of methanol. The mixture was cooled to 0°C, sodium borohydride (0.2 g, 5.7 mol) was slowly added, and the mixture was reacted at room temperature for 2 h. The reaction solution was concentrated under reduced pressure, diluted with 100 mL of ethyl acetate, and washed by adding 100 mL of water. The organic phase was dried over anhydrous sodium sulfate, and concentrated under reduced pressure, to obtain tert-butyl 4-hydroxyl-4'-(trifluoromethyl)-[1,4'-bipiperidin]-1'-carboxylate (70e) (0.9g, yield: 89%).

### Step 5:

### tert-butyl 4-(4-amino-3-(4-phenoxyphenyl)-1H-pyrazolo[3,4-d]pyrimidin-1-yl)-4'-(trifluoromethyl)-[1,4'-bipiperidin]-1'-carboxylate (70f)

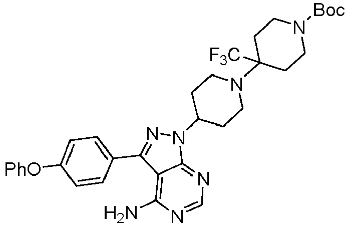

Tert-butyl 4-hydroxyl-4'-(trifluoromethyl)-[1,4'-bipiperidin]-1'-carboxylate (70e) (0.9 g, 2.57 mmol) was added to 15 mL of anhydrous pyridine, TsCl (1.0 g, 5.2 mmol) was added, and the mixture was reacted at 60°C for 3 h. The reaction solution was cooled to room temperature, concentrated under reduced pressure, diluted with 100 mL of ethyl acetate, and washed by adding 100 mL of saturated sodium bicarbonate aqueous solution. The organic phase was dried over anhydrous sodium sulfate, and concentrated under reduced pressure to obtain a crude product. The crude product was dissolved in 20 mL of DMF, 3-(4-phenoxyphenyl)-1H-pyrazolo[3,4-d]pyrimidin-4-amine (0.61 g, 2.0 mmol) and Cs₂CO₃ (1.7 g, 5.2 mmol) were added, and the reaction was stirred at 80°C for 3 h. The reaction solution was cooled to room temperature, diluted by adding 100 mL of ethyl acetate, and washed by adding 100 mL of water. The organic phase was dried over anhydrous sodium sulfate, and concentrated under reduced pressure, then the crude product was separated and purified by silica gel column chromatography (petroleum ether/ethyl acetate (v/v) = 1 : 1), to obtain tert-butyl 4-(4-amino-3-(4-phenoxyphenyl)-1H-pyrazolo[3,4-d]pyrimidin-1-yl)-4'-(trifluoromethyl)-[1,4'-bipiperidin]-1'-carboxylate (70f) (0.4g, total yield: 24%).
¹H NMR (400 MHz, CDCl₃) δ 8.37 (s, 1H), 7.70 - 7.63 (m, 2H), 7.42 - 7.34 (m, 2H), 7.20 - 7.12 (m, 3H), 7.11 - 7.04 (m, 2H), 5.58 (br.s, 2H), 4.92 - 4.79 (m, 1H), 4.08 - 3.75 (m, 2H), 3.37 - 3.22 (m, 2H), 3.20 - 2.92 (m, 2H), 2.79 - 2.65 (m, 2H), 2.35 - 2.19 (m, 2H), 2.14 - 2.98 (m, 4H), 1.74 - 1.61 (m, 2H), 1.48 (s, 9H).
LCMS m/z = 638.3 [M+1]⁺.

### Step 6:

### 3-(4-phenoxyphenyl)-1-(4'-(trifluoromethyl)-[1,4'-bipiperidin]-4-yl)-1H-pyrazolo[3,4-d]pyrimidin-4-amine (70g)

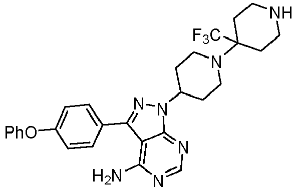

Tert-butyl 4-(4-amino-3-(4-phenoxyphenyl)-1H-pyrazolo[3,4-d]pyrimidin-1-yl)-4'-(trifluoromethyl)-[1,4'-bipiperidin]-1'-carboxylate (70f) (0.4 g, 0.63 mmol) was dissolved in 10 mL of DCM, 3 mL of trifluoroacetic acid was added, and the mixture was stirred at room temperature for 3 h. To the reaction solution was slowly added dropwise 30 mL of saturated sodium bicarbonate aqueous solution, the mixed solution was extracted with 50 mL of dichloromethane, and washed with 50 mL of water. The organic phase was dried over anhydrous sodium sulfate, and concentrated under reduced pressure, to obtain 3-(4-phenoxyphenyl)-1-(4'-(trifluoromethyl)-[1,4'-bipiperidin]-4-yl)-1H-pyrazolo[3,4-d]pyrimidin-4-amine (70g) (0.32 g, yield: 95%).
LCMS m/z = 538.6 [M+1]⁺.

### Step 7:

### tert-butyl 3-(4-(4-amino-3-(4-phenoxyphenyl)-1H-pyrazolo[3,4-d]pyrimidin-1-yl)-4'-(trifluoromethyl)-[1,4'-bipiperidin]-1'-yl)azetidine-1-carboxylate (70h)

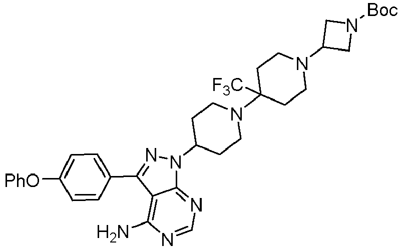

3-(4-phenoxyphenyl)-1-(4'-(trifluoromethyl)-[1,4'-bipiperidin]-4-yl)-1H-pyrazolo[3,4-d]pyrimidin-4-amine (70g) (0.32 g, 0.6 mmol) was dissolved in 20 mL of DCE, and tert-butyl 3-oxoazetidine-1-carboxylate (0.2 g, 1.2 mmol) was added. The mixture was stirred at room temperature for 1 h, then sodium triacetoxyborohydride (0.6 g, 3.0 mmol) was added, and the mixture was stirred at room temperature for 16 h. To the reaction solution was slowly added dropwise 30 mL of saturated sodium bicarbonate solution, and the mixed solution was extracted by adding 30 mL of dichloromethane. The organic phase was washed with 50 mL of water, separated, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-phenoxyphenyl)-1H-pyrazolo[3,4-d]pyrimidin-1-yl)-4'-(trifluoromethyl)-[1,4' - bipiperidin]-1'-yl)azetidine-1'-carboxylate (70h) (0.3 g, yield: 70%).
LCMS m/z = 693.3 [M+1]⁺.

### Step 8:

### 1-(1'-(azetidin-3-yl)-4'-(trifluoromethyl)-[1,4'-bipiperidin]-4-yl)-3-(4-phenoxyphenyl)-1H-pyrazolo[3,4-d]pyrimidin-4-amine (70i)

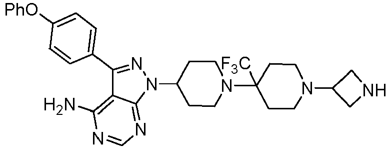

Tert-butyl 3-(4-(4-amino-3-(4-phenoxyphenyl)-1H-pyrazolo[3,4-d]pyrimidin-1-yl)-4'-(trifluoromethyl)-[1,4'-bipiperidin]-1'-yl)azetidine-1'-carboxylate (70h) (0.3 g, 0.43 mmol) was dissolved in 10 mL of DCM, 3 mL of trifluoroacetic acid was added, and the mixture was stirred at room temperature for 3 h. To the reaction solution was slowly added dropwise 30 mL of saturated sodium bicarbonate solution, and the mixed solution was extracted by adding 50 mL of dichloromethane. The organic phase was washed with 50 mL of water, separated, dried over anhydrous sodium sulfate, and concentrated under reduced pressure, to obtain 1-(1'-(azetidin-3-yl)-4'-(trifluoromethyl)-[1,4'-bipiperidin]-4-yl)-3-(4-phenoxyphenyl)-1H-pyrazolo[3,4-d]pyrimidin-4-amine (70i) (0.25 g, yield: 96%).
LCMS m/z = 593.3 [M+1]⁺.

### Step 9:

### 5-(3-(4-(4-amino-3-(4-phenoxyphenyl)-1H-pyrazolo[3,4-d]pyrimidin-1-yl)-4'-(trifluoromethyl)-[1,4'-bipiperidin]-1'-yl)azetidin-1-yl)-2-(2,6-dioxopiperidin-3 - yl)isoindoline-1,3-dione (compound 70)

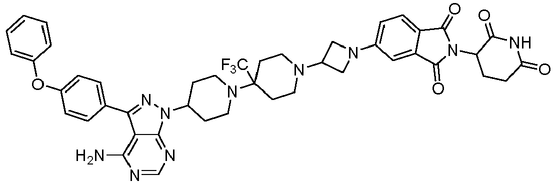

1-(1'-(azetidin-3-yl)-4'-(trifluoromethyl)-[1,4'-bipiperidin]-4-yl)-3-(4-phenoxyphenyl)-1H-pyrazolo[3,4-d]pyrimidin-4-amine (70i) (0.12 g, 0.2 mmol) was dissolved in 5 mL of DMSO, DIPEA (0.13 g, 0.1 mmol) and 2-(2,6-dioxopiperidin-3-yl)-5-fluoroisoindoline-1,3-dione (see WO 2017197056 for the synthetic method) (90 mg, 0.30 mmol) were added, and the mixture was warmed to 90°C and reacted for 5 h. The reaction solution was cooled to room temperature, and 50 mL of ethyl acetate and 50 mL of water were added. The organic phase was separated, dried over anhydrous sodium sulfate, and concentrated under reduced pressure, and then the crude product was separated and purified by silica gel column chromatography (dichloromethane/methanol (v/v) = 20 : 1), to obtain 5-(3-(4-(4-amino-3-(4-phenoxyphenyl)-1H-pyrazolo[3,4-d]pyrimidin-1-yl)-4'-(trifluoromethyl)-[1,4'-bipiperidin]-1'-yl)azetidin-1-yl)-2-(2,6-dioxopiperidin-3-yl)isoindoline-1,3-dione (compound 70) (20 mg, yield: 11%).
¹H NMR (400 MHz, CDCl₃) δ 8.50 - 8.28 (m, 2H), 7.69 - 7.60 (m, 3H), 7.43 - 7.35 (m, 2H), 7.21 - 7.12 (m, 3H), 7.11 - 7.04 (m, 2H), 6.80 (d, 1H), 6.53 (dd, 1H), 5.78 (br.s, 2H), 4.98 - 4.78 (m, 2H), 4.16 - 4.07 (m, 2H), 3.93 - 3.85 (m, 2H), 3.52 - 3.43 (m, 1H), 3.36 - 3.24 (m, 2H), 2.94 - 2.62 (m, 7H), 2.42 - 2.21 (m, 4H), 2.18 - 2.10 (m, 3H), 2.08 - 2.02 (m, 2H), 1.92 - 1.81 (m, 2H).
LCMS m/z = 849.3 [M+1]⁺.

**Example 71:**

### 2-((3r, Sr,7r)-adamantan-1-yl)-1-(2-(4-(4-amino-3-(4-phenoxyphenyl)-1H-pyrazolo[3,4-d]pyrimidin-1-yl)piperidin-1-yl)-7-azaspiro[3.5]nonan-7-yl)ethan-1-one (compound 71) trifluoroacetate

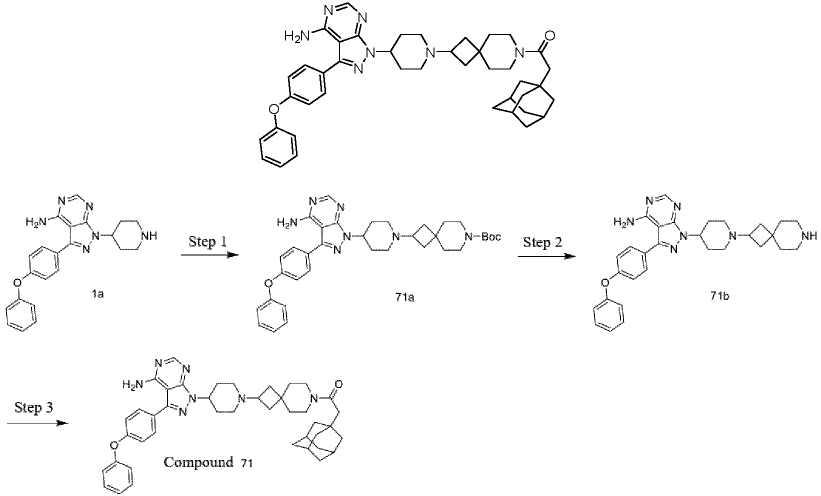

### Step 1:

### Tert-butyl 2-[4-[4-amino-3-(4-phenoxyphenyl)pyrazolo[3,4-d]pyrimidin-1-yl]-1-piperidyl]-7-azaspiro[3.5]nonan-7-carboxylate (71a)

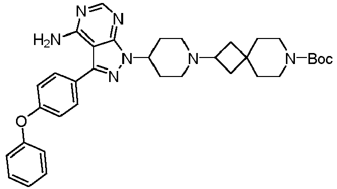

3-(4-phenoxyphenyl)-1-(piperidine-4-yl)-1H-pyrazolo[3,4-d]pyrimidin-4-amine (1a) (see J. Med. Chem. 2015, 58, 9625-9638 for the synthetic method) (1.1 g, 2.85 mmol) was dissolved in 30 mL of 1,2-dichloroethane, tert-butyl 2-oxo-7-azaspiro[3.5]nonan-7-carboxylate (0.67 g, 3.92 mmol) and glacial acetic acid (0.342 g, 5.70 mmol) were added, and the mixture was stirred at room temperature for 1 h, and then sodium triacetoxyborohydride (1.068 g, 5.04 mmol) was added. Upon completion of the addition, the reaction was carried out at room temperature overnight. To the reaction solution was added dropwise saturated sodium bicarbonate solution to adjust the pH to 10, and the reaction solution was concentrated under reduced pressure, then the crude product was separated and purified by silica gel column chromatography (dichloromethane/methanol (v/v) = 100 : 0-9 : 1), to obtain tert-butyl 2-[4-[4-amino-3-(4-phenoxyphenyl)pyrazolo[3,4-d]pyrimidin-1-yl]-1-piperidyl]-7-azaspiro[3.5]nonan-7-carboxylate (71a) (1.11 g, yield: 64%).
LCMS m/z = 610.3 [M+1]⁺.

### Step 2:

### 1-[1-(7-Azaspiro[3.5]nonan-2-yl)-4-piperidyl]-3-(4-phenoxyphenyl)pyrazolo[3,4-d]pyrimidin-4-amine (71b) trifluoroacetate

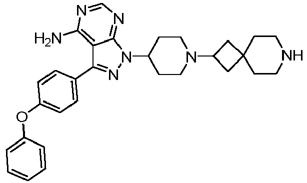

Tert-butyl 2-[4-[4-amino-3-(4-phenoxyphenyl)pyrazolo[3,4-d]pyrimidin-1-yl]-1-piperidyl]-7-azaspiro[3.5]nonan-7-carboxylate (71a) (1.1 g, 1.81 mmol) was dissolved in 15 mL of dichloromethane, 40 mL of 4 N ethyl acetate hydrochloride solution was added, and the mixture was stirred at room temperature for 2 h. The reaction solution was concentrated under reduced pressure, to obtain crude product 1-[1-(7-azaspiro[3.5]nonan-2-yl)-4-piperidyl]-3-(4-phenoxyphenyl)pyrazolo[3,4-d]pyrimidin-4-amine (71b) trifluoroacetate (0.65 g).
LCMS m/z = 510.3 [M+1]⁺.

### Step 3:

### 2-((3r, Sr,7r)-adamantan-1-yl)-1-(2-(4-(4-amino-3-(4-phenoxyphenyl)-1H-pyrazolo[3,4-d]pyrimidin-1-yl)piperidin-1-yl)-7-azaspiro[3.5]nonan-7-yl)ethan-1-one (compound 71) trifluoroacetate

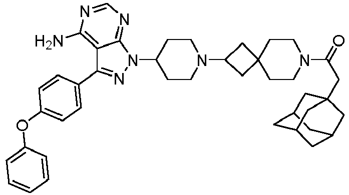

Adamantaneacetic acid (92 mg, 0.47 mmol) was dissolved in 15 mL of DCM, and DIPEA (152 mg, 1.18 mmol) and HATU (224 mg, 0.59 mmol) were successively added. The mixture was stirred at room temperature for 30 min, the above crude product 1-[1-(7-azaspiro[3.5]nonan-2-yl)-4-piperidyl]-3-(4-phenoxyphenyl)pyrazolo[3,4-d]pyrimidin-4-amine (71b) trifluoroacetate (200 mg) was added, and the mixture was stirred at room temperature for 18 h. The reaction system was concentrated under reduced pressure. The residue was separated and purified by silica gel column chromatography (dichloromethane/methanol (v/v) = 20 : 1), to obtain 2-((3r, Sr,7r)-adamantan-1-yl)-1-(2-(4-(4-amino-3-(4-phenoxyphenyl)-1H-pyrazolo[3,4-d]pyrimidin-1-yl)piperidine-1-yl)-7-azaspiro[3.5]nonan-7-yl)ethan-1-one (compound 71) trifluoroacetate (100 mg).
¹H NMR (400 MHz, CDCl₃) δ 8.36 (s, 1H), 7.68 - 7.60 (m, 2H), 7.44 - 7.35 (m, 2H), 7.22 - 7.05 (m, 5H), 5.52 (s, 2H), 4.90 - 4.71 (m, 1H), 3.66 - 3.30 (m, 4H), 3.18 - 3.00 (m, 2H), 2.94 - 2.73 (m, 1H), 2.52 - 2.34 (m, 2H), 2.18 - 2.00 (m, 8H), 1.96 (s, 3H), 1.85 - 1.73 (m, 2H), 1.73 - 1.46 (m, 16H).
LCMS m/z = 686.4 [M+1]⁺.

### Example 72:

### 2-(1-adamantyl)-1-[3-[3-[4-[4-amino-3-(4-phenoxyphenyl)pyrazolo[3,4-d]pyrimidin-1-yl]-1-piperidyl]azetidin-1-yl]azetidin-1-yl]ethanone (compound 72)

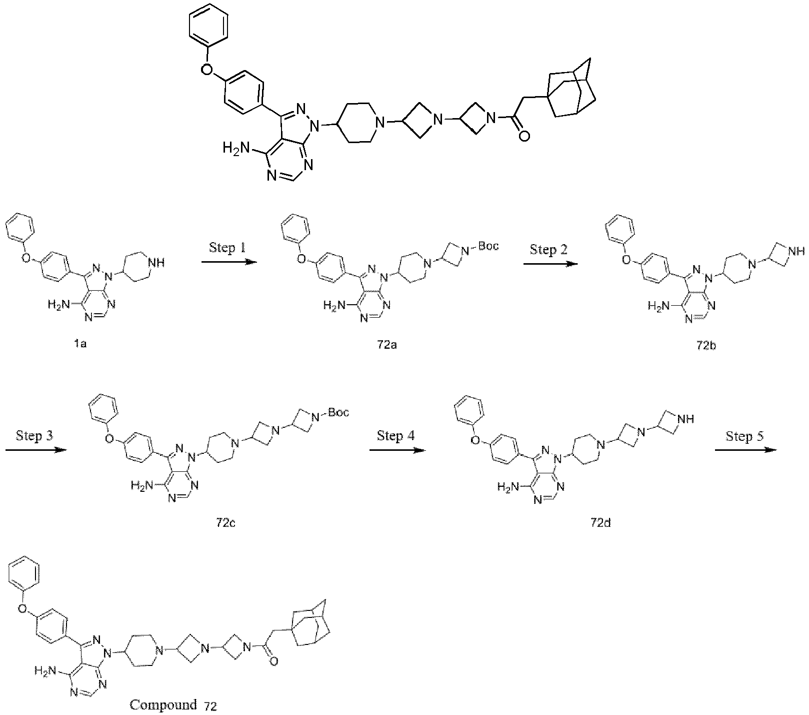

### Step 1:

### tert-butyl 3-[4-[4-amino-3-(4-phenoxyphenyl)pyrazolo[3,4-d]pyrimidin-1-yl]-1-piperidyl] azetidine-1-carboxylate (72a)

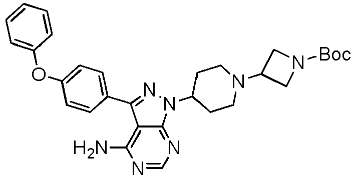

3-(4-phenoxyphenyl)-1-(piperidine-4-yl)-1H-pyrazolo[3,4-d]pyrimidin-4-amine (1a) (see J. Med. Chem. 2015, 58, 9625-9638 for the synthetic method) (1.5 g, 3.88 mmol) was dissolved in 15 mL of 1,2-dichloroethane, and tert-butyl 3-oxoazetidine-1-carboxylate (0.797 g, 4.66 mmol) and glacial acetic acid (1.24 g, 20.63 mmol) were successively added. Upon completion of the addition, the reaction was stirred at 65°C for 3 h. The reaction solution was cooled to room temperature, and sodium triacetoxyborohydride (1.651 g, 7.79 mmol) was added. Upon completion of the addition, the reaction was carried out at room temperature overnight. To the reaction solution was added dropwise saturated sodium bicarbonate solution to adjust the pH to 10, and the reaction solution was concentrated under reduced pressure, then the crude product was separated and purified by silica gel column chromatography (dichloromethane/methanol (v/v) = 100 : 0-19 : 1), to obtain tert-butyl 3-[4-[4-amino-3-(4-phenoxyphenyl)pyrazolo[3,4-d]pyrimidin-1-yl]-1-piperidyl]azetidine-1-carboxylate (72a) (1.932 g, yield: 92%).
LCMS m/z = 542.3 [M+1]⁺.

### Step 2:

### 1-[1-(azetidin-3-yl)-4-piperidyl]-3-(4-phenoxyphenyl)pyrazolo[3,4-d]pyrimidin-4-amine (72b)

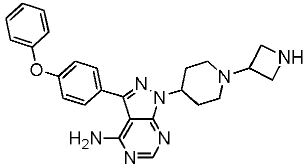

Tert-butyl 3-[4-[4-amino-3-(4-phenoxyphenyl)pyrazolo[3,4-d]pyrimidin-1-yl]-1-piperidyl]azetidine-1-carboxylate (72a) (1.6 g, 2.96 mmol) was dissolved in 5 mL of dichloromethane, 12 mL of 4 N ethyl acetate hydrochloride solution was added, and the mixture was stirred at room temperature for 2 h. The reaction solution was concentrated under reduced pressure. To the residue was added 50 mL of dichloromethane. The pH was adjusted to 10 with saturated sodium bicarbonate solution. The liquid separation was conducted, and the organic layer was dried over anhydrous sodium sulfate, and concentrated under reduced pressure, to obtain crude product 1-[1-(azetidin-3-yl)-4-piperidyl]-3-(4-phenoxyphenyl)pyrazolo[3,4-d]pyrimidin-4-amine (72b) (0.888 g).
LCMS m/z = 442.2 [M+1]⁺.

### Step 3:

### tert-butyl 3-[3-[4-[4-amino-3-(4-phenoxyphenyl)pyrazolo[3,4-d]pyrimidin-1-yl]-1-piperidyl] azetidin-1-yl]azetidine-1-carboxylate (72c)

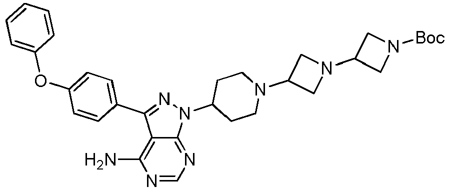

The above crude product 1-[1-(azetidin-3-yl)-4-piperidyl]-3-(4-phenoxyphenyl)pyrazolo[3,4-d]pyrimidin-4-amine (72b) (0.7 g) was dissolved in 20 mL of 1,2-dichloroethane, and tert-butyl 3-oxoazetidine-1-carboxylate (0.407 g, 2.38 mmol) and glacial acetic acid (0.190 g, 3.17 mmol) were successively added. Upon completion of the addition, the reaction was stirred at 65°C for 3 h. The reaction solution was cooled to room temperature, and sodium triacetoxyborohydride (0.672 g, 3.17 mmol) was added. Upon completion of the addition, the reaction was carried out at room temperature overnight. The pH was adjusted to 10 by adding dropwise saturated sodium bicarbonate solution to the reaction solution. The reaction solution was concentrated under reduced pressure, and then the crude product was separated and purified by silica gel column chromatography (dichloromethane/methanol (v/v) = 100 : 0-19 : 1), to obtain tert-butyl 3-[3-[4-[4-amino-3-(4-phenoxyphenyl)pyrazolo[3,4-d]pyrimidin-1-yl]-1-piperidyl]azetidin-1-yl]azetidine-1-carboxylate (72c) (0.918 g, two-step yield from compound 72a: 66%).
LCMS m/z = 597.3 [M+1]⁺.

### Step 4:

### 1-[1-[1-(azetidin-3-yl)azetidin-3-yl]-4-piperidyl]-3-(4-phenoxyphenyl)pyrazolo[3,4-d]pyrimidin-4-amine (72d)

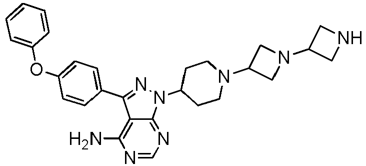

Tert-butyl 3 -[3 -[4-[4-amino-3 -(4-phenoxyphenyl)pyrazolo[3,4-d]pyrimidin-1-yl]-1-piperidyl]azetidine-1-yl]azetidine-1-carboxylate (72c) (0.8 g, 1.342 mmol) was dissolved in 10 mL of dichloromethane, 30 mL of 4 N ethyl acetate hydrochloride solution was added, and the mixture was stirred at room temperature for 2 h. The reaction solution was concentrated under reduced pressure. To the residue was added 100 mL of dichloromethane. The pH was adjusted to 10 with saturated sodium bicarbonate solution. The liquid separation was conducted, and the organic layer was dried over anhydrous sodium sulfate, and concentrated under reduced pressure, to obtain crude product 1-[1-[1-(azetidin-3-yl)azetidin-3-yl]-4-piperidyl]-3-(4-phenoxyphenyl)pyrazolo[3,4-d]pyrimidin-4-amine (72d) (0.600 g).
LCMS m/z = 497.3 [M+1]⁺.

### Step 5:

### 2-(1-adamantyl)-1-[3-[3-[4-[4-amino-3-(4-phenoxyphenyl)pyrazolo[3,4-d]pyrimidin-1-yl]-1-piperidyl]azetidin-1-yl]azetidin-1-yl]ethanone (compound 72)

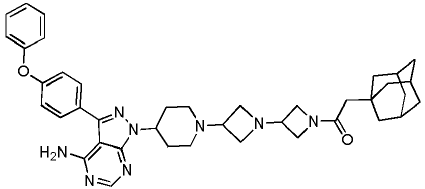

2-(1-adamantyl)acetic acid (0.050 g, 0.257 mmol) was dissolved in 2 mL of dichloromethane, and the above crude product 1-[1-[1-(azetidin-3-yl)azetidin-3-yl]-4-piperidyl]-3-(4-phenoxyphenyl)pyrazolo[3,4-d]pyrimidin-4-amine (72d) (0.141 g), HATU (0.108 g, 0.284 mmol) and diisopropylethylamine (0.0665 g, 0.515 mmol) were successively added. Upon completion of the addition, the reaction was carried out at room temperature for 3 hours. To the reaction solution was added 10 mL of water and 20 mL of dichloromethane. The liquid separation was conducted, and the organic layer was dried over anhydrous sodium sulfate, and concentrated under reduced pressure. The resulting crude product was separated and purified by silica gel column chromatography (dichloromethane/methanol (v/v) = 98 : 2-92 : 8), to obtain 2-(1-adamantyl)-1-[3-[3-[4-[4-amino-3-(4-phenoxyphenyl)]pyrazolo[3,4-d]pyrimidin-1-yl]-1-piperidyl]azetidin-1-yl]azetidin-1-yl]ethanone (compound 72) (0.102 g, two-step yield from compound 72c: 48%).
¹H NMR (400 MHz, CDCl₃) δ 8.36 (s, 1H), 7.69 - 7.60 (m, 2H), 7.44 - 7.35 (m, 2H), 7.21 - 7.11 (m, 3H), 7.10 - 7.04 (m, 2H), 5.57 (s, 2H), 4.89 - 4.73 (m, 1H), 4.14 - 4.06 (m, 1H), 4.05 - 3.91 (m, 2H), 3.83 - 3.72 (m, 1H), 3.59 - 3.50 (m, 2H), 3.50 - 3.40 (m, 1H), 3.22 - 2.84 (m, 5H), 2.53 - 2.37 (m, 2H), 2.29 - 1.99 (m, 4H), 1.99 - 1.91 (m, 3H), 1.85 (s, 2H), 1.73 - 1.57 (m, 12H).
LCMS m/z = 673.4 [M+1]⁺.

### Example 73:

### 2-(1-adamantyl)-1-(3-(4-(4-amino-3-(4-phenoxyphenyl)pyrazolo[3,4-d]pyrimidin-1-yl)-[1,4'-bipiperidin]-1'-yl)azetidin-1-yl)ethan-1-one (compound 73)

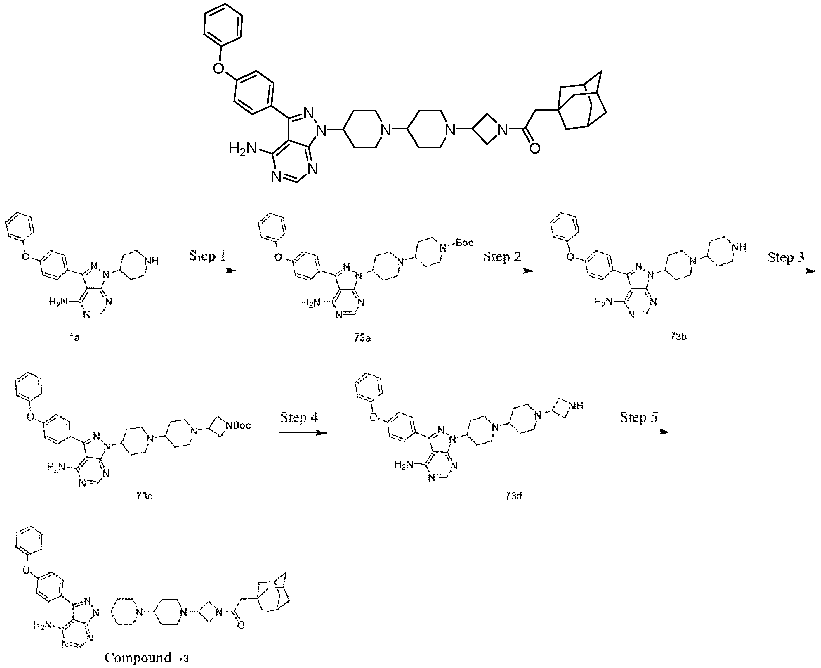

### Step 1:

### Tert-butyl 4-[4-[4-amino-3-(4-phenoxyphenyl)pyrazolo[3,4-d]pyrimidin-1-yl]-1-piperidyl] piperidine-1-carboxylate (73a)

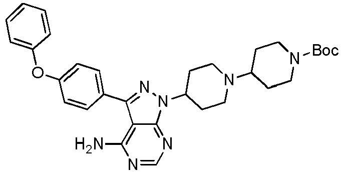

3-(4-phenoxyphenyl)-1-(piperidin-4-yl)-1H-pyrazolo[3,4-d]pyrimidin-4-amine (1a) (see J. Med. Chem. 2015, 58, 9625-9638 for the synthetic method) (1.72 g, 4.45 mmol) was dissolved in 18 mL of 1,2-dichloroethane, to which was added tert-butyl 4-oxopiperidine-1-carboxylate (1.065 g, 5.35 mmol) and glacial acetic acid (1.42 g, 23.66 mmol). Upon completion of the addition, the reaction was stirred at 65°C for 3 h, then the reaction system was cooled to room temperature, and sodium triacetoxyborohydride (1.894 g, 8.93 mmol) was added. Upon completion of the addition, the reaction was carried out at room temperature overnight. The pH was adjusted to 10 by adding dropwise saturated sodium bicarbonate solution to the reaction solution. The reaction solution was concentrated under reduced pressure, and then the crude product was separated and purified by silica gel column chromatography (dichloromethane/methanol (v/v) = 100 : 0-19 : 1), to obtain tert-butyl 4-[4-[4-amino-3-(4-phenoxyphenyl)pyrazolo[3,4-d]pyrimidin-1-yl]-1-piperidyl]piperidine-1-carboxylate (73a) (1.22 g, yield: 48%).

### Step 2:

### 3-(4-phenoxyphenyl)-1-[1-(4-piperidyl)-4-piperidyl]pyrazolo[3,4-d]pyrimidin-4-amine (73b)

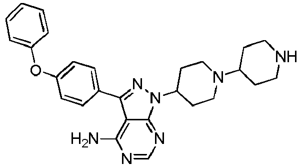

Tert-butyl 4-[4-[4-amino-3-(4-phenoxyphenyl)pyrazolo[3,4-d]pyrimidin-1-yl]-1-piperidyl]piperidine-1-carboxylate (73a) (1.05 g, 1.84 mmol) was dissolved in 5 mL of dichloromethane, 17 mL of 4 N ethyl acetate hydrochloride solution was added, and the mixture was stirred at room temperature for 2 h. The reaction solution was concentrated under reduced pressure, and to the residue was added 100 mL of dichloromethane. The pH was adjusted to 10 with saturated sodium bicarbonate solution. The liquid separation was conducted, and the organic layer was dried over anhydrous sodium sulfate, and concentrated under reduced pressure to obtain crude product 3-(4-phenoxyphenyl)-1-[1-(4-piperidyl)-4-piperidyl]pyrazolo[3,4-d]pyrimidin-4-amine (73b) (0.83 g).

### Step 3:

### Tert-butyl 3-(4-(4-amino-3-(4-phenoxyphenyl)-1H-pyrazolo[3,4-d]pyrimidin-1-yl)-[1,4'-bipiperidin]-1'-yl)azetidine-1-carboxylate (73c)

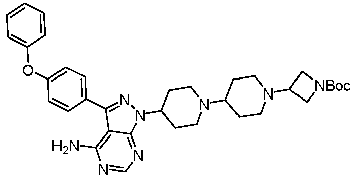

The above crude product 3-(4-phenoxyphenyl)-1-[1-(4-piperidyl)-4-piperidyl]pyrazolo[3,4-d]pyrimidin-4-amine (73b) (0.8 g) was dissolved in 60 mL of 1,2-dichloroethane, and glacial acetic acid (0.154 g, 2.56 mmol) and tert-butyl 3-oxoazetidine-1-carboxylate (0.408 g, 2.38 mmol) were successively added. Under nitrogen atmosphere, the reaction was heated to 65°C and stirred for 3 h, and the reaction solution was cooled to room temperature, sodium triacetoxyborohydride (2.166 g, 10.22 mmol) was added, and the mixture was stirred at room temperature overnight. The reaction was quenched by adding 70 mL of saturated sodium bicarbonate solution to the reaction solution, stirred, and allowed to stand for layer separation. The aqueous phase was extracted with dichloromethane (50 mL × 2), and the organic phase was combined, washed with 100 mL of saturated saline, dried over anhydrous sodium sulfate, filtered, and concentrated under reduced pressure. The crude product was separated and purified by silica gel column chromatography (dichloromethane/methanol (v/v) = 100/1-15/1), to obtain tert-butyl 3-(4-(4-amino-3-(4-phenoxyphenyl)-1H-pyrazolo[3,4-d]pyrimidin-1-yl)-[1,4'-bipiperidin]-1'-yl)azetidine-1-carboxylate (73c) (0.897 g, two-step yield from compound 73a: 81%).

### Step 4:

### 1-(1'-(azetidin-3-yl)-[1,4'-bipiperidin]-4-yl)-3-(4-phenoxyphenyl)-1H-pyrazolo[3,4-d]pyrimidin-4-amine (73d)

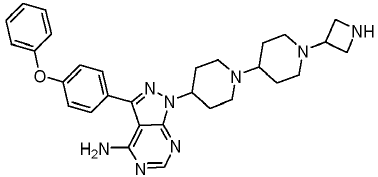

Tert-butyl 3-(4-(4-amino-3-(4-phenoxyphenyl)-1H-pyrazolo[3,4-d]pyrimidin-1-yl)-[1,4'-bipiperidin]-1'-yl)azetidine-1-carboxylate (73c) (0.5 g, 0.80 mmol) was dissolved with 3 mL of methanol, 18 mL of 4 N ethyl acetate hydrochloride solution was added dropwise, and the reaction was carried out at room temperature overnight. The reaction solution was concentrated under reduced pressure, and the pH of the crude product was adjusted to 10 with saturated sodium bicarbonate solution, and extraction was performed with dichloromethane (40 mL × 3). The organic phase was combined, dried over anhydrous sodium sulfate, and concentrated under reduced pressure, to obtain crude product 1-(1'-(azetidin-3-yl)-[1,4'-bipiperidin]-4-yl)-3-(4-phenoxyphenyl)-1H-pyrazolo[3,4-d]pyrimidin-4-amine (73d) (0.3 g).

### Step 5:

### 2-(1-adamantyl)-1-(3-(4-(4-amino-3-(4-phenoxyphenyl)pyrazolo[3,4-d]pyrimidin-1-yl)-[1,4'-bipiperidin]-1'-yl)azetidin-1-yl)ethan-1-one (compound 73)

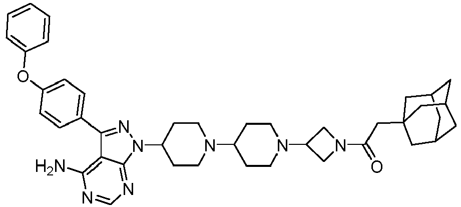

2-(1-adamantyl)acetic acid (0.02 g, 0.103 mmol) was dissolved in 2 mL of dichloromethane, and the above crude product 1-(1'-(azetidin-3-yl)-[1,4'-bipiperidin]-4-yl)-3-(4-phenoxyphenyl)-1H-pyrazolo[3,4-d]pyrimidin-4-amine (73d) (0.0594 g), HATU (0.0431 g, 0.113 mmol) and diisopropylethylamine (0.0266 g, 0.206 mmol) were successively added. Upon completion of the addition, the reaction was carried out at room temperature for 3 hours. To the reaction solution was added 10 mL of water and 20 mL of dichloromethane. The liquid separation was conducted, and the organic layer was dried over anhydrous sodium sulfate, and concentrated under reduced pressure. The resulting crude product was separated and purified by silica gel column chromatography (dichloromethane/methanol (v/v) = 98 : 2-92 : 8), to obtain 2-(1-adamantyl)-1-(3-(4-(4-amino-3-(4-phenoxyphenyl)pyrazolo[3,4-d]pyrimidin-1-yl)-[1,4'-bipiperidin]-1'-yl)azetidin-1-yl)ethan-1-one (compound 73) (0.052 g, two-step yield from compound 73c: 47%).
¹H NMR (400 MHz, CDCl₃) δ 8.37 (s, 1H), 7.70 - 7.62 (m, 2H), 7.45 - 7.35 (m, 2H), 7.22 - 7.12 (m, 3H), 7.12 - 7.05 (m, 2H), 5.52 (s, 2H), 4.81 - 4.70 (m, 1H), 4.17 - 4.07 (m, 1H), 4.07 - 3.94 (m, 2H), 3.90 - 3.79 (m, 1H), 3.15 - 3.02 (m, 3H), 2.96 - 2.81 (m, 2H), 2.54 - 2.33 (m, 5H), 2.10 - 2.00 (m, 2H), 2.00 - 1.93 (m, 3H), 1.93 - 1.82 (m, 6H), 1.74 - 1.60 (m, 14H).
LCMS m/z = 701.5 [M+1]⁺.

### Example 74:

### 2-((3r, Sr,7r)-adamantan-1-yl)-1-(3-(4-(4-amino-3-(4-phenoxyphenyl)-1H-pyrazolo[3,4-d]pyrimidin-1-yl)piperidin-1-yl)azetidin-1-yl)ethanone (compound 74)

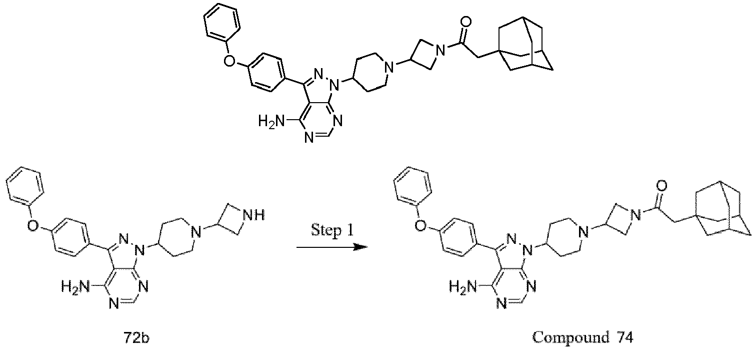

Adamantaneacetic acid (66 mg, 0.34 mmol) was dissolved in 15 mL of DCM, and DIPEA (58 mg, 0.45 mmol) and HATU (172 mg, 0.45 mmol) were successively added. The mixture was stirred at room temperature for 30 min, the above crude product 1-[1-(azetidin-3-yl)-4-piperidyl]-3-(4-phenoxyphenyl)pyrazolo[3,4-d]pyrimidin-4-amine (72b) (100 mg) was added, and the mixture was stirred at room temperature for 18 h. The reaction system was concentrated under reduced pressure. The residue was separated and purified by silica gel column chromatography (dichloromethane/methanol (v/v) = 20 : 1), to obtain 2-((3r,5r,7r)-adamantan-1-yl)-1-(3-(4-(4-amino-3-(4-phenoxyphenyl)-1H-pyrazolo[3,4-d]pyrimidin-1-yl)piperidine-1-yl)azetidin-1-yl)ethanone (compound 74) (50 mg, two-step yield from compound 72a: 24%).
¹H NMR (400 MHz, CDCl₃) δ 8.36 (s, 1H), 7.65 (d, 2H), 7.44 - 7.33 (m, 2H), 7.22 - 7.02 (m, 5H), 5.57 (s, 2H), 4.88 - 4.74 (m, 1H), 4.20 - 4.10 (m, 1H), 4.09 - 3.96 (m, 2H), 3.92 - 3.83 (m, 1H), 3.24 - 3.13 (m, 1H), 3.06 - 2.88 (m, 2H), 2.53 - 2.37 (m, 2H), 2.20 - 2.02 (m, 4H), 2.01 - 1.92 (m, 3H), 1.87 (s, 2H), 1.76 - 1.58 (m, 12H).
LCMS m/z = 618.3 [M+1]⁺.

### Example 75:

### 5-(3-(7-(4-amino-3-(4-phenoxyphenyl)-1H-pyrazolo[3,4-d]pyrimidin-1-yl)-2-azaspiro[3. 5]nonan-2-yl)azetidin-1-yl)-2-(2,6-dioxopiperidin-3-yl)isoindoline-1,3-dione (compound 75)

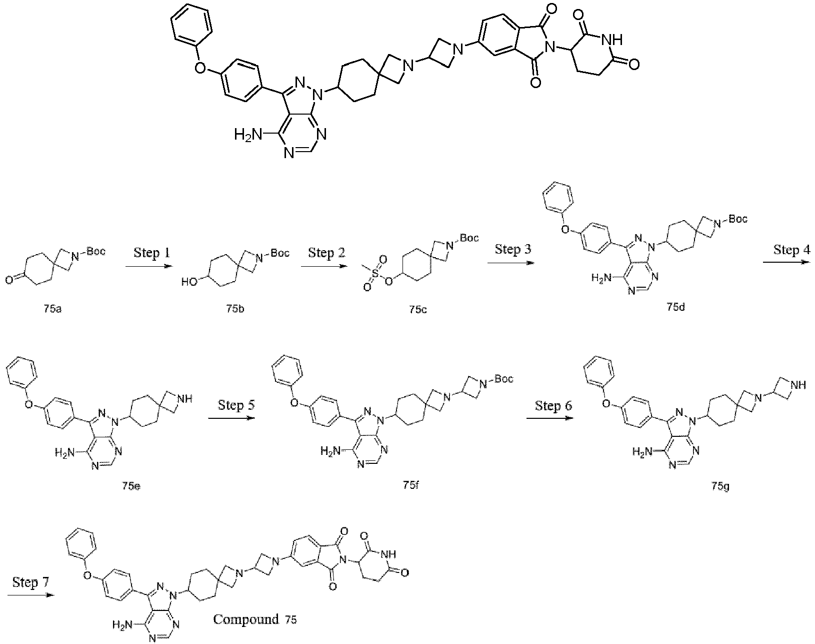

### Step 1:

### tert-butyl 7-hydroxyl-2-azaspiro[3.5]nonane-2-carboxylate (63b)

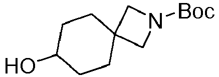

Tert-butyl 7-oxo-2-azaspiro[3.5]nonan-2-carboxylate (75a) (380 mg, 1.59 mmol) was dissolved in 8 mL of methanol, sodium borohydride (120 mg, 3.18 mmol) was added, and the reaction was carried out at room temperature for 0.5 hours. The reaction solution was quenched with 5 mL of water. The aqueous phase was extracted with ethyl acetate (20 mL × 3). The organic phase was combined, dried over anhydrous sodium sulfate, and concentrated under reduced pressure, to obtain tert-butyl 7-hydroxyl-2-azaspiro[3.5]nonan-2-carboxylate (75b) (360 mg, yield: 94%).

### Step 2:

### Tert-butyl 7-((methylsulfonyl)oxy)-2-azaspiro[3.5]nonan-2-carboxylate (75c)

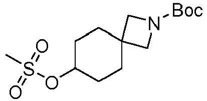

Tert-butyl 7-hydroxyl-2-azaspiro[3.5]nonan-2-carboxylate (75b) (360 mg, 1.49 mmol) was dissolved in 10 mL of dichloromethane. The mixture was cooled to 0°C, triethylamine (302 mg, 2.98 mmol) and methanesulfonyl chloride (0.17 mL, 2.24 mmol) were successively added , and the reaction was cooled to room temperature and stirred for 1.5 hours. The reaction solution was quenched with 10 mL of water. The aqueous phase was extracted with dichloromethane (20 mL × 3). The organic phase was combined, dried over anhydrous sodium sulfate, and concentrated under reduced pressure, to obtain tert-butyl 7-((methylsulfonyl)oxy)-2-azaspiro[3.5]nonan-2-carboxylate (75c) (461 mg, yield: 97%).

### Step 3:

### tert-butyl 7-(4-amino-3-(4-phenoxyphenyl)-1H-pyrazolo[3,4-d]pyrimidin-1-yl)-2-azaspiro [3.5]nonane-2-carboxylate (75d)

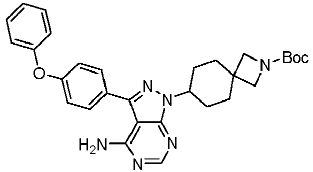

Tert-butyl 7-((methylsulfonyl)oxy)-2-azaspiro[3.5]nonan-2-carboxylate (75c) (461 mg, 1.44 mmol) was dissolved in 10 mL of DMF, 3-(4-phenoxyphenyl)-1H-pyrazolo[3,4-d]pyrimidin-4-amine (524 mg, 1.73 mmol) and cesium carbonate (1.4 g, 4.32 mmol) were successively added, and the reaction was warmed to 80°C and stirred for 5 hours. The reaction solution was cooled to room temperature, and diluted with 10 mL of water. The aqueous phase was extracted with dichloromethane (20 mL × 3). The organic phase was combined, dried over anhydrous sodium sulfate, and concentrated under reduced pressure. The crude product was separated and purified by silica gel column chromatography (dichloromethane/methanol (v/v) = 10 : 1-8 : 1), to obtain tert-butyl 7-(4-amino-3-(4-phenoxyphenyl)-1H-pyrazolo[3,4-d]pyrimidin-1-yl)-2-azaspiro[3.5]nonane-2-carboxylate (75d) (305 mg, yield: 40%).
LCMS m/z = 527.3 [M+1]⁺.

### Step 4:

### 3-(4-phenoxyphenyl)-1-(2-azaspiro[3.5]nonan-7-yl)-1H-pyrazolo[3,4-d]pyrimidin-4-amine (75e)

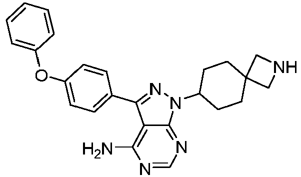

Tert-butyl 7-(4-amino-3-(4-phenoxyphenyl)-1H-pyrazolo[3,4-d]pyrimidin-1-yl)-2-azaspiro[3.5]nonane-2-carboxylate (75d) (305 mg, 0.58 mmol) was dissolved in 6 mL of dichloromethane, 3 mL of trifluoroacetic acid was added, and the reaction was carried out at room temperature for 2.5 hours. The pH of the reaction solution was adjusted to 10 with 2 N sodium hydroxide aqueous solution. The aqueous phase was extracted with dichloromethane (10 mL × 3). The organic phase was combined, dried over anhydrous sodium sulfate, and concentrated under reduced pressure, to obtain 3-(4-phenoxyphenyl)-1-(2-azaspiro[3.5]nonane-7-yl)-1H-pyrazolo[3,4-d]pyrimidin-4-amine (75e) (209 mg, yield: 85%).
LCMS m/z = 427.3 [M+1]⁺.

### Step 5:

### tert-butyl 3-(7-(4-amino-3-(4-phenoxyphenyl)-1H-pyrazolo[3,4-d]pyrimidin-1-yl)-2- azaspiro[3.5]nonan-2-yl)azetidine-1-carboxylate (75f)

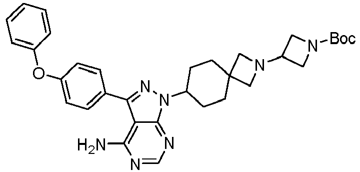

The 3-(4-phenoxyphenyl)-1-(2-azaspiro[3.5]nonane-7-yl)-1H-pyrazolo[3,4-d]pyrimidin-4-amine (75e) (115 mg, 0.27 mmol) was dissolved in 5 mL of DCE, and 1-Boc-3-azetidinone (92 mg, 0.54 mmol), glacial acetic acid (0.04 mL, 0.68 mmol) and anhydrous sodium sulfate (200 mg) were successively added at room temperature, the mixture was stirred for 5 minutes, then sodium triacetoxyborohydride (172 mg, 0.81 mmol) was added, and the mixture was stirred at room temperature overnight. To the reaction solution was added 20 mL of water, the pH was adjusted to 10 with 2 N sodium hydroxide aqueous solution, and the resulted solution was extracted with dichloromethane (30 mL × 3). The organic phase was combined, washed with water (20 mL × 2), dried over anhydrous sodium sulfate, and concentrated under reduced pressure. The crude product was separated and purified by silica gel column chromatography (dichloromethane/methanol (v/v) = 10 : 1-8 : 1), to obtain tert-butyl 3-(7-(4-amino-3-(4-phenoxyphenyl)-1H-pyrazolo[3,4-d]pyrimidin-1-yl)-2-azaspiro[3.5]nonan-2-yl)azetidine-1-carboxylate (75f) (130 mg, yield: 83%).
LCMS m/z = 582.3 [M+1]⁺.

### Step 6:

### 1-(2-(azetidin-3-yl)-2-azaspiro[3.5]nonan-7-yl)-3-(4-phenoxyphenyl)-1H-pyrazolo[3,4-d]pyrimidin-4-amine (75g)

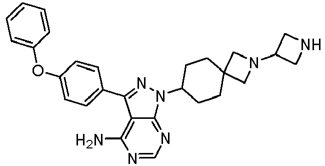

Tert-butyl 3-(7-(4-amino-3-(4-phenoxyphenyl)-1H-pyrazolo[3,4-d]pyrimidin-1-yl)-2-azaspiro[3.5]nonan-2-yl)azetidine-1-carboxylate (75f) (130 mg, 0.22 mmol) was dissolved in 4 mL of dichloromethane, 2 mL of trifluoroacetic acid was added, and the reaction was carried out at room temperature for 2.5 hours. The pH of the reaction solution was adjusted to 10 with 2 N sodium hydroxide aqueous solution. The aqueous phase was extracted with dichloromethane (10 mL × 3). The organic phase was combined, dried over anhydrous sodium sulfate, and concentrated under reduced pressure, to obtain 1-(2-(azetidine-3-yl)-2-azaspiro[3.5]nonan-7-yl)-3-(4-phenoxyphenyl)-1H-pyrazolo[3,4-d]pyrimidin-4-amine (75g) (95 mg, yield: 90%).

### Step 7:

### 5-(3-(7-(4-amino-3-(4-phenoxyphenyl)-1H-pyrazolo[3,4-d]pyrimidin-1-yl)-2-azaspiro[3. 5]nonan-2-yl)azetidin-1-yl)-2-(2,6-dioxopiperidin-3-yl)isoindoline-1,3-dione (compound 75)

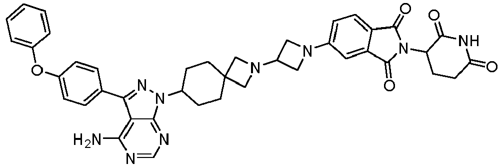

1-(2-(azetidin-3-yl)-2-azaspiro[3.5]nonan-7-yl)-3-(4-phenoxyphenyl)-1H-pyrazolo[3,4-d]pyrimidin-4-amine (75g) (95 mg, 0.20 mmol) was dissolved in 5 mL of DMSO, 2-(2,6-dioxopiperidin-3-yl)-5-fluoroisoindoline-1,3-dione (see WO 2017197056 for the synthetic method) (55 mg, 0.20 mmol) and diisopropylethylamine (129 mg, 1.00 mmol) were added at room temperature, and the mixture was warmed to 80°C and reacted for 4 hours. The reaction solution was cooled to room temperature, poured into 20 mL of water, and the aqueous phase was extracted with a mixed solvent of dichloromethane/methanol (v/v) = 10 : 1 (30 mL × 3). The organic phase was combined, washed with 50 mL of water, dried over anhydrous sodium sulfate, and concentrated under reduced pressure, and then the crude product was separated and purified by silica gel column chromatography (dichloromethane/methanol (v/v) = 10 : 1-5 : 1), to obtain 5-(3-(7-(4-amino-3-(4-phenoxyphenyl)-1H-pyrazolo[3,4-d]pyrimidin-1-yl)-2-azaspiro[3.5]nonan-2-yl)azetidin-1-yl)-2-(2, 6-dioxopiperidin-3-yl)isoindoline-1,3-dione (compound 75) (81 mg, yield: 55%).
¹H NMR (400 MHz, CDCl₃) δ 8.82 (br.s, 1H), 8.37 (s, 1H), 7.69 - 7.59 (m, 3H), 7.43 - 7.34 (m, 2H), 7.21 - 7.03 (m, 5H), 6.79 (d, 1H), 6.53 (dd, 1H), 5.60 (br.s, 2H), 4.92 (dd, 1H), 4.81 - 4.70 (m, 1H), 4.11 - 4.03 (m, 2H), 3.95 - 3.86 (m, 2H), 3.80 - 3.71 (m, 1H), 3.32 (s, 2H), 3.19 (s, 2H), 2.93 - 2.65 (m, 3H), 2.24 - 2.06 (m, 5H), 2.06 - 1.95 (m, 2H), 1.78 - 1.67 (m, 2H).
LCMS m/z = 738.3 [M+1]⁺.

### Example 76:

### 3-(2-(3-(4-(4-amino-3-(4-phenoxyphenyl)-1H-pyrazolo[3,4-d]pyrimidin-1-yl)piperidin-1-yl)-[1,3'-biazetidin]-1'-yl)-5-oxo-5,7-dihydro-6H-pyrrolo[3,4-b]pyridin-6-yl)piperidine-2,6-dione (compound 76)

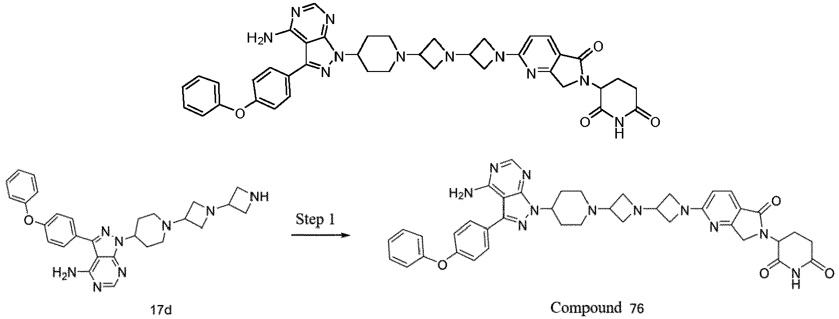

3-(2-chloro-5-oxo-7H-pyrrolo[3,4-b]pyridine-6-yl)piperidine-2,6-dione (see WO 2017197056 for the synthetic method) (0.1 g, 0.36 mmol) was dissolved in 8 mL of DMSO, 0.5 mL DIPEA and 1-[1-[1-(azetidin-3-yl)azetidin-3-yl]-4-piperidyl]-3-(4-phenoxyphenyl)pyrazolo[3,4-d]pyrimidin-4-amine (17d) (see WO 2020239103 for the synthetic method) (0.53 g, 1.07 mmol) were added, and the reaction was stirred at 80°C for 5 h. The reaction solution was cooled to room temperature, 10 mL of water was added, and the reaction solution was extracted with ethyl acetate (40 mL × 3). The organic phase was washed with saturated saline (20 mL × 3), dried over anhydrous sodium sulfate, and concentrated under reduced pressure, and then the crude product was separated and purified by silica gel column chromatography (dichloromethane/methanol (v/v) = 100 : 0-10 : 1), to obtain 3-(2-(3-(4-(4-amino-3-(4-phenoxyphenyl)-1H-pyrazolo[3,4-d]pyrimidin-1-yl)piperidine-1-yl)-[1,3'-biazetidin]-1'-yl)-5-oxo-5,7-dihydro-6H-pyrrolo[3,4-b]pyridine-6-yl)piperidine-2,6-dione (compound 76) (0.1 g, yield: 38%).
¹H NMR (400 MHz, CDCl₃) δ 9.33 (s, 1H), 8.38 (s, 1H), 7.81 (d, 1H), 7.68 - 7.60 (m, 2H), 7.43 - 7.34 (m, 2H), 7.20 - 7.03 (m, 5H), 6.26 (d, 1H), 5.69 (s, 2H), 5.17 (dd, 1H), 4.90 - 4.73 (m, 1H), 4.36 - 4.08 (m, 4H), 4.02 - 3.94 (m, 2H), 3.77 - 3.65 (m, 1H), 3.63 - 3.52 (m, 2H), 3.26 - 3.03 (m, 3H), 3.03 - 2.73 (m, 4H), 2.53 - 1.75 (m, 8H).
LC-MS m/z = 740.3 [M+1]⁺.

### Example 77:

### 5-(4-((4-(4-amino-3-(4-phenoxyphenyl)-1H-pyrazolo[3,4-d]pyrimidin-1-yl)piperidin-1 -yl)methyl)piperidin-1 -yl)-2-(2,6-dioxopiperi din-3 -yl)isoindoline-1,3-dione (compound 77)

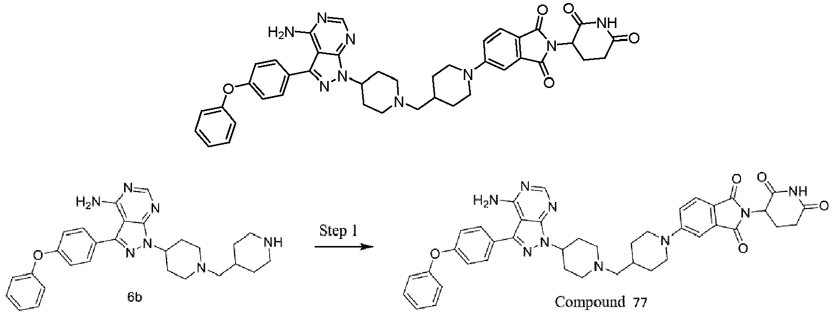

3-(4-phenoxyphenyl)-1-(1-(piperidine-4-ylmethyl)piperidin-4-yl)-1H-pyrazolo[3,4-d]pyrimidin-4-amine (6b) (see WO 2020239103 for the synthetic method) (150 mg, 0.31 mmol) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (128 mg, 0.46 mmol) were added, and the reaction was stirred at 80°C for 5 h. The reaction solution was cooled to room temperature, 50 mL of water was added, and the reaction solution was filtered. The solid was collected, washed with 50 mL of water, and dissolved with 100 mL of dichloromethane, dried over anhydrous sodium sulfate, and concentrated under reduced pressure, then the crude product was separated and purified by silica gel column chromatography (dichloromethane/methanol (v/v) = 15 : 1), to obtain 5-(4-((4-(4-amino-3-(4-phenoxyphenyl)-1H-pyrazolo[3,4-d]pyrimidin-1-yl)piperidine-1-yl)methyl)piperidine-1-yl)-2-(2,6-dioxopiperidin-3-yl)isoindoline-1,3-dione (compound 77) (160 mg, yield: 70%).
¹H NMR (400 MHz, CDCl₃) δ 9.18 (s, 1H), 8.40 (s, 1H), 7.71 - 7.61 (m, 3H), 7.43 - 7.34 (m, 2H), 7.28 (d, 1H), 7.21 - 7.11 (m, 3H), 7.11-7.01 (m, 3H), 5.72 (br.s, 2H), 4.94 (dd, 1H), 4.88 - 4.70 (m, 1H), 4.01 - 3.87 (m, 2H), 3.10 - 2.66 (m, 7H), 2.52 - 2.37 (m, 2H), 2.34 - 2.20 (m, 2H), 2.17 - 2.08 (m, 1H), 2.07 - 1.66 (m, 7H), 1.37 - 1.22 (m, 2H).
LCMS m/z = 740.3 [M+1]⁺.

### Example 78:

### 5-(3-((4-(4-amino-3-(4-phenoxyphenyl)-1H-pyrazolo[3,4-d]pyrimidin-1-yl)piperi din-1 -yl)methyl)pyrrol e-1 -yl)-2-(2,6-dioxopiperi din-3 -yl)i soindoline-1,3 -di one (compound 78)

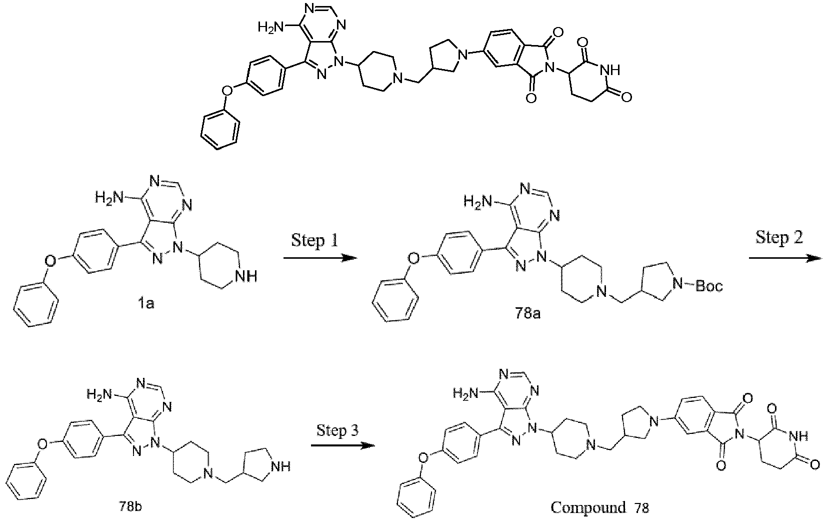

### Step 1:

### tert-butyl 3-((4-(4-amino-3-(4-phenoxyphenyl)-1H-pyrazolo[3,4-d]pyrimidin-1-yl)piperidin-1-yl)methyl)pyrrole-1-carboxylate (78a)

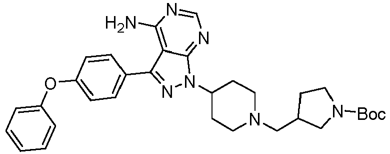

3-(4-phenoxyphenyl)-1-(piperidine-4-yl)-1H-pyrazolo[3,4-d]pyrimidin-4-amine (1a) (see J. Med. Chem. 2015, 58, 9625-9638 for the synthetic method) (310 mg, 0.80 mmol) was dissolved in 15 mL of DCE, tert-butyl 3-formylpyrrole-1-carboxylate (240 mg, 1.20 mmol) was added, and the mixture was stirred at room temperature for 10 min, then sodium triacetoxyborohydride (340 mg, 1.60 mmol) was added, and the mixture was stirred at room temperature for 16 h. To the reaction system was slowly added 20 mL of sodium bicarbonate aqueous solution, and the mixed solution was extracted with DCM (50 mL × 3). The organic phase was washed with 50 mL of water, dried over anhydrous sodium sulfate, and concentrated under reduced pressure. The crude product was separated and purified by silica gel column chromatography (dichloromethane/methanol (v/v) = 15 : 1), to obtain tert-butyl 3-((4-(4-amino-3-(4-phenoxyphenyl)-1H-pyrazolo[3,4-d]pyrimidin-1-yl)piperidine-1-yl)methyl )pyrrole-1-carboxylate (78a) (460 mg, yield: > 99%).
LCMS m/z = 570.3 [M+1]⁺.

### Step 2:

### 3-(4-phenoxyphenyl)-1-(1-(pyrrole-3-ylmethyl)piperidin-4-yl)-1H-pyrazolo[3,4-d]pyrimidin-4-amine (78b)

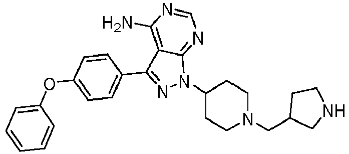

Tert-butyl 3-((4-(4-amino-3-(4-phenoxyphenyl)-1H-pyrazolo[3,4-d]pyrimidin-1-yl)piperidine-1-yl)methyl)pyrrole-1-carboxylate (78a) (460 mg, 0.8 mmol) was dissolved in 10 mL of dichloromethane, 5 mL of trifluoroacetic acid was added, and the mixture was stirred at room temperature for 2 h. The reaction system was concentrated under reduced pressure. The residue was dissolved with 30 mL of 4 mol/L NaOH aqueous solution, extracted with DCM (40 mL × 3). The organic phase was washed with 40 mL of water, dried over anhydrous sodium sulfate, and concentrated under reduced pressure, to obtain 3-(4-phenoxyphenyl)-1-(1-(pyrrole-3-ylmethyl)piperidine-4-yl)-1H-pyrazolo[3,4-d]pyrimidin-4-amine (78b) (360 mg, yield: 96%).
LCMS m/z = 470.2 [M+1]⁺.

### Step 3:

### 5-(3-((4-(4-amino-3-(4-phenoxyphenyl)-1H-pyrazolo[3,4-d]pyrimidin-1-yl)piperi din-1 -yl)methyl)pyrrol e-1 -yl)-2-(2,6-dioxopiperi din-3 -yl)i soindoline-1,3 -di one (compound 78)

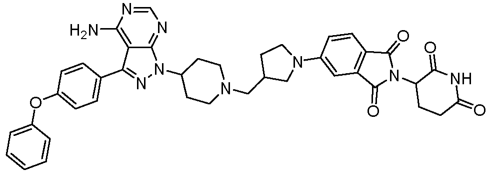

3-(4-phenoxyphenyl)-1-(1-(pyrrole-3-ylmethyl)piperidine-4-yl)-1H-pyrazolo[3,4-d]pyrimidin-4-amine (78b) (150 mg, 0.32 mmol) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (132 mg, 0.478 mmol) were added, and the reaction was stirred at 80°C for 5 h. The reaction solution was cooled to room temperature, 50 mL of water was added, and the reaction solution was filtered. The solid was collected, washed with 50 mL of water, and dissolved with 100 mL of dichloromethane, dried over anhydrous sodium sulfate, and concentrated under reduced pressure, then the crude product was separated and purified by silica gel column chromatography (dichloromethane/methanol (v/v) = 15 : 1), to obtain 5-(3-((4-(4-amino-3-(4-phenoxyphenyl)-1H-pyrazolo[3,4-d]pyrimidin-1-yl)piperidine-1-yl)methyl)pyrrole-1-yl)-2-(2,6-dioxopiperidin-3-yl)isoindoline-1,3-dione (compound 78) (120 mg, yield: 52%).
¹H NMR (400 MHz, CDCl₃) δ 9.12 (s, 1H), 8.40 (s, 1H), 7.69 - 7.61 (m, 3H), 7.43 - 7.35 (m, 2H), 7.20 - 7.11 (m, 3H), 7.11 - 7.04 (m, 2H), 6.96 (d, 1H), 6.70 (dd, 1H), 5.71 (br.s, 2H), 4.94 (dd, 1H), 4.89 - 4.75 (m, 1H), 3.64 - 3.55 (m, 1H), 3.54 - 3.46 (m, 1H), 3.46 - 3.37 (m, 1H), 3.27 - 3.00 (m, 3H), 2.94 - 2.62 (m, 4H), 2.58 - 2.40 (m, 4H), 2.36 - 2.18 (m, 2H), 2.18 - 1.97 (m, 3H), 1.93 - 1.80 (m, 2H).
LCMS m/z = 726.3 [M+1]⁺.

### Example 79:

### 5-(3-((4-(4-amino-3-(4-phenoxyphenyl)-1H-pyrazolo[3,4-d]pyrimidin-1-yl)piperidin-1-yl)methyl)azetidin-1-yl)-2-(2,6-dioxopiperidin-3-yl)isoindoline-1,3-dione (compound 79)

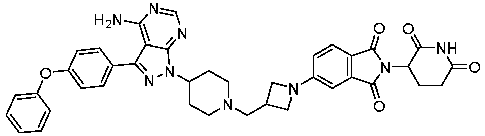

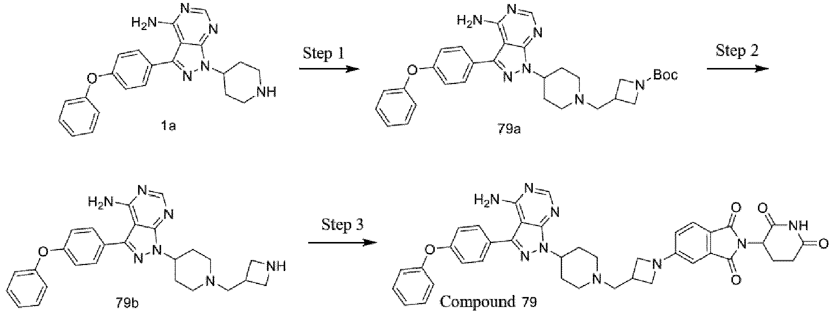

### Step 1:

### tert-butyl 3-((4-(4-amino-3-(4-phenoxyphenyl)-1H-pyrazolo[3,4-d]pyrimidin-1-yl)piperidin-1-yl)methyl)azetidine-1-carboxylate (79a)

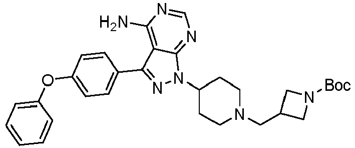

3-(4-phenoxyphenyl)-1-(piperidine-4-yl)-1H-pyrazolo[3,4-d]pyrimidin-4-amine (1a) (see J. Med. Chem. 2015, 58, 9625-9638 for the synthetic method) (775 mg, 2.00 mmol) was dissolved in 25 mL of DCE, tert-butyl 3-formylazetidine-1-carboxylate (555 mg, 3.00 mmol) was added, and the mixture was stirred at room temperature for 10 min, then sodium triacetoxyborohydride (850 mg, 4.01 mmol) was added, and the mixture was stirred at room temperature for 15 h. To the reaction system was slowly added 40 mL of sodium bicarbonate aqueous solution, and the mixed solution was extracted with DCM (50 mL × 3). The organic phase was washed with 50 mL of water, dried over anhydrous sodium sulfate, and concentrated under reduced pressure. The crude product was separated and purified by silica gel column chromatography (dichloromethane/methanol (v/v) = 15 : 1), to obtain tert-butyl 3-((4-(4-amino-3-(4-phenoxyphenyl)-1H-pyrazolo[3,4-d]pyrimidin-1-yl)piperidine-1-yl)methyl)azetidine-1-carboxylate (79a) (1.1 g, yield: 99%).
LCMS m/z = 556.3 [M+1]⁺.

### Step 2:

### 1-(1-(azetidin-3-ylmethyl)piperidin-4-yl)-3-(4-phenoxyphenyl)-1H-pyrazolo[3,4-d]pyrimidin-4-amine (79b)

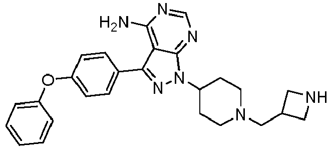

Tert-butyl 3-((4-(4-amino-3-(4-phenoxyphenyl)-1H-pyrazolo[3,4-d]pyrimidin-1-yl)piperidine-1-yl)methyl)azetidine-1-carboxylate (79a) (1.1 g, 1.98 mmol) was dissolved in 20 mL of dichloromethane, 9 mL of trifluoroacetic acid was added, and the mixture was stirred at room temperature for 2 h. The reaction system was concentrated under reduced pressure. The residue was dissolved with 30 mL of 4 mol/L NaOH aqueous solution, extracted with DCM (40 mL × 3). The organic phase was washed with 40 mL of water, dried over anhydrous sodium sulfate, and concentrated under reduced pressure, to obtain 1-(1-(azetidine-3-ylmethyl)piperidine-4-yl)-3-(4-phenoxyphenyl)-1H-pyrazolo[3,4-d]pyrimidin-4-amine (79b) (0.9 g, yield: > 99%).
LCMS m/z = 456.2 [M+1]⁺.

### Step 3:

### 5-(3-((4-(4-amino-3-(4-phenoxyphenyl)-1H-pyrazolo[3,4-d]pyrimidin-1-yl)piperidin-1-yl)methyl)azetidin-1-yl)-2-(2,6-dioxopiperidin-3-yl)isoindoline-1,3-dione (compound 79)

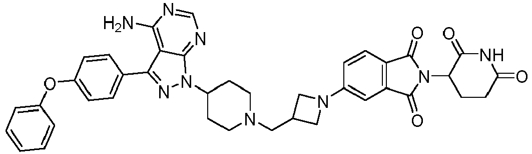

1-(1-(azetidin-3-ylmethyl)piperidine-4-yl)-3-(4-phenoxyphenyl)-1H-pyrazolo[3,4-d]pyrimidin-4-amine (79b) (180 mg, 0.40 mmol) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (163 mg, 0.59 mmol) were added, and the reaction was stirred at 80°C for 5 h. The reaction solution was cooled to room temperature, 50 mL of water was added, and the reaction solution was filtered. The solid was collected, washed with 50 mL of water, and dissolved with 100 mL of dichloromethane, dried over anhydrous sodium sulfate, and concentrated under reduced pressure, then the crude product was separated and purified by silica gel column chromatography (dichloromethane/methanol (v/v) = 15 : 1), to obtain 5-(3-((4-(4-amino-3-(4-phenoxyphenyl)-1H-pyrazolo[3,4-d]pyrimidin-1-yl)piperidine-1-yl)methyl)azetidin-1-yl)-2-(2,6-dioxopiperidin-3-yl)isoindoline-1,3-dione (compound 79) (80 mg, yield: 28%).
¹H NMR (400 MHz, CDCl₃) δ 9.07 (s, 1H), 8.40 (s, 1H), 7.69 - 7.61 (m, 3H), 7.43 - 7.35 (m, 2H), 7.21 - 7.12 (m, 3H), 7.11 - 7.05 (m, 2H), 6.79 (d, 1H), 6.50 (dd, 1H), 5.66 (br.s, 2H), 4.93 (dd, 1H), 4.89 - 4.75 (m, 1H), 4.21 - 4.09 (m, 2H), 3.80 - 3.68 (m, 2H), 3.18 - 3.00 (m, 3H), 2.93 - 2.66 (m, 5H), 2.54 - 2.26 (m, 4H), 2.18 - 1.98 (m, 3H).
LCMS m/z = 712.3 [M+1]⁺.

### Example 80:

### (7S)-7-(1-(1-((1-(2-(2,6-dioxopiperidin-3-yl)-1,3-dioxoisoindolin-5-yl)azetidin-3-yl)methyl)azetidin-3-yl)piperidin-4-yl)-2-(4-phenoxyphenyl)-4,5,6,7-tetrahydropyrazolo[1,5-a]pyrimidine-3-carboxamide (compound 80)

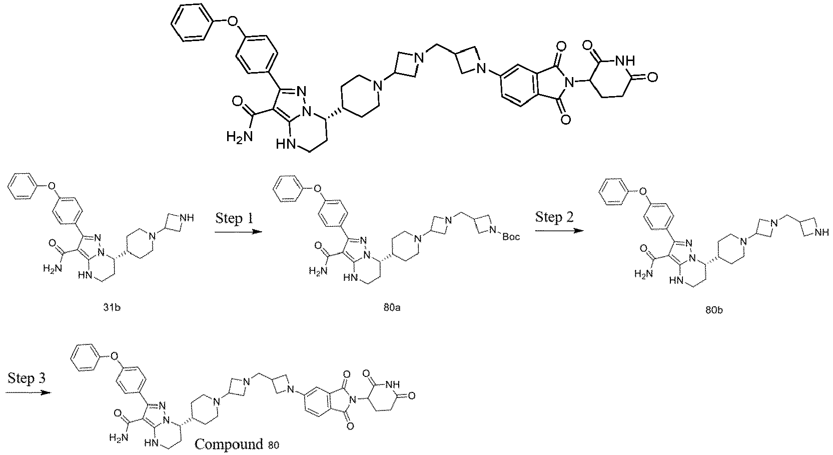

### Step 1:

### tert-butyl (S)-3-((3-(4-(3-carbamoyl-2-(4-phenoxyphenyl)-4,5,6,7-tetrahydropyrazolo[1,5-a] pyrimidin-7-yl)piperidin-1-yl)azetidin-1-yl)methyl)azetidine-1-carboxylate (80a)

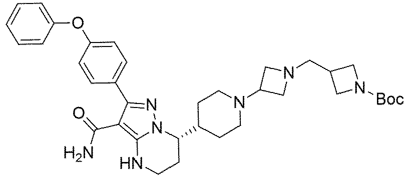

(7S)-7-[1-(azetidin-3-yl)-4-piperidyl]-2-(4-phenoxyphenyl)-4,5,6,7-tetrahydropyrazolo[1,5-a]pyrimidin-3-carboxamide (31b) (see WO 2020239103 for the synthetic method) (140 mg, 0.30 mmol) was dissolved in 15 mL of DCE, tert-butyl 3-formylazetidine-1-carboxylate (100 mg, 0.54 mmol) was added, the mixture was stirred at room temperature for 10 min, then sodium triacetoxyborohydride (191 mg, 0.90 mmol) was added, and the mixture was stirred at room temperature for 16 h. To the reaction system was slowly added 20 mL of saturated sodium bicarbonate aqueous solution, and the mixed solution was extracted with DCM (50 mL × 3). The organic phase was washed with 50 mL of water, dried over anhydrous sodium sulfate, and concentrated under reduced pressure, and then the crude product was separated and purified by silica gel column chromatography (dichloromethane/methanol (v/v) = 10 : 1-4 : 1), to obtain tert-butyl (S)-3-((3-(4-(3-carbamoyl-2-(4-phenoxyphenyl)-4,5,6,7-tetrahydropyrazolo[1,5-a]pyrimidin-7-yl)piperidine-1-yl)azetidin-1-yl)methyl)azetidine-1-carboxylate (80a) (140 mg, yield: 73%).
LCMS m/z = 642.3 [M+1]⁺.

### Step 2:

### (S)-7-(1-(1-(azetidin-3-ylmethyl)azetidin-3-yl)piperidin-4-yl)-2-(4-phenoxyphenyl)-4,5,6,7-tetrahydropyrazolo[1,5-a]pyrimidine-3-carboxamide (80b) trifluoroacetate

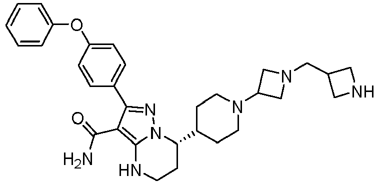

Tert-butyl (S)-3-((3-(4-(3-carbamoyl-2-(4-phenoxyphenyl)-4,5,6,7-tetrahydropyrazolo[1,5-a]pyrimidin-7-yl)piperidine-1-yl)azetidin-1-yl)methyl)azetidine-1-carboxylate (80a) (140 mg, 0.22 mmol) was dissolved in 10 mL of dichloromethane, 5 mL of trifluoroacetic acid was added, and the mixture was stirred at room temperature for 2 h. The reaction system was concentrated under reduced pressure, to obtain crude product (S)-7-(1-(1-(1-(azetidin-3-ylmethyl)azetidin-3-yl)piperidine-4-yl)-2-(4-phenoxyphenyl)-4,5,6,7-tetrahydropyrazolo[1,5-a]pyrimidine-3-carboxamide (80b) trifluoroacetate (190 mg).
LCMS m/z = 542.3 [M+1]⁺.

### Step 5:

### (7S)-7-(1-(1-((1-(2-(2,6-dioxopiperidin-3-yl)-1,3-dioxoisoindolin-5-yl)azetidin-3-yl)methyl)azetidin-3-yl)piperidin-4-yl)-2-(4-phenoxyphenyl)-4,5,6,7-tetrahydropyrazolo[1,5-a]pyrimidine-3-carboxamide (compound 80)

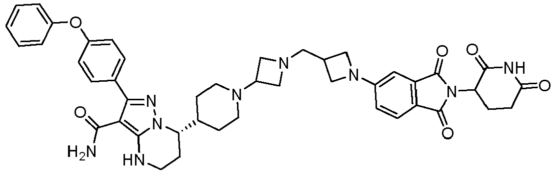

The above crude product (S)-7-(1-(1-(1-(azetidin-3-ylmethyl)azetidin-3-yl)piperidine-4-yl)-2-(4-phenoxyphenyl)-4,5,6,7-tetrahydropyrazolo[1,5-a]pyrimidine-3-carboxamide (80b) trifluoroacetate (190 mg) was dissolved in 10 mL of DMSO, and solid sodium bicarbonate (67 mg, 0.80 mmol) was added. The mixture was stirred at room temperature for 10 min, then 1.0 mL of DIPEA and 2-(2,6-dioxopiperidin-3-yl)-5-fluoroisoindoline-1,3-dione (see WO 2017197056 for the synthetic method) (83 mg, 0.30 mmol) were added, and the reaction was stirred at 80°C for 5 h. The reaction solution was cooled to room temperature, 50 mL of water was added, and the reaction solution was filtered. The solid was collected, washed with 50 mL of water, dissolved with 100 mL of dichloromethane, dried over anhydrous sodium sulfate, and concentrated under reduced pressure, and then the crude product was separated and purified by silica gel column chromatography (dichloromethane/methanol (v/v) = 10 : 1), to obtain (7S)-7-(1-(1-((1-(2-(2,6-dioxopiperidin-3-yl)-1,3-dioxoisoindolin-5-yl)azetidin-3-yl)methyl)azetidin-3-yl)piperidine-4-yl)-2-(4-phenoxyphenyl)-4,5,6,7-tetrahydropyrazolo[1,5-a]pyrimidine-3-carboxamide (compound 80) (80 mg, two-step yield from compound 80a: 46%).
¹H NMR (400 MHz, CDCl₃) δ 9.45 (br.s, 1H), 7.62 (d, 1H), 7.53 - 7.46 (m, 2H), 7.40 - 7.32 (m, 2H), 7.18 - 7.11 (m, 1H), 7.10 - 7.01 (m, 4H), 6.87 - 6.82 (m, 1H), 6.58 (s, 1H), 6.47 (dd, 1H), 5.17 (br.s, 2H), 4.91 (dd, 1H), 4.12 - 4.04 (m, 3H), 3.73 - 3.66 (m, 2H), 3.66 - 3.54 (m, 2H), 3.46 - 3.36 (m, 2H), 3.07 - 2.92 (m, 2H), 2.90 - 2.64 (m, 7H), 2.25 - 2.00 (m, 4H), 1.95 - 1.65 (m, 6H), 1.64 - 1.53 (m, 2H).
LCMS m/z = 798.3 [M+1]⁺.

### Example 81:

### (7S)-7-(1'-((1-(2-(2,6-dioxopiperidin-3-yl)-1,3-dioxoisoindolin-5-yl)piperidin-4-yl)methyl)-[1,4'-bipiperidin]-4-yl)-2-(4-phenoxyphenyl)-4,5,6,7-tetrahydropyrazolo[1,5-a]pyrimidine-3-carboxamide (compound 81)

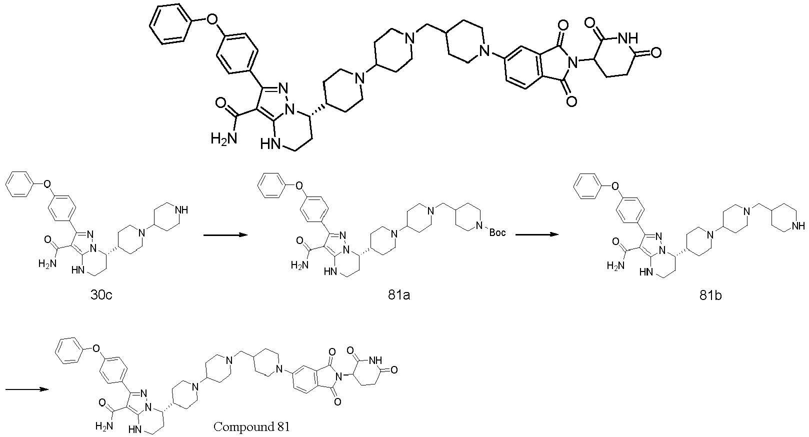

### Step 1:

### tert-butyl (S)-4-((4-(3-carbamoyl-2-(4-phenoxyphenyl)-4,5,6,7-tetrahydropyrazolo[1,5-a] pyrimidin-7-yl)-[1,4'-bipiperidin]-1'-yl)methyl)piperidine-1 - carboxylate (81a)

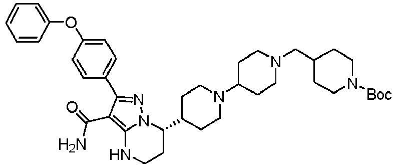

(7S)-2-(4-phenoxyphenyl)-7-[1-(4-piperidyl)-4-piperidyl]-4,5,6,7-tetrahydropyrazolo[1,5-a]pyrimidine-3-carboxamide (30c) (see WO 2020239103 for the synthetic method) (230 mg, 0.46 mmol) was dissolved in 15 mL of DCE, tert-butyl 4-formylpiperidine-1-carboxylate (150 mg, 0.70 mmol) was added, the mixture was stirred at room temperature for 10 min, then sodium triacetoxyborohydride (195 mg, 0.92 mmol) was added, and the mixture was stirred at room temperature for 16 h. To the reaction system was slowly added 20 mL of sodium bicarbonate aqueous solution, and the mixed solution was extracted with DCM (50 mL × 3). The organic phase was washed with 50 mL of water, dried over anhydrous sodium sulfate, and concentrated under reduced pressure, and then the crude product was separated and purified by silica gel column chromatography (dichloromethane/methanol (v/v) = 10 : 1-4 : 1), to obtain tert-butyl (S)-4-((4-(3-carbamoyl-2-(4-phenoxyphenyl)-4,5,6,7-tetrahydropyrazolo[1,5-a]pyrimidin-7-yl)-[1,4'-bipiperidin]- 1-yl)methyl)piperidine-1-carboxylate (81a) (250 mg, yield: 78%).
LCMS m/z = 698.5 [M+1]⁺.

### Step 2:

### (S)-2-(4-phenoxyphenyl)-7-(1'-(piperidin-4-ylmethyl)-[1,4'-bipiperidin]-4-yl)-4,5,6,7-tetrahydropyrazolo[1,5-a]pyrimidine-3-carboxamide (81b) trifluoroacetate

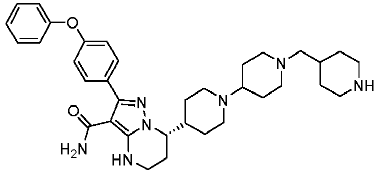

Tert-butyl (S)-4-((4-(3-carbamoyl-2-(4-phenoxyphenyl)-4,5,6,7-tetrahydropyrazolo[1,5-a]pyrimidin-7-yl)-[1,4'-bipiperidin]-1'-yl)methyl)piperidine-1-carboxylate (81a) (230 mg, 0.33 mmol) was dissolved in 10 mL of dichloromethane, 5 mL of trifluoroacetic acid was added, and the mixture was stirred at room temperature for 2 h. The reaction system was directly concentrated under reduced pressure, to obtain crude product (S)-2-(4-phenoxyphenyl)-7-(1'-(piperidin-4-ylmethyl)-[1,4'-bipiperidin]-4-yl)-4,5,6,7-tetrahydropyrazolo[1,5-a]pyrimidine-3-carboxamide (81b) trifluoroacetate (270 mg).
LCMS m/z = 598.3 [M+1]⁺.

### Step 3:

### (7S)-7-(1'-((1-(2-(2,6-dioxopiperidin-3-yl)-1,3-dioxoisoindolin-5-yl)piperidin-4-yl)methyl)-[1,4'-bipiperidin]-4-yl)-2-(4-phenoxyphenyl)-4,5,6,7-tetrahydropyrazolo[1,5-a]pyrimidine-3-carboxamide (compound 81)

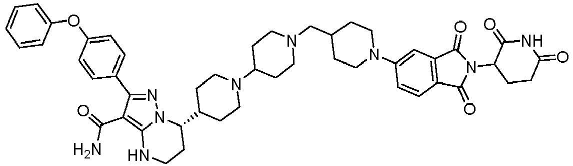

The above crude product (S)-2-(4-phenoxyphenyl)-7-(1'-(piperidin-4-ylmethyl)-[1,4'-bipiperidin]-4-yl)-4,5,6,7-tetrahydropyrazolo[1,5-a]pyrimidine-3-carboxamide (81b) trifluoroacetate (270 mg) was dissolved in 10 mL of DMSO, and solid sodium bicarbonate (143 mg, 1.70 mmol) was added. The mixture was stirred at room temperature for 10 min, then 1.0 mL of DIPEA and 2-(2,6-dioxopiperidin-3-yl)-5-fluoroisoindoline-1,3-dione (see WO 2017197056 for the synthetic method) (142 mg, 0.51 mmol) were added, and the reaction was stirred at 80°C for 5 h. The reaction solution was cooled to room temperature, 50 mL of water was added, and the reaction solution was filtered. The solid was collected, washed with 50 mL of water, dissolved with 100 mL of dichloromethane, dried over anhydrous sodium sulfate, and concentrated under reduced pressure, and then the crude product was separated and purified by silica gel column chromatography (dichloromethane/methanol (v/v) = 10 : 1), to obtain (7S)-7-(1'-((1-(2-(2,6-dioxopiperidin-3-yl)-1,3-dioxoisoindolin-5-yl)piperidin-4-yl)methyl)-[1,4'-bipiperidin]-4-yl)-2-(4-phenoxyphenyl)-4,5,6,7-tetrahydropyrazolo[1,5-a]pyrimidine-3-carboxamide (compound 81) (140 mg, two-step yield from compound 81a: 50%).
¹H NMR (400 MHz, CDCl₃) δ 7.66 (d, 1H), 7.54 - 7.47 (m, 2H), 7.40 - 7.32 (m, 2H), 7.29 - 7.26 (m, 1H), 7.18 - 7.11 (m, 1H), 7.10 - 6.98 (m, 5H), 6.57 (s, 1H), 5.18 (br.s, 2H), 4.93 (dd, 1H), 4.11 - 4.03 (m, 1H), 3.98 - 3.87 (m, 2H), 3.47 - 3.35 (m, 2H), 3.06 - 2.65 (m, 9H), 2.35 - 2.00 (m, 9H), 1.96 - 1.68 (m, 8H), 1.64 - 1.52 (m, 4H), 1.50 - 1.40 (m, 1H), 1.32 - 1.19 (m, 2H).
LCMS m/z = 854.4 [M+1]⁺.

### Example 82:

### (7S)-7-(1'-((1-(2-(2,6-dioxopiperidin-3-yl)-1,3-dioxoisoindolin-5-yl)azetidin-3-yl)methyl)-[1,4'-bipiperidin]-4-yl)-2-(4-phenoxyphenyl)-4,5,6,7-tetrahydropyrazolo [1,5-a]pyrimidine-3-carboxamide (compound 82)

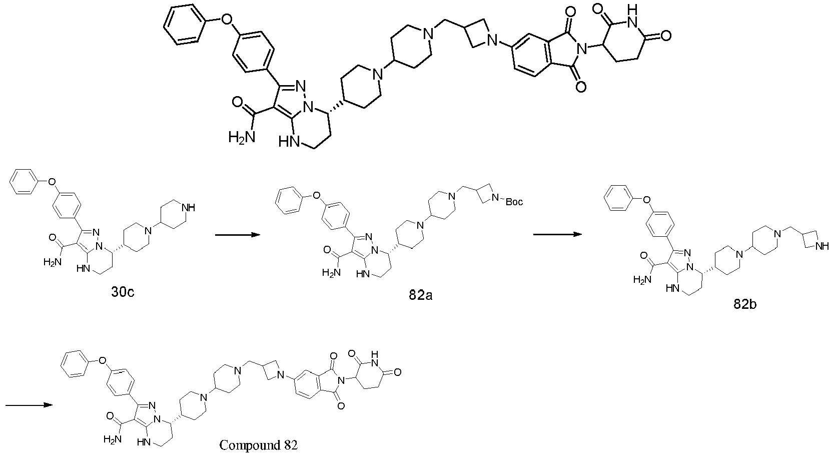

### Step 1:

### tert-butyl (S)-3 -((4-(3 -carbamoyl-2-(4-phenoxyphenyl)-4,5,6,7-tetrahydropyrazolo[1,5-a]pyrimidin-7-yl)-[1,4'-bipiperidin]-1'-yl)methyl)azetidine-1-carboxylate (82a)

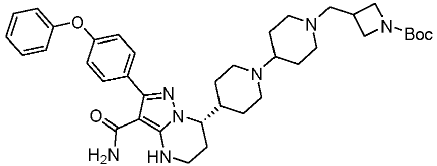

(7S)-2-(4-phenoxyphenyl)-7-[1-(4-piperidyl)-4-piperidyl]-4,5,6,7-tetrahydropyrazolo[1,5-a]pyrimidine-3-carboxamide (30c) (see WO 2020239103 for the synthetic method) (230 mg, 0.46 mmol) was dissolved in 15 mL of DCE, tert-butyl 3-formylazetidine-1-carboxylate (128 mg, 0.69 mmol) was added, the mixture was stirred at room temperature for 10 min, then sodium triacetoxyborohydride (195 mg, 0.92 mmol) was added, and the mixture was stirred at room temperature for 16 h. To the reaction system was slowly added 20 mL of sodium bicarbonate aqueous solution, and the mixed solution was extracted with DCM (50 mL × 3). The organic phase was washed with 50 mL of water, dried over anhydrous sodium sulfate, and concentrated under reduced pressure, and then the crude product was separated and purified by silica gel column chromatography (dichloromethane/methanol (v/v) = 10 : 1-4 : 1), to obtain tert-butyl (S)-3-((4-(3-carbamoyl-2-(4-phenoxyphenyl)-4,5,6,7-tetrahydropyrazolo[1,5-a]pyrimidin-7-yl)-[1,4'-bipiperidin]-1'-yl)methyl)azetidine-1-carboxylate (82a) (280 mg, yield: 91%).
LCMS m/z = 670.5 [M+1]⁺.

### Step 2:

### (S)-7-(1'-(azetidin-3-ylmethyl)-[1,4'-bipiperidin]-4-yl)-2-(4-phenoxyphenyl)-4,5,6,7-tetrahydropyrazolo[1,5-a]pyrimidine-3-carboxamide (82b) trifluoroacetate

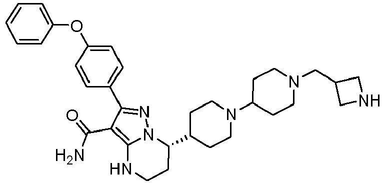

Tert-butyl (S)-3-((4-(3-carbamoyl-2-(4-phenoxyphenyl)-4,5,6,7-tetrahydropyrazolo[1,5-a]pyrimidin-7-yl)-[1,4'-bipiperidin]-1'-yl)methyl)azetidine-1-carboxylate (82a) (260 mg, 0.39 mmol) was dissolved in 10 mL of dichloromethane, 5 mL of trifluoroacetic acid was added, and the mixture was stirred at room temperature for 2 h. The reaction system was directly concentrated under reduced pressure, to obtain crude product (S)-7-(1'-(azetidin-3-ylmethyl)-[1,4'-bipiperidin]-4-yl)-2-(4-phenoxyphenyl)-4,5,6,7-tetrahydropyrazolo[1,5-a]pyrimidine-3-carboxamide (82b) trifluoroacetate (320 mg).
LCMS m/z = 570.3 [M+1]⁺.

### Step 3:

### (7S)-7-(1'-((1-(2-(2,6-dioxopiperidin-3-yl)-1,3-dioxoisoindolin-5-yl)azetidin-3-yl)methyl)-[1,4'-bipiperidin]-4-yl)-2-(4-phenoxyphenyl)-4,5,6,7-tetrahydropyrazolo [1,5-a]pyrimidine-3-carboxamide (compound 82)

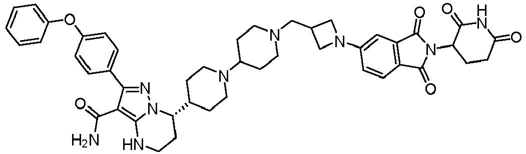

The above crude product (S)-7-(1'-(azetidin-3-ylmethyl)-[1,4'-bipiperidin]-4-yl)-2-(4-phenoxyphenyl)-4,5,6,7-tetrahydropyrazolo[1,5-a]pyrimidine-3-carboxamide (82b) trifluoroacetate (320 mg) was dissolved in 10 mL of DMSO, and solid sodium bicarbonate (173 mg, 2.06 mmol) was added. The mixture was stirred at room temperature for 10 min, 1.0 mL of DIPEA and 2-(2,6-dioxopiperidin-3-yl)-5-fluoroisoindoline-1,3-dione (see WO 2017197056 for the synthetic method) (168 mg, 0.61 mmol) were added, and the reaction was stirred at 80°C for 5 h. The reaction solution was cooled to room temperature, 50 mL of water was added, and the reaction solution was filtered. The solid was collected, washed with 50 mL of water, dissolved with 100 mL of dichloromethane, dried over anhydrous sodium sulfate, and concentrated under reduced pressure, and then the crude product was separated and purified by silica gel column chromatography (dichloromethane/methanol (v/v) = 10 : 1), to obtain (7S)-7-(1'-((1-(2-(2,6-dioxopiperidin-3-yl)-1,3-dioxoisoindolin-5-yl)azetidin-3-yl)methyl)-[1,4'-bipiperidin]-4-yl)-2-(4-phenoxyphenyl)-4,5,6,7-tetrahydropyrazolo[1,5-a]pyrimidine-3-carboxamide (compound 82) (140 mg, two-step yield from compound 82a: 43%).
¹H NMR (400 MHz, CDCl₃) δ 7.63 (d, 1H), 7.55 - 7.47 (m, 2H), 7.41 - 7.32 (m, 2H), 7.18 - 7.11 (m, 1H), 7.10 - 7.01 (m, 4H), 6.82 (d, 1H), 6.57 (s, 1H), 6.48 (dd, 1H), 5.19 (br.s, 2H), 4.92 (dd, 1H), 4.16 - 4.04 (m, 3H), 3.73 - 3.63 (m, 2H), 3.47 - 3.35 (m, 2H), 3.08 - 2.68 (m, 8H), 2.68 - 2.59 (m, 2H), 2.35 - 1.94 (m, 10H), 1.91 - 1.79 (m, 4H), 1.75 - 1.71 (m, 1H), 1.65 - 1.50 (m, 4H), 1.48 - 1.39 (m, 1H).
LCMS m/z = 826.4 [M+1]⁺.

### Example 83:

### (7S)-7-(1-((1-(2-(2,6-dioxopiperidin-3-yl)-1,3-dioxoisoindolin-5-yl)piperidin-4-yl)methyl)piperidin-4-yl)-2-(4-phenoxyphenyl)-4,5,6,7-tetrahydropyrazolo[1,5-a]pyrimidine-3-carboxamide (compound 83)

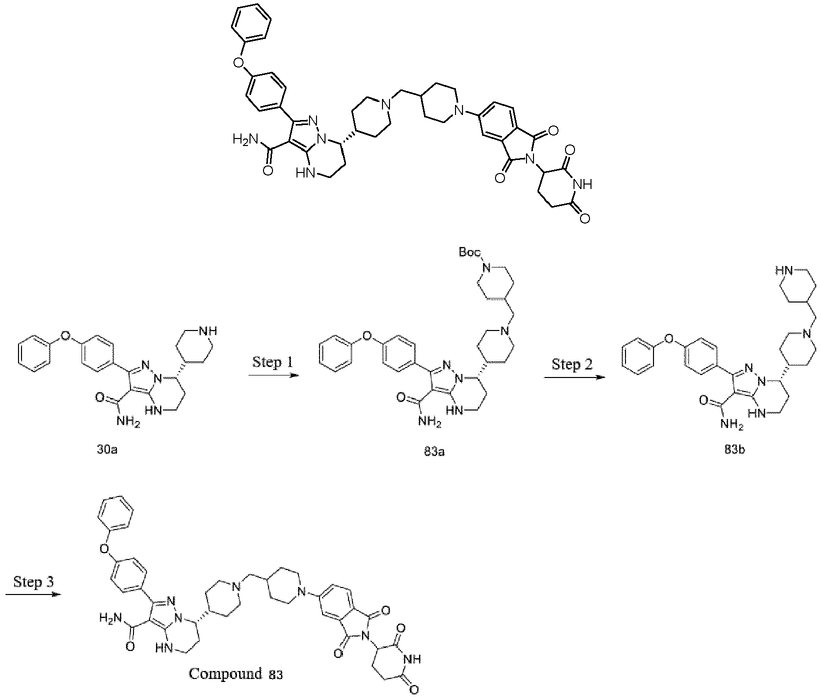

### Step 1:

### tert-butyl (S)-4-((4-(3-carbamoyl-2-(4-phenoxyphenyl)-4,5,6,7-tetrahydropyrazolo[1,5-a] pyrimidin-7-yl)piperidin-1-yl)methyl)piperidine-1-carboxylate (83a)

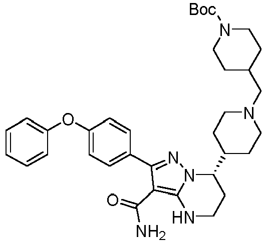

(S)-2-(4-phenoxyphenyl)-7-(piperidin-4-yl)-4,5,6,7-tetrahydropyrazolo[1,5-a]pyrimidine-3-carboxamide (30a) (95% ee) (see WO 2018033853 for the synthetic method) (417 mg, 1.00 mmol) was dissolved in 15 mL of chloroform, 1-tertbutoxycarbonylpiperidine-4-carbaldehyde (1.07 g, 5.00 mmol), glacial acetic acid (0.23 mL, 4.00 mmol) and anhydrous sodium sulfate (800 mg) were successively added at room temperature, and the mixture was stirred at 60°C for 2 h, then sodium triacetoxyborohydride (1.27 g, 6.00 mmol) was added, and the mixture was stirred at room temperature for 16 h. To the reaction solution was added 50 mL of water, the pH of the aqueous phase was adjusted to 10 with 2 mol/L sodium hydroxide aqueous solution, and the resulted solution was extracted with dichloromethane (30 mL × 3). The organic phase was combined, washed with water (20 mL × 2), dried over anhydrous sodium sulfate, and concentrated under reduced pressure. The crude product was separated and purified by silica gel column chromatography (dichloromethane/methanol (v/v) = 10 : 1-8 : 1), to obtain tert-butyl (S)-4-((4-(3-carbamoyl-2-(4-phenoxyphenyl)-4,5,6,7-tetrahydropyrazolo[1,5-a]pyrimidin-7-yl)piperidin-1-yl)methyl)piperidine-1-carboxylate (83a) (240 mg, yield: 39%).
LCMS m/z = 615.3 [M+1]⁺.

### Step 2:

### (S)-2-(4-phenoxyphenyl)-7-(1-(piperidin-4-ylmethyl)piperidin-4-yl)-4,5,6,7-tetrahydropyrazolo[1,5-a]pyrimidine-3-carboxamide (83b)

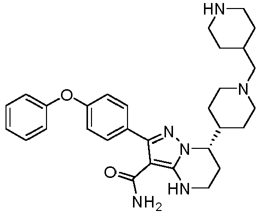

Tert-butyl (S)-4-((4-(3-carbamoyl-2-(4-phenoxyphenyl)-4,5,6,7-tetrahydropyrazolo[1,5-a]pyrimidin-7-yl)piperidin-1-yl)methyl)piperidine-1-carboxylate (83a) (240 mg, 0.39 mmol) was dissolved in 4 mL of dichloromethane, 2 mL of trifluoroacetic acid was added, and the reaction was carried out at room temperature for 1.5 h. The pH of the reaction solution was adjusted to 10 with 2 mol/L sodium hydroxide solution. The aqueous phase was extracted with dichloromethane (10 mL × 3). The organic phase was combined, dried over anhydrous sodium sulfate, and concentrated under reduced pressure, to obtain (S)-2-(4-phenoxyphenyl)-7-(1-(piperidin-4-ylmethyl)piperidin-4-yl)-4,5,6,7-tetrahydropyrazolo[1,5-a]pyrimidine-3-carboxamide (83b) (190 mg, yield: 95%).
LCMS m/z = 515.3 [M+1]⁺.

### Step 3:

### (7S)-7-(1-((1-(2-(2,6-dioxopiperidin-3-yl)-1,3-dioxoisoindolin-5-yl)piperidin-4-yl)methyl)piperidin-4-yl)-2-(4-phenoxyphenyl)-4,5,6,7-tetrahydropyrazolo[1,5-a]pyrimidine-3-carboxamide (compound 83)

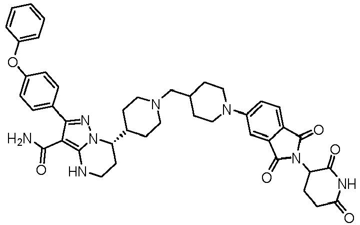

(S)-2-(4-phenoxyphenyl)-7-(1-(piperidin-4-ylmethyl)piperidin-4-yl)-4,5,6,7-tetrahydropyrazolo[1,5-a]pyrimidine-3-carboxamide (83b) (190 mg, 0.37 mmol) was dissolved in 5 mL of DMSO, 2-(2,6-dioxopiperidin-3-yl)-5-fluoroisoindoline-1,3-dione (see WO 2017197056 for the synthetic method) (129 mg, 0.47 mmol) and diisopropylethylamine (255 mg, 1.97 mmol) were added at room temperature, and the mixture was warmed to 80°C and reacted for 4 h. The reaction solution was cooled to room temperature, poured into 20 mL of water. The aqueous phase was extracted with dichloromethane/methanol (v/v) =10 : 1 (30 mL × 3). The organic phase was combined, washed with 40 mL of water, dried over anhydrous sodium sulfate, and concentrated under reduced pressure, and then the crude product was separated and purified by silica gel column chromatography (dichloromethane/methanol (v/v) = 15 : 1-8 : 1), to obtain (7S)-7-(1-((1-(2-(2,6-dioxopiperidin-3-yl)-1,3-dioxoisoindolin-5-yl)piperidin-4-yl)methyl)piperidin-4-yl)-2-(4-phenoxyphenyl)-4,5,6,7-tetrahydropyrazolo[1,5-a]pyrimidine-3-carboxamide (compound 83) (90 mg, yield: 32%).
¹H NMR (400 MHz, CDCl₃) δ 8.66 (br.s, 1H), 7.66 (d, 1H), 7.55 - 7.47 (m, 2H), 7.40 - 7.32 (m, 2H), 7.29 - 7.25 (m, 1H), 7.20 - 6.98 (m, 6H), 6.59 (s, 1H), 5.23 (br.s, 2H), 4.93 (dd, 1H), 4.11 - 4.03 (m, 1H), 3.99 - 3.88 (m, 2H), 3.46 - 3.37 (m, 2H), 3.05 - 2.65 (m, 7H), 2.26 - 2.02 (m, 6H), 2.01 - 1.84 (m, 4H), 1.83 - 1.41 (m, 5H), 1.33 - 1.20 (m, 2H).
LCMS m/z = 771.3 [M+1]⁺.

### Example 84:

### 5-(3-((4-(4-amino-3-(4-phenoxyphenyl)-1H-pyrazolo[3,4-d]pyrimidin-1-yl)-[1,4'-bipiperidin]-1'-yl)methyl)azetidin-1-yl)-2-(2,6-dioxopiperidin-3-yl)isoindoline-1,3-dione (compound 84)

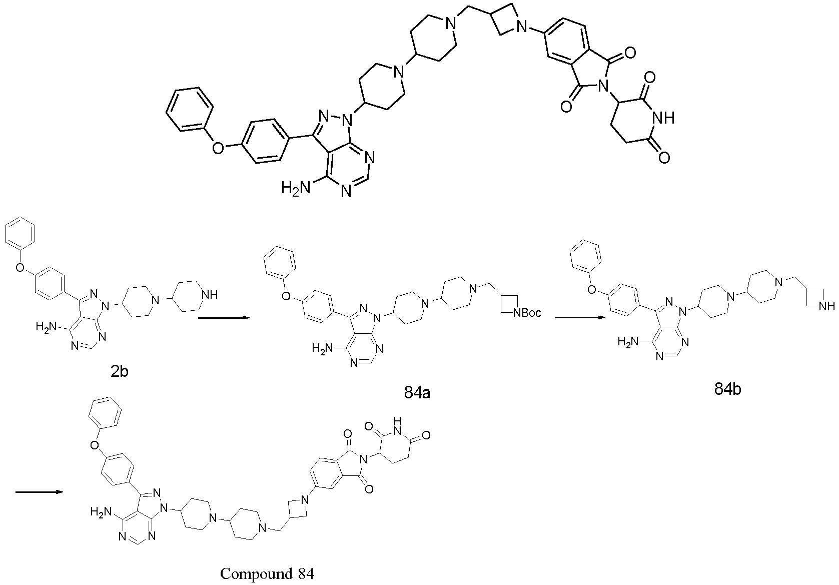

### Step 1:

### tert-butyl 3-((4-(4-amino-3-(4-phenoxyphenyl)-1H-pyrazolo[3,4-d]pyrimidin-1-yl)-[1,4'-bipiperidin]-1'-yl)methyl)azetidine-1-carboxylate (84a)

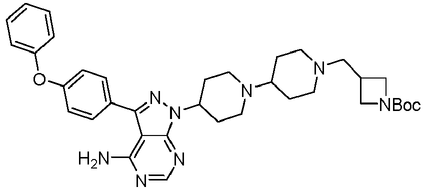

3-(4-phenoxyphenyl)-1-[1-(4-piperidyl)-4-piperidyl]pyrazolo[3,4-d]pyrimidine-4-amine (2b) (see WO 2020239103 for the synthetic method) (0.52 g, 1.11 mmol) was dissolved in 20 mL of DCE, tert-butyl 3-formylazetidine-1-carboxylate (0.37 g, 2.0 mmol) was added, the mixture was stirred at room temperature for 10 min, then sodium triacetoxyborohydride (0.7 g, 3.3 mmol) was added. The reaction was carried out under stirring at room temperature for 12 h. To the reaction solution was slowly added dropwise 50 mL of saturated sodium bicarbonate solution. The aqueous phase was extracted with 30 mL of dichloromethane, dried over anhydrous sodium sulfate, and concentrated under reduced pressure, and then the crude product was separated and purified by silica gel column chromatography (dichloromethane/methanol (v/v) = 20 : 1), to obtain tert-butyl 3-((4-(4-amino-3-(4-phenoxyphenyl)-1H-pyrazolo[3,4-d]pyrimidin-1-yl)-[1,4'-bipiperidin]-1 yl)methyl)azetidine-1-carboxylate (84a) (0.5 g, yield: 71%).
LC-MS m/z = 639.4 [M+1]⁺.

### Step 2:

### 1-(1'-(azetidin-3-ylmethyl)-[1,4'-bipiperidin]-4-yl)-3-(4-phenoxyphenyl)-1H-pyrazolo[3,4-d]pyrimidin-4-amine (84b)

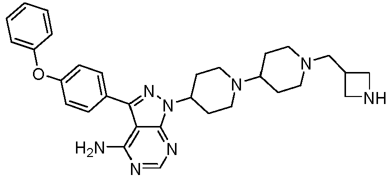

Tert-butyl 3-((4-(4-amino-3-(4-phenoxyphenyl)-1H-pyrazolo[3,4-d]pyrimidin-1-yl)-[1,4'-bipiperidin]-1'-yl)methyl)azetidine-1-carboxylate (84a) (0.5 g, 0.78 mmol) was dissolved in 15 mL of DCM, 5 mL of trifluoroacetic acid was added, and the mixture was stirred at room temperature for 3 h. To the reaction solution was slowly added dropwise 30 mL of saturated sodium bicarbonate solution. The aqueous phase was extracted with 30 mL of dichloromethane, dried over anhydrous sodium sulfate, and concentrated under reduced pressure, to obtain crude product 1-(1'-(azetidin-3-ylmethyl)-[1,4'-bipiperidin]-4-yl)-3-(4-phenoxyphenyl)-1H-pyrazolo[3,4-d]pyrimidine-4-amine (84b) (0.4 g).
LCMS m/z = 539.3 [M+1]⁺.

### Step 3:

### 5-(3-((4-(4-amino-3-(4-phenoxyphenyl)-1H-pyrazolo[3,4-d]pyrimidin-1-yl)-[1,4'-bipiperidin]-1'-yl)methyl)azetidin-1-yl)-2-(2,6-dioxopiperidin-3-yl)isoindoline-1,3-dione (compound 84)

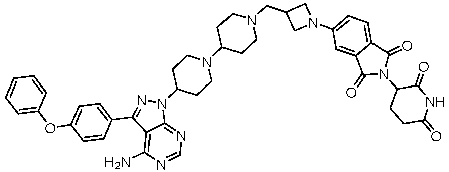

The above crude product 1-(1'-(azetidin-3-ylmethyl)-[1,4'-bipiperidin]-4-yl)-3-(4-phenoxyphenyl)-1H-pyrazolo[3,4-d]pyrimidine-4-amine (84b) (0.4 g) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (0.35 g, 1.27 mmol) were added, and the reaction was stirred at 90°C for 5 h. The reaction solution was cooled to room temperature, and diluted with 50 mL of ethyl acetate. The organic phase was washed with 50 mL of water, dried over anhydrous sodium sulfate, and concentrated under reduced pressure, and then the crude product was separated and purified by silica gel column chromatography (dichloromethane /methanol (v/v) = 20 : 1), to obtain 5-(3-((4-(4-amino-3-(4-phenoxyphenyl)-1H-pyrazolo[3,4-d]pyrimidin-1-yl)-[1,4'-bipiperidin]-1'-yl)methyl)azetidin-1-yl)-2-(2,6-dioxopiperidin-3-yl)isoindoline-1,3-dione (compound 84) (50 mg, two-step yield from compound 84a: 8%).
¹H NMR (400 MHz, CDCl₃) δ 8.37 (s, 1H), 7.69 - 7.60 (m, 3H), 7.43 - 7.34 (m, 2H), 7.21 - 7.03 (m, 5H), 6.81 (d, 1H), 6.49 (dd, 1H), 5.60 (br.s, 2H), 4.93 (dd, 1H), 4.82 - 4.70 (m, 1H), 4.19 - 4.09 (m, 2H), 3.75 - 3.65 (m, 2H), 3.17 - 3.07 (m, 2H), 3.07 - 2.91 (m, 3H), 2.91 - 2.68 (m, 3H), 2.68 - 2.61 (m, 2H), 2.51 - 2.31 (m, 5H), 2.17 - 2.08 (m, 1H), 2.08 - 1.99 (m, 4H), 1.88 - 1.79 (m, 2H), 1.68 - 1.55 (m, 2H).
LCMS m/z = 795.4 [M+1]⁺.

### Example 85:

### 5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)isoindoline-1,3-dione (compound 85)

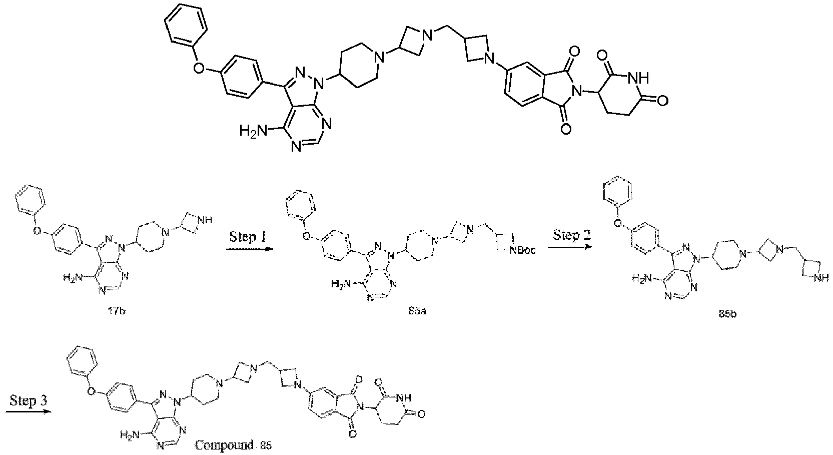

### Step 1:

### tert-butyl 3-((3-(4-(4-amino-3-(4-phenoxyphenyl)-1H-pyrazolo[3,4-d]pyrimidin-1-yl) piperidin-1-yl)azetidin-1-yl)methyl)azetidine-1-carboxylate (85a)

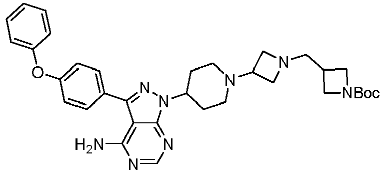

1-[1-(azetidin-3-yl)-4-piperidyl]-3-(4-phenoxyphenyl)pyrazolo[3,4-d]pyrimidine-4-amine (17b) (see WO 2020239103 for the synthetic method) (0.7 g, 1.59 mmol) was dissolved in 20 mL of DCE, tert-butyl 3-formylazetidine-1-carboxylate (0.37 g, 2.0 mmol) was added, the mixture was stirred at room temperature for 10 min, then sodium triacetoxyborohydride (0.7 g, 3.3 mmol) was added. The reaction was carried out under stirring at room temperature for 12 h. To the reaction solution was slowly added dropwise 50 mL of saturated sodium bicarbonate solution. The aqueous phase was extracted with 30 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain tert-butyl 3-((3-(4-(4-amino-3-(4-phenoxyphenyl)-1H-pyrazolo[3,4-d]pyrimidin-1-yl)piperidin-1-yl)azetidin-1-yl)methyl )azetidin-1-carboxylate (85a) (0.6 g, yield: 62%).
LC-MS m/z = 611.3 [M+1]⁺.

### Step 2:

### 1-(1-(1-(azetidin-3-ylmethyl)azetidin-3-yl)piperidin-4-yl)-3-(4-phenoxyphenyl)-1H-pyrazolo[3,4-d]pyrimidin-4-amine (85b)

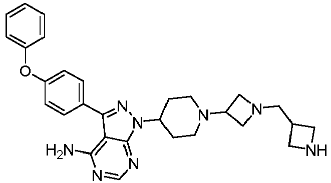

Tert-butyl 3-((3-(4-(4-amino-3-(4-phenoxyphenyl)-1H-pyrazolo[3,4-d]pyrimidin-1-yl)piperidin-1-yl)azetidin-1-yl)methyl )azetidine-1-carboxylate (85a) (0.6 g, 0.98 mmol) was dissolved in 15 mL of DCM, 5 mL of trifluoroacetic acid was added, and the mixture was stirred at room temperature for 3 h. To the reaction solution was slowly added dropwise 30 mL of saturated sodium bicarbonate solution. The aqueous phase was extracted with 30 mL of dichloromethane, dried over anhydrous sodium sulfate, and concentrated under reduced pressure, to obtain crude product 1-(1-(1-(azetidin-3-ylmethyl)azetidin-3-yl)piperidin-4-yl)-3-(4-phenoxyphenyl)-1H-pyrazolo[3,4-d]pyrimidine-4-amine (85b) (0.45 g).
LCMS m/z = 511.3 [M+1]⁺.

### Step 3:

### 5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)isoindoline-1,3-dione (compound 85)

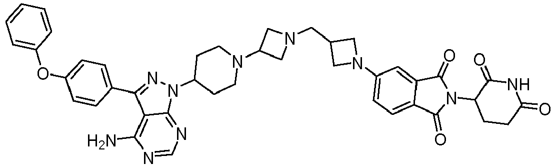

The above crude product 1-(1-(1-(azetidin-3-ylmethyl)azetidin-3-yl)piperidin-4-yl)-3-(4-phenoxyphenyl)-1H-pyrazolo[3,4-d]pyrimidine-4-amine (85b) (0.45 g) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (0.35 g, 1.27 mmol) were added, and the reaction was stirred at 90°C for 5 h. The reaction solution was cooled to room temperature, and diluted with 50 mL of ethyl acetate. The organic phase was washed with 50 mL of water, dried over anhydrous sodium sulfate, and concentrated under reduced pressure, and then the crude product was separated and purified by silica gel column chromatography (dichloromethane /methanol (v/v) = 20 : 1), to obtain 5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)isoindoline-1,3-dione (compound 85) (0.11 g, two-step yield from compound 85a: 15%).
¹H NMR (400 MHz, CDCl₃) δ 11.24 (br.s, 1H), 8.38 (s, 1H), 7.69 - 7.59 (m, 3H), 7.42 - 7.34 (m, 2H), 7.21 - 7.03 (m, 5H), 6.93 (d, 1H), 6.46 (dd, 1H), 5.82 (br.s, 2H), 4.94 (dd, 1H), 4.84 - 4.71 (m, 1H), 4.14 - 4.03 (m, 2H), 3.76 - 3.53 (m, 4H), 3.12 - 2.97 (m, 3H), 2.96 - 2.66 (m, 8H), 2.48 - 2.31 (m, 2H), 2.16 - 1.94 (m, 5H).
LCMS m/z = 767.3 [M+1]⁺.

### Example 86:

### 5-(4-((4-(4-amino-3-(4-phenoxyphenyl)-1H-pyrazolo[3,4-d]pyrimidin-1-yl)-[1,4'-bipiperidin]-1'-yl)methyl)piperidin-1-yl)-2-(2,6-dioxopiperidin-3-yl)isoindoline-1,3-dione (compound 86)

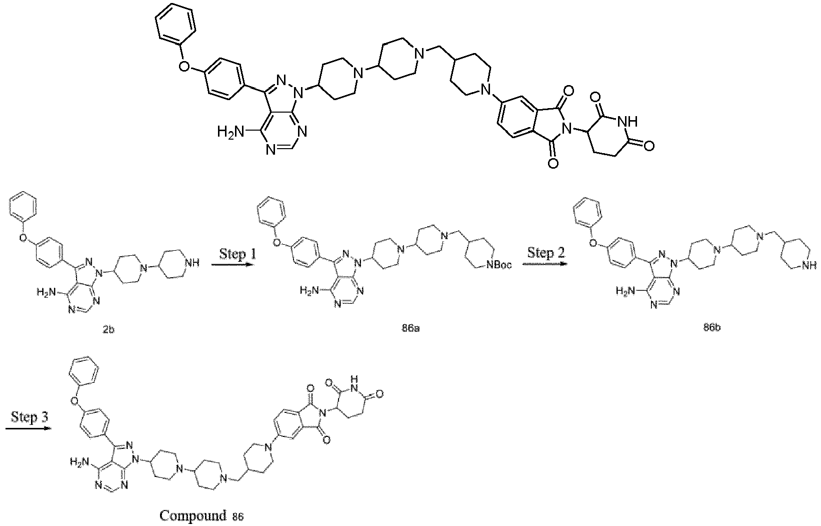

### Step 1:

### tert-butyl 4-((4-(4-amino-3-(4-phenoxyphenyl)-1H-pyrazolo[3,4-d]pyrimidin-1-yl)-[1,4'-bipiperidin]-1'-yl)methyl)piperidine-1 -carboxylate (86a)

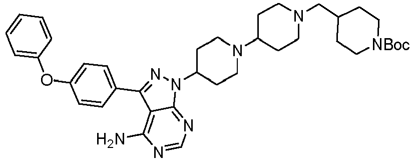

3-(4-phenoxyphenyl)-1-[1-(4-piperidyl)-4-piperidyl]pyrazolo[3,4-d]pyrimidine-4-amine (2b) (see WO 2020239103 for the synthetic method) (0.7 g, 1.49 mmol) was dissolved in 20 mL of DCE, tert-butyl 4-formylpiperidine-1-carboxylate (0.5 g, 2.34 mmol) was added, the mixture was stirred at room temperature for 10 min, then sodium triacetoxyborohydride (0.7 g, 3.3 mmol) was added. The reaction was carried out under stirring at room temperature for 12 h. To the reaction solution was slowly added dropwise 50 mL of saturated sodium bicarbonate solution. The aqueous phase was extracted with 30 mL of dichloromethane, dried over anhydrous sodium sulfate, and concentrated under reduced pressure, and then the crude product was separated and purified by silica gel column chromatography (dichloromethane/methanol (v/v) = 20 : 1), to obtain tert-butyl 4-((4-(4-amino-3-(4-phenoxyphenyl)-1H-pyrazolo[3,4-d]pyrimidin-1-yl)-[1,4'-bipiperidin]-1'-yl)methyl)piperidine-1-carboxylate (86a) (0.55 g, yield: 55%).
LC-MS m/z = 667.4 [M+1]⁺.

### Step 2:

### 3-(4-phenoxyphenyl)-1-(1'-(piperidin-4-ylmethyl)-[1,4'-bipiperidin]-4-yl)-1H-pyrazolo[3,4-d]pyrimidin-4-amine (86b)

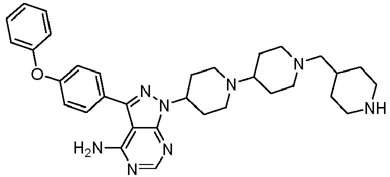

Tert-butyl 4-((4-(4-amino-3-(4-phenoxyphenyl)-1H-pyrazolo[3,4-d]pyrimidin-1-yl)-[1,4'-bipiperidin]-1'-yl)methyl)piperidine-1 -carboxylate (86a) (0.55 g, 0.83 mmol) was dissolved in 15 mL of DCM, 5 mL of trifluoroacetic acid was added, and the mixture was stirred at room temperature for 3 h. To the reaction solution was slowly added dropwise 30 mL of saturated sodium bicarbonate solution. The aqueous phase was extracted with 30 mL of dichloromethane, dried over anhydrous sodium sulfate, and concentrated under reduced pressure, to obtain 3-(4-phenoxyphenyl)-1-(1'-(piperidin-4-ylmethyl)-[1,4'-bipiperidin]-4-yl)-1H-pyrazolo[3,4-d]pyrimidine-4-amine (86b) (0.43 g).
LCMS m/z = 567.3 [M+1]⁺.

### Step 3:

### 5-(4-((4-(4-amino-3-(4-phenoxyphenyl)-1H-pyrazolo[3,4-d]pyrimidin-1-yl)-[1,4'-bipiperidin]-1'-yl)methyl)piperidin-1-yl)-2-(2,6-dioxopiperidin-3-yl)isoindoline-1,3-dione (compound 86)

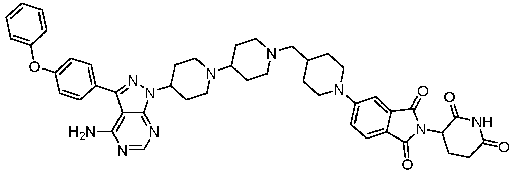

The above crude product 3-(4-phenoxyphenyl)-1-(1'-(piperidin-4-ylmethyl)-[1,4'-bipiperidin]-4-yl)-1H-pyrazolo[3,4-d]pyrimidine-4-amine (86b) (0.43 g) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (0.35 g, 1.27 mmol) were added, and the reaction was stirred at 90°C for 5 h. The reaction solution was cooled to room temperature, and diluted with 50 mL of ethyl acetate. The organic phase was washed with 50 mL of water, dried over anhydrous sodium sulfate, and concentrated under reduced pressure, and then the crude product was separated and purified by silica gel column chromatography (dichloromethane /methanol (v/v) = 15 : 1), to obtain 5-(4-((4-(4-amino-3-(4-phenoxyphenyl)-1H-pyrazolo[3,4-d]pyrimidin-1-yl)-[1,4'-bipiperidin]-1'-yl)methyl)piperidin-1-yl)-2-(2,6-dioxopiperidin-3-yl)isoindoline-1,3-dione (compound 86) (40 mg, two-step yield from compound 86a: 6%).
¹H NMR (400 MHz, CDCl₃) δ 8.39 (s, 1H), 7.70 - 7.60 (m, 3H), 7.42 - 7.34 (m, 2H), 7.30 - 7.26 (m, 1H), 7.20 - 6.99 (m, 6H), 5.78 (br.s, 2H), 4.94 (dd, 1H), 4.83 - 4.69 (m, 1H), 3.99 - 3.89 (m, 2H), 3.17 - 3.05 (m, 2H), 3.02 - 2.89 (m, 4H), 2.89 - 2.65 (m, 3H), 2.50 - 2.29 (m, 5H), 2.23 - 2.15 (m, 2H), 2.15 - 2.08 (m, 1H), 2.03 - 1.99 (m, 1H), 1.99 - 1.93 (m, 3H), 1.93 - 1.88 (m, 2H), 1.88 - 1.84 (m, 1H), 1.84 - 1.76 (m, 2H), 1.68 - 1.54 (m, 2H), 1.34 - 1.19 (m, 2H).
LCMS m/z = 823.4 [M+1]⁺.

### Example 87:

### 5-(3-(4-(4-amino-3-(4-(4-(trifluoromethyl)phenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 87)

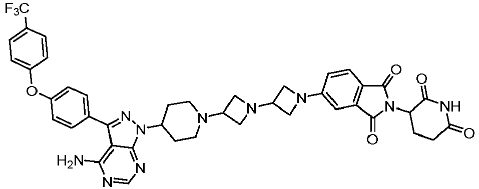

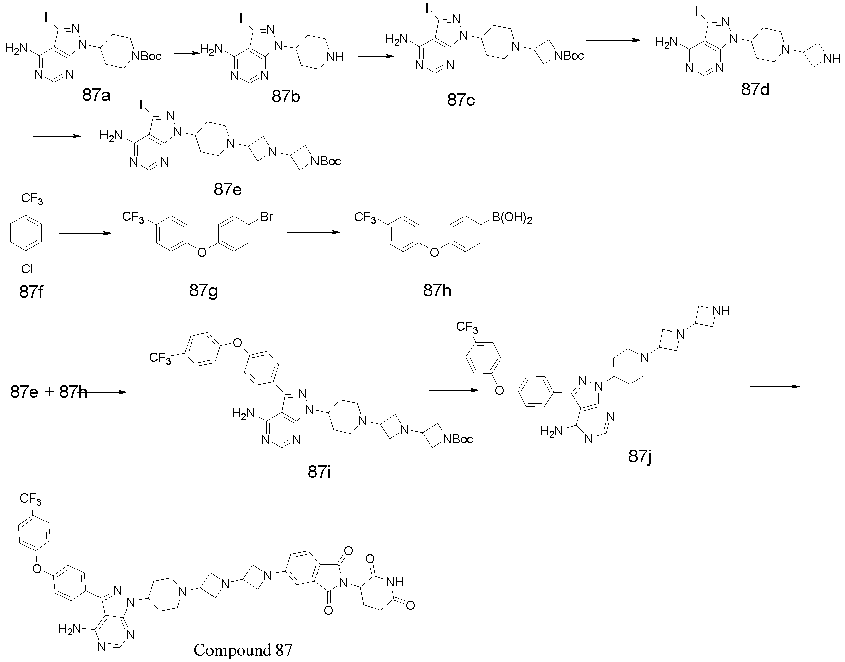

### Step 1:

### 3-iodo-1-(piperidin-4-yl)-1H-pyrazolo[3,4-d]pyrimidin-4-amine (87b)

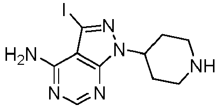

Tert-butyl 4-(4-amino-3-iodo-1H-pyrazolo[3,4-d]pyrimidin-1-yl)piperidine-1-carboxylate (87a) (see CN 110627796 for the synthetic method) (2 g, 4.50 mmol) was dissolved in 20 mL of DCM, 7.0 mL of trifluoroacetic acid was added, and the reaction was stirred at room temperature for 4 h. The reaction solution was concentrated under reduced pressure. The residue was dissolved in 50 mL of DCM, and 50 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 3-iodo-1-(piperidin-4-yl)-1H-pyrazolo[3,4-d]pyrimidine-4-amine (87b) (1.61 g).
LCMS m/z = 345.0 [M+1]⁺.

### Step 2:

### tert-butyl 3-(4-(4-amino-3-iodo-1H-pyrazolo[3,4-d]pyrimidin-1-yl)piperidin-1-yl) azetidine-1-carboxylate (87c)

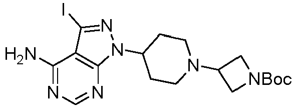

The above crude product 3-iodo-1-(piperidin-4-yl)-1H-pyrazolo[3,4-d]pyrimidine-4-amine (87b) (1.61 g) was dissolved in 20 mL of DCE, and tert-butyl 3-oxoazetidine-1-carboxylate (1.31 g, 7.66 mmol) and acetic acid (0.05 mL, 0.875 mmol) were added. The reaction was stirred at room temperature for 1 h, sodium triacetoxyborohydride (2.44 g, 11.5 mmol) was added, and the reaction was stirred at room temperature for 16 h. The reaction was quenched by adding 50 mL of water to the reaction solution. The organic layer was separated. The aqueous layer was extracted with dichloromethane (70 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-iodo-1H-pyrazolo[3,4-d]pyrimidin-1-yl)piperidin-1-yl)azetidine-1-carboxylate (87c) (1.00 g, two-step yield from compound 87a: 45%).
LCMS m/z = 500.1 [M+1]⁺.

### Step 3:

### 1-(1-(azetidin-3-yl)piperidin-4-yl)-3-iodo-1H-pyrazolo[3,4-d]pyrimidin-4-amine (87d)

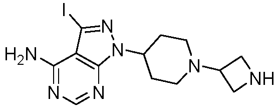

Tert-butyl 3-(4-(4-amino-3-iodo-1H-pyrazolo[3,4-d]pyrimidin-1-yl)piperidin-1-yl)azetidine-1-carboxylate (87c) (1 g, 2.0 mmol) was dissolved in 15 mL of DCM, 6.0 mL of trifluoroacetic acid was added, and the reaction was stirred at room temperature for 4 h. The reaction solution was concentrated under reduced pressure. The residue was dissolved in 50 mL of DCM, and 50 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-(1-(azetidin-3-yl)piperidin-4-yl)-3-iodo-1H-pyrazolo[3,4-d]pyrimidin-4-amine (87d) (696 mg).
LCMS m/z = 400.1 [M+1]⁺.

### Step 4:

### tert-butyl 3-(4-(4-amino-3-iodo-1H-pyrazolo[3,4-d]pyrimidin-1-yl)piperidin-1-yl)-[1,3'-biazetidine]-1'-carboxylate (87e)

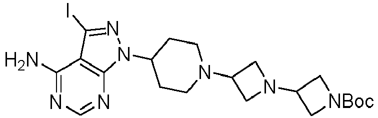

The above crude product 1-(1-(azetidin-3-yl)piperidin-4-yl)-3-iodo-1H-pyrazolo[3,4-d]pyrimidin-4-amine (87d) (696 mg) was dissolved in 20 mL of DCE, and tert-butyl 3-oxoazetidine-1-carboxylate (596 mg, 3.48 mmol) and acetic acid (0.05 mL, 0.875 mmol) were added. The reaction was stirred at room temperature for 1 h, sodium triacetoxyborohydride (1.1 g, 5.19 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-iodo-1H-pyrazolo[3,4-d]pyrimidin-1-yl)piperidin-1-yl)-[1,3'-biazetidin]-1'-carboxylate (87e) (120 mg, two-step yield from compound 87c: 11%).
LCMS m/z = 555.2 [M+1]⁺.

### Step 5:

### 1-bromo-4-(4-(trifluoromethyl)phenoxy)benzene (87g)

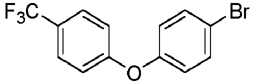

To 1-chloro-4-(trifluoromethyl)benzene (87f) (5 g, 27.7 mmol) in DMSO (70 mL) solution was added potassium tert-butoxide (3.26 g, 29.1 mmol) and 4-bromophenol (5.03 g, 29.1 mmol), and the reaction system was warmed to 160°C, heated and reacted for 28 h. The reaction was cooled to room temperature, and the reaction solution was slowly poured into 100 mL of ice water, extracted with ethyl acetate (100 mL × 3). The organic layer was combined, dried over anhydrous sodium sulfate, and concentrated under reduced pressure, and then the crude product was separated and purified by silica gel column chromatography (petroleum ether), to obtain 1-bromo-4-(4-(trifluoromethyl)phenoxy)benzene (87g) (3.1 g, yield: 35%).

### Step 6:

### (4-(4-(trifluoromethyl)phenoxy)phenyl)boronic acid (87h)

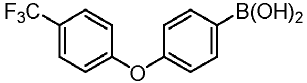

To a three-necked flask filled with nitrogen were added 1-bromo-4-(4-(trifluoromethyl)phenoxy)benzene (87g) (1 g, 3.15 mmol) and dry tetrahydrofuran (20 mL), the mixture was cooled to -78°C, and 2.5 mol/L n-butyllithium solution (1.89 mL) was slowly added dropwise. Upon completion of the addition, the reaction was carried out at -78°C for 30 min, and triisopropyl borate (0.88 mL, 3.81 mmol) was added dropwise, and the mixture was slowly warmed to room temperature and reacted for 3 h. Upon completion of the reaction, the reaction was quenched with 2 mol/L hydrochloric acid solution (50 mL), and extracted with ethyl acetate (50 mL × 3). The organic phase was washed with saturated sodium chloride solution (50 mL), dried over anhydrous sodium sulfate, and concentrated under reduced pressure to obtain crude product (4-(4-(trifluoromethyl)phenoxy)phenyl)boronic acid (87h) (100 mg).

### Step 7:

### tert-butyl 3-(4-(4-amino-3-(4-(4-(trifluoromethyl)phenoxy)phenyl)-1H-pyrazolo[3,4-d]pyrimidin-1-yl)piperidin-1-yl)-[1,3'-biazetidine]-1'-carboxylate (87i)

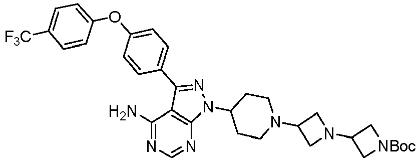

Tert-butyl 3-(4-(4-amino-3-iodo-1H-pyrazolo[3,4-d]pyrimidin-1-yl)piperidin-1-yl)-[1,3'-biazetidin]-1'-carboxylate (87e) (132 mg, 0.24 mmol), Pd(dppf)Cl₂ (9 mg, 0.012 mmol), potassium carbonate (100 mg, 0.72 mmol), and the above crude product (4-(4-(trifluoromethyl)phenoxy)phenyl )boronic acid (87h) (100 mg) were successively added to a three-necked flask. Nitrogen replacement was carried out three times, a mixed solvent of 10 mL of 1,4-dioxane and water (v/v) = 7 : 3 was added, and the reaction was warmed to 120°C and refluxed with stirring for 4 h. The reaction system was cooled to room temperature, and 30 mL of ethyl acetate and 30 mL of water were added. The organic phase was separated. The aqueous phase was extracted with ethyl acetate (30 mL × 3). The organic phase was combined, dried over anhydrous sodium sulfate, and concentrated under reduced pressure, and then the crude product was purified by silica gel column chromatographic column (dichloromethane/methanol (v/v) = 20 : 1), to obtain tert-butyl 3-(4-(4-amino-3-(4-(4-(trifluoromethyl)phenoxy)phenyl )-1H-pyrazolo[3,4-d]pyrimidin-1-yl)piperidin-1-yl)-[1,3'-biazetidin]-1'-carboxylate (87i) (100 mg, yield from compound 87e: 63%).
LCMS m/z = 665.3 [M+1]⁺.

### Step 8:

### 1-(1-([1,3'-biazetidin]-3-yl)piperidin-4-yl)-3-(4-(4-(trifluoromethyl)phenoxy)phenyl)-1H-pyrazolo[3,4-d]pyrimidin-4-amine (87j)

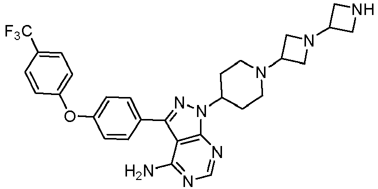

Tert-butyl 3-(4-(4-amino-3-(4-(4-(trifluoromethyl)phenoxy )phenyl)-1H-pyrazolo[3,4-d]pyrimidin-1-yl)pipeirdin-1-yl)-[1,3'-biazetidin]-1'-carboxylate (87i) (100 mg, 0.15 mmol) was dissolved in 10 mL of DCM, 2.0 mL of trifluoroacetic acid was added, and the reaction was stirred at room temperature for 4 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-(1-([1,3'-biazetidin]-3-yl)piperidin-4-yl)-3-(4-(4-(trifluoromethyl)phenoxy)phenyl)-1H-pyrazolo[3,4-d]pyrimidine-4-amine (87j) (85 mg).

### Step 9:

### 5-(3-(4-(4-amino-3-(4-(4-(trifluoromethyl)phenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 87)

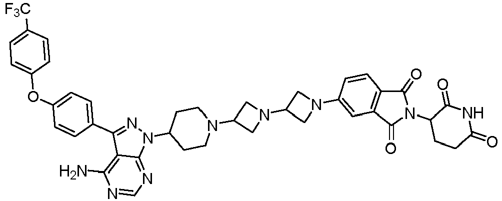

The above crude product 1-(1-([1,3'-biazetidin]-3-yl)piperidin-4-yl)-3-(4-(4-(trifluoromethyl)phenoxy)phenyl)-1H-pyrazolo[3,4-d]pyrimidine-4-amine (87j) (85 mg) was dissolved in 5 mL of DMSO, and 0.3 mL of DIPEA and 2-(2,6-dioxopiperidin-3-yl)-5-fluoroisoindoline-1,3-dione (see WO 2017197056 for the synthetic method) (42 mg, 0.15 mmol) were added, and the reaction was stirred at 80°C for 5 h. The reaction solution was cooled to room temperature, 5 mL of water was added, and the reaction solution was filtered. The filter cake was collected, washed with 20 mL of water. The solid was dissolved with 30 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(4-(trifluoromethyl)phenoxy)phenyl)-1H-pyrazolo[3,4-d]pyrimidin-1-yl)piperidin-1-yl]-[1,3'-biazetidin]-1'-yl)-2-(2,6-dioxopiperidin-3-yl)isoindoline-1,3-dione (compound 87) (20 mg, two-step yield from compound 87i: 16%).
¹H NMR (400 MHz, CDCl₃) δ 8.32 (s, 1H), 7.68 - 7.60 (m, 2H), 7.60 - 7.52 (m, 3H), 7.13 (d, 2H), 7.06 (d, 2H), 6.74 - 6.69 (m, 1H), 6.45 (dd, 1H), 5.59 (br.s, 2H), 4.85 (dd, 1H), 4.79 - 4.66 (m, 1H), 4.02 - 3.91 (m, 2H), 3.84 - 3.75 (m, 2H), 3.66 - 3.58 (m, 1H), 3.54 - 3.43 (m, 2H), 3.09 - 2.94 (m, 3H), 2.91 - 2.81 (m, 2H), 2.81 - 2.58 (m, 3H), 2.46 - 2.27 (m, 2H), 2.09 - 1.90 (m, 5H).
LCMS m/z = 821.3 [M+1]⁺.

### Example 88:

### 5-(3-(4-(4-amino-3-(4-(4-(trifluoromethyl)phenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 88)

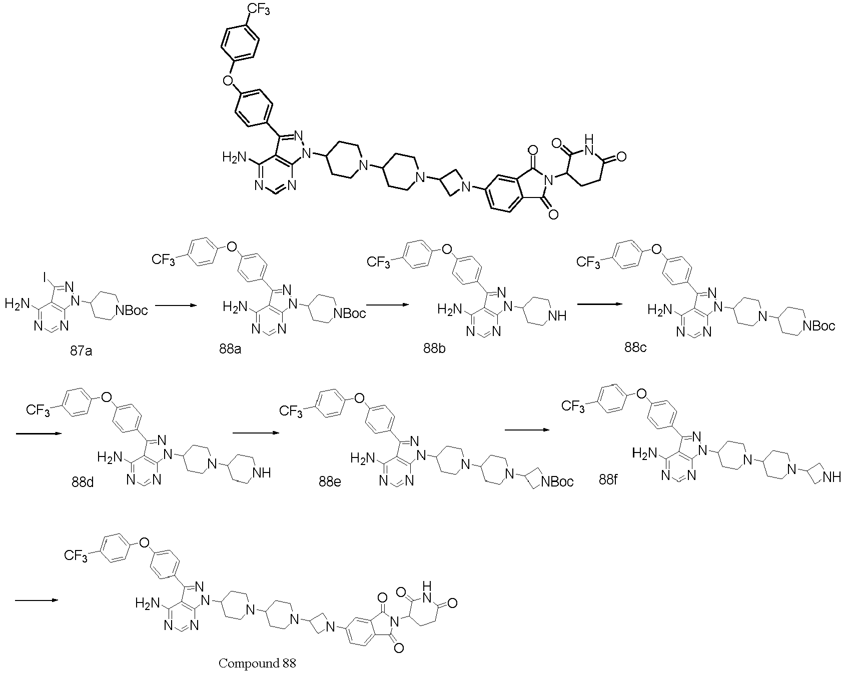

### Step 1:

### tert-butyl 4-(4-amino-3-(4-(4-(trifluoromethyl)phenoxy)phenyl)-1H-pyrazolo[3,4-d]pyrimidin-1-yl)piperidine-1-carboxylate (88a)

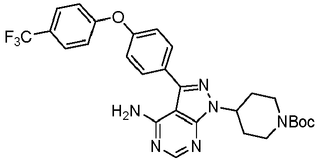

Tert-butyl 4-(4-amino-3-iodo-1H-pyrazolo[3,4-d]pyrimidin-1-yl)piperidin-1-carboxylate (87a) (see CN110627796 for the synthetic method) (0.88 g, 2 mmol), Pd(dppf)Cl₂ (73 mg, 0.1 mmol), potassium carbonate (0.83 g, 6.0 mmol), and the above crude product (4-(4-(trifluoromethyl)phenoxy)phenyl )boronic acid (87h) (0.62 g) were added to a three-necked flask. Nitrogen replacement was carried out three times, a mixed solvent of 22 mL of 1,4-dioxane and water (v/v) = 8 : 3 was added, and the reaction was warmed to 120°C and refluxed with stirring for 4 h. The reaction system was cooled to room temperature, and 50 mL of ethyl acetate and 50 mL of water were added. The organic phase was separated. The aqueous phase was extracted with ethyl acetate (50 mL × 3). The organic phase was combined, dried over anhydrous sodium sulfate, and concentrated under reduced pressure, and then the crude product was purified by silica gel column chromatography (dichloromethane/methanol (v/v) = 20 : 1), to obtain tert-butyl 4-(4-amino-3-(4-(4-(trifluoromethyl)phenoxy)phenyl)-1H-pyrazolo[3,4-d]pyrimidin-1-yl)piperidin-1-carboxylate (88a) (450 mg, yield from compound 87a: 41%).

### Step 2:

### 1-(piperidin-4-yl)-3-(4-(4-(trifluoromethyl)phenoxy)phenyl)-1H-pyrazolo[3,4-d]pyrimidin-4-amine (88b)

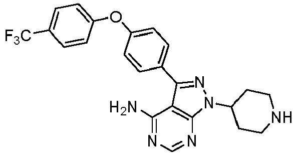

Tert-butyl 4-(4-amino-3-(4-(4-(trifluoromethyl)phenoxy )phenyl)-1H-pyrazolo[3,4-d]pyrimidin-1-yl)piperidine-1-carboxylate (88a) (450 mg, 0.81 mmol) was dissolved in 15 mL of DCM, 5.0 mL of trifluoroacetic acid was added, and the reaction was stirred at room temperature for 4 h. The reaction system was concentrated under reduced pressure. The residue was dissolved in 20 mL of DCM, and 20 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-(piperidin-4-yl)-3-(4-(4-(trifluoromethyl)phenoxy )phenyl)-1H-pyrazolo[3,4-d]pyrimidine-4-amine (88b) (370 mg).

### Step 3:

### tert-butyl 4-(4-amino-3-(4-(4-(trifluoromethyl)phenoxy)phenyl)-1H-pyrazolo[3,4-d] pyrimidin-1-yl)-[1,4'-bipiperidine]-1'-carboxylate (88c)

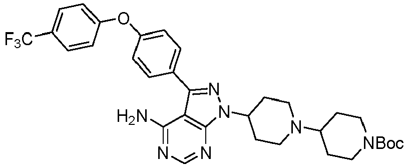

The above crude product 1-(piperidin-4-yl)-3-(4-(4-(trifluoromethyl)phenoxy)phenyl)-1H-pyrazolo[3,4-d]pyrimidine-4-amine (88b) (370 mg) was dissolved in 30 mL of DCE, and tert-butyl 4-oxopiperidine-1-carboxylate (320 mg, 1.61 mmol) and acetic acid (0.05 mL, 0.875 mmol) were added. The reaction was stirred at room temperature for 1 h, sodium triacetoxyborohydride (520 mg, 2.45 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 4-(4-amino-3-(4-(4-(trifluoromethyl)phenoxy)phenyl)-1H-pyrazolo[3,4-d]pyrimidin-1-yl)-[1,4'-bipiperidin]-1'-carboxylate (88c) (330 mg, two-step yield from compound 88a: 64%).
LCMS m/z = 638.3 [M+1]⁺.

### Step 4:

### 1-([1,4'-bipiperidin]-4-yl)-3-(4-(4-(trifluoromethyl)phenoxy)phenyl)-1H-pyrazolo[3,4-d]pyrimidin-4-amine (88d)

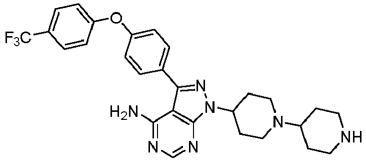

Tert-butyl 4-(4-amino-3-(4-(4-(trifluoromethyl)phenoxy)phenyl)-1H-pyrazolo[3,4-d]pyrimidin-1-yl)-[1,4'-bipiperidin]-1'-carboxylate (88c) (330 mg, 0.52 mmol) was dissolved in 20 mL of DCM, 8.0 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-([1,4'-bipiperidin]-4-yl)-3-(4-(4-(trifluoromethyl)phenoxy)phenyl)-1H-pyrazolo[3,4-d]pyrimidine-4-amine (88d) (280 mg).

### Step 5:

### tert-butyl 3-(4-(4-amino-3-(4-(4-(trifluoromethyl)phenoxy)phenyl)-1H-pyrazolo[3,4-d]pyrimidin-1-yl)-[1,4'-bipiperidin]-1'-yl)azetidine-1-carboxylate (88e)

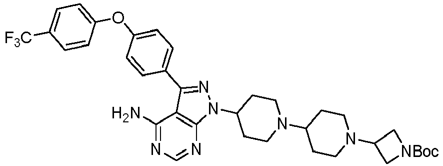

The above crude product 1-([1,4'-bipiperidin]-4-yl)-3-(4-(4-(trifluoromethyl)phenoxy)phenyl)-1H-pyrazolo[3,4-d]pyrimidine-4-amine (88d) (280 mg) was dissolved in 20 mL of DCE, and tert-butyl 3-oxoazetidine-1-carboxylate (178 mg, 1.04 mmol) and acetic acid (0.05 mL, 0.875 mmol) were added. The reaction was stirred at room temperature for 1 h, sodium triacetoxyborohydride (331 mg, 1.56 mmol) was added, and the reaction was stirred at room temperature for 16 h. The reaction was quenched by adding 20 mL of water to the reaction solution. The organic layer was separated. The aqueous layer was extracted with dichloromethane (30 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-(trifluoromethyl)phenoxy )phenyl)-1H-pyrazolo[3,4-d]pyrimidin-1-yl)-[1,4'-bipiperidin]-1'-yl)azetidine-1-carboxylate (88e) (100 mg, two-step yield from compound 88c: 28%).

### Step 6:

### 1-(1'-(azetidin-3-yl)-[1,4'-bipiperidin]-4-yl)-3 -(4-(4-(trifluoromethyl)phenoxy)phenyl)-1H-pyrazolo[3,4-d]pyrimidin-4-amine (88f)

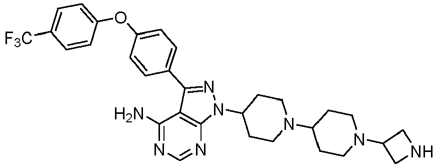

Tert-butyl 3-(4-(4-amino-3-(4-(4-(trifluoromethyl)phenoxy )phenyl)-1H-pyrazolo[3,4-d]pyrimidin-1-yl)-[1,4'-bipiperidin]-1'-yl)azetidine-1-carboxylate (88e) (100 mg, 0.14 mmol) was dissolved in 10 mL of DCM, 3 mL of trifluoroacetic acid was added, and the reaction was stirred at room temperature for 2 h. The reaction system was concentrated under reduced pressure. The residue was dissolved in 15 mL of DCM, and 15 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-(1'-(azetidin-3-yl)-[1,4'-bipiperidin]-4-yl)-3-(4-(4-(trifluoromethyl)phenoxy)phenyl)-1H-pyrazolo[3,4-d]pyrimidine-4-amine (88f) (83 mg).

### Step 7:

### 5-(3-(4-(4-amino-3-(4-(4-(trifluoromethyl)phenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 88)

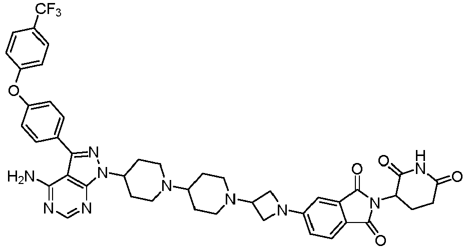

The above crude product 1-(1'-(azetidin-3-yl)-[1,4'-bipiperidin]-4-yl)-3-(4-(4-(trifluoromethyl)phenoxy)phenyl)-1H-pyrazolo[3,4-d]pyrimidine-4-amine (88f) (83 mg) was dissolved in 5 mL of DMSO, and 0.5 mL of DIPEA and 2-(2,6-dioxopiperidin-3-yl)-5-fluoroisoindoline-1,3-dione (see WO 2017197056 for the synthetic method) (43 mg, 0.16 mmol) were added, and the reaction was stirred at 80°C for 5 h. The reaction solution was cooled to room temperature, 5 mL of water was added, and the reaction solution was filtered. The filter cake was collected, washed with 20 mL of water. The solid was dissolved with 30 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(4-(trifluoromethyl)phenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 88) (81 mg, two-step yield from compound 88e: 68%).
¹H NMR (400 MHz, CDCl₃) δ 8.84 (s, 1H), 8.39 (s, 1H), 7.76 - 7.67 (m, 2H), 7.67 - 7.58 (m, 3H), 7.19 (d, 2H), 7.13 (d, 2H), 6.81 - 6.76 (m, 1H), 6.52 (dd, 1H), 5.61 (br.s, 2H), 4.93 (dd, 1H), 4.86 - 4.68 (m, 1H), 4.16 - 4.04 (m, 2H), 3.94 - 3.83 (m, 2H), 3.41 - 3.31 (m, 1H), 3.21 - 3.03 (m, 2H), 3.01 - 2.92 (m, 2H), 2.92 - 2.64 (m, 3H), 2.62 - 2.30 (m, 5H), 2.20 - 1.82 (m, 7H), 1.78 - 1.54 (m, 2H).
LCMS m/z = 849.3 [M+1]⁺.

### Example 89:

### 5-(3-(4-(4-amino-3-(4-(4-fluorophenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 89)

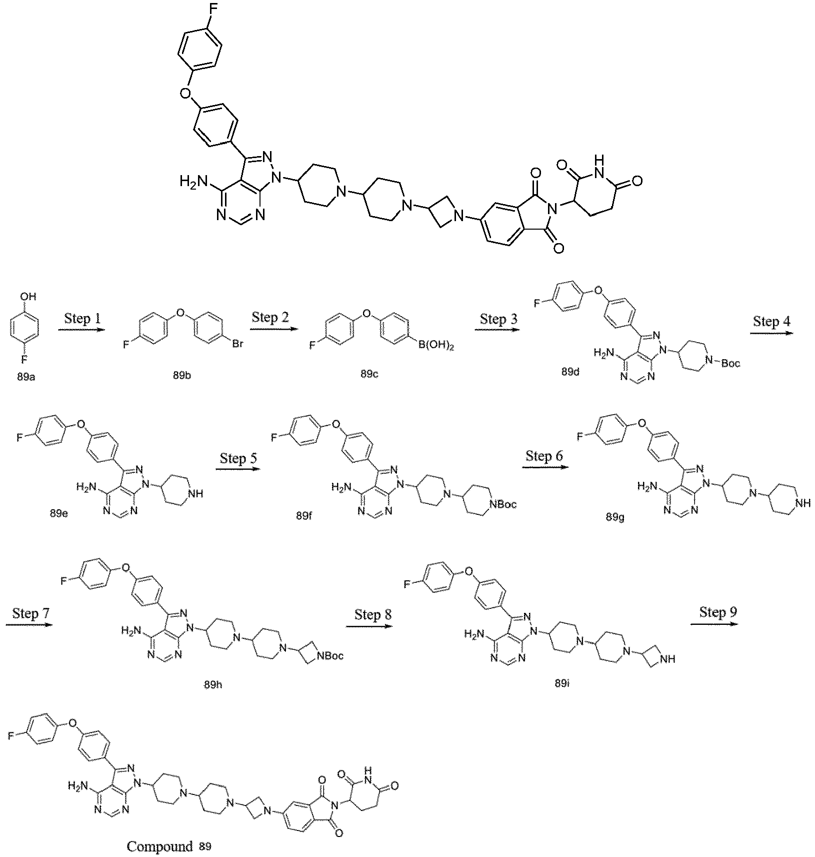

### Step 1:

### 1-bromo-4-(4-fluorophenoxy)benzene (89b)

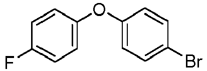

To a dry three-necked flask were successively added 4-fluorophenol (89a) (0.87 g, 7.8 mmol), 1-bromo-4-iodobenzene (2.0 g, 7.07 mmol), CuI (27 mg, 0.142 mmol), *N,N'*-dimethylglycine (55 mg, 0.53 mmol) and Cs₂CO₃ (4.6 g, 14.1 mmol). Nitrogen replacement was carried out 3 times, 50 mL of 1,4-dioxane was added, and the reaction was warmed to 90°C and stirred for 12 h. The reaction system was cooled to room temperature, and 50 mL of ethyl acetate and 50 mL of water were added. The organic layer was separated. The aqueous layer was extracted with ethyl acetate (50 mL × 3). The organic layer was combined, dried over anhydrous sodium sulfate, and concentrated under reduced pressure, and then the crude product was separated and purified by silica gel column chromatography (petroleum ether), to obtain 1-bromo-4-(4-fluorophenoxy)benzene (89b) (1.27 g, yield: 67%).

### Step 2:

### (4-(4-fluorophenoxy)phenyl)boronic acid (89c)

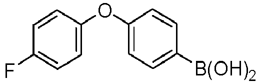

To a three-necked flask filled with nitrogen were added 1-bromo-4-(4-fluorophenoxy)benzene (89b) (0.5 g, 1.87 mmol) and 30 mL of dry tetrahydrofuran, the mixture was cooled to -78°C, and 2.5 mol/L n-butyllithium solution (1.12 mL) was slowly added dropwise. Upon completion of the addition, the reaction was carried out at -78°C for 30 min, and triisopropyl borate (0.52 mL, 2.24 mmol) was added dropwise, and the mixture was slowly warmed to room temperature, then reacted for 3 h. Upon completion of the reaction, the reaction was quenched with 2 mol/L hydrochloric acid solution (40 mL), and extracted with ethyl acetate (40 mL × 3). The organic phase was washed with saturated sodium chloride solution (40 mL), dried over anhydrous sodium sulfate, and concentrated under reduced pressure, to obtain crude product (4-(4-fluorophenoxy)phenyl)boronic acid (89c) (0.26 g).

### Step 3:

### tert-butyl 4-(4-amino-3-(4-(4-fluorophenoxy)phenyl)-1H-pyrazolo[3,4-d]pyrimidin-1-yl)piperidine-1-carboxylate (89d)

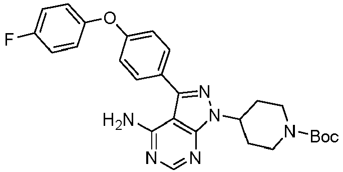

Tert-butyl 4-(4-amino-3-iodo-1H-pyrazolo[3,4-d]pyrimidin-1-yl)piperidin-1-carboxylate (87a) (see CN 110627796 for the synthetic method) (0.44 g, 1 mmol), Pd(dppf)Cl₂ (37 mg, 0.05 mmol), potassium carbonate (0.41 g, 3.0 mmol), and the above crude product (4-(4-fluorophenoxy)phenyl)boronic acid (89c) (0.26 g) were successively added to a three-necked flask. Nitrogen replacement was carried out 3 times, a mixed solvent of 10 mL of 1,4-dioxane and water (v/v) = 7 : 3 was added, and the reaction was warmed to 120°C and refluxed with stirring for 4 h. The reaction system was cooled to room temperature, and 30 mL of ethyl acetate and 30 mL of water were added. The organic phase was separated. The aqueous phase was extracted with ethyl acetate (30 mL × 3). The organic phase was combined, dried over anhydrous sodium sulfate, and concentrated under reduced pressure, and then the crude product was purified by silica gel column chromatography (dichloromethane/methanol (v/v) = 20 : 1), to obtain tert-butyl 4-(4-amino-3-(4-(4-fluorophenoxy)phenyl)-1H-pyrazolo[3,4-d]pyrimidin-1-yl)piperidine-1-carboxylate (89d) (340 mg, yield from compound 87a: 67%).
LCMS m/z = 505.2 [M+1]⁺.

### Step 4:

### 3-(4-(4-fluorophenoxy)phenyl)-1-(piperidin-4-yl)-1H-pyrazolo[3,4-d]pyrimidin-4-amine (89e)

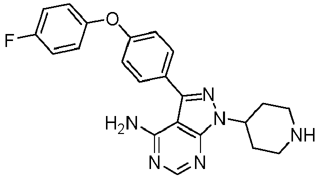

Tert-butyl 4-(4-amino-3-(4-(4-fluorophenoxy)phenyl)-1H-pyrazolo[3,4-d]pyrimidin-1-yl)piperidine-1-carboxylate (89d) (342 mg, 0.68 mmol) was dissolved in 10 mL of DCM, 2.0 mL of trifluoroacetic acid was added, and the reaction was stirred at room temperature for 4 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 3-(4-(4-fluorophenoxy)phenyl)-1-(piperidin-4-yl)-1H-pyrazolo[3,4-d]pyrimidine-4-amine (89e) (320 mg).

### Step 5:

### tert-butyl 4-(4-amino-3-(4-(4-fluorophenoxy)phenyl)-1H-pyrazolo[3,4-d]pyrimidin-1-yl)-[1,4'-bipiperidine]-1'-carboxylate (89f)

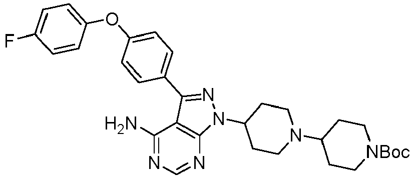

The above crude product 3-(4-(4-fluorophenoxy)phenyl)-1-(piperidin-4-yl)-1H-pyrazolo[3,4-d]pyrimidine-4-amine (89e) (320 mg) was dissolved in 20 mL of DCE, and tert-butyl 4-oxopiperidine-1-carboxylate (310 mg, 1.56 mmol) and acetic acid (0.05 mL, 0.875 mmol) were added. The reaction was stirred at room temperature for 1 h, sodium triacetoxyborohydride (500 mg, 2.36 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 4-(4-amino-3-(4-(4-fluorophenoxy)phenyl)-1H-pyrazolo[3,4-d]pyrimidin-1-yl)-[1,4'-bipiperidin]-1'-carboxylate (89f) (230 mg, two-step yield from compound 89d: 58%).

### Step 6:

### 1-([1,4'-bipiperidin]-4-yl)-3-(4-(4-fluorophenoxy)phenyl)-1H-pyrazolo[3,4-d]pyrimidin-4-amine (89g)

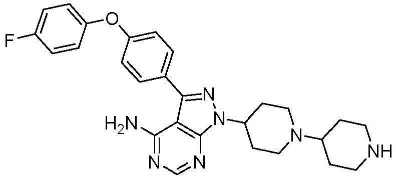

Tert-butyl 4-(4-amino-3-(4-(4-fluorophenoxy)phenyl)-1H-pyrazolo[3,4-d]pyrimidin-1-yl)-[1,4'-bipiperidin]-1'-carboxylate(89f) (226 mg, 0.38 mmol) was dissolved in 10 mL of DCM, 3.0 mL of trifluoroacetic acid was added, and the reaction was stirred at room temperature for 2 h. The reaction system was concentrated under reduced pressure. The residue was dissolved in 20 mL of DCM, and 20 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-([1,4'-bipiperidin]-4-yl)-3-(4-(4-fluorophenoxy)phenyl)-1H-pyrazolo[3,4-d]pyrimidin-4-amine (89g) (190 mg).

### Step 7:

### tert-butyl 3-(4-(4-amino-3-(4-(4-fluorophenoxy)phenyl)-1H-pyrazolo[3,4-d]pyrimidin-1-yl)-[1,4'-bipiperidin]-1'-yl)azetidine-1-carboxylate (89h)

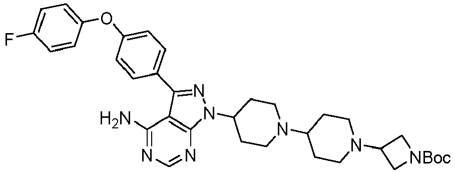

The above crude product 1-([1,4'-bipiperidin]-4-yl)-3-(4-(4-fluorophenoxy)phenyl)-1H-pyrazolo[3,4-d]pyrimidin-4-amine (89g) (190 mg) was dissolved in 20 mL of DCE, and tert-butyl 3-oxoazetidine-1-carboxylate (130 mg, 0.76 mmol) and acetic acid (0.05 mL, 0.875 mmol) were added. The reaction was stirred at room temperature for 1 h, sodium triacetoxyborohydride (240 mg, 1.13 mmol) was added, and the reaction was stirred at room temperature for 16 h. The reaction was quenched by adding 20 mL of water to the reaction solution. The organic layer was separated. The aqueous layer was extracted with dichloromethane (30 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-fluorophenoxy)phenyl)-1H-pyrazolo[3,4-d]pyrimidin-1-yl)-[1,4'-bipiperidin]-1'-yl)azetidine-1-carboxylate (89h) (120 mg, two-step yield from compound 89f: 49%).

### Step 8:

### 1-(1 '-(azeti din-3 -yl)-[1,4'-bipiperidin]-4-yl)-3-(4-(4-fluorophenoxy)phenyl)-1H-pyrazolo[3,4-d]pyrimidin-4-amine (89i)

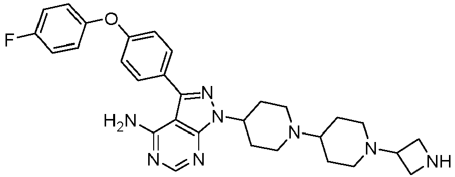

Tert-butyl 3-(4-(4-amino-3-(4-(4-fluorophenoxy)phenyl)-1H-pyrazolo[3,4-d]pyrimidin-1-yl)-[1,4'-bipiperidin]-1'-yl)azetidine-1-carboxylate (89h) (120 mg, 0.19 mmol) was dissolved in 10 mL of DCM, 3 mL of trifluoroacetic acid was added, and the reaction was stirred at room temperature for 2 h. The reaction system was concentrated under reduced pressure. The residue was dissolved in 15 mL of DCM, and 15 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-(1'-(azetidin-3-yl)-[1,4'-bipiperidin]-4-yl)-3-(4-(4-fluorophenoxy)phenyl)-1H-pyrazolo[3,4-d]pyrimidin-4-amine (89i) (100 mg).

### Step 9:

### 5-(3-(4-(4-amino-3-(4-(4-fluorophenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 89)

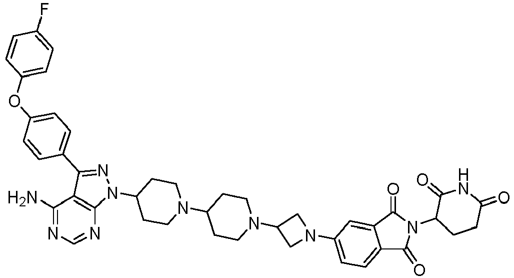

The above crude product 1-(1'-(azetidin-3-yl)-[1,4'-bipiperidin]-4-yl)-3-(4-(4-fluorophenoxy)phenyl)-1H-pyrazolo[3,4-d]pyrimidine-4-amine (89i) (100 mg) was dissolved in 5 mL of DMSO, and 0.5 mL of DIPEA and 2-(2,6-dioxopiperidin-3-yl)-5-fluoroisoindoline-1,3-dione (see WO 2017197056 for the synthetic method) (58 mg, 0.21 mmol) were added, and the reaction was stirred at 80°C for 5 h. The reaction solution was cooled to room temperature, 5 mL of water was added, and the reaction solution was filtered. The filter cake was collected, washed with 20 mL of water. The solid was dissolved with 30 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(4-fluorophenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 89) (81 mg, two-step yield from compound 89h: 53%).
¹H NMR (400 MHz, CDCl₃) δ 8.71 (s, 1H), 8.41 - 8.35 (m, 1H), 7.68 - 7.32 (m, 3H), 7.26 - 7.00 (m, 6H), 6.79 (d, 1H), 6.52 (dd, 1H), 5.59 (br.s, 2H), 4.93 (dd, 1H), 4.86 - 4.66 (m, 1H), 4.14 - 4.06 (m, 2H), 3.93 - 3.83 (m, 2H), 3.40 - 3.31 (m, 1H), 3.23 - 3.03 (m, 2H), 3.01 - 2.92 (m, 2H), 2.92 - 2.65 (m, 3H), 2.62 - 2.26 (m, 5H), 2.18 - 1.80 (m, 7H), 1.78 - 1.57 (m, 2H).
LCMS m/z = 799.3 [M+1]⁺.

### Example 90:

### 5-(3-(4-(4-amino-3-(4-(4-methoxyphenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 90)

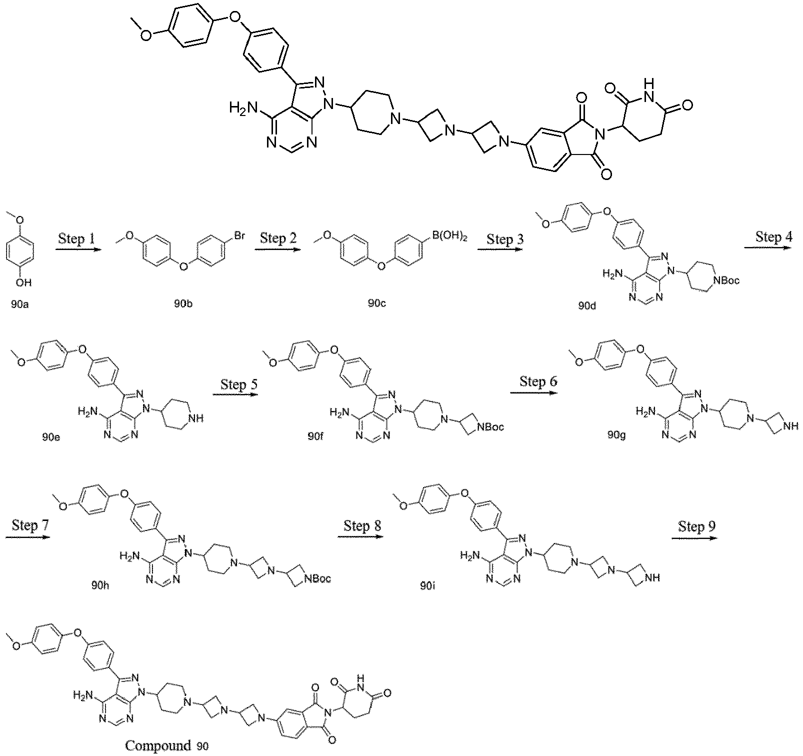

### Step 1:

### 1-bromo-4-(4-methoxyphenoxy)benzene (90b)

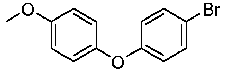

To a dry three-necked flask were successively added 4-methoxyphenol (90a) (2.41 g, 19.4 mmol), 1-bromo-4-iodobenzene (5.0 g, 17.67 mmol), CuI (67 mg, 0.35 mmol), *N,N'*-dimethylglycine (140 mg, 1.36 mmol) and Cs₂CO₃ (11.51 g, 35.33 mmol). Nitrogen replacement was carried out three times, then 50 mL of 1,4-dioxane was added, and the reaction system was warmed to 90°C and stirred for 12 h. The reaction system was cooled to room temperature, and 100 mL of ethyl acetate and 100 mL of water were added. The organic layer was separated. The aqueous layer was extracted with ethyl acetate (100 mL × 3). The organic layer was combined, dried over anhydrous sodium sulfate, and concentrated under reduced pressure, and then the crude product was separated and purified by silica gel column chromatography (petroleum ether), to obtain 1-bromo-4-(4-methoxyphenoxy)benzene (90b) (2.0 g, yield: 37%).

### Step 2:

### (4-(4-methoxyphenoxy)phenyl)boronic acid (90c)

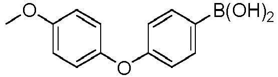

To a three-necked flask filled with nitrogen were added 1-bromo-4-(4-methoxyphenoxy)benzene (90b) (2.0 g, 7.17 mmol) and 30 mL of dry tetrahydrofuran, the reaction solution was cooled to -78°C, and 1.6 mol/L n-butyllithium solution (6.72 mL) was slowly added dropwise. Upon completion of the addition, the reaction was carried out at -78°C for 30 min, and triisopropyl borate (2.00 mL, 8.61 mmol) was added dropwise, and the reaction was slowly warmed to room temperature and stirred for 3 h. Upon completion of the reaction, the reaction was quenched with 2 mol/L hydrochloric acid solution (50 mL), and extracted with ethyl acetate (50 mL × 3). The organic phase was washed with saturated sodium chloride solution (50 mL), dried over anhydrous sodium sulfate, and concentrated under reduced pressure, to obtain crude product (4-(4-methoxyphenoxy)phenyl)boronic acid (90c) (0.94 g).

### Step 3:

### tert-butyl 4-(4-amino-3-(4-(4-methoxyphenoxy)phenyl)-1H-pyrazolo[3,4-d]pyrimidin-1-yl)piperidine-1-carboxylate (90d)

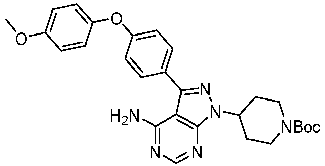

Tert-butyl 4-(4-amino-3-iodo-1H-pyrazolo[3,4-d]pyrimidin-1-yl)piperidin-1-carboxylate (87a) (see CN110627796 for the synthetic method) (1.55 g, 3.49 mmol), Pd(dppf)Cl₂ (130 mg, 0.18 mmol), potassium carbonate (1.45 g, 10.49 mmol), and the above crude product (4-(4-methoxyphenoxy)phenyl)boronic acid (90c) (0.94 g) were successively added to a three-necked flask. Nitrogen replacement was carried out three times, a mixed solvent of 50 mL of 1,4-dioxane and water (v/v) = 7 : 3 was added, and the reaction was warmed to 120°C and refluxed with stirring for 4 h. The reaction system was cooled to room temperature, and 100 mL of ethyl acetate and 100 mL of water were added. The organic phase was separated. The aqueous phase was extracted with ethyl acetate (100 mL × 3). The organic phase was combined, dried over anhydrous sodium sulfate, and concentrated under reduced pressure, and then the crude product was purified by silica gel column chromatography (dichloromethane/methanol (v/v) = 20 : 1), to obtain tert-butyl 4-(4-amino-3-(4-(4-methoxyphenoxy)phenyl)-1H-pyrazolo[3,4-d]pyrimidin-1-yl)piperidine-1-carboxylate (90d) (1.54 g, yield from compound 87a: 85%).

### Step 4:

### 3-(4-(4-methoxyphenoxy)phenyl)-1-(piperidin-4-yl)-1H-pyrazolo[3,4-d]pyrimidin-4-amine (90e)

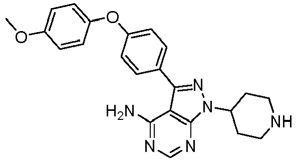

Tert-butyl 4-(4-amino-3-(4-(4-methoxyphenoxy)phenyl)-1H-pyrazolo[3,4-d]pyrimidin-1-yl)piperidine-1-carboxylate (90d) (1.54 g, 2.98 mmol) was dissolved in 30 mL of DCM, 11.0 mL of trifluoroacetic acid was added, and the reaction was stirred at room temperature for 4 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 3-(4-(4-methoxyphenoxy)phenyl)-1-(piperidin-4-yl)-1H-pyrazolo[3,4-d]pyrimidin-4-amine (90e) (600 mg).

### Step 5:

### tert-butyl 3-(4-(4-amino-3-(4-(4-methoxyphenoxy)phenyl)-1H-pyrazolo[3,4-d]pyrimidin-1-yl)piperidin-1-yl)azetidine-1 -carboxylate (90f)

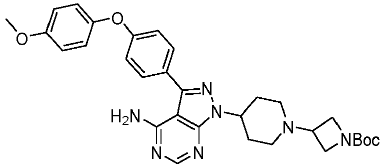

The above crude product 3-(4-(4-methoxyphenoxy)phenyl)-1-(piperidin-4-yl)-1H-pyrazolo[3,4-d]pyrimidin-4-amine (90e) (600 mg) was dissolved in 20 mL of DCE, and tert-butyl 3-oxoazetidine-1-carboxylate (490 mg, 2.87 mmol) and acetic acid (0.05 mL, 0.875 mmol) were added. The reaction was stirred at room temperature for 1 h, sodium triacetoxyborohydride (920 mg, 4.34 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-methoxyphenoxy)phenyl)-1H-pyrazolo[3,4-d]pyrimidin-1-yl)piperidin-1-yl)azetidine-1-carboxylate (90f) (800 mg, two-step yield from compound 90d: 47%).
LCMS m/z = 572.3 [M+1]⁺.

### Step 6:

### 1-(1-(azetidin-3-yl)piperidin-4-yl)-3-(4-(4-methoxyphenoxy)phenyl)-1H-pyrazolo[3,4-d]pyrimidin-4-amine (90g)

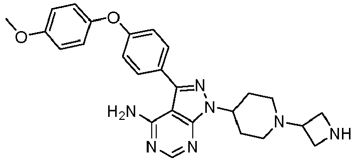

Tert-butyl 3-(4-(4-amino-3-(4-(4-methoxyphenoxy)phenyl)-1H-pyrazolo[3,4-d]pyrimidin-1-yl)piperidin-1-yl)azetidine-1-carboxylate (90f) (800 mg, 1.40 mmol) was dissolved in 20 mL of DCM, 7.0 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-(1-(azetidin-3-yl)piperidin-4-yl)-3-(4-(4-methoxyphenoxy)phenyl)-1H-pyrazolo[3,4-d]pyrimidin-4-amine (90g) (660 mg).
LCMS m/z = 472.2 [M+1]⁺.

### Step 7:

### tert-butyl 3-(4-(4-amino-3-(4-(4-methoxyphenoxy)phenyl)-1H-pyrazolo[3,4-d]pyrimidin-1-yl)piperidin-1-yl)-[1,3'-biazetidine]-1'-carboxylate (90h)

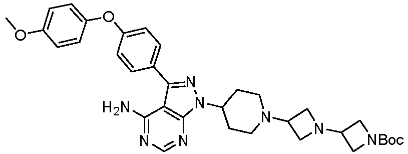

The above crude product 1-(1-(azetidin-3-yl)piperidin-4-yl)-3-(4-(4-methoxyphenoxy)phenyl)-1H-pyrazolo[3,4-d]pyrimidin-4-amine (90g) (660 mg) was dissolved in 30 mL of DCE, and tert-butyl 3-oxoazetidine-1-carboxylate (480 mg, 2.80 mmol) and acetic acid (0.05 mL, 0.875 mmol) were added. The reaction was stirred at room temperature for 1 h, sodium triacetoxyborohydride (890 mg, 4.20 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-methoxyphenoxy)phenyl)-1H-pyrazolo[3,4-d]pyrimidin-1-yl)piperidin-1-yl)-[1,3'-biazetidin]-1'-carboxylate (90h) (430 mg, two-step yield from compound 90f: 49%).

### Step 8:

### 1-(1-([1,3'-biazetidin]-3-yl)piperidin-4-yl)-3-(4-(4-methoxyphenoxy)phenyl)-1H-pyrazolo[3,4-d]pyrimidin-4-amine (90i)

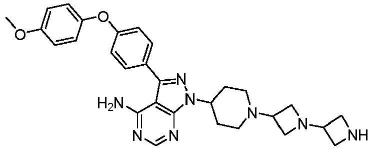

Tert-butyl 3-(4-(4-amino-3-(4-(4-methoxyphenoxy)phenyl)-1H-pyrazolo[3,4-d]pyrimidin-1-yl)piperidin-1-yl)-[1,3'-biazetidin]-1'-carboxylate (90h) (430 mg, 0.69 mmol) was dissolved in 20 mL of DCM, 5.5 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-(1-([1,3'-biazetidin]-3-yl)piperidin-4-yl)-3-(4-(4-methoxyphenoxy)phenyl)-1H-pyrazolo[3,4-d]pyrimidin-4-amine (90i) (320 mg).

### Step 9:

### 5-(3-(4-(4-amino-3-(4-(4-methoxyphenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 90)

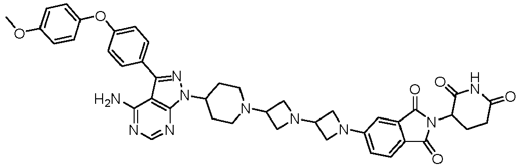

The above crude product 1-(1-([1,3'-biazetidin]-3-yl)piperidin-4-yl)-3-(4-(4-methoxyphenoxy)phenyl)-1H-pyrazolo[3,4-d]pyrimidin-4-amine (90i) (320 mg) was dissolved in 10 mL of DMSO, and 1 mL of DIPEA and 2-(2,6-dioxopiperidin-3-yl)-5-fluoroisoindoline-1,3-dione (see WO 2017197056 for the synthetic method) (190 mg, 0.69 mmol) were added, and the reaction was stirred at 80°C for 5 h. The reaction solution was cooled to room temperature, 20 mL of water was added, and the reaction solution was filtered. The filter cake was collected, washed with 50 mL of water. The solid was dissolved with 50 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(4-methoxyphenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 90) (200 mg, two-step yield from compound 90h: 37%).
¹H NMR (400 MHz, CDCl₃) δ 9.51 (s, 1H), 8.38 (s, 1H), 7.68 - 7.56 (m, 3H), 7.10 - 6.99 (m, 4H), 6.96 - 6.89 (m, 2H), 6.78 (d, 1H), 6.52 (dd, 1H), 5.70 (br.s, 2H), 4.92 (dd, 1H), 4.86 - 4.71 (m, 1H), 4.09 - 3.99 (m, 2H), 3.92 - 3.85 (m, 2H), 3.83 (s, 3H), 3.76 - 3.65 (m, 1H), 3.62 - 3.52 (m, 2H), 3.20 - 3.00 (m, 3H), 3.00 - 2.64 (m, 5H), 2.51 - 2.35 (m, 2H), 2.18 - 1.96 (m, 5H).
LCMS m/z = 783.3 [M+1]⁺.

### Example 91:

### 5-(3-(4-(4-amino-3-(4-(4-methoxyphenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 91)

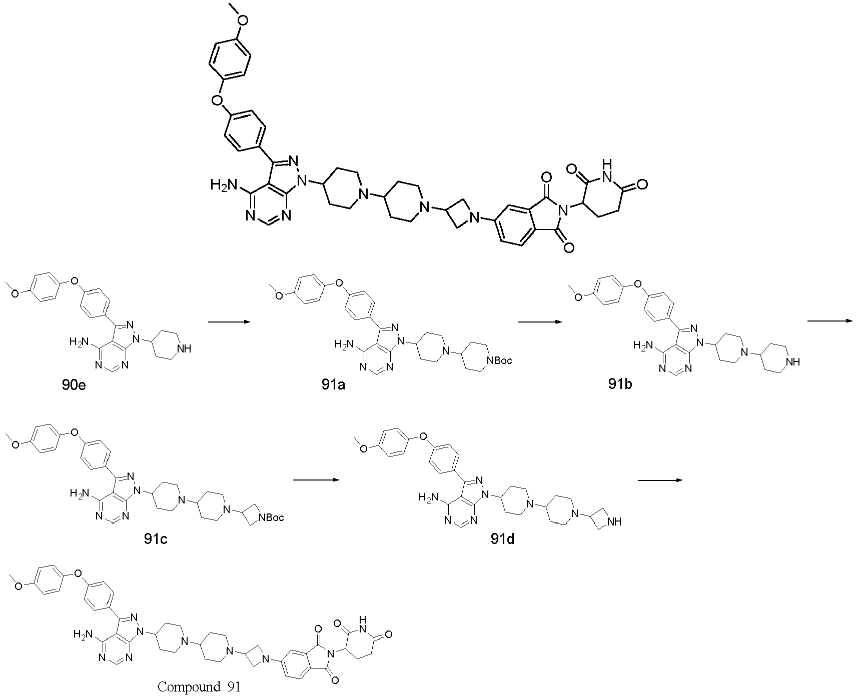

### Step 1:

### tert-butyl 4-(4-amino-3-(4-(4-methoxyphenoxy)phenyl)-1H-pyrazolo[3,4-d]pyrimidin-1-yl)-[1,4'-bipiperidine]-1'-carboxylate (91a)

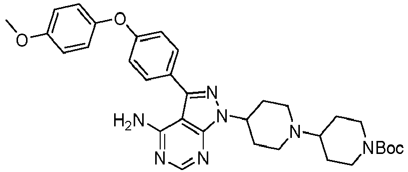

The above crude product 3-(4-(4-methoxyphenoxy)phenyl)-1-(piperidin-4-yl)-1H-pyrazolo[3,4-d]pyrimidin-4-amine (90e) (700 mg) was dissolved in 20 mL of DCE, and tert-butyl 4-oxopiperidine-1-carboxylate (670 mg, 3.37 mmol) and acetic acid (0.05 mL, 0.875 mmol) were added. The reaction was stirred at room temperature for 1 h, sodium triacetoxyborohydride (1.07 g, 5.05 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 4-(4-amino-3-(4-(4-methoxyphenoxy)phenyl)-1H-pyrazolo[3,4-d]pyrimidin-1-yl)-[1,4'-bipiperidin]-1'-carboxylate (91a) (330 mg, two-step yield from compound 90d: 16%).
LCMS m/z = 600.3 [M+1]⁺.

### Step 2:

### 1-([1,4'-bipiperidin]-4-yl)-3-(4-(4-methoxyphenoxy)phenyl)-1H-pyrazolo[3,4-d]pyrimidin-4-amine (91b)

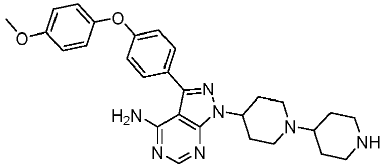

Tert-butyl 4-(4-amino-3-(4-(4-methoxyphenoxy)phenyl)-1H-pyrazolo[3,4-d]pyrimidin-1-yl)-[1,4'-bipiperidin]-1'-carboxylate (91a) (330 mg, 0.55 mmol) was dissolved in 10 mL of DCM, 3.0 mL of trifluoroacetic acid was added, and the reaction was stirred at room temperature for 2 h. The reaction solution was concentrated under reduced pressure. The residue was dissolved in 20 mL of DCM, and 20 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-([1,4'-bipiperidin]-4-yl)-3-(4-(4-methoxyphenoxy)phenyl)-1H-pyrazolo[3,4-d]pyrimidin-4-amine (91b) (280 mg).
LCMS m/z = 500.3 [M+1]⁺.

### Step 3:

### tert-butyl 3-(4-(4-amino-3-(4-(4-methoxyphenoxy)phenyl)-1H-pyrazolo[3,4-d]pyrimidin-1-yl)-[1,4'-bipiperidin]-1'-yl)azetidine-1-carboxylate (91c)

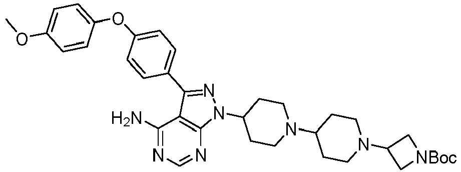

The crude product 1-([1,4'-bipiperidin]-4-yl)-3-(4-(4-methoxyphenoxy)phenyl)-1H-pyrazolo[3,4-d]pyrimidin-4-amine (91b) (280 mg) was dissolved in 30 mL of DCE, and tert-butyl 3-oxoazetidine-1-carboxylate (190 mg, 1.11 mmol) and acetic acid (0.05 mL, 0.875 mmol) were added. The reaction was stirred at room temperature for 1 h, sodium triacetoxyborohydride (360 mg, 1.70 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-methoxyphenoxy)phenyl)-1H-pyrazolo[3,4-d]pyrimidin-1-yl)-[1,4'-bipiperidin]-1'-yl)azetidine-1-carboxylate (91c) (360 mg, two-step yield from compound 91a: > 99%).

### Step 4:

### 1-(1'-(azetidin-3-yl)-[1,4'-bipiperidin]-4-yl)-3-(4-(4-methoxyphenoxy)phenyl)-1H-pyrazolo[3,4-d]pyrimidin-4-amine (91d)

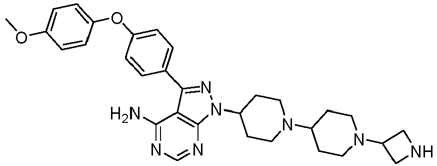

Tert-butyl 3-(4-(4-amino-3-(4-(4-methoxyphenoxy)phenyl)-1H-pyrazolo[3,4-d]pyrimidin-1-yl)-[1,4'-bipiperidin] -1'-yl)azetidine-1-carboxylate (91c) (360 mg, 0.55 mmol) was dissolved in 20 mL of DCM, 8.2 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-(1'-(azetidin-3-yl)-[1,4'-bipiperidin]-4-yl)-3-(4-(4-methoxyphenoxy)phenyl)-1H-pyrazolo[3,4-d]pyrimidin-4-amine (91d) (310 mg).

### Step 5:

### 5-(3-(4-(4-amino-3-(4-(4-methoxyphenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 91)

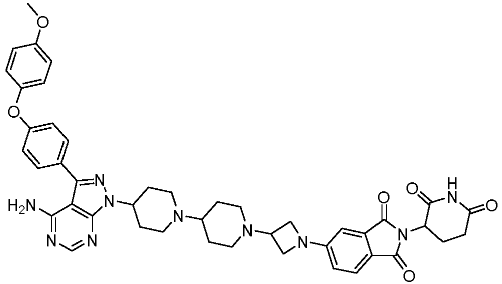

The above crude product 1-(1'-(azetidin-3-yl)-[1,4'-bipiperidin]-4-yl)-3-(4-(4-methoxyphenoxy)phenyl)-1H-pyrazolo[3,4-d]pyrimidin-4-amine (91d) (310 mg) was dissolved in 10 mL of DMSO, and 1 mL of DIPEA and 2-(2,6-dioxopiperidin-3-yl)-5-fluoroisoindoline-1,3-dione (see WO 2017197056 for the synthetic method) (170 mg, 0.62 mmol) were added, and the reaction was stirred at 80°C for 5 h. The reaction solution was cooled to room temperature, 10 mL of water was added, and the reaction solution was filtered. The filter cake was collected, washed with 30 mL of water. The solid was dissolved with 50 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(4-methoxyphenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline -1,3-dione (compound 91) (130 mg, two-step yield from compound 91c: 29%).
¹H NMR (400 MHz, CDCl₃) δ 8.56 (s, 1H), 8.37 (s, 1H), 7.70 - 7.55 (m, 3H), 7.12 - 6.98 (m, 4H), 6.96 - 6.89 (m, 2H), 6.79 (d, 1H), 6.52 (dd, 1H), 5.54 (br.s, 2H), 4.93 (dd, 1H), 4.84 - 4.67 (m, 1H), 4.15 - 4.05 (m, 2H), 3.94 - 3.84 (m, 2H), 3.83 (s, 3H), 3.40 - 3.32 (m, 1H), 3.21 - 3.03 (m, 2H), 3.02 - 2.65 (m, 5H), 2.60 - 2.30 (m, 5H), 2.18 - 1.83 (m, 7H), 1.79 - 1.55 (m, 2H).
LCMS m/z = 811.4 [M+1]⁺.

### Example 92:

### 5-(3-(4-(4-amino-3-(4-(2-fluorophenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 92)

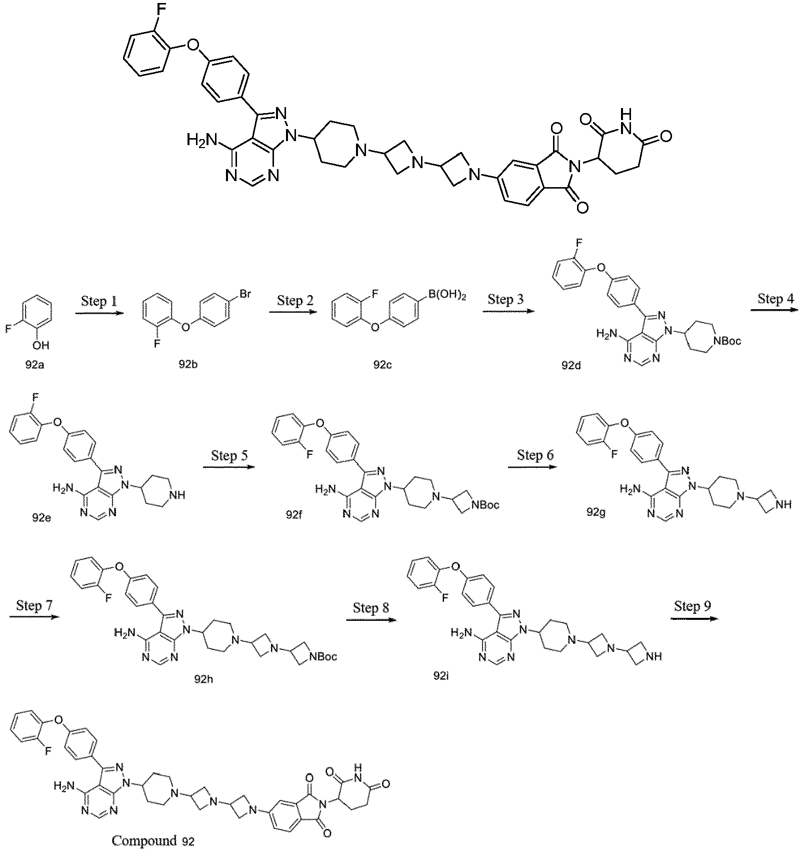

### Step 1:

### 1-(4-bromophenoxy)-2-fluorobenzene (92b)

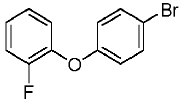

To a dry three-necked flask were successively added 2-fluorophenol (92a) (2.38 g, 21.23 mmol), 1-bromo-4-iodobenzene (4.0 g, 14.14 mmol), CuI (54 mg, 0.28 mmol), *N,N'*-dimethylglycine (110 mg, 1.07 mmol) and Cs₂CO₃ (9.2 g, 28.24 mmol). Nitrogen replacement was carried out three times, then 50 mL of 1,4-dioxane was added, and the reaction was warmed to 90°C and stirred for 12 h. The reaction system was cooled to room temperature, and 50 mL of ethyl acetate and 50 mL of water were added. The organic layer was separated. The aqueous layer was extracted with ethyl acetate (50 mL × 3). The organic layer was combined, dried over anhydrous sodium sulfate, and concentrated under reduced pressure, and then the crude product was separated and purified by silica gel column chromatography (petroleum ether), to obtain 1-(4-bromophenoxy)-2-fluorobenzene (92b) (1.23 g, yield: 33%).

### Step 2:

### (4-(2-fluorophenoxy)phenyl)boronic acid (92c)

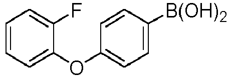

To a three-necked flask filled with nitrogen were added 1-(4-bromophenoxy)-2-fluorobenzene (92b) (2.3 g, 8.61 mmol) and 30 mL of dry tetrahydrofuran, the mixture was cooled to -78°C, and 1.6 mol/L n-butyllithium solution (8 mL) was slowly added dropwise. Upon completion of the addition, the reaction was carried out at -78°C for 30 min, and triisopropyl borate (2.4 mL, 10.33 mmol) was added dropwise, and the mixture was slowly warmed to room temperature and reacted for 3 h. Upon completion of the reaction, the reaction was quenched with 2 mol/L hydrochloric acid solution (50 mL), and extracted with ethyl acetate (50 mL × 3). The organic phase was washed with saturated sodium chloride solution (40 mL), dried over anhydrous sodium sulfate, and concentrated under reduced pressure, to obtain crude product (4-(2-fluorophenoxy)phenyl)boronic acid (92c) (890 mg).

### Step 3:

### tert-butyl 4-(4-amino-3-(4-(2-fluorophenoxy)phenyl)-1H-pyrazolo[3,4-d]pyrimidin-1-yl)piperidine-1-carboxylate (92d)

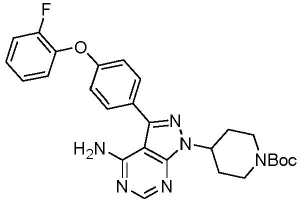

Tert-butyl 4-(4-amino-3-iodo-1H-pyrazolo[3,4-d]pyrimidin-1-yl)piperidin-1-carboxylate (87a) (see CN110627796 for the synthetic method) (1.55 g, 3.49 mmol), Pd(dppf)Cl₂ (130 mg, 0.18 mmol), potassium carbonate (1.45 g, 10.49 mmol), and the above crude product (4-(2-fluorophenoxy)phenyl)boronic acid (92c) (890 mg) were successively added to a three-necked flask. Nitrogen replacement was carried out three times, a mixed solvent of 50 mL of 1,4-dioxane and water (v/v) = 7 : 3 was added, and the reaction was warmed to 120°C and refluxed with stirring for 4 h. The reaction system was cooled to room temperature, and 100 mL of ethyl acetate and 100 mL of water were added. The organic phase was separated. The aqueous phase was extracted with ethyl acetate (100 mL × 3). The organic phase was combined, dried over anhydrous sodium sulfate, and concentrated under reduced pressure, and then the crude product was purified by silica gel column chromatography (dichloromethane/methanol (v/v) = 20 : 1), to obtain tert-butyl 4-(4-amino-3-(4-(2-fluorophenoxy)phenyl)-1H-pyrazolo[3,4-d]pyrimidin-1-yl)piperidine-1-carboxylate (92d) (1.6 g, yield from compound 87a: 91%).

### Step 4:

### 3-(4-(2-fluorophenoxy)phenyl)-1-(piperidin-4-yl)-1H-pyrazolo[3,4-d]pyrimidin-4-amine (92e)

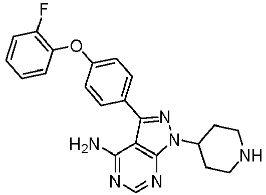

Tert-butyl 4-(4-amino-3-(4-(2-fluorophenoxy)phenyl)-1H-pyrazolo[3,4-d]pyrimidin-1-yl)piperidine-1-carboxylate (92d) (1.6 g, 3.17 mmol) was dissolved in 30 mL of DCM, 10.0 mL of trifluoroacetic acid was added, and the reaction was stirred at room temperature for 4 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (500 mL × 4). The organic layer was combined, dried over anhydrous sodium sulfate, and concentrated under reduced pressure, to obtain crude product 3-(4-(2-fluorophenoxy)phenyl)-1-(piperidin-4-yl)-1H-pyrazolo[3,4-d]pyrimidine-4-amine (92e) (700 mg).

### Step 5:

### tert-butyl 3-(4-(4-amino-3-(4-(2-fluorophenoxy)phenyl)-1H-pyrazolo[3,4-d]pyrimidin-1-yl)piperidin-1-yl)azetidine-1 -carboxylate (92f)

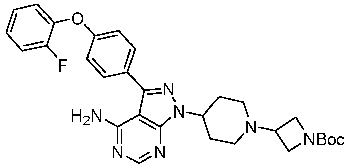

The above crude product 3-(4-(2-fluorophenoxy)phenyl)-1-(piperidin-4-yl)-1H-pyrazolo[3,4-d]pyrimidin-4-amine (92e) (700 mg) was dissolved in 30 mL of DCE, and tert-butyl 3-oxoazetidine-1-carboxylate (590 mg, 3.45 mmol) and acetic acid (0.05 mL, 0.875 mmol) were added. The reaction was stirred at room temperature for 1 h, sodium triacetoxyborohydride (1.10 g, 5.19 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(2-fluorophenoxy)phenyl)-1H-pyrazolo[3,4-d]pyrimidin-1-yl)piperidin-1-yl)azetidine-1-carboxylate (92f) (600 mg, two-step yield from compound 92d: 34%).
LCMS m/z = 560.2 [M+1]⁺.

### Step 6:

### 1-(1-(azetidin-3-yl)piperidin-4-yl)-3-(4-(2-fluorophenoxy)phenyl)-1H-pyrazolo[3,4-d]pyrimidin-4-amine (92g)

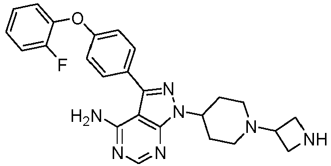

Tert-butyl 3-(4-(4-amino-3-(4-(2-fluorophenoxy)phenyl)-1H-pyrazolo[3,4-d]pyrimidin-1-yl)piperidin-1-yl)azetidine-1-carboxylate (92f) (600 mg, 1.07 mmol) was dissolved in 30 mL of DCM, 10.0 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 4). The organic layer was combined, dried over anhydrous sodium sulfate, and concentrated under reduced pressure, to obtain crude product 1-(1-(azetidin-3-yl)piperidin-4-yl)-3-(4-(2-fluorophenoxy)phenyl)-1H-pyrazolo[3,4-d]pyrimidin-4-amine (92g) (490 mg).

### Step 7:

### tert-butyl 3-(4-(4-amino-3-(4-(2-fluorophenoxy)phenyl)-1H-pyrazolo[3,4-d]pyrimidin-1-yl)piperidin-1-yl)-[1,3'-biazetidin]-1'-carboxylate (92h)

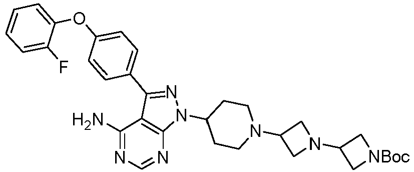

The above crude product 1-(1-(azetidin-3-yl)piperidin-4-yl)-3-(4-(2-fluorophenoxy)phenyl)-1H-pyrazolo[3,4-d]pyrimidin-4-amine (92g) (490 mg) was dissolved in 30 mL of DCE, and tert-butyl 3-oxoazetidine-1-carboxylate (370 mg, 2.16 mmol) and acetic acid (0.05 mL, 0.875 mmol) were added. The reaction was stirred at room temperature for 1 h, sodium triacetoxyborohydride (680 mg, 3.21 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(2-fluorophenoxy)phenyl)-1H-pyrazolo[3,4-d]pyrimidin-1-yl)piperidin-1-yl)-[1,3'-biazetidin]-1'-carboxylate (92h) (600 mg, two-step yield from compound 92f: 91%).
LCMS m/z = 615.4 [M+1]⁺.

### Step 8:

### 1-(1-([1,3'-biazetidin]-3-yl)piperidin-4-yl)-3-(4-(2-fluorophenoxy)phenyl)-1H-pyrazolo[3,4-d]pyrimidin-4-amine (92i)

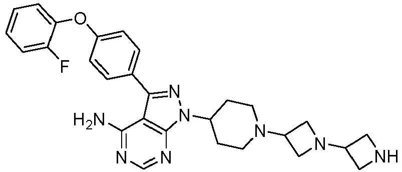

Tert-butyl 3-(4-(4-amino-3-(4-(2-fluorophenoxy)phenyl)-1H-pyrazolo[3,4-d]pyrimidin-1-yl)piperidin-1-yl)-[1,3'-biazetidin]-1'-carboxylate (92h) (600 mg, 0.98 mmol) was dissolved in 20 mL of DCM, 7 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 4). The organic layer was combined, dried over anhydrous sodium sulfate, and concentrated under reduced pressure, to obtain crude product 1-(1-([1,3'-biazetidin]-3-yl)piperidin-4-yl)-3-(4-(2-fluorophenoxy)phenyl)-1H-pyrazolo[3,4-d]pyrimidin-4-amine (92i) (450 mg).

### Step 9:

### 5-(3-(4-(4-amino-3-(4-(2-fluorophenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 92)

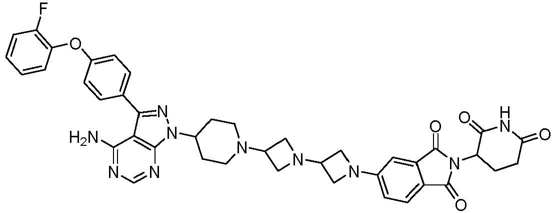

The above crude product 1-(1-([1,3'-biazetidin]-3-yl)piperidin-4-yl)-3-(4-(2-fluorophenoxy)phenyl)-1H-pyrazolo[3,4-d]pyrimidin-4-amine (92i) (450 mg) was dissolved in 10 mL of DMSO, and 1 mL of DIPEA and 2-(2,6-dioxopiperidin-3-yl)-5-fluoroisoindoline-1,3-dione (see WO 2017197056 for the synthetic method) (260 mg, 0.94 mmol) were added, and the reaction was stirred at 80°C for 5 h. The reaction solution was cooled to room temperature, 20 mL of water was added, and the reaction solution was filtered. The filter cake was collected, washed with 50 mL of water. The solid was dissolved with 50 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(2-fluorophenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 92) (215 mg, two-step yield from compound 92h: 28%).
¹H NMR (400 MHz, CDCl₃) δ 8.70 (s, 1H), 8.38 (s, 1H), 7.68 - 7.30 (m, 3H), 7.28 - 7.02 (m, 6H), 6.78 (d, 1H), 6.53 (dd, 1H), 5.55 (br.s, 2H), 4.92 (dd, 1H), 4.87 - 4.71 (m, 1H), 4.08 - 4.00 (m, 2H), 3.96 - 3.83 (m, 2H), 3.78 - 3.65 (m, 1H), 3.64 - 3.52 (m, 2H), 3.25 - 3.03 (m, 3H), 3.03 - 2.63 (m, 5H), 2.54 - 2.36 (m, 2H), 2.24 - 1.94 (m, 5H).
LCMS m/z = 771.3 [M+1]⁺.

### Example 93:

### 5-(3-(4-(4-amino-3-(4-(2-fluorophenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 93)

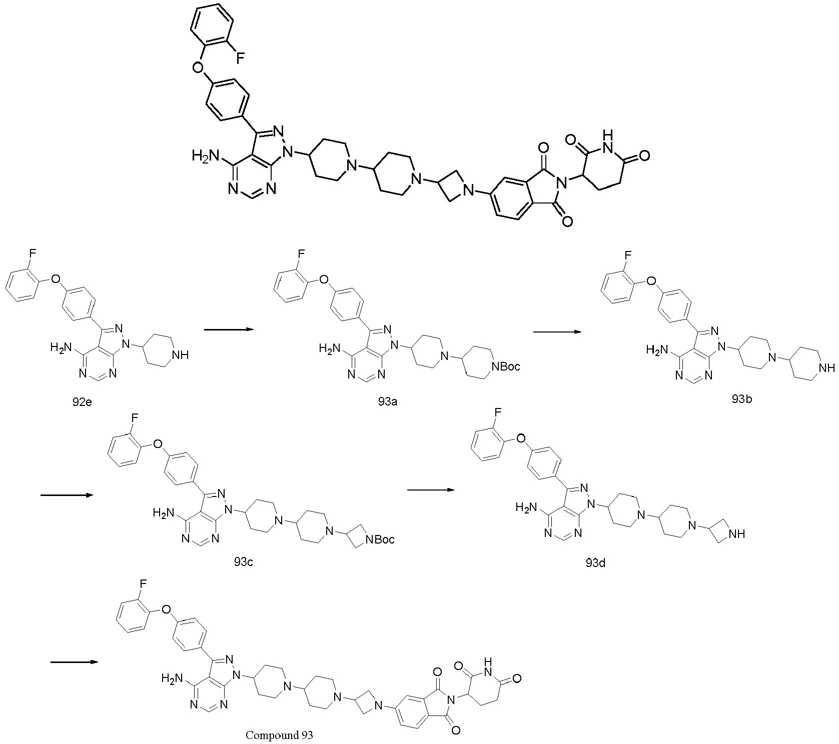

### Step 1:

### tert-butyl 4-(4-amino-3-(4-(2-fluorophenoxy)phenyl)-1H-pyrazolo[3,4-d]pyrimidin-1-yl)-[1,4'-bipiperidine]-1'-carboxylate (93a)

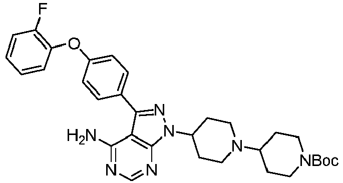

The above crude product 3-(4-(2-fluorophenoxy)phenyl)-1-(piperidin-4-yl)-1H-pyrazolo[3,4-d]pyrimidine-4-amine (92e) (700 mg) was dissolved in 20 mL of DCE, and tert-butyl 4-oxopiperidine-1-carboxylate (690 mg, 3.46 mmol) and acetic acid (0.05 mL, 0.875 mmol) were added. The reaction was stirred at room temperature for 1 h, sodium triacetoxyborohydride (1.10 g, 5.19 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 4-(4-amino-3-(4-(2-fluorophenoxy)phenyl)-1H-pyrazolo[3,4-d]pyrimidin-1-yl)-[1,4'-bipiperidin]-1'-carboxylate (93a) (560 mg, two-step yield from compound 92d: 30%).
LCMS m/z = 588.3 [M+1]⁺.

### Step 2:

### 1-([1,4'-bipiperidin]-4-yl)-3-(4-(2-fluorophenoxy)phenyl)-1H-pyrazole[3,4-d]pyrimidin-4-amine (93b)

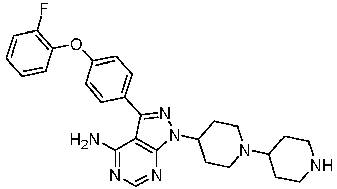

Tert-butyl 4-(4-amino-3-(4-(2-fluorophenoxy)phenyl)-1H-pyrazolo[3,4-d]pyrimidin-1-yl)-[1,4'-bipiperidin]-1'-carboxylate(93a) (560 mg, 0.95 mmol) was dissolved in 30 mL of DCM, 10.0 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-([1,4'-bipiperidin]-4-yl)-3-(4-(2-fluorophenoxy)phenyl)-1H-pyrazole[3,4-d]pyrimidin-4-amine (93b) (460 mg).

### Step 3:

### tert-butyl 3-(4-(4-amino-3-(4-(2-fluorophenoxy)phenyl)-1H-pyrazolo[3,4-d]pyrimidin-1-yl)-[1,4'-bipiperidin]-1'-yl)azetidine-1-carboxylate (93c)

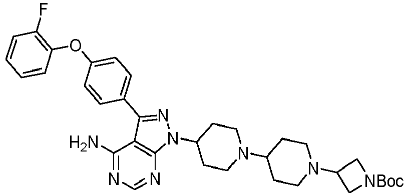

The above crude product 1-([1,4'-bipiperidin]-4-yl)-3-(4-(2-fluorophenoxy)phenyl)-1H-pyrazole[3,4-d]pyrimidin-4-amine (93b) (460 mg) was dissolved in 30 mL of DCE, and tert-butyl 3-oxoazetidine-1-carboxylate (330 mg, 1.9 mmol) and acetic acid (0.05 mL, 0.875 mmol) were added. The reaction was stirred at room temperature for 1 h, sodium triacetoxyborohydride (600 mg, 2.83 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(2-fluorophenoxy)phenyl)-1H-pyrazolo[3,4-d]pyrimidin-1-yl)-[1,4'-bipiperidin]-1'-yl)azetidine-1-carboxylate (93c) (530 mg, two-step yield from compound 93a: 87%).
LCMS m/z = 643.4 [M+1]⁺.

### Step 4:

### 1-(1'-(azetidin-3-yl)-[1,4'-bipiperidin]-4-yl)-3-(4-(2-fluorophenoxy)phenyl)-1H-pyrazolo[3,4-d]pyrimidin-4-amine (93d)

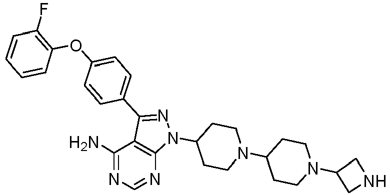

Tert-butyl 3-(4-(4-amino-3-(4-(2-fluorophenoxy)phenyl)-1H-pyrazolo[3,4-d]pyrimidin-1-yl)-[1,4'-bipiperidin]-1'-yl)azetidine-1-carboxylate (93c) (500 mg, 0.78 mmol) was dissolved in 30 mL of DCM, 8.2 mL of trifluoroacetic acid was added, and the reaction was stirred at room temperature for 2 h. The reaction system was concentrated under reduced pressure. The residue was dissolved in 30 mL of DCM, and 30 mL of water was added. The pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 1-(1'-(azetidin-3-yl)-[1,4'-bipiperidin]-4-yl)-3-(4-(2-fluorophenoxy)phenyl)-1H-pyrazolo[3,4-d]pyrimidin-4-amine (93d) (420 mg).

### Step 5:

### 5-(3-(4-(4-amino-3-(4-(2-fluorophenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 93)

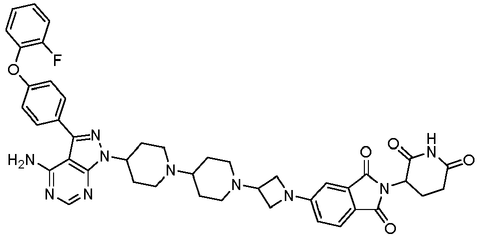

The above crude product 1-(1'-(azetidin-3-yl)-[1,4'-bipiperidin]-4-yl)-3-(4-(2-fluorophenoxy)phenyl)-1H-pyrazolo[3,4-d]pyrimidine-4-amine (93d) (420 mg) was dissolved in 10 mL of DMSO, and 1 mL of DIPEA and 2-(2,6-dioxopiperidin-3-yl)-5-fluoroisoindoline-1,3-dione (see WO 2017197056 for the synthetic method) (240 mg, 0.87 mmol) were added, and the reaction was stirred at 80°C for 5 h. The reaction solution was cooled to room temperature, 10 mL of water was added, and the reaction solution was filtered. The filter cake was collected, washed with 30 mL of water. The solid was dissolved with 50 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(2-fluorophenoxy)phenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 93) (130 mg, two-step yield from compound 93c: 21%).
¹H NMR (400 MHz, CDCl₃) δ 8.82 (s, 1H), 8.38 (s, 1H), 7.70 - 7.30 (m, 3H), 7.28 - 6.99 (m, 6H), 6.78 (d, 1H), 6.52 (dd, 1H), 5.59 (br.s, 2H), 4.93 (dd, 1H), 4.85 - 4.67 (m, 1H), 4.16 - 4.04 (m, 2H), 3.93 - 3.82 (m, 2H), 3.41 - 3.31 (m, 1H), 3.28 - 3.03 (m, 2H), 3.03 - 2.92 (m, 2H), 2.92 - 2.65 (m, 3H), 2.65 - 2.25 (m, 5H), 2.18 - 1.84 (m, 7H), 1.77 - 1.57 (m, 2H).
LCMS m/z = 799.3 [M+1]⁺.

### Example 94:

### 5-(3-(4-(4-amino-3-(4-(4-fluorophenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 94)

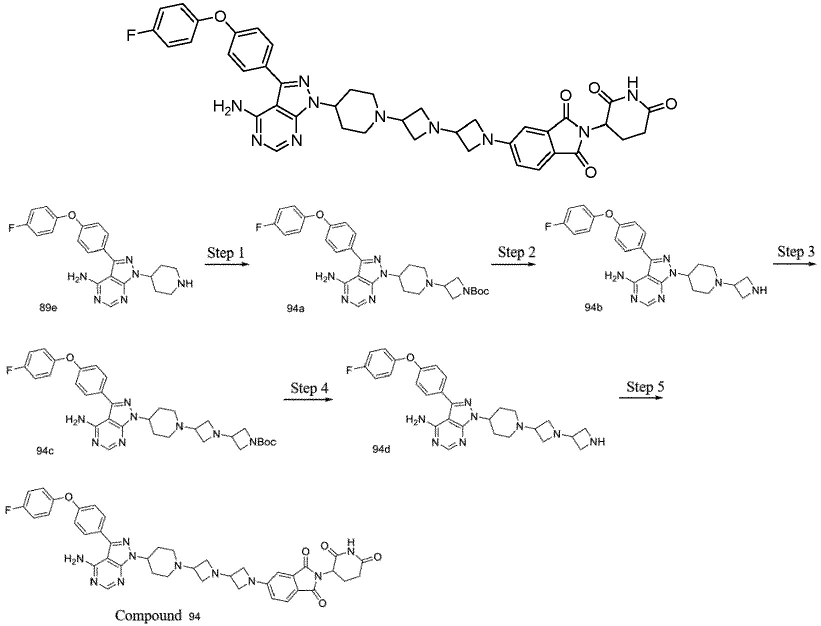

### Step 1:

### tert-butyl 3-(4-(4-amino-3-(4-(4-fluorophenoxy)phenyl)-1H-pyrazolo[3,4-d]pyrimidin-1-yl)piperidin-1-yl)azetidine-1-carboxylate (94a)

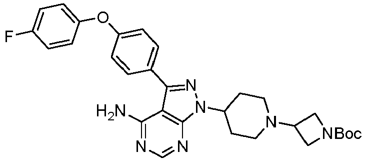

The above crude product 3-(4-(4-fluorophenoxy)phenyl)-1-(piperidin-4-yl)-1H-pyrazolo[3,4-d]pyrimidin-4-amine (89e) (280 mg) was dissolved in 10 mL of DCE, and tert-butyl 3-oxoazetidine-1-carboxylate (240 mg, 1.40 mmol) and acetic acid (0.05 mL, 0.875 mmol) were added. The reaction was stirred at room temperature for 1 h, sodium triacetoxyborohydride (430 mg, 2.03 mmol) was added, and the reaction was stirred at room temperature for 16 h. The reaction was quenched by adding 10 mL of water to the reaction solution. The organic layer was separated. The aqueous layer was extracted with dichloromethane (30 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-fluorophenoxy)phenyl)-1H-pyrazolo[3,4-d]pyrimidin-1-yl)piperidin-1-yl)azetidine-1-carboxylate (94a) (170 mg, two-step yield from compound 89d: 51%).
LCMS m/z = 560.2 [M+1]⁺.

### Step 2:

### 1-(1-(azetidin-3-yl)piperidin-4-yl)-3-(4-(4-fluorophenoxy)phenyl)-1H-pyrazolo[3,4-d]pyrimidin-4-amine (94b)

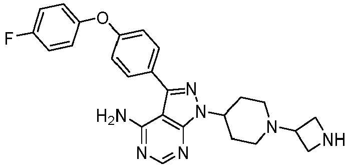

Tert-butyl 3-(4-(4-amino-3-(4-(4-fluorophenoxy)phenyl)-1H-pyrazolo[3,4-d]pyrimidin-1-yl)piperidin-1-yl)azetidine-1-carboxylate (94a) (170 mg, 0.30 mmol) was dissolved in 5 mL of DCM, 1.0 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (20 mL × 3). The organic layer was combined, dried over anhydrous sodium sulfate, and concentrated under reduced pressure, to obtain crude product 1-(1-(azetidin-3-yl)piperidin-4-yl)-3-(4-(4-fluorophenoxy)phenyl)-1H-pyrazolo[3,4-d]pyrimidin-4-amine (94b) (124 mg).
LCMS m/z = 460.2 [M+1]⁺.

### Step 3:

### tert-butyl 3-(4-(4-amino-3-(4-(4-fluorophenoxy)phenyl)-1H-pyrazolo[3,4-d]pyrimidin-1-yl)piperidin-1-yl)-[1,3'-biazetidine]-1'-carboxylate (94c)

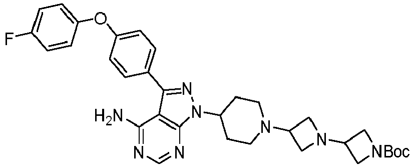

The crude product 1-(1-(azetidin-3-yl)piperidin-4-yl)-3-(4-(4-fluorophenoxy)phenyl)-1H-pyrazolo[3,4-d]pyrimidin-4-amine (94b) (124 mg) was dissolved in 10 mL of DCE, and tert-butyl 3-oxoazetidine-1-carboxylate (92 mg, 0.54 mmol) and acetic acid (0.05 mL, 0.875 mmol) were added. The reaction was stirred at room temperature for 1 h, sodium triacetoxyborohydride (151 mg, 0.71 mmol) was added, and the reaction was stirred at room temperature for 16 h. The reaction was quenched by adding 10 mL of water to the reaction solution. The organic layer was separated. The aqueous layer was extracted with dichloromethane (30 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(4-(4-fluorophenoxy)phenyl)-1H-pyrazolo[3,4-d]pyrimidin-1-yl)piperidin-1-yl)-[1,3'-biazetidin]-1'-carboxylate (94c) (65 mg, two-step yield from compound 94a: 35%).
LCMS m/z = 615.3 [M+1]⁺.

### Step 4:

### 1-(1-([1,3'-biazetidin]-3-yl)piperidin-4-yl)-3-(4-(4-fluorophenoxy)phenyl)-1H-pyrazolo[3,4-d]pyrimidin-4-amine (94d)

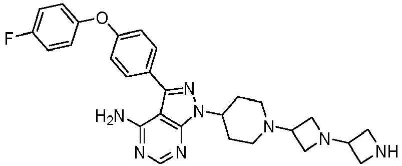

Tert-butyl 3-(4-(4-amino-3-(4-(4-fluorophenoxy)phenyl)-1H-pyrazolo[3,4-d]pyrimidin-1-yl)piperidin-1-yl)-[1,3'-biazetidin]-1'-carboxylate (94c) (65 mg, 0.11 mmol) was dissolved in 5 mL of DCM, 0.5 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (20 mL × 3). The organic layer was combined, dried over anhydrous sodium sulfate, and concentrated under reduced pressure, to obtain crude product 1-(1-([1,3'-biazetidin]-3-yl)piperidin-4-yl)-3-(4-(4-fluorophenoxy)phenyl)-1H-pyrazolo[3,4-d]pyrimidin-4-amine (94d) (56 mg).

### Step 5:

### 5-(3-(4-(4-amino-3-(4-(4-fluorophenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 94)

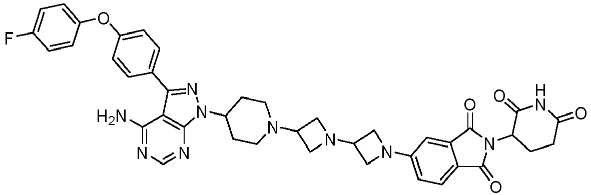

The above crude product 1-(1-([1,3'-biazetidin]-3-yl)piperidin-4-yl)-3-(4-(4-fluorophenoxy)phenyl)-1H-pyrazolo[3,4-d]pyrimidin-4-amine (94d) (56 mg) was dissolved in 2 mL of DMSO, and 0.2 mL of DIPEA and 2-(2,6-dioxopiperidin-3-yl)-5-fluoroisoindoline-1,3-dione (see WO 2017197056 for the synthetic method) (30 mg, 0.11 mmol) were added, and the reaction was stirred at 80°C for 5 h. The reaction solution was cooled to room temperature, 5 mL of water was added, and the reaction solution was filtered. The filter cake was collected, washed with 20 mL of water. The solid was dissolved with 30 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(4-(4-fluorophenoxy)phenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 94) (20 mg, two-step yield from compound 94c: 24%).
¹H NMR (400 MHz, CDCl₃) δ 8.40 - 8.34 (m, 1H), 7.67 - 7.34 (m, 3H), 7.27 - 6.99 (m, 6H), 6.78 (d, 1H), 6.52 (dd, 1H), 5.56 (br.s, 2H), 4.92 (dd, 1H), 4.84 - 4.72 (m, 1H), 4.08 - 3.99 (m, 2H), 3.91 - 3.83 (m, 2H), 3.74 - 3.63 (m, 1H), 3.60 - 3.52 (m, 2H), 3.15 - 3.02 (m, 3H), 2.98 - 2.64 (m, 5H), 2.51 - 2.33 (m, 2H), 2.17 - 1.95 (m, 5H).
LCMS m/z = 771.3 [M+1]⁺.

### Example 95:

### 5-(3-(4-(4-amino-3-(3-fluoro-4-phenoxyphenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 95)

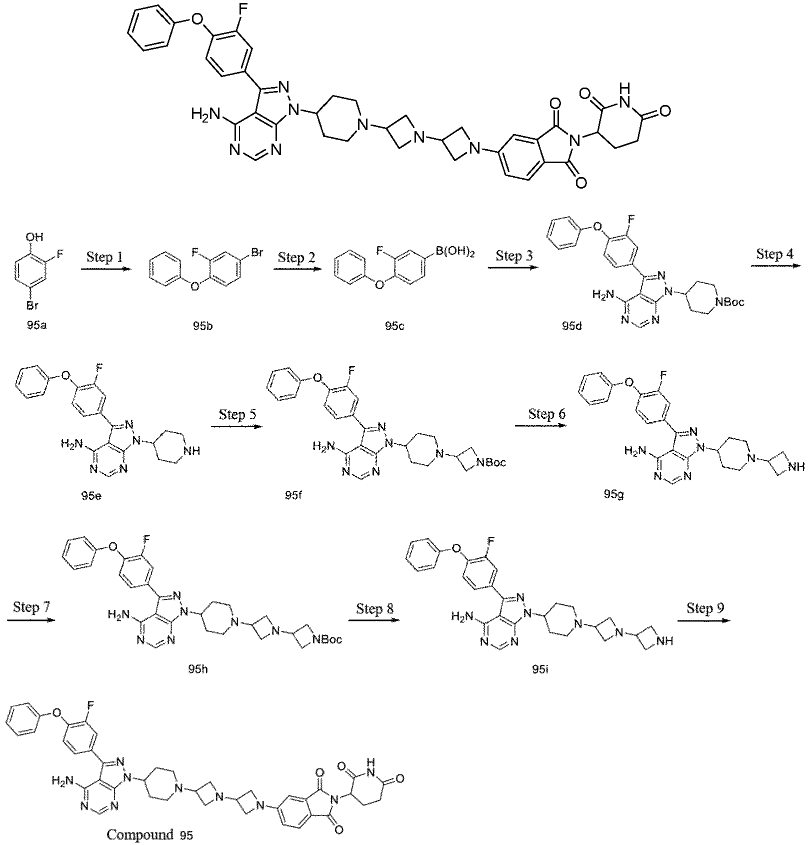

### Step 1:

### 4-bromo-2-fluoro-1-phenoxybenzene (95b)

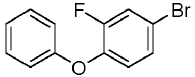

To an aqueous solution (20 mL) of 4-bromo-2-fluorophenol (95a) (1.2 g, 6.3 mmol) and diphenyliodonium bromide (2.5 g, 6.9 mmol) was added 1 mol/L NaOH solution (6.9 mL), and the reaction mixture was warmed to 110°C and stirred for 5 h. The reaction system was cooled to room temperature, diluted by adding 50 mL of methyl tert-butyl ether, and filtered. The organic layer was separated from filtrate, washed with 30 mL of saturated sodium chloride solution, dried over anhydrous sodium sulfate, and concentrated under reduced pressure, and then the crude product was separated and purified by silica gel column chromatography (petroleum ether), to obtain 4-bromo-2-fluoro-1-phenoxybenzene (95b) (1.2 g, yield: 71%).

### Step 2:

### (3-fluoro-4-phenoxyphenyl)boronic acid (95c)

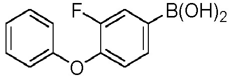

To a three-necked flask filled with nitrogen were added 4-bromo-2-fluoro-1-phenoxybenzene (95b) (1.18 g, 4.42 mmol) and dry tetrahydrofuran (30 mL), the mixture was cooled to -78°C, and 1.6 mol/L n-butyllithium solution (4.1 mL) was slowly added dropwise. Upon completion of the addition, the reaction was carried out at -78°C for 30 min, and triisopropyl borate (1.23 mL, 5.33 mmol) was added dropwise, and the mixture was slowly warmed to room temperature and reacted for 3 h. Upon completion of the reaction, the reaction was quenched with 2 mol/L hydrochloric acid solution (50 mL), and extracted with ethyl acetate (50 mL × 3). The organic phase was washed with saturated sodium chloride solution (50 mL), dried over anhydrous sodium sulfate, and concentrated under reduced pressure, to obtain crude product (3-fluoro-4-phenoxyphenyl)boronic acid (95c) (0.57 g).

### Step 3:

### tert-butyl 4-(4-amino-3-(3-fluoro-4-phenoxyphenyl)-1H-pyrazolo[3,4-d]pyrimidin-1-yl)piperidine-1-carboxylate (95d)

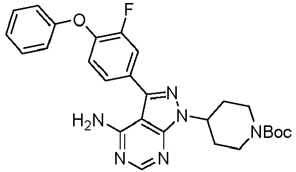

Tert-butyl 4-(4-amino-3-iodo-1H-pyrazolo[3,4-d]pyrimidin-1-yl)piperidin-1-carboxylate (87a) (see CN 110627796 for the synthetic method) (1.0 g, 2.25 mmol), Pd(dppf)Cl₂ (82 mg, 0.11 mmol), potassium carbonate (0.93 g, 6.73 mmol), and crude product (3-fluoro-4-phenoxyphenyl)boronic acid (95c) (0.57 g) were successively added to a three-necked flask. Nitrogen replacement was carried out 3 times, a mixed solvent of 30 mL of 1,4-dioxane and water (v/v) = 7 : 3 was added, and the reaction was warmed to 120°C and refluxed with stirring for 4 h. The reaction system was cooled to room temperature, and 100 mL of ethyl acetate and 100 mL of water were added. The organic phase was separated. The aqueous phase was extracted with ethyl acetate (100 mL × 3). The organic phase was combined, dried over anhydrous sodium sulfate, and concentrated under reduced pressure, and then the crude product was purified by silica gel column chromatography (dichloromethane/methanol (v/v) = 20 : 1), to obtain tert-butyl 4-(4-amino-3-(3-fluoro-4-phenoxyphenyl)-1H-pyrazolo[3,4-d]pyrimidin-1-yl)piperidine-1-carboxylate (95d) (920 mg, a yield calculated from compound 87a: 81%).
LCMS m/z = 505.3 [M+1]⁺.

### Step 4:

### 3-(3-fluoro-4-phenoxyphenyl)-1-(piperidin-4-yl)-1H-pyrazolo[3,4-d]pyrimidin-4-amine (95e)

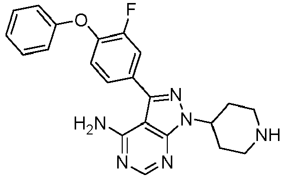

Tert-butyl 4-(4-amino-3-(3-fluoro-4-phenoxyphenyl)-1H-pyrazolo[3,4-d]pyrimidin-1-yl)piperidine-1-carboxylate (95d) (920 mg, 1.82 mmol) was dissolved in 20 mL of DCM, 7.0 mL of trifluoroacetic acid was added, and the reaction was stirred at room temperature for 4 h. The reaction system was concentrated under reduced pressure. The residue was dissolved in 30 mL of DCM, and 30 mL of water was added, and then the pH was adjusted to 12 by adding dropwise 1 mol/L NaOH solution. The organic layer was separated. The aqueous layer was extracted with dichloromethane (50 mL × 3). The organic layer was combined, dried over anhydrous sodium sulfate, and concentrated under reduced pressure, to obtain crude product 3-(3-fluoro-4-phenoxyphenyl)-1-(piperidin-4-yl)-1H-pyrazolo[3,4-d]pyrimidin-4-amine (95e) (370 mg).

### Step 5:

### tert-butyl 3-(4-(4-amino-3-(3-fluoro-4-phenoxyphenyl)-1H-pyrazolo[3,4-d]pyrimidin-1-yl)piperidin-1-yl)azetidine-1-carboxylate (95f)

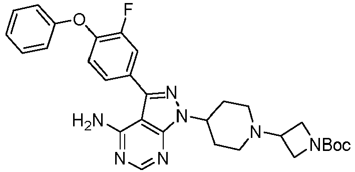

The above crude product 3-(3-fluoro-4-phenoxyphenyl)-1-(piperidin-4-yl)-1H-pyrazolo[3,4-d]pyrimidin-4-amine (95e) (370 mg) was dissolved in 20 mL of DCE, and tert-butyl 3-oxoazetidine-1-carboxylate (310 mg, 1.81 mmol) and acetic acid (0.05 mL, 0.875 mmol) were added. The reaction was stirred at room temperature for 1 h, sodium triacetoxyborohydride (580 mg, 2.74 mmol) was added, and the reaction was stirred at room temperature for 16 h. The reaction was quenched by adding 20 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(3-fluoro-4-phenoxyphenyl)-1H-pyrazolo[3,4-d]pyrimidin-1-yl)piperidin-1-yl)azetidine-1-carboxylate (95f) (350 mg, two-step yield from compound 95d: 34%).
LCMS m/z = 560.3 [M+1]⁺.

### Step 6:

### 1-(1-(azetidin-3-yl)piperidin-4-yl)-3-(3-fluoro-4-phenoxyphenyl)-1H-pyrazolo[3,4-d]pyrimidin-4-amine (95g)

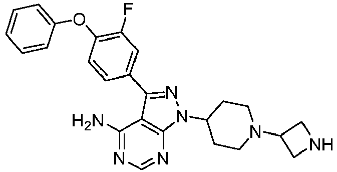

Tert-butyl 3-(4-(4-amino-3-(3-fluoro-4-phenoxyphenyl)-1H-pyrazolo[3,4-d]pyrimidin-1-yl)piperidin-1-yl)azetidine-1-carboxylate (95f) (350 mg, 0.63 mmol) was dissolved in 10 mL of DCM, 3.0 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (20 mL × 3). The organic layer was combined, dried over anhydrous sodium sulfate, and concentrated under reduced pressure, to obtain crude product 1-(1-(azetidin-3-yl)piperidin-4-yl)-3-(3-fluoro-4-phenoxyphenyl)-1H-pyrazolo[3,4-d]pyrimidin-4-amine (95g) (290 mg).

### Step 7:

### tert-butyl 3-(4-(4-amino-3-(3-fluoro-4-phenoxyphenyl)-1H-pyrazolo[3,4-d]pyrimidin-1-yl)piperidin-1-yl)-[1,3'-biazetidine]-1'-carboxylate (95h)

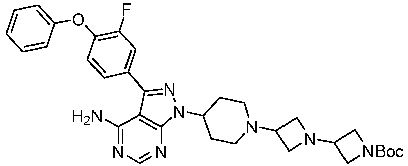

The above crude product 1-(1-(azetidin-3-yl)piperidin-4-yl)-3-(3-fluoro-4-phenoxyphenyl)-1H-pyrazolo[3,4-d]pyrimidin-4-amine (95g) (290 mg) was dissolved in 20 mL of DCE, and tert-butyl 3-oxoazetidine-1-carboxylate (220 mg, 1.29 mmol) and acetic acid (0.05 mL, 0.875 mmol) were added. The reaction was stirred at room temperature for 1 h, sodium triacetoxyborohydride (400 mg, 1.89 mmol) was added, and the reaction was stirred at room temperature for 16 h. The reaction was quenched by adding 10 mL of water to the reaction solution. The organic layer was separated. The aqueous layer was extracted with dichloromethane (50 mL × 4), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(3-fluoro-4-phenoxyphenyl)-1H-pyrazolo[3,4-d]pyrimidin-1-yl)piperidin-1-yl)-[1,3'-biazetidin]-1'-carboxylate (95h) (300 mg, two-step yield from compound 95f: 77%).

### Step 8:

### 1-(1-([1,3'-biazetidin]-3-yl)piperidin-4-yl)-3-(3-fluoro-4-phenoxyphenyl)-1H-pyrazolo[3,4-d]pyrimidin-4-amine (95i)

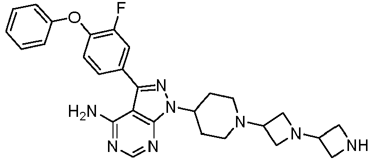

Tert-butyl 3-(4-(4-amino-3-(3-fluoro-4-phenoxyphenyl)-1H-pyrazolo[3,4-d]pyrimidin-1-yl)piperidin-1-yl)-[1,3'-biazetidin]-1'-carboxylate (95h) (300 mg, 0.49 mmol) was dissolved in 10 mL of DCM, 3 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-(1-([1,3'-biazetidin]-3-yl)piperidin-4-yl)-3-(3-fluoro-4-phenoxyphenyl)-1H-pyrazolo[3,4-d]pyrimidin-4-amine (95i) (220 mg).

### Step 9:

### 5-(3-(4-(4-amino-3-(3-fluoro-4-phenoxyphenyl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 95)

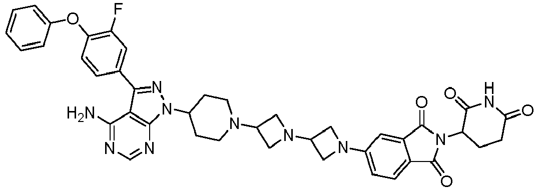

The above crude product 1-(1-([1,3'-biazetidin]-3-yl)piperidin-4-yl)-3-(3-fluoro-4-phenoxyphenyl)-1H-pyrazolo[3,4-d]pyrimidin-4-amine (95i) (220 mg) was dissolved in 5 mL of DMSO, and 1.0 mL of DIPEA and 2-(2,6-dioxopiperidin-3-yl)-5-fluoroisoindoline-1,3-dione (see WO 2017197056 for the synthetic method) (130 mg, 0.47 mmol) were added, and the reaction was stirred at 80°C for 5 h. The reaction solution was cooled to room temperature, 20 mL of water was added, and the reaction solution was filtered. The filter cake was collected, washed with 30 mL of water. The solid was dissolved with 30 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(3-fluoro-4-phenoxyphenyl)-1H-pyrazolo[3,4-d]pyrimidin-1-yl)piperidin-1-yl]-[1,3'-biazetidin]-1'-yl)-2-(2,6-dioxopiperidin-3-yl)isoindoline-1,3-dione (compound 95) (100 mg, two-step yield from compound 95h: 26%).
¹H NMR (400 MHz, CDCl₃) δ 8.68 (br.s, 1H), 8.37 (s, 1H), 7.64 (d, 1H), 7.55 (dd, 1H), 7.44 - 7.34 (m, 3H), 7.20 - 7.13 (m, 2H), 7.09 - 7.03 (m, 2H), 6.79 (d, 1H), 6.53 (dd, 1H), 5.74 (br.s, 2H), 4.97 - 4.72 (m, 2H), 4.10 - 4.01 (m, 2H), 3.95 - 3.86 (m, 2H), 3.79 - 3.68 (m, 1H), 3.65 - 3.55 (m, 2H), 3.30 - 2.92 (m, 5H), 2.92 - 2.64 (m, 3H), 2.52 - 2.37 (m, 2H), 2.33 - 2.00 (m, 5H).
LCMS m/z = 771.3 [M+1]⁺.

### Example 96:

### 5-(3-(4-(4-amino-3-(3-fluoro-4-phenoxyphenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 96)

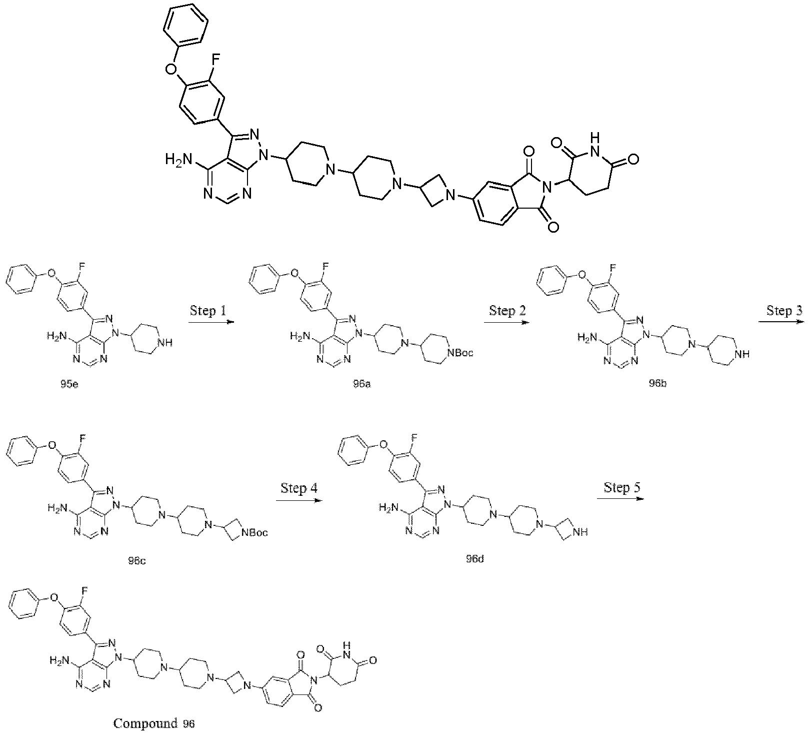

### Step 1:

### tert-butyl 4-(4-amino-3-(3-fluoro-4-phenoxyphenyl)-1H-pyrazolo[3,4-d]pyrimidin-1-yl)-[1,4'-bipiperidine]-1'-carboxylate (96a)

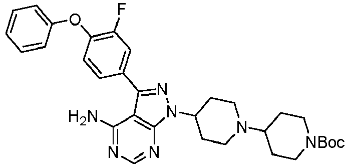

The above crude product 3-(3-fluoro-4-phenoxyphenyl)-1-(piperidin-4-yl)-1H-pyrazolo[3,4-d]pyrimidin-4-amine (95e) (370 mg) was dissolved in 20 mL of DCE, and tert-butyl 4-oxopiperidine-1-carboxylate (360 mg, 1.81 mmol) and acetic acid (0.05 mL, 0.875 mmol) were added. The reaction was stirred at room temperature for 1 h, sodium triacetoxyborohydride (580 mg, 2.74 mmol) was added, and the reaction was stirred at room temperature for 16 h. The reaction was quenched by adding 30 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 4-(4-amino-3-(3-fluoro-4-phenoxyphenyl)-1H-pyrazolo[3,4-d]pyrimidin-1-yl)-[1,4'-bipiperidin]-1'-carboxylate (96a) (250 mg, two-step yield from compound 95d: 23%).
LCMS m/z = 588.3 [M+1]⁺.

### Step 2:

### 1-([1,4'-bipiperidin]-4-yl)-3-(3-fluoro-4-phenoxyphenyl)-1H-pyrazolo[3,4-d]pyrimidin-4-amine (96b)

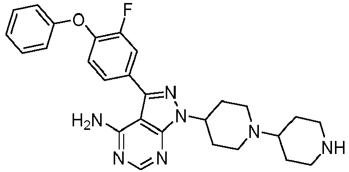

Tert-butyl 4-(4-amino-3-(3-fluoro-4-phenoxyphenyl)-1H-pyrazolo[3,4-d]pyrimidin-1-yl)-[1,4'-bipiperidin]-1'-carboxylate (96a) (250 mg, 0.43 mmol) was dissolved in 10 mL of DCM, 3.0 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-([1,4'-bipiperidin]-4-yl)-3-(3-fluoro-4-phenoxyphenyl)-1H-pyrazolo[3,4-d]pyrimidin-4-amine (96b) (210 mg).

### Step 3:

### tert-butyl 3-(4-(4-amino-3-(3-fluoro-4-phenoxyphenyl)-1H-pyrazolo[3,4-d]pyrimidin-1-yl)-[1,4'-bipiperidin]-1'-yl)azetidine-1-carboxylate (96c)

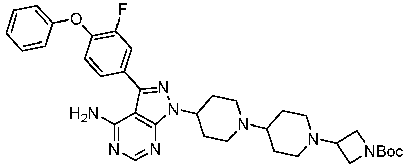

The above crude product 1-([1,4'-bipiperidin]-4-yl)-3-(3-fluoro-4-phenoxyphenyl)-1H-pyrazolo[3,4-d]pyrimidin-4-amine (96b) (210 mg) was dissolved in 10 mL of DCE, and tert-butyl 3-oxoazetidine-1-carboxylate (150 mg, 0.88 mmol) and acetic acid (0.05 mL, 0.875 mmol) were added. The reaction was stirred at room temperature for 1 h, sodium triacetoxyborohydride (270 mg, 1.27 mmol) was added, and the reaction was stirred at room temperature for 16 h. The reaction was quenched by adding 20 mL of water to the reaction solution. The organic layer was separated. The aqueous layer was extracted with dichloromethane (30 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(3-fluoro-4-phenoxyphenyl)-1H-pyrazolo[3,4-d]pyrimidin-1-yl)-[1,4'-bipiperidin]-1'-yl)azetidin-1-carboxylate (96c) (200 mg, two-step yield from compound 96a: 72%).

### Step 4:

### 1-(1'-(azetidin-3-yl)-[1,4'-bipiperidin]-4-yl)-3-(3-fluoro-4-phenoxyphenyl)-1H-pyrazolo[3,4-d]pyrimidin-4-amine (96d)

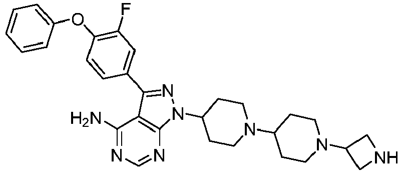

Tert-butyl 3-(4-(4-amino-3-(3-fluoro-4-phenoxyphenyl)-1H-pyrazolo[3,4-d]pyrimidin-1-yl)-[1,4'-bipiperidin]-1'-yl)azetidine-1-carboxylate (96c) (200 mg, 0.31 mmol) was dissolved in 10 mL of DCM, 3.0 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 1-(1'-(azetidin-3-yl)-[1,4'-bipiperidin]-4-yl)-3-(3-fluoro-4-phenoxyphenyl)-1H-pyrazolo[3,4-d]pyrimidin-4-amine (96d) (170 mg).

### Step 5:

### 5-(3-(4-(4-amino-3-(3-fluoro-4-phenoxyphenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 96)

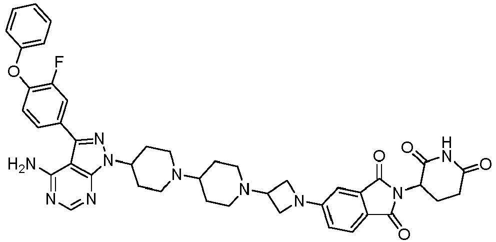

The above crude product 1-(1'-(azetidin-3-yl)-[1,4'-bipiperidin]-4-yl)-3-(3-fluoro-4-phenoxyphenyl)-1H-pyrazolo[3,4-d]pyrimidin-4-amine (96d) (170 mg) was dissolved in 10 mL of DMSO, and 0.6 mL of DIPEA and 2-(2,6-dioxopiperidin-3-yl)-5-fluoroisoindoline-1,3-dione (see WO 2017197056 for the synthetic method) (94 mg, 0.34 mmol) were added, and the reaction was stirred at 80°C for 5 h. The reaction solution was cooled to room temperature, 10 mL of water was added, and the reaction solution was filtered. The filter cake was collected, washed with 30 mL of water. The solid was dissolved with 50 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(3-fluoro-4-phenoxyphenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 96) (66 mg, two-step yield from compound 96c: 27%).
¹H NMR (400 MHz, CDCl₃) δ 8.36 (s, 1H), 7.64 (d, 1H), 7.54 (dd, 1H), 7.43 - 7.34 (m, 3H), 7.20 - 7.12 (m, 2H), 7.10 - 7.03 (m, 2H), 6.79 (d, 1H), 6.53 (dd, 1H), 6.05 - 5.60 (m, 2H), 4.98 - 4.78 (m, 2H), 4.15 - 4.06 (m, 2H), 3.92 - 3.84 (m, 2H), 3.42 - 3.33 (m, 1H), 3.32 - 3.18 (m, 2H), 3.03 - 2.93 (m, 2H), 2.93 - 2.60 (m, 5H), 2.57 - 2.39 (m, 3H), 2.29 - 2.16 (m, 2H), 2.20 - 2.08 (m, 1H), 2.06 - 1.93 (m, 4H), 1.81 - 1.65 (m, 2H).
LCMS m/z = 799.3 [M+1]⁺.

### Example 97:

### 5-(3-(4-(4-amino-3-(6-phenoxypyridin-3-yl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 97)

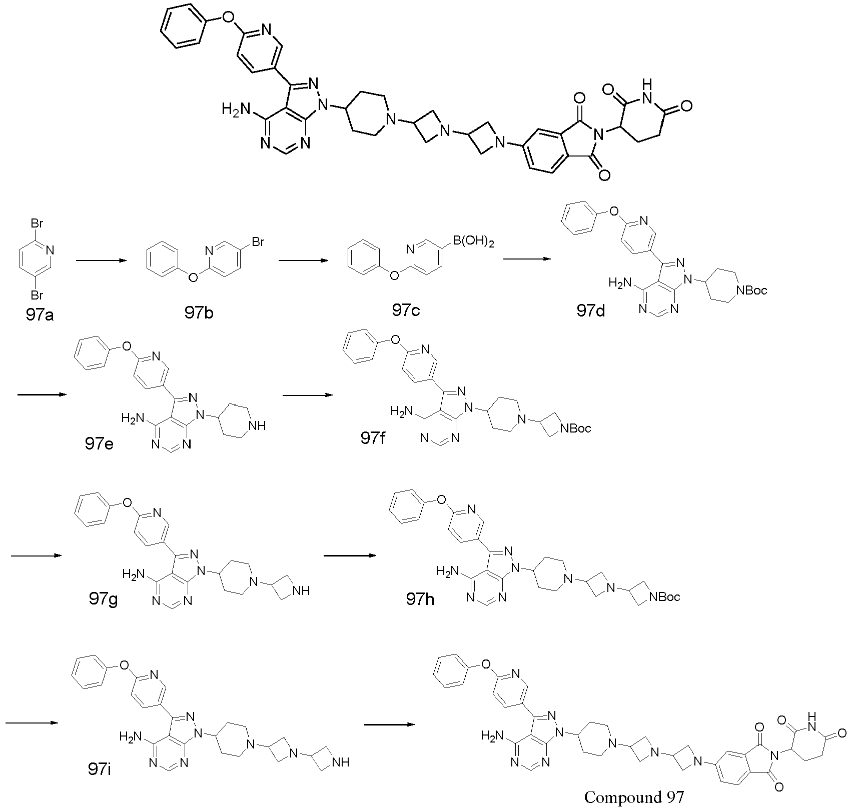

### Step 1:

### 5-bromo-2-phenoxypyridine (97b)

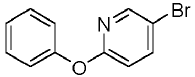

To a dry three-necked flask were successively added 2,5-bibromopyridine (97a) (5.0 g, 21.1 mmol), phenol (2.38 g, 25.29 mmol), CuI (401 mg, 2.11 mmol), TMEDA (244 mg, 2.10 mmol) and Cs₂CO₃ (13.7 g, 42.0 mmol). Nitrogen replacement was carried out 3 times, 100 mL of DMSO was added, and the reaction mixture was warmed to 110°C and stirred for 24 h. The reaction system was cooled to room temperature, slowly poured into 200 mL of ice water, and extracted by adding 300 mL of ethyl acetate. The organic layer was separated. The organic layer was washed with water (50 mL × 3), dried over anhydrous sodium sulfate, and concentrated under reduced pressure, and then the crude product was separated and purified by silica gel column chromatography (petroleum ether), to obtain 5-bromo-2-phenoxypyridine (97b) (4.0 g, yield: 76%).

### Step 2:

### (6-phenoxypyridin-3-yl)boronic acid (97c)

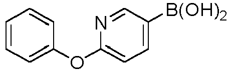

To a three-necked flask filled with nitrogen were added 5-bromo-2-phenoxypyridine (97b) (2.0 g, 8.00 mmol) and dry tetrahydrofuran (30 mL), the mixture was cooled to -78°C, and 1.6 mol/L n-butyllithium solution (7.5 mL) was slowly added dropwise. Upon completion of the addition, the reaction was carried out at -78°C for 30 min, and triisopropyl borate (2.2 mL, 9.5 mmol) was added dropwise, and the mixture was slowly warmed to room temperature and reacted for 3 h. Upon completion of the reaction, the reaction was quenched with 2 mol/L hydrochloric acid solution (50 mL), and extracted with ethyl acetate (50 mL × 3). The organic phase was washed with saturated sodium chloride solution (50 mL), dried over anhydrous sodium sulfate, and concentrated under reduced pressure, to obtain crude product (6-phenoxypyridin-3-yl)boronic acid (97c) (0.83 g).

### Step 3:

### tert-butyl 4-(4-amino-3-(6-phenoxypyridin-3-yl)-1H-pyrazolo[3,4-d]pyrimidin-1-yl)piperidine-1-carboxylate (97d)

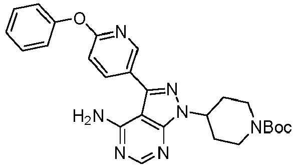

Tert-butyl 4-(4-amino-3-iodo-1H-pyrazolo[3,4-d]pyrimidin-1-yl)piperidin-1-carboxylate (87a) (see CN110627796 for the synthetic method) (1.55 g, 3.38 mmol), Pd(dppf)Cl₂ (130 mg, 0.18 mmol), potassium carbonate (1.45 g, 10.49 mmol), and the above crude product (6-phenoxypyridin-3-yl)boronic acid (97c) (0.83 g) were successively added to a three-necked flask. Nitrogen replacement was carried out 3 times, a mixed solvent of 50 mL of 1,4-dioxane and water (v/v) = 7 : 3 was added, and the reaction mixture was warmed to 120°C and refluxed with stirring for 4 h. The reaction system was cooled to room temperature, and 100 mL of ethyl acetate and 100 mL of water were added. The organic phase was separated. The aqueous phase was extracted with ethyl acetate (100 mL × 3). The organic phase was combined, dried over anhydrous sodium sulfate, and concentrated under reduced pressure, and then the crude product was purified by silica gel column chromatography (dichloromethane/methanol (v/v) = 20 : 1), to obtain 4-(4-amino-3-(6-phenoxypyridin-3-yl)-1H-pyrazolo[3,4-d]pyrimidin-1-yl)piperidine-1-carboxylate tert-butyl (97d) (584 mg, yield from compound 87a: 35%).
LCMS m/z = 488.2 [M+1]⁺.

### Step 4:

### 3-(6-phenoxypyridin-3-yl)-1-(piperidin-4-yl)-1H-pyrazolo[3,4-d]pyrimidin-4-amine (97e)

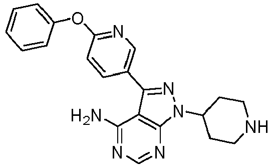

Tert-butyl 4-(4-amino-3-(6-phenoxypyridine-3-yl)-1H-pyrazolo[3,4-d]pyrimidin-1-yl)piperidine-1-carboxylate (97d) (487 mg, 1.0 mmol) was dissolved in 10 mL of DCM, 3.0 mL of trifluoroacetic acid was added, and the reaction was stirred at room temperature for 4 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (30 mL × 3). The organic layer was combined, dried over anhydrous sodium sulfate, and concentrated under reduced pressure, to obtain crude product 3-(6-phenoxypyridin-3-yl)-1-(piperidin-4-yl)-1H-pyrazolo[3,4-d]pyrimidin-4-amine (97e) (390 mg).

### Step 5:

### tert-butyl 3-(4-(4-amino-3-(6-phenoxypyridin-3-yl)-1H-pyrazolo[3,4-d]pyrimidin-1-yl)piperidin-1-yl)azetidine-1-carboxylate (97f)

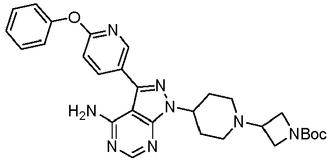

The above crude product 3-(6-phenoxypyridin-3-yl)-1-(piperidin-4-yl)-1H-pyrazolo[3,4-d]pyrimidin-4-amine (97e) (390 mg) was dissolved in 20 mL of DCE, and tert-butyl 3-oxoazetidine-1-carboxylate (340 mg, 2.0 mmol) and acetic acid (0.05 mL, 0.875 mmol) were added. The reaction was stirred at room temperature for 1 h, sodium triacetoxyborohydride (640 mg, 3.0 mmol) was added, and the reaction was stirred at room temperature for 16 h. The reaction was quenched by adding 20 mL of water to the reaction solution. The organic layer was separated. The aqueous layer was extracted with dichloromethane (50 mL × 3), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(6-phenoxypyridin-3-yl)-1H-pyrazolo[3,4-d]pyrimidin-1-yl)piperidin-1-yl)azetidine-1-carboxylate (97f) (175 mg, two-step yield from compound 97d: 32%).
LCMS m/z = 543.3 [M+1]⁺.

### Step 6:

### 1-(1-(azetidin-3-yl)piperidin-4-yl)-3-(6-phenoxypyridin-3-yl)-1H-pyrazolo[3,4-d]pyrimidin-4-amine (97g)

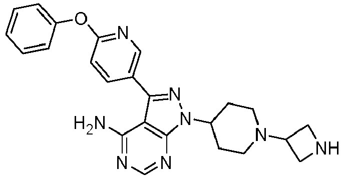

Tert-butyl 3-(4-(4-amino-3 -(6-phenoxypyridin-3 -yl)-1H-pyrazolo[3,4-d]pyrimidin-1-yl)piperidin-1-yl)azetidine-1-carboxylate (97f) (175 mg, 0.32 mmol) was dissolved in 10 mL of DCM, 3.0 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (20 mL × 3). The organic layer was combined, dried over anhydrous sodium sulfate, and concentrated under reduced pressure, to obtain crude product 1-(1-(azetidin-3-yl)piperidin-4-yl)-3-(6-phenoxypyridin-3-yl)-1H-pyrazolo[3,4-d]pyrimidin-4-amine (97g) (140 mg).

### Step 7:

### tert-butyl 3-(4-(4-amino-3-(6-phenoxypyridin-3-yl)-1H-pyrazolo[3,4-d]pyrimidin-1-yl)piperidin-1-yl)-[1,3'-biazetidine]-1'-carboxylate (97h)

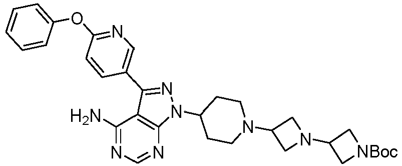

The above crude product 1-(1-(azetidin-3-yl)piperidin-4-yl)-3-(6-phenoxypyridin-3-yl)-1H-pyrazolo[3,4-d]pyrimidin-4-amine (97g) (140 mg) was dissolved in 10 mL of DCE, and tert-butyl 3-oxoazetidine-1-carboxylate (110 mg, 0.64 mmol) and acetic acid (0.05 mL, 0.875 mmol) were added. The reaction was stirred at room temperature for 1 h, sodium triacetoxyborohydride (200 mg, 0.94 mmol) was added, and the reaction was stirred at room temperature for 16 h. The reaction was quenched by adding 10 mL of water to the reaction solution. The organic layer was separated. The aqueous layer was extracted with dichloromethane (20 mL × 4), and the organic layer was combined, dried over anhydrous sodium sulfate, and concentrated under reduced pressure, and then the crude product was separated and purified by silica gel column chromatography (dichloromethane/methanol (v/v) = 20 : 1), to obtain tert-butyl 3-(4-(4-amino-3-(6-phenoxypyridin-3-yl)-1H-pyrazolo[3,4-d]pyrimidin-1-yl)piperidin-1-yl)-[1,3'-biazetidin]-1'-carboxylate (97h) (44 mg, two-step yield from compound 97f: 23%).

### Step 8:

### 1-(1-([1,3'-biazetidin]-3-yl)piperidin-4-yl)-3-(6-phenoxypyridin-3-yl)-1H-pyrazolo[3,4-d]pyrimidin-4-amine (97i)

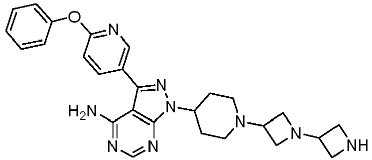

Tert-butyl 3 -(4-(4-amino-3 -(6-phenoxypyridin-3-yl)-1H-pyrazolo[3,4-d]pyrimidin-1-yl)piperidin-1-yl)-[1,3'-biazetidin]-1'-carboxylate (97h) (44 mg, 0.074 mmol) was dissolved in 5 mL of DCM, 2 mL of trifluoroacetic acid was added, and the reaction was stirred at room temperature for 2 h. The reaction system was concentrated under reduced pressure. The residue was dissolved in 10 mL of DCM, and 10 mL of water was added. The pH was adjusted to 12 by adding dropwise 1 mol/L NaOH solution. The organic layer was separated. The aqueous layer was extracted with dichloromethane (20 mL × 3). The organic layer was combined, dried over anhydrous sodium sulfate, and concentrated under reduced pressure, to obtain crude product 1-(1-([1,3'-biazetidin]-3-yl)piperidin-4-yl)-3-(6-phenoxypyridin-3-yl)-1H-pyrazolo[3,4-d]pyrimidin-4-amine (97i) (32 mg).

### Step 9:

### 5-(3-(4-(4-amino-3-(6-phenoxypyridin-3-yl)-1H-pyrazolo[3,4-d]pyrimidin-1-yl)piperidin-1-yl)-[1,3'-biazetidin]-1'-yl)-2-(2,6-dioxopiperidin-3-yl)isoindoline-1,3-dione (compound 97)

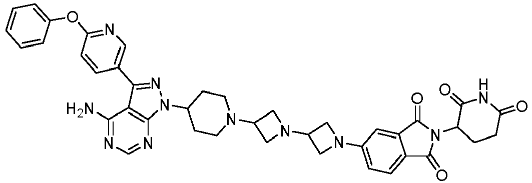

The above crude product 1-(1-([1,3'-biazetidin]-3-yl)piperidin-4-yl)-3-(6-phenoxypyridin-3-yl)-1H-pyrazolo[3,4-d]pyrimidin-4-amine (97i) (32 mg) was dissolved in 3 mL of DMSO, and 0.1 mL of DIPEA and 2-(2,6-dioxopiperidin-3-yl)-5-fluoroisoindoline-1,3-dione (see WO 2017197056 for the synthetic method) (20 mg, 0.07 mmol) were added, and the reaction was stirred at 80°C for 5 h. The reaction solution was cooled to room temperature, 5 mL of water was added, and the reaction solution was filtered. The filter cake was collected, washed with 10 mL of water. The solid was dissolved with 10 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-(4-(4-amino-3-(6-phenoxypyridin-3-yl)-1H-pyrazolo[3,4-d]pyrimidin-1-yl)piperidin-1-yl]-[1,3'-biazetidin]-1'-yl)-2-(2,6-dioxopiperidin-3-yl)isoindoline-1,3-dione (compound 97) (17 mg, two-step yield from compound 97h: 30%).
¹H NMR (400 MHz, CDCl₃) δ 8.51 (s, 1H), 8.45 - 8.41 (m, 1H), 8.32 (s, 1H), 7.96 (dd, 1H), 7.57 (d, 1H), 7.42 - 7.34 (m, 2H), 7.22 - 7.15 (m, 1H), 7.15 - 7.09 (m, 2H), 7.01 (d, 1H), 6.72 (d, 1H), 6.46 (dd, 1H), 5.46 (br.s, 2H), 4.93 - 4.70 (m, 2H), 4.05 - 3.93 (m, 2H), 3.90 - 3.78 (m, 2H), 3.75 - 3.62 (m, 1H), 3.59 - 3.50 (m, 2H), 3.28 - 3.02 (m, 3H), 3.02 - 2.85 (m, 2H), 2.85 - 2.58 (m, 3H), 2.46 - 2.30 (m, 2H), 2.26 - 1.90 (m, 5H).
LCMS m/z = 754.3 [M+1]⁺.

### Example 98:

### 5-(4-((3-(4-(4-amino-3-(4-phenoxyphenyl)-1H-pyrazolo[3,4-d]pyrimidin-1-yl)piperidin-1-yl)azetidin-1-yl)methyl)piperidin-1-yl)-2-(2,6-dioxopiperidin-3-yl)isoindoline-1,3-dione (compound 98) trifluoroacetate

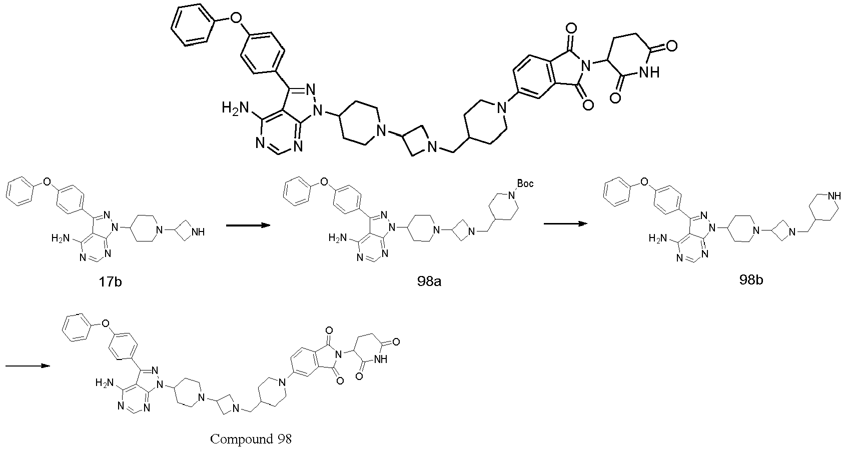

### Step 1:

### tert-butyl 4-((3-(4-(4-amino-3-(4-phenoxyphenyl)-1H-pyrazolo[3,4-d]pyrimidin-1-yl) piperidin-1-yl)azetidin-1-yl)methyl)piperidine-1-carboxylate (98a)

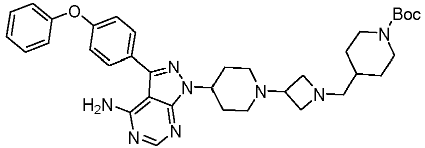

1-[1-(azetidin-3-yl)-4-piperidyl]-3-(4-phenoxyphenyl)pyrazolo[3,4-d]pyrimidin-4-amine (17b) (see WO 2020239103 for the synthetic method) (100 mg, 0.23 mmol) was dissolved in 15 mL of DCE, tert-butyl 4-formylpiperidine-1-carboxylate (74 mg, 0.35 mmol) and 0.05 mL of acetic acid were successively added, and the mixture was stirred at room temperature for 60 min, then sodium triacetoxyborohydride (97 mg, 0.46 mmol) was added. The reaction was carried out at room temperature for 16 h. To the reaction system was slowly added 20 mL of saturated sodium bicarbonate solution, and the mixed solution was extracted with 80 mL of DCM. The organic phase was washed with 50 mL of water, dried over anhydrous sodium sulfate, and concentrated under reduced pressure. The crude product was separated and purified by silica gel column chromatography (dichloromethane/methanol (v/v) = 8 : 1), to obtain tert-butyl 4-((3-(4-(4-amino-3-(4-phenoxyphenyl)-1H-pyrazolo[3,4-d]pyrimidin-1-yl)piperidin-1-yl)azetidin-1-yl)methyl)piperidine-1-carboxylate (98a) (100 mg, yield: 68%).
LCMS m/z = 639.5 [M+1]⁺.

### Step 2:

### 3-(4-phenoxyphenyl)-1-(1-(1-(piperidin-4-ylmethyl)azetidin-3-yl)piperidin-4-yl)-1H-pyrazolo[3,4-d]pyrimidin-4-amine (98b) trifluoroacetate

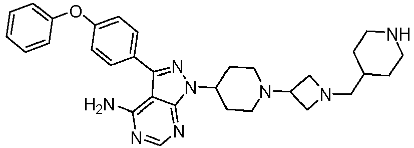

Tert-butyl 4-((3-(4-(4-amino-3-(4-phenoxyphenyl)-1H-pyrazolo[3,4-d]pyrimidin-1-yl)piperidin-1-yl)azetidin-1-yl)methyl)piperidine-1-carboxylate (98a) (100 mg, 0.16 mmol) was dissolved in 5 mL of DCM, 2 mL of trifluoroacetic acid was added, and the mixture was stirred at room temperature for 2 h. The reaction solution was concentrated under reduced pressure, to obtain crude product 3-(4-phenoxyphenyl)-1-(1-(1-(piperidin-4-ylmethyl)azetidin-3-yl)piperidin-4-yl)-1H-pyrazolo[3,4-d]pyrimidin-4-amine (98b) trifluoroacetate (120 mg).
LCMS m/z = 539.3 [M+1]⁺.

### Step 3:

### 5-(4-((3-(4-(4-amino-3-(4-phenoxyphenyl)-1H-pyrazolo[3,4-d]pyrimidin-1-yl)piperidin-1-yl)azetidin-1-yl)methyl)piperidin-1-yl)-2-(2,6-dioxopiperidin-3-yl)isoindoline-1,3-dione (compound 98) trifluoroacetate

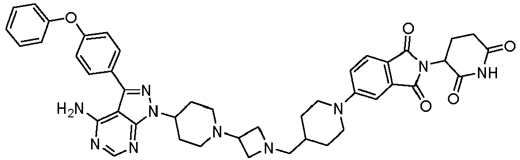

The above crude product 3-(4-phenoxyphenyl)-1-(1-(1-(piperidin-4-ylmethyl)azetidin-3-yl)piperidin-4-yl)-1H-pyrazolo[3,4-d]pyrimidin-4-amine (98b) trifluoroacetate (120 mg) was dissolved in 4 mL of DMSO, and solid sodium bicarbonate (71 mg, 0.85 mmol) was added. The mixture was stirred at room temperature for 10 min, 0.2 mL of DIPEA and 2-(2,6-dioxopiperidin-3-yl)-5-fluoroisoindoline-1,3-dione (see WO 2017197056 for the synthetic method) (94 mg, 0.34 mmol) were added, and the reaction was stirred at 80°C for 5 h. The reaction solution was cooled to room temperature, 50 mL of water was added, and the reaction solution was filtered. The solid was collected, washed with 20 mL of water, dissolved with 100 mL of DCM, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-(4-((3-(4-(4-amino-3-(4-phenoxyphenyl)-1H-pyrazolo[3,4-d]pyrimidin-1-yl)piperidin-1-yl)azetidin-1-yl)methyl)piperidin-1-yl)-2-(2,6-dioxopiperidin-3-yl)isoindoline-1,3-dione (compound 98) trifluoroacetate (40 mg).
¹H NMR (400 MHz, DMSO-*d₆*) δ 11.06 (s, 1H), 8.36 (s, 1H), 7.72 - 7.63 (m, 3H), 7.50 - 7.41 (m, 2H), 7.38 - 7.32 (m, 1H), 7.30 - 7.24 (m, 1H), 7.24 - 7.19 (m, 1H), 7.19 - 7.11 (m, 4H), 5.07 (dd, 1H), 5.00 - 4.86 (m, 1H), 4.52 - 4.34 (m, 2H), 4.34 - 4.20 (m, 2H), 4.14 - 4.04 (m, 2H), 3.92 - 3.80 (m, 1H), 3.34 - 3.22 (m, 2H), 3.22 - 3.13 (m, 2H), 3.04 - 2.82 (m, 3H), 2.80 - 2.52 (m, 4H), 2.45 - 2.28 (m, 2H), 2.20 - 2.08 (m, 2H), 2.08 - 1.98 (m, 1H), 1.98 - 1.86 (m, 1H), 1.83 - 1.69 (m, 2H), 1.34 - 1.20 (m, 2H).
LCMS m/z = 795.4 [M +1]⁺.

### Example 99:

### 5-(3-((3-(4-(4-amino-3-(4-phenoxyphenyl)-1H-pyrazolo[3,4-d]pyrimidin-1-yl)piperidin-1-yl)azetidin-1-yl)methyl)pyrrolidine-1-yl)-2-(2,6-dioxopiperidin-3-yl)isoindoline-1,3-dione (compound 99) trifluoroacetate

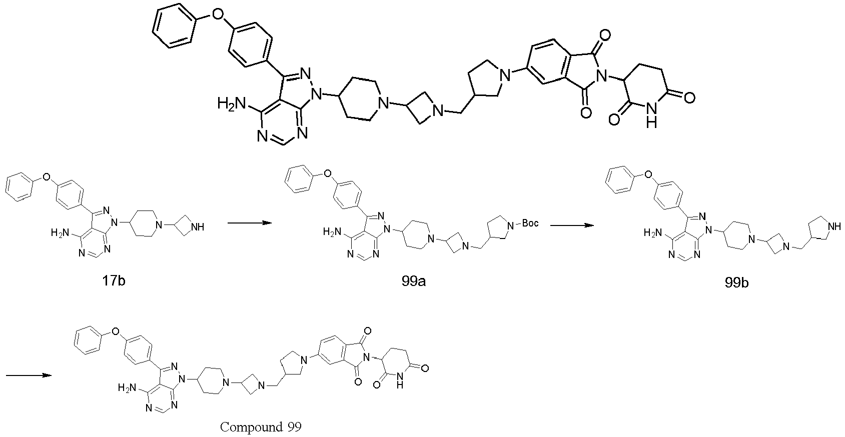

### Step 1:

### tert-butyl 3-((3-(4-(4-amino-3-(4-phenoxyphenyl)-1H-pyrazolo[3,4-d]pyrimidin-1-yl) piperidin-1-yl)azetidin-1-yl)methyl)pyrrolidine-1-carboxylate (99a)

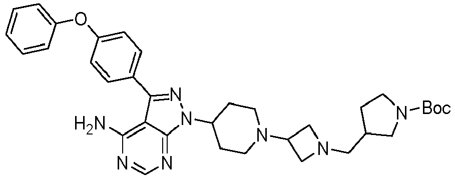

1-[1-(azetidin-3-yl)-4-piperidyl]-3-(4-phenoxyphenyl)pyrazolo[3,4-d]pyrimidin-4-amine (17b) (see WO 2020239103 for the synthetic method) (100 mg, 0.23 mmol) was dissolved in 15 mL of DCE, tert-butyl 3-formylpyrrolidine-1-carboxylate (90 mg, 0.45 mmol) and 0.05 mL of acetic acid were successively added, and the mixture was stirred at room temperature for 60 min, then sodium triacetoxyborohydride (97 mg, 0.46 mmol) was added. The reaction was carried out at room temperature for 16 h. To the reaction system was slowly added 20 mL of saturated sodium bicarbonate solution, and the mixed solution was extracted with 80 mL of DCM. The organic phase was washed with 50 mL of water, dried over anhydrous sodium sulfate, and concentrated under reduced pressure. The crude product was separated and purified by silica gel column chromatography (dichloromethane/methanol (v/v) = 8 : 1), to obtain tert-butyl 3-((3-(4-(4-amino-3-(4-phenoxyphenyl)-1H-pyrazolo[3,4-d]pyrimidin-1-yl)piperidin-1-yl)azetidin-1-yl)methyl)pyrrolidine-1-carboxylate (99a) (110 mg, yield: 77%).
LCMS m/z = 625.4 [M+1]⁺.

### Step 2:

### 3-(4-phenoxyphenyl)-1-(1-(1-(pyrrolidine-3-ylmethyl)azetidin-3-yl)piperidin-4-yl)-1H-pyrazolo[3,4-d]pyrimidin-4-amine (99b) trifluoroacetate

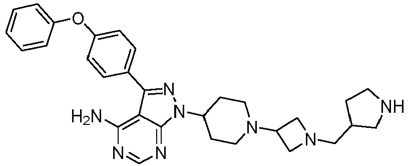

Tert-butyl 3-((3-(4-(4-amino-3-(4-phenoxyphenyl)-1H-pyrazolo[3,4-d]pyrimidin-1-yl)piperidin-1-yl)azetidin-1-yl)methyl)pyrrolidine-1-carboxylate (99a) (100 mg, 0.16 mmol) was dissolved in 5 mL of DCM, 2 mL of trifluoroacetic acid was added, and the mixture was stirred at room temperature for 2 h. The reaction solution was concentrated under reduced pressure, to obtain crude product 3-(4-phenoxyphenyl)-1-(1-(1-(pyrrolidine-3-ylmethyl)azetidin-3-yl)piperidin-4-yl)-1H-pyrazolo[3,4-d]pyrimidin-4-amine (99b) trifluoroacetate (120 mg).
LCMS m/z = 525.3 [M+1]⁺.

### Step 3:

### 5-(3-((3-(4-(4-amino-3-(4-phenoxyphenyl)-1H-pyrazolo[3,4-d]pyrimidin-1-yl)piperidin-1-yl)azetidin-1-yl)methyl)pyrrolidine-1-yl)-2-(2,6-dioxopiperidin-3-yl)isoindoline-1,3-dione (compound 99) trifluoroacetate

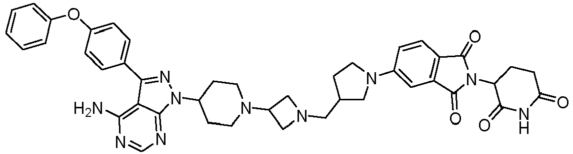

The above crude product 3-(4-phenoxyphenyl)-1-(1-(1-(pyrrolidine-3-ylmethyl)azetidin-3-yl)piperidin-4-yl)-1H-pyrazolo[3,4-d]pyrimidin-4-amine (99b) trifluoroacetate (120 mg) was dissolved in 4 mL of DMSO, and solid sodium bicarbonate (71 mg, 0.85 mmol) was added. The mixture was stirred at room temperature for 10 min, 0.2 mL of DIPEA and 2-(2,6-dioxopiperidin-3-yl)-5-fluoroisoindoline-1,3-dione (see WO 2017197056 for the synthetic method) (94 mg, 0.34 mmol) were added, and the reaction was stirred at 80°C for 5 h. The reaction solution was cooled to room temperature, 50 mL of water was added, and the reaction solution was filtered. The solid was collected, washed with 20 mL of water, dissolved with 100 mL of DCM, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-(3-((3-(4-(4-amino-3-(4-phenoxyphenyl)-1H-pyrazolo[3,4-d]pyrimidin-1-yl)piperidin-1-yl)azetidin-1-yl)methyl)pyrrolidine-1-yl)-2-(2,6-dioxopiperidin-3-yl)isoindoline-1,3-dione (compound 99) trifluoroacetate (35 mg).
¹H NMR (400 MHz, DMSO-*d₆*) δ 11.06 (s, 1H), 8.35 (s, 1H), 7.72 - 7.63 (m, 3H), 7.51 - 7.41 (m, 2H), 7.28 - 7.09 (m, 5H), 6.96 - 6.90 (m, 1H), 6.83 (dd, 1H), 5.06 (dd, 1H), 5.00 - 4.82 (m, 1H), 4.50 - 4.33 (m, 2H), 4.33 - 4.14 (m, 2H), 3.90 - 3.65 (m, 2H), 3.50 - 3.31 (m, 3H), 3.30 - 3.15 (m, 4H), 2.96 - 2.81 (m, 1H), 2.74 - 2.53 (m, 5H), 2.45 - 2.29 (m, 2H), 2.28 - 2.07 (m, 3H), 2.07 - 1.96 (m, 1H), 1.90 - 1.74 (m, 1H).
LCMS m/z = 781.3 [M +1]⁺.

### Example 100:

### 5-(3-(((3aR,6aS)-5-(4-(4-amino-3-(4-phenoxyphenyl)-1H-pyrazolo[3,4-d]pyrimidin-1-yl)piperidin-1-yl)hexahydrocyclopenta[c]pyrrol-2(1H)-yl)methyl)azetidin-1-yl)-2-(2,6-dioxopiperidin-3-yl)isoindoline-1,3-dione (compound 100) trifluoroacetate

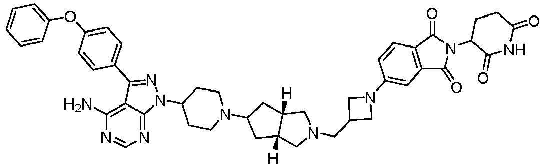

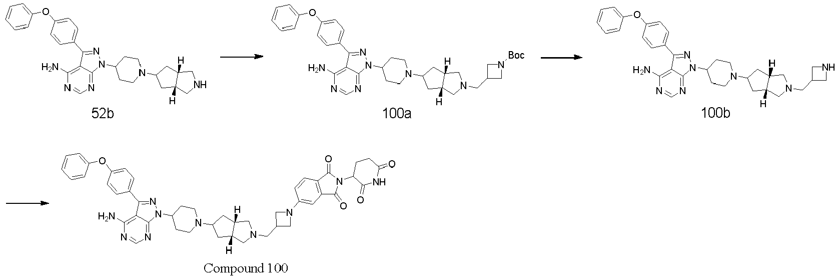

### Step 1:

### tert-butyl 3-(((3 aR,6aS)-5-(4-(4-amino-3 -(4-phenoxyphenyl)-1H-pyrazolo[3,4-d]pyrimidin-1- yl)piperidin-1-yl)hexahydrocyclopenta[c]pyrrol-2(1H)-yl)methyl)azetidine-1-carboxylate (100a)

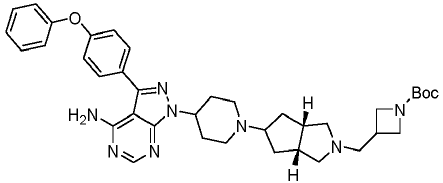

1-(1-((3aR,6aS)-octahydrocyclopenta[c]pyrrol-5-yl)piperidin-4-yl)-3-(4-phenoxyphenyl)-1H-pyrazolo[3,4-d]pyrimidin-4-amine trifluoroacetate (52b) (see WO 2020239103 for the synthetic method) (120 mg) was dissolved in 15 mL of THF, tert-butyl 3-formylazetidine-1-carboxylate (90 mg, 0.49 mmol) and 0.05 mL of acetic acid were successively added, and the mixture was stirred at room temperature for 60 min, then sodium triacetoxyborohydride (102 mg, 0.48 mmol) was added. The reaction was carried out at room temperature for 16 h. To the reaction system was slowly added 20 mL of saturated sodium bicarbonate solution, and the mixed solution was extracted with 80 mL of DCM. The organic phase was washed with 50 mL of water, dried over anhydrous sodium sulfate, and concentrated under reduced pressure. The crude product was separated and purified by silica gel column chromatography (dichloromethane/methanol (v/v) = 8 : 1), to obtain tert-butyl 3-(((3aR,6aS)-5-(4-(4-amino-3-(4-phenoxyphenyl)-1H-pyrazolo[3,4-d]pyrimidin-1-yl)piperidin-1-yl)hexahydrocyclopenta[c]pyrrol-2(1H)-yl)methyl)azetidin-1-carboxylate (100a) (160 mg).
LCMS m/z = 665.4 [M+1]⁺.

### Step 2:

### 1-(1-((3 aR,6aS)-2-(azetidin-3-ylmethyl)octahydrocyclopenta[c]pyrrol-5-yl)piperidin-4-yl)-3-(4-phenoxyphenyl)-1H-pyrazolo[3,4-d]pyrimidin-4-amine (100b) trifluoroacetate

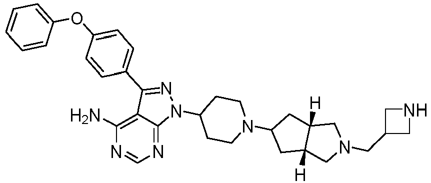

Tert-butyl 3 -(((3 aR,6aS)-5-(4-(4-amino-3 -(4-phenoxyphenyl)-1H-pyrazolo[3,4-d]pyrimidin-1-yl)piperidin-1-yl)hexahydrocyclopenta[c]pyrrol-2(1H)-yl)methyl)azetidine-1-carboxylate (100a) (150 mg, 0.23 mmol) was dissolved in 5 mL of DCM, and 2 mL of trifluoroacetic acid was added, the mixture was stirred at room temperature for 2 h. The reaction solution was concentrated under reduced pressure, to obtain crude product 1-(1-((3aR,6aS)-2-(azetidin-3-ylmethyl)octahydrocyclopenta[c]pyrrol-5-yl)piperidin-4-yl)-3-(4-phenoxyphenyl)-1H-pyrazolo[3,4-d]pyrimidin-4-amine (100b) trifluoroacetate (180 mg).
LCMS m/z = 565.3 [M+1]⁺.

### Step 3:

### 5-(3-(((3aR,6aS)-5-(4-(4-amino-3-(4-phenoxyphenyl)-1H-pyrazolo[3,4-d]pyrimidin-1-yl)piperidin-1-yl)hexahydrocyclopenta[c]pyrrol-2(1H)-yl)methyl)azetidin-1-yl)-2-(2,6-dioxopiperidin-3-yl)isoindoline-1,3-dione (compound 100) trifluoroacetate

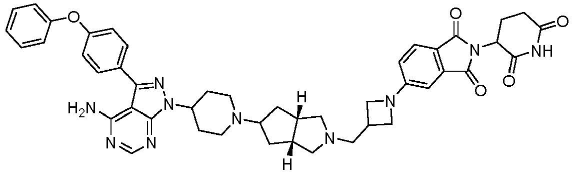

The above crude product 1-(1-((3aR,6aS)-2-(azetidin-3-ylmethyl)octahydrocyclopenta[c]pyrrol-5-yl)piperidin-4-yl)-3-(4-phenoxyphenyl)-1H-pyrazolo[3,4-d]pyrimidin-4-amine (100b) trifluoroacetate (180 mg) was dissolved in 4 mL of DMSO, and solid sodium bicarbonate (85 mg, 1.0 mmol) was added, the mixture was stirred at room temperature for 10 min, 0.2 mL of DIPEA and 2-(2,6-dioxopiperidin-3-yl)-5-fluoroisoindoline-1,3-dione (see WO 2017197056 for the synthetic method) (127 mg, 0.46 mmol) were added, and the reaction was stirred at 80°C for 5 h. The reaction solution was cooled to room temperature, 50 mL of water was added, and the reaction solution was filtered. The solid was collected, washed with 20 mL of water, dissolved with 100 mL of DCM, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-(3-(((3aR,6aS)-5-(4-(4-amino-3-(4-phenoxyphenyl)-1H-pyrazolo[3,4-d]pyrimidin-1-yl)piperidin-1-yl)hexahydrocyclopenta[c]pyrrol-2(1H)-yl)methyl)azetidin-1-yl)-2-(2,6-dioxopiperidin-3-yl)isoindoline-1,3-dione (compound 100) trifluoroacetate (40 mg).
¹H NMR (400 MHz, DMSO-*d₆*) δ 11.15 - 11.00 (m, 1H), 8.38 - 8.30 (m, 1H), 7.76 - 7.60 (m, 3H), 7.49 - 7.41 (m, 2H), 7.25 - 7.10 (m, 5H), 6.84 - 6.75 (m, 1H), 6.72 - 6.60 (m, 1H), 5.23 - 5.00 (m, 2H), 4.30 - 4.15 (m, 2H), 3.93 - 3.68 (m, 3H), 3.68 - 3.08 (m, 9H), 3.02 - 2.86 (m, 2H), 2.84 - 2.68 (m, 1H), 2.68 - 2.51 (m, 2H), 2.51 - 2.43 (m, 4H), 2.42 - 2.28 (m, 2H), 2.28 - 2.13 (m, 2H), 2.12 - 1.95 (m, 1H), 1.85 - 1.60 (m, 2H).
LCMS m/z = 821.4 [M +1]⁺.

### Example 101:

### 5-(3-((2-(4-(4-amino-3-(4-phenoxyphenyl)-1H-pyrazolo[3,4-d]pyrimidin-1-yl)piperidin-1-yl)-7-azaspiro[3. 5]nonan-7-yl)methyl)azetidin-1-yl)-2-(2,6-dioxopiperidin-3-yl)isoindoline-1,3-dione (compound 101) trifluoroacetate

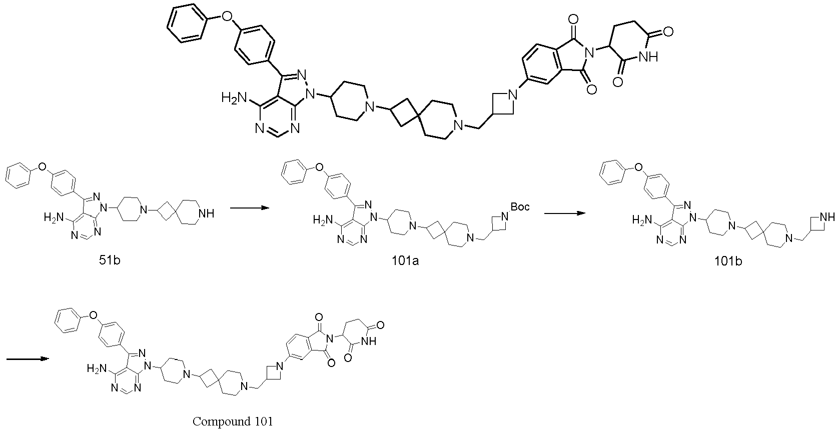

### Step 1:

### tert-butyl 3-((2-(4-(4-amino-3-(4-phenoxyphenyl)-1H-pyrazolo[3,4-d]pyrimidin-1-yl) piperidin-1-yl)-7-azaspiro[3.5]nonan-7-yl)methyl)azetidine-1-carboxylate (101a)

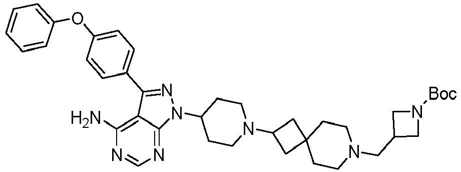

1-(1-(1-(7-azaspiro[3.5]nonan-2-yl)piperidin-4-yl)-3-(4-phenoxyphenyl)-1H-pyrazolo[3,4-d]pyrimidin-4-amine trifluoroacetate (51b) (see WO 2020239103 for the synthetic method) (120 mg) was dissolved in 15 mL of THF, tert-butyl 3-formylazetidine-1-carboxylate (90 mg, 0.49 mmol) and 0.05 mL of acetic acid were successively added, and the mixture was stirred at room temperature for 60 min, then sodium triacetoxyborohydride (102 mg, 0.48 mmol) was added. The reaction was carried out at room temperature for 16 h. To the reaction system was slowly added 20 mL of saturated sodium bicarbonate solution, and the mixed solution was extracted with 80 mL of DCM. The organic phase was washed with 50 mL of water, dried over anhydrous sodium sulfate, and concentrated under reduced pressure. The crude product was separated and purified by silica gel column chromatography (dichloromethane/methanol (v/v) = 8 : 1), to obtain tert-butyl 3-((2-(4-(4-amino-3-(4-phenoxyphenyl)-1H-pyrazolo[3,4-d]pyrimidin-1-yl)piperidin-1-yl)-7-azaspiro[3.5]nonan-7-yl)methyl)azetidine-1-carboxylate (101a) (160 mg).
LCMS m/z = 679.4 [M+1]⁺.

### Step 2:

### 1-(1-(7-(azetidin-3-ylmethyl)-7-azaspiro[3.5]nonan-2-yl)piperidin-4-yl)-3-(4-phenoxyphenyl)-1H-pyrazolo[3,4-d]pyrimidin-4-amine (101b) trifluoroacetate

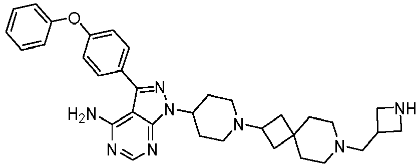

Tert-butyl 3-((2-(4-(4-amino-3-(4-phenoxyphenyl)-1H-pyrazolo[3,4-d]pyrimidin-1-yl)piperidin-1-yl)-7-azaspiro[3. 5]nonan-7-yl)methyl)azetidine-1-carboxylate (101a) (150 mg, 0.22 mmol) was dissolved in 5 mL of DCM, 2 mL of trifluoroacetic acid was added, and the mixture was stirred at room temperature for 2 h. The reaction solution was concentrated under reduced pressure, to obtain crude product 1-(1-(7-(azetidin-3-ylmethyl)-7-azaspiro[3.5]nonan-2-yl)piperidin-4-yl)-3-(4-phenoxyphenyl)-1H-pyrazolo[3,4 -d]pyrimidin-4-amine (101b) trifluoroacetate (180 mg).
LCMS m/z = 579.4 [M+1]⁺.

### Step 3:

### 5-(3-((2-(4-(4-amino-3-(4-phenoxyphenyl)-1H-pyrazolo[3,4-d]pyrimidin-1-yl)piperidin-1-yl)-7-azaspiro[3. 5]nonan-7-yl)methyl)azetidin-1-yl)-2-(2,6-dioxopiperidin-3-yl)isoindoline-1,3-dione (compound 101) trifluoroacetate

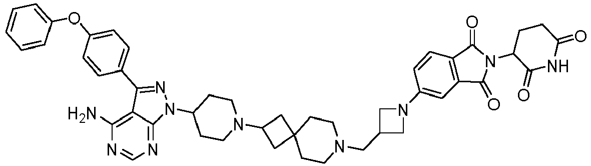

The above crude product 1-(1-(7-(azetidin-3-ylmethyl)-7-azaspiro[3.5]nonan-2-yl)piperidin-4-yl)-3-(4-phenoxyphenyl)-1H-pyrazolo[3,4 - d]pyrimidin-4-amine (101b) trifluoroacetate (180 mg) was dissolved in 4 mL of DMSO, and solid sodium bicarbonate (85 mg, 1.0 mmol) was added, the mixture was stirred at room temperature for 10 min, 0.2 mL of DIPEA and 2-(2,6-dioxopiperidin-3-yl)-5-fluoroisoindoline-1,3-dione (see WO 2017197056 for the synthetic method) (127 mg, 0.46 mmol) were added, and the reaction was stirred at 80°C for 5 h. The reaction solution was cooled to room temperature, 50 mL of water was added, and the reaction solution was filtered. The solid was collected, washed with 20 mL of water, dissolved with 100 mL of DCM, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-(3-((2-(4-(4-amino-3-(4-phenoxyphenyl)-1H-pyrazolo[3,4-d]pyrimidin-1-yl)piperidin-1-yl)-7-azaspiro[3.5]nonan-7-yl)methyl)azetidin-1-yl)-2-(2,6-dioxopiperidin-3-yl)isoindoline-1,3-dione (compound 101) trifluoroacetate (60 mg).
¹H NMR (400 MHz, DMSO-*d₆*) δ 11.06 (s, 1H), 8.36 (s, 1H), 7.76 - 7.62 (m, 3H), 7.50 - 7.41 (m, 2H), 7.25 - 7.10 (m, 5H), 6.81 (d, 1H), 6.68 (dd, 1H), 5.24 - 5.00 (m, 2H), 4.30 - 4.19 (m, 2H), 3.90 - 3.80 (m, 2H), 3.80 - 3.68 (m, 4H), 3.58 - 3.50 (m, 2H), 3.50 - 3.43 (m, 2H), 3.43 - 3.32 (m, 2H), 3.32 - 3.20 (m, 1H), 3.18 - 3.04 (m, 2H), 3.04 - 2.95 (m, 1H), 2.95 - 2.82 (m, 1H), 2.70 - 2.52 (m, 2H), 2.50 - 2.34 (m, 4H), 2.32 - 2.21 (m, 2H), 2.21 - 2.13 (m, 1H), 2.13 - 1.89 (m, 2H), 1.88 - 1.70 (m, 2H).
LCMS m/z = 835.3 [M +1]⁺.

### Example 102:

### 5-(3-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)azetidin-1-yl)-2-(2,6-dioxopiperidin-3-yl)isoindoline-1,3-dione (compound 102) trifluoroacetate

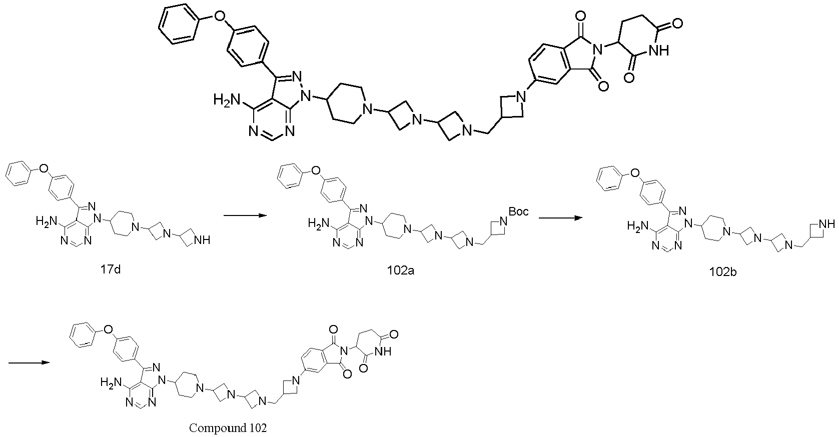

### Step 1:

### tert-butyl 3-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)azetidine-1-carboxylate (102a)

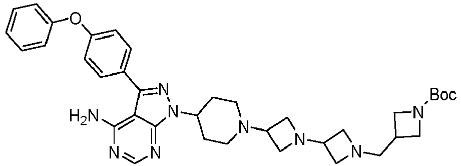

1-[1-[1-(azetidin-3-yl)azetidin-3-yl]-4-piperidyl]-3-(4-phenoxyphenyl)pyrazolo[3,4-d]pyrimidin-4-amine (17d) (see WO 2020239103 for the synthetic method) (200 mg, 0.40 mmol) was dissolved in 15 mL of THF, tert-butyl 3-formylazetidine-1-carboxylate (150 mg, 0.81 mmol) and 0.05 mL of acetic acid were successively added, and the mixture was stirred at room temperature for 60 min, then sodium triacetoxyborohydride (171 mg, 0.81 mmol) was added. The reaction was carried out at room temperature for 16 h. To the reaction system was slowly added 20 mL of saturated sodium bicarbonate solution, and the mixed solution was extracted with 80 mL of DCM. The organic phase was washed with 50 mL of water, dried over anhydrous sodium sulfate, and concentrated under reduced pressure. The crude product was separated and purified by silica gel column chromatography (dichloromethane/methanol (v/v) = 8 : 1), to obtain tert-butyl 3-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)azetidine-1-carboxylate (102a) (150 mg, yield: 56%).
LCMS m/z = 666.4 [M+1]⁺.

### Step 2:

### 1-(1-(1'-(azetidin-3-ylmethyl)-[1,3'-biazetidin]-3-yl)piperidin-4-yl)-3-(4-phenoxyphenyl)-1H-pyrazolo[3,4-d]pyrimidin-4-amine (102b) trifluoroacetate

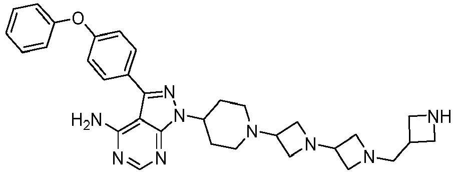

Tert-butyl 3 -((3 -(4-(4-amino-3-(4-phenoxyphenyl)-1H-pyrazolo[3,4-d]pyrimidin-1-yl)piperidin-1-yl)-[1,3'-biazetidin]-1'-yl)methyl)azetidine-1-carboxylate (102a) (150 mg, 0.23 mmol) was dissolved in 5 mL of DCM, 2 mL of trifluoroacetic acid was added, and the mixture was stirred at room temperature for 2 h. The reaction solution was concentrated under reduced pressure, to obtain crude product 1-(1-(1'-(azetidin-3-ylmethyl)-[1,3'-biazetidin]-3-yl)piperidin-4-yl)-3-(4-phenoxyphenyl)-1H-pyrazolo[3,4-d]pyrimidin-4-amine (102b) trifluoroacetate (180 mg).
LCMS m/z = 566.3 [M+1]⁺.

### Step 3:

### 5-(3-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)azetidin-1-yl)-2-(2,6-dioxopiperidin-3-yl)isoindoline-1,3-dione (compound 102) trifluoroacetate

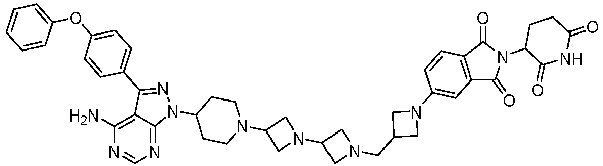

The above crude product 1-(1-(1'-(azetidin-3-ylmethyl)-[1,3'-biazetidin]-3-yl)piperidin-4-yl)-3-(4-phenoxyphenyl)-1H-pyrazolo[3,4-d]pyrimidin-4-amine (102b) trifluoroacetate (180 mg) was dissolved in 4 mL of DMSO, and solid sodium bicarbonate (85 mg, 1.0 mmol) was added, the mixture was stirred at room temperature for 10 min, 0.2 mL of DIPEA and 2-(2,6-dioxopiperidin-3-yl)-5-fluoroisoindoline-1,3-dione (see WO 2017197056 for the synthetic method) (127 mg, 0.46 mmol) were added, and the reaction was stirred at 80°C for 5 h. The reaction solution was cooled to room temperature, 50 mL of water was added, and the reaction solution was filtered. The solid was collected, washed with 20 mL of water, dissolved with 100 mL of DCM, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-(3-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)azetidin-1-yl)-2-(2,6-dioxopiperidin-3-yl)isoindoline-1,3-dione (compound 102) trifluoroacetate (20 mg).
¹H NMR (400 MHz, DMSO-*d₆*) δ 8.36 (s, 1H), 7.73 - 7.63 (m, 3H), 7.45 - 7.38 (m, 2H), 7.23 - 7.07 (m, 5H), 6.83 (d, 1H), 6.67 (dd, 1H), 5.26 - 5.12 (m, 1H), 5.05 (dd, 1H), 4.37 - 4.16 (m, 4H), 4.10 - 3.95 (m, 3H), 3.89 - 3.75 (m, 4H), 3.74 - 3.57 (m, 5H), 3.57 - 3.45 (m, 2H), 3.30 - 3.18 (m, 2H), 3.16 - 3.03 (m, 1H), 2.92 - 2.79 (m, 1H), 2.79 - 2.56 (m, 4H), 2.45 - 2.31 (m, 2H), 2.14 - 2.04 (m, 1H).
LCMS m/z = 822.4 [M +1]⁺.

### Example 103:

### 5-(3-((7-(4-(4-amino-3-(4-phenoxyphenyl)-1H-pyrazolo[3,4-d]pyrimidin-1-yl)piperidin-1-yl)-2-azaspiro[3. 5]nonan-2-yl)methyl)azetidin-1-yl)-2-(2,6-dioxopiperidin-3-yl)isoindoline-1,3-dione (compound 103) trifluoroacetate

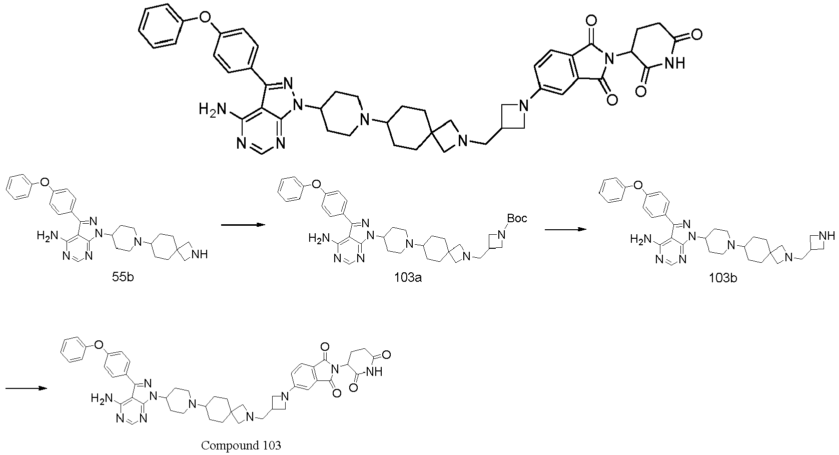

### Step 1:

### tert-butyl 3-((7-(4-(4-amino-3-(4-phenoxyphenyl)-1H-pyrazolo[3,4-d]pyrimidin-1-yl)piperidin-1-yl)-2-azaspiro[3.5]nonan-2-yl)methyl)azetidine-1-carboxylate (103a)

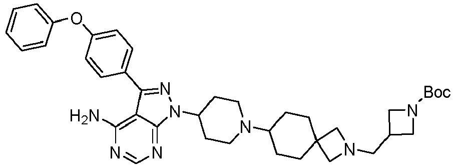

1-(1-(2-azaspiro[3.5]nonan-7-yl)piperidin-4-yl)-3-(4-phenoxyphenyl)-1H-pyrazolo[3,4-d]pyrimidin-4-amine hydrochloride (55b) (see WO 2020239103 for the synthetic method) (120 mg) was dissolved in 15 mL of THF, tert-butyl 3-formylazetidine-1-carboxylate (91 mg, 0.49 mmol) and 0.05 mL of acetic acid were successively added, and the mixture was stirred at room temperature for 60 min, then sodium triacetoxyborohydride (105 mg, 0.50 mmol) was added. The reaction was carried out at room temperature for 16 h. To the reaction system was slowly added 20 mL of saturated sodium bicarbonate solution, and the mixed solution was extracted with 80 mL of DCM. The organic phase was washed with 50 mL of water, dried over anhydrous sodium sulfate, and concentrated under reduced pressure. The crude product was separated and purified by silica gel column chromatography (dichloromethane/methanol (v/v) = 8 : 1), to obtain tert-butyl 3-((7-(4-(4-amino-3-(4-phenoxyphenyl)-1H-pyrazolo[3,4-d]pyrimidin-1-yl)piperidin-1-yl)-2-azaspiro[3.5]nonan-2-yl)methyl)azetidine-1-carboxylate (103a) (150 mg).
LCMS m/z = 679.4 [M+1]⁺.

### Step 2:

### 1-(1-(2-(azetidin-3-ylmethyl)-2-azaspiro[3.5]nonan-7-yl)piperidin-4-yl)-3-(4-phenoxyphenyl)-1H-pyrazolo[3,4-d]pyrimidin-4-amine (103b) trifluoroacetate

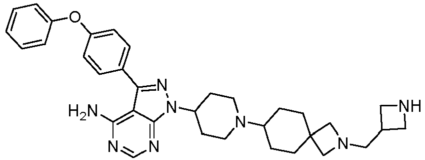

Tert-butyl 3-((7-(4-(4-amino-3-(4-phenoxyphenyl)-1H-pyrazolo[3,4-d]pyrimidin-1-yl)piperidin-1-yl)-2-azaspiro[3.5]nonan-2-yl)methyl)azetidine-1-carboxylate (103a) (150 mg, 0.22 mmol) was dissolved in 5 mL of DCM, 2 mL of trifluoroacetic acid was added, and the mixture was stirred at room temperature for 2 h. The reaction solution was concentrated under reduced pressure, to obtain crude product 1-(1-(2-(azetidin-3-ylmethyl)-2-azaspiro[3.5]nonan-7-yl)piperidin-4-yl)-3-(4-phenoxyphenyl)-1H-pyrazolo[3,4 -d]pyrimidin-4-amine (103b) trifluoroacetate (180 mg).
LCMS m/z = 579.3 [M+1]⁺.

### Step 3:

### 5-(3-((7-(4-(4-amino-3-(4-phenoxyphenyl)-1H-pyrazolo[3,4-d]pyrimidin-1-yl)piperidin-1-yl)-2-azaspiro[3. 5]nonan-2-yl)methyl)azetidin-1-yl)-2-(2,6-dioxopiperidin-3-yl)isoindoline-1,3-dione (compound 103) trifluoroacetate

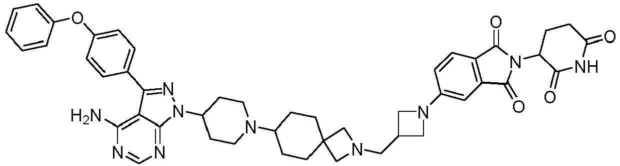

The above crude product 1-(1-(2-(azetidin-3-ylmethyl)-2-azaspiro[3.5]nonan-7-yl)piperidin-4-yl)-3-(4-phenoxyphenyl)-1H-pyrazolo[3,4 - d]pyrimidin-4-amine (103b) trifluoroacetate (180 mg) was dissolved in 4 mL of DMSO, and solid sodium bicarbonate (85 mg, 1.0 mmol) was added, the mixture was stirred at room temperature for 10 min, 0.2 mL of DIPEA and 2-(2,6-dioxopiperidin-3-yl)-5-fluoroisoindoline-1,3-dione (see WO 2017197056 for the synthetic method) (127 mg, 0.46 mmol) were added, and the reaction was stirred at 80°C for 5 h. The reaction solution was cooled to room temperature, 50 mL of water was added, and the reaction solution was filtered. The solid was collected, washed with 20 mL of water, dissolved with 100 mL of DCM, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-(3-((7-(4-(4-amino-3-(4-phenoxyphenyl)-1H-pyrazolo[3,4-d]pyrimidin-1-yl)piperidin-1-yl)-2-azaspiro[3.5]nonan-2-yl)methyl)azetidin-1-yl)-2-(2,6-dioxopiperidin-3-yl)isoindoline-1,3-dione (compound 103) trifluoroacetate (15 mg).
¹H NMR (400 MHz, DMSO-*d₆*) δ 11.20 - 11.00 (m, 1H), 8.40 - 8.33 (m, 1H), 7.75 - 7.60 (m, 3H), 7.50 - 7.40 (m, 2H), 7.29 - 7.08 (m, 5H), 6.83 - 6.73 (m, 1H), 6.71 - 6.62 (m, 1H), 5.28 - 5.01 (m, 2H), 4.25 - 4.13 (m, 2H), 3.99 - 3.94 (m, 2H), 3.92 - 3.84 (m, 2H), 3.80 - 3.68 (m, 2H), 3.64 - 3.49 (m, 2H), 3.49 - 3.40 (m, 2H), 3.40 - 3.27 (m, 2H), 3.25 - 3.11 (m, 1H), 2.98 - 2.81 (m, 3H), 2.65 - 2.51 (m, 2H), 2.45 - 2.14 (m, 5H), 2.08 - 1.95 (m, 1H), 1.95 - 1.80 (m, 2H), 1.80 - 1.50 (m, 4H).
LCMS m/z = 835.3 [M +1]⁺.

### Example 104:

### 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 104)

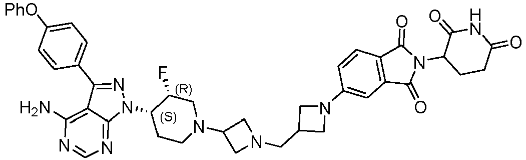

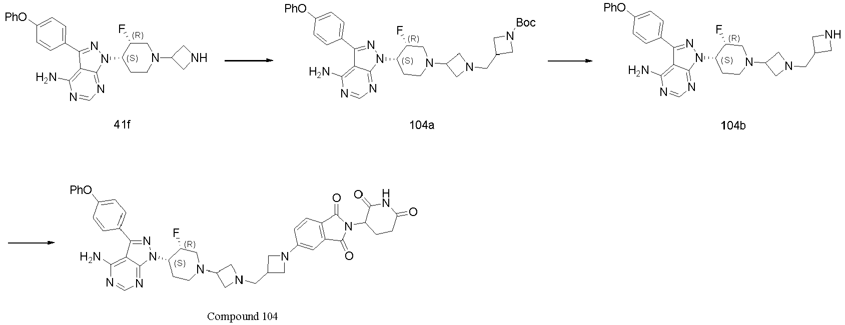

### Step 1:

### tert-butyl 3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidine-1-carboxylate (104a)

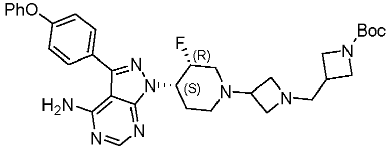

1-((3R,4S)-1-(azetidin-3-yl)-3 -fluoropiperidin-4-yl)-3 -(4-phenoxyphenyl)-1H-pyrazolo[3,4-d]pyrimidin-4-amine (41f) (see WO 2020239103 for the synthetic method) (0.46 g, 1.0 mmol) was dissolved in 20 mL of THF, tert-butyl 3-formylazetidine-1-carboxylate (0.37 g, 2.0 mmol) was added, the mixture was stirred at room temperature for 1 h, and sodium triacetoxyborohydride (0.43 g , 2.0 mmol) was added. The reaction was carried out at room temperature for 16 h. To the reaction solution was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidine-1-carboxylate (104a) (0.46 g, yield: 73%).
LCMS m/z = 629.3 [M+1]⁺.

### Step 2:

### 1-((3R,4S)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (104b)

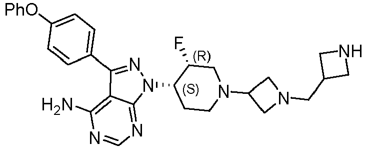

Tert-butyl 3 -((3 -((3R,4S)-4-(4-amino-3 -(4-phenoxyphenyl)-1H-pyrazolo[3,4-d]pyrimidin-1-yl)-3-fluoropiperidin-1-yl)azetidin-1-yl)methyl)azetidine-1-carboxylate (104a) (0.45 g, 0.72 mmol) was dissolved in 10 mL of methanol, 10 mL of 4 mol/L hydrochloric acid in 1,4-dioxane solution was added. The reaction was carried out at normal temperature for 1 h. The reaction solution was concentrated under reduced pressure, 100 mL of dichloromethane was added, and the pH was adjusted to 12 with 3 mol/L sodium hydroxide solution. The organic phase was separated, dried over anhydrous sodium sulfate, and concentrated under reduced pressure, to obtain crude product 1-((3R,4S)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (104b) (0.36 g).

### Step 3:

### 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 104)

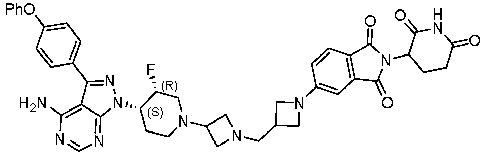

The above crude product 1-((3R,4S)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (104b) (0.36 g) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (0.37 g, 1.34 mmol) were added, and the reaction was warmed to 85°C and reacted for 3 h. The reaction solution was cooled to room temperature, 50 mL of water was added, and the reaction solution was filtered. The solid was collected, washed with 20 mL of water, and dissolved with 100 mL of DCM, dried over anhydrous sodium sulfate, and concentrated under reduced pressure. The crude product was separated and purified by silica gel column chromatography (dichloromethane/methanol (v/v) = 15 : 1), to obtain 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 104) (0.18 g, two-step yield from compound 104a: 32%).
¹H NMR (400 MHz, CDCl₃) δ 8.38 (d, 1H), 7.70 - 7.59 (m, 3H), 7.43 - 7.33 (m, 2H), 7.21 - 7.03 (m, 5H), 6.97 - 6.88 (m, 1H), 6.51 - 6.41 (m, 1H), 5.64 (br.s, 2H), 5.18 - 4.98 (m, 1H), 4.98 - 4.78 (m, 2H), 4.18 - 4.00 (m, 2H), 3.78 - 3.48 (m, 4H), 3.25 - 2.94 (m, 6H), 2.90 - 2.66 (m, 6H), 2.42 - 2.22 (m, 1H), 2.22 - 2.14 (m, 1H), 2.14 - 2.04 (m, 2H).
LCMS m/z = 785.3 [M+1]⁺.

### Example 105:

### 4-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)isoindoline-1,3-dione (compound 105)

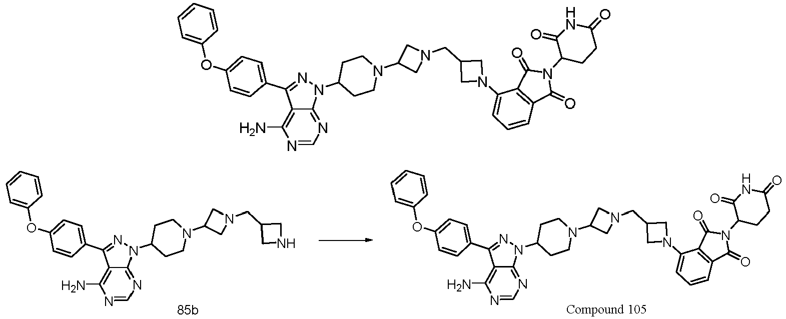

The above crude product 1-(1-(1-(azetidin-3-ylmethyl)azetidin-3-yl)piperidin-4-yl)-3-(4-phenoxyphenyl)-1H-pyrazolo[3,4-d]pyrimidine-4-amine (85b) (110 mg) was dissolved in 5 mL of DMSO, and 0.2 mL of DIPEA and 2-(2,6-dioxopiperidin-3-yl)-4-fluoroisoindoline-1,3-dione (see WO 2017197056 for the synthetic method) (83 mg, 0.3 mmol) were added, and the reaction was stirred at 85°C for 3 h. The reaction solution was cooled to room temperature, and diluted with 50 mL of ethyl acetate. The organic phase was washed with 50 mL of water, dried over anhydrous sodium sulfate, and concentrated under reduced pressure, and then the crude product was separated and purified by silica gel column chromatography (dichloromethane /methanol (v/v) = 15 : 1), to obtain 4-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)isoindoline-1,3-dione (compound 105) (60 mg, two-step yield from compound 85a: 33%).
¹H NMR (400 MHz, CDCl₃) δ 9.29 (br.s, 1H), 8.31 (s, 1H), 7.61 - 7.53 (m, 2H), 7.40 - 7.25 (m, 3H), 7.14 - 6.96 (m, 6H), 6.49 (d, 1H), 5.58 (br.s, 2H), 4.92 - 4.82 (m, 1H), 4.77 - 4.64 (m, 1H), 4.35 - 4.20 (m, 2H), 3.93 - 3.81 (m, 2H), 3.68 - 3.52 (m, 2H), 3.10 - 2.90 (m, 3H), 2.90 - 2.56 (m, 8H), 2.42 - 2.27 (m, 2H), 2.12 - 1.88 (m, 5H).
LCMS m/z = 767.3 [M+1]⁺.

### Example 106:

### 5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)-6-fluoroisoindoline-1,3-dione (compound 106)

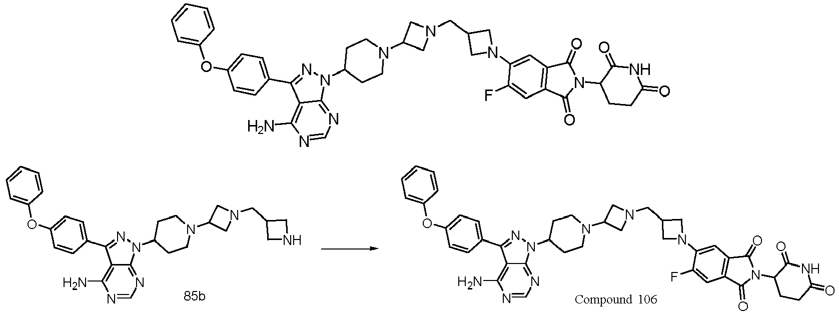

The above crude product 1-(1-(1-(azetidin-3-ylmethyl)azetidin-3-yl)piperidin-4-yl)-3-(4-phenoxyphenyl)-1H-pyrazolo[3,4-d]pyrimidine-4-amine (85b) (150 mg) was dissolved in 5 mL of DMSO, and 0.2 mL of DIPEA and 2-(2,6-dioxopiperidin-3-yl)-5.6-difluoroisoindoline-1,3-dione (see WO 2020239103 for the synthetic method) (118 mg, 0.4 mmol) were added, and the reaction was stirred at 85°C for 3 h. The reaction solution was cooled to room temperature, and diluted with 50 mL of ethyl acetate. The organic phase was washed with 50 mL of water, dried over anhydrous sodium sulfate, and concentrated under reduced pressure, and then the crude product was separated and purified by silica gel column chromatography (dichloromethane /methanol (v/v) = 15 : 1), to obtain 5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-2-(2,6-dioxopiperidin-3-yl)-6-fluoroisoindoline-1,3-dione (compound 106) (120 mg, two-step yield from compound 85a: 47%).
¹H NMR (400 MHz, DMSO-*d₆*) δ 11.06 (br.s, 1H), 8.23 (s, 1H), 7.70 - 7.62 (m, 2H), 7.57 (d, 1H), 7.48 - 7.40 (m, 2H), 7.23 - 7.09 (m, 5H), 6.88 (d, 1H), 5.05 (dd, 1H), 4.72 - 4.59 (m, 1H), 4.27 - 4.16 (m, 2H), 3.88 - 3.78 (m, 2H), 3.48 - 3.39 (m, 2H), 2.98 - 2.78 (m, 6H), 2.78 - 2.63 (m, 3H), 2.63 - 2.51 (m, 2H), 2.27 - 2.11 (m, 2H), 2.07 - 1.83 (m, 5H).
LCMS m/z = 785.3 [M +1]⁺.

### Example 107:

### 3-(5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-1-oxoisoindolin-2-yl)piperidine-2,6-dione (compound 107) trifluoroacetate

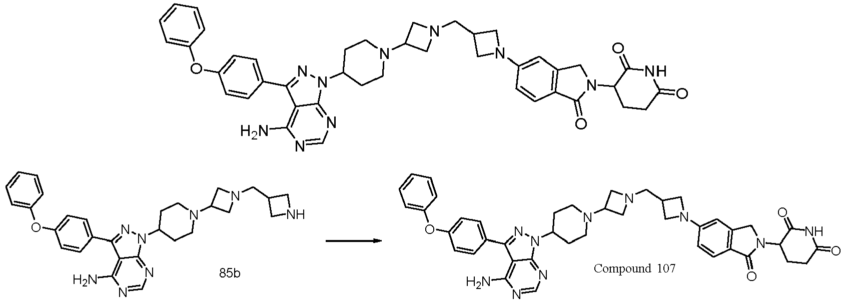

1-(1-(1-(azetidin-3-ylmethyl)azetidin-3-yl)piperidin-4-yl)-3-(4-phenoxyphenyl)-1H-pyrazolo[3,4-d]pyrimidin-4-amine (85b) (510 mg) was dissolved in 10 mL of DMSO, cesium carbonate (980 mg, 3.0 mmol), cuprous iodide (190 mg, 1.0 mmol) and L-proline (230 mg, 2 mmol) were added, and the mixture was warmed to 100°C and reacted for 2 h. The reaction system was cooled to room temperature, 100 mL of ethyl acetate was added, and the reaction solution was filtered. The filtrate was washed with 100 mL of purified water, dried over anhydrous sodium sulfate, and concentrated under reduced pressure. The crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 3-(5-3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-1-oxoisoindolin-2-yl)piperidine-2,6-dione (compound 107) trifluoroacetate (130 mg).
¹H NMR (400 MHz, DMSO-*d₆*) δ 10.92 (br.s, 1H), 8.33 - 8.28 (m, 1H), 7.70 - 7.62 (m, 2H), 7.52 (d, 1H), 7.49 - 7.41 (m, 2H), 7.25 - 7.09 (m, 5H), 6.56 - 6.46 (m, 2H), 5.03 (dd, 1H), 4.93 - 4.77 (m, 1H), 4.36 - 4.26 (m, 3H), 4.25 - 4.16 (m, 1H), 4.16 - 3.99 (m, 5H), 3.74 - 3.66 (m, 2H), 3.59 - 3.49 (m, 2H), 3.24 - 3.06 (m, 2H), 3.04 - 2.82 (m, 2H), 2.70 - 2.50 (m, 2H), 2.45 - 2.22 (m, 4H), 2.15 - 2.00 (m, 2H), 2.00 - 1.87 (m, 1H).
LCMS m/z = 753.2 [M +1]⁺.

### Example 107-1:

### 3-(5-(3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-1-oxoisoindolin-2-yl)piperidine-2,6-dione (compound 107)

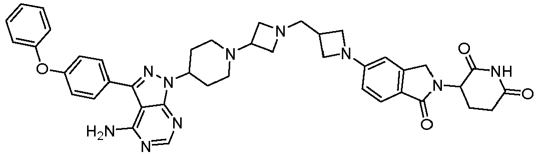

To 3-(5-3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-1-oxoisoindolin-2-yl)piperidine-2,6-dione (compound 107) trifluoroacetate (80 mg) was added 30 mL of dichloromethane, 30 mL of 25% ammonia water was added, and the mixture was continuously stirred for 1 h until a clear system was obtained. The organic phase was separated, dried over anhydrous sodium sulfate, and concentrated under reduced pressure, to obtain 3-(5-3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)-1-oxoisoindolin-2-yl)piperidine-2,6-dione (compound 107) (30 mg).
¹H NMR (400 MHz, CDCl₃) δ 8.46 - 8.30 (m, 2H), 7.72 - 7.56 (m, 3H), 7.44 - 7.33 (m, 2H), 7.22 - 7.03 (m, 5H), 6.43 (dd, 1H), 6.39 - 6.33 (m, 1H), 5.52 (br.s, 2H), 5.18 (dd, 1H), 4.87 - 4.72 (m, 1H), 4.42 - 4.33 (m, 1H), 4.27 - 4.17 (m, 1H), 4.13 - 4.01 (m, 2H), 3.96 - 3.57 (m, 4H), 3.35 - 3.04 (m, 3H), 3.04 - 2.70 (m, 6H), 2.51 - 2.29 (m, 3H), 2.29 - 1.94 (m, 6H).
LCMS m/z = 753.2 [M +1]⁺.

### Example 108:

### 5-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)-2-(2,6-dioxopiperidin-3-yl)isoindoline-1,3-dione (compound 108) trifluoroacetate

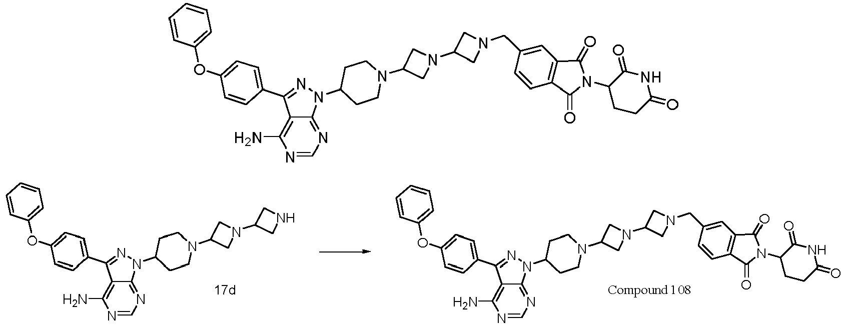

1-[1-[1-(azetidin-3 -yl)azetidin-3-yl]-4-piperidyl]-3 -(4-phenoxyphenyl)pyrazolo[3,4-d]pyrimidin-4-amine (17d) (see WO 2020239103 for the synthetic method) (100 mg, 0.2 mmol) was added to a mixed solvent of 10 mL of 1,2-dichloroethane and 2 mL of DMSO, 2-(2,6-dioxopiperidin-3-yl)-1,3-dioxoisoindolin-5-carbaldehyde (see Org. Biomol. Chem., 2013, 11, 4757-4763 for the synthetic method) (86 mg, 0.3 mmol) was added, and the reaction was carried out at room temperature for 1 h, sodium triacetoxyborohydride (63 mg, 0.3 mmol) was added, and the mixture was reacted at room temperature for another 12 h. To the reaction solution was added 30 mL of dichloromethane, and the pH of the system was adjusted to 9.0 with saturated sodium bicarbonate solution. The organic phase was separated, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), the reaction system was lyophilized to obtain 5-((3-(4-(4-amino-3-(4-phenoxyphenyl)-1H-pyrazolo[3,4-d]pyrimidin-1-yl)piperidin-1-yl)-[1,3'-biazetidin]-1'-yl)methyl)-2-(2,6-dioxopiperidin-3-yl)isoindoline-1,3-dione (compound 108) trifluoroacetate (45 mg).
¹H NMR (400 MHz, DMSO-*d₆*) δ 11.13 (br.s, 1H), 8.37 - 8.31 (m, 1H), 8.08 (s, 1H), 8.06 - 7.94 (m, 2H), 7.71 - 7.63 (m, 2H), 7.49 - 7.41 (m, 2H), 7.27 - 7.10 (m, 5H), 5.18 (dd, 1H), 5.14 - 5.00 (m, 1H), 4.57 (s, 2H), 4.24 - 4.08 (m, 2H), 4.02 - 3.86 (m, 4H), 3.76 - 3.64 (m, 4H), 3.48 - 3.40 (m, 2H), 3.25 - 3.10 (m, 2H), 2.97 - 2.83 (m, 1H), 2.69 - 2.51 (m, 2H), 2.50 - 2.38 (m, 2H), 2.30 - 2.18 (m, 2H), 2.13 - 2.02 (m, 1H).
LCMS m/z = 767.3 [M+1]⁺.

### Example 109:

### 5-((3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)methyl)-2-(2, 6-dioxopiperidin-3-yl)isoindoline-1,3-dione (compound 109) trifluoroacetate

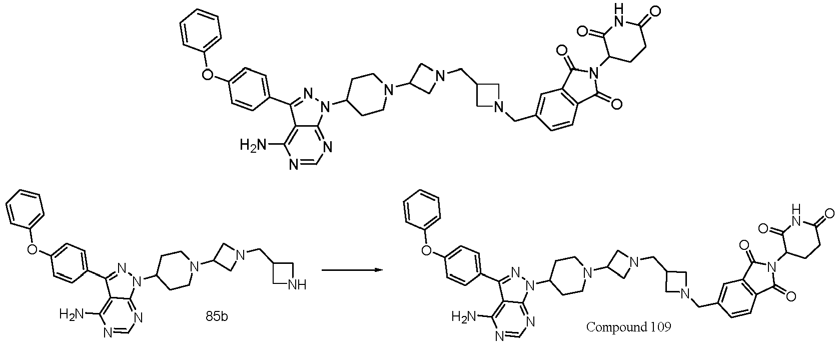

The above crude product 1-(1-(1-(azetidine-3-ylmethyl)azetidin-3-yl)piperidin-4-yl)-3-(4-phenoxyphenyl)-1H-pyrazolo[3,4-d]pyrimidin-4-amine (85b) (110 mg) was added to a mixed solvent of 10 mL of 1,2-dichloroethane and 2 mL of DMSO, 2-(2,6-dioxopiperidin-3-yl)-1,3-dioxoisoindolin-5-carbaldehyde (see Org. Biomol. Chem., 2013, 11, 4757-4763 for the synthetic method) (86 mg, 0.3 mmol) was added, and the reaction was carried out at room temperature for 1 h, sodium triacetoxyborohydride (63 mg, 0.3 mmol) was added, and the mixture was reacted at room temperature for another 12 h with stirring. To the reaction solution was added 30 mL of dichloromethane, and the pH of the system was adjusted to 9.0 with saturated sodium bicarbonate solution. The organic phase was separated, dried over anhydrous sodium sulfate, and concentrated under reduced pressure, and then the crude product was passed through Pre-HPLC (instrument and preparative column: using Glison GX-281 to prepare the liquid phase, preparative column model: Sunfire C18, 5 µm, inner diameter × length = 30 mm × 150 mm). Preparation method: The crude product was dissolved with methanol and dimethyl sulfoxide, and filtered with 0.45 µm filter membrane, to prepare into a sample solution. Mobile phase system: acetonitrile/water (containing 0.1% TFA). Gradient elution method: gradient elution with acetonitrile from 5% to 60% (elution time: 15 min), lyophilization was performed to obtain 5-((3-((3-(4-(4-amino-3-(4-phenoxyphenyl)-1H-pyrazolo[3,4-d]pyrimidin-1-yl)piperidin-1-yl)azetidin-1-yl)methyl)azetidin-1-yl)methyl)-2-(2,6-dioxopiperidin-3-yl)isoindoline-1,3-dione (compound 109) trifluoroacetate (40 mg).
¹H NMR (400 MHz, DMSO-*d₆*) 11.13 (br.s, 1H), 8.32 (s, 1H), 8.08 - 8.01 (m, 2H), 7.98 - 7.91 (m, 1H), 7.70 - 7.63 (m, 2H), 7.49 - 7.41 (m, 2H), 7.27 - 7.09 (m, 5H), 5.18 (dd, 1H), 4.95 - 4.80 (m, 1H), 4.59 (s, 2H), 4.32 - 4.08 (m, 5H), 4.08 - 3.96 (m, 4H), 3.49 - 3.39 (m, 2H), 3.22 - 3.11 (m, 2H), 3.06 - 2.82 (m, 2H), 2.70 - 2.51 (m, 4H), 2.40 - 2.24 (m, 2H), 2.14 - 2.00 (m, 3H).
LCMS m/z = 781.3 [M+1]⁺.

### Example 110:

### 5-(3-((3-((3 S,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 110)

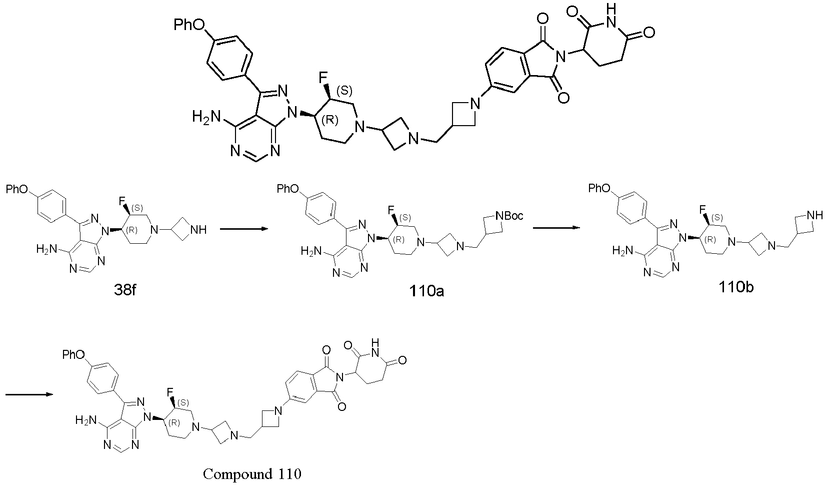

### Step 1:

### tert-butyl 3-((3-((3 S,4R)-4-(4-amino-3-(4-phenoxyphenyl)-1H-pyrazolo[3,4-d]pyrimidin-1-yl) -3-fluoropiperidin-1-yl)azetidin-1-yl)methyl)azetidine-1-carboxylate (110a)

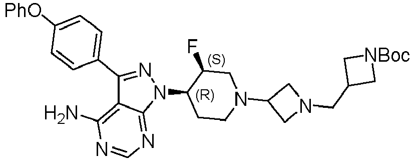

1-((3S,4R)-1-(azetidin-3-yl)-3 -fluoropiperidin-4-yl)-3 -(4-phenoxyphenyl)-1H-pyrazole[3,4-d]pyrimidin-4-amine (38f) (see WO 2020239103 for the synthetic method) (1.4 g, 3.0 mmol) was added to 50 mL of 1,2-dichloroethane, tert-butyl 3-formylazetidine-1-carboxylate (0.63 g, 3.4 mmol) was added, the mixture was stirred at room temperature for 1 h, and sodium triacetoxyborohydride (0.72 g, 3.4 mmol) was added. The reaction was carried out at room temperature for 12 h. To the reaction solution was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 3-((3-((3S,4R)-4-(4-amino-3-(4-phenoxyphenyl)-1H-pyrazolo[3,4-d]pyrimidin-1-yl)-3-fluoropiperidin-1-yl)azetidin-1-yl)methylazetidine-1-carboxylate (110a) (1.8 g, yield: 95%).
LCMS m/z = 629.3 [M+1]⁺.

### Step 2:

### 1-((3S,4R)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (110b)

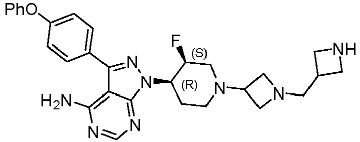

Tert-butyl 3-((3-((3S,4R)-4-(4-amino-3-(4-phenoxyphenyl)-1H-pyrazolo[3,4-d]pyrimidin-1-yl)-3-fluoropiperidin-1-yl)azetidin-1-yl)methylazetidine-1-carboxylate (110a) (1.8 g, 2.9 mmol) was dissolved in 20 mL of methanol, 20 mL of 4 mol/L hydrochloric acid in 1,4-dioxane was added. The reaction was carried out at normal temperature for 1 h. The reaction solution was concentrated under reduced pressure, dissolved by adding 100 mL of dichloromethane, and the pH was adjusted to 12 with 3 mol/L sodium hydroxide solution. The organic phase was separated, dried over anhydrous sodium sulfate, and concentrated under reduced pressure, to obtain crude product 1-((3S,4R)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (110b) (1.5 g).

### Step 3:

### 5-(3-((3-((3 S,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 110)

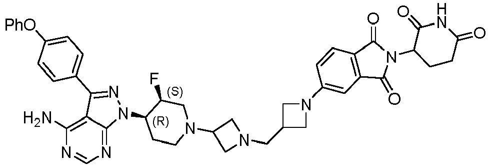

The above crude product 1-((3S,4R)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (110b) (0.62 g) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (0.5 g, 1.8 mmol) were added, and the reaction was warmed to 85°C and reacted for 3 h. The reaction solution was cooled to room temperature, and diluted with 100 mL of ethyl acetate. The organic phase was washed with 100 mL of water, dried over anhydrous sodium sulfate, and concentrated under reduced pressure. The crude product was separated and purified by silica gel column chromatography (dichloromethane /methanol (v/v) = 15 : 1), to obtain 5-(3-((3-((3S,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 110) (0.31 g, two-step yield from compound 110a: 33%).
¹H NMR (400 MHz, CDCl₃) δ 8.37 (d, 1H), 7.70 - 7.59 (m, 3H), 7.42 - 7.34 (m, 2H), 7.21 - 7.03 (m, 5H), 6.95 - 6.86 (m, 1H), 6.51 - 6.42 (m, 1H), 5.72 (br.s, 2H), 5.18 - 4.98 (m, 1H), 4.98 - 4.78 (m, 2H), 4.14 - 4.03 (m, 2H), 3.78 - 3.52 (m, 4H), 3.25 - 2.94 (m, 6H), 2.91 - 2.67 (m, 6H), 2.44 - 2.23 (m, 1H), 2.22 - 2.04 (m, 3H).
LCMS m/z = 785.3 [M+1]⁺.

### Example 111:

### 5-(3-((3-((3R,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 111)

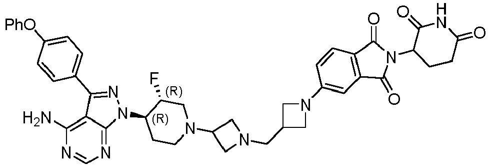

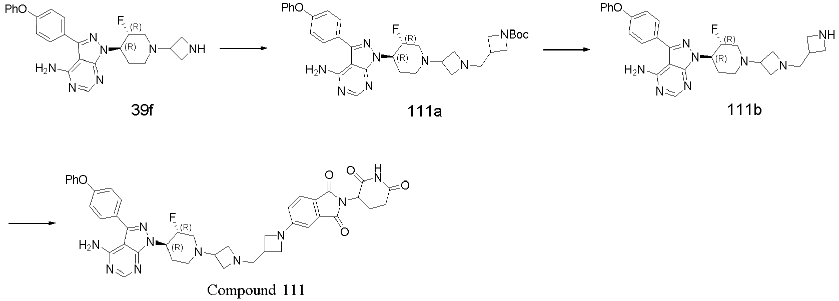

### Step 1:

### tert-butyl 3-((3-((3R,4R)-4-(4-amino-3-(4-phenoxyphenyl)-1H-pyrazolo[3,4-d]pyrimidin-1-yl)- 3-fluoropiperidin-1-yl)azetidin-1-yl)methyl)azetidine-1-carboxylate (111a)

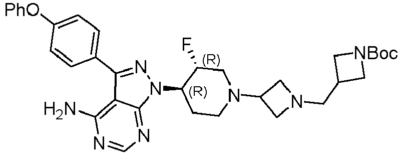

1-((3R,4R)-1-(azetidin-3-yl)-3-fluoropiperidin-4-yl)-3-(4-phenoxyphenyl)-1H-pyrazolo[3,4-d]pyrimidin-4-amine (39f) (see WO 2020239103 for the synthetic method) (2.5 g, 5.4 mmol) was added to 80 mL of 1,2-dichloroethane, tert-butyl 3-formylazetidine 1-carboxylate (1.1 g, 5.9 mmol) was added, the mixture was stirred at room temperature for 1 h, and sodium triacetoxyborohydride (1.3 g, 6.1 mmol) was added. The reaction was carried out at room temperature for 12 h. To the reaction solution was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 3-((3-((3R,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidine-1-carboxylate (111a) (3.3 g, yield: 97%).
LCMS m/z = 629.3 [M+1]⁺.

### Step 2:

### 1-((3R,4R)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (111b)

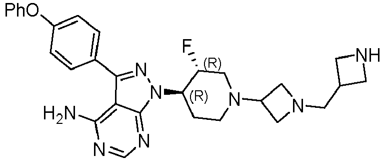

Tert-butyl 3-((3-((3R,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidine-1-carboxylate (111a) (3.3 g, 5.3 mmol) was dissolved in 50 mL of methanol, 50 mL of 4 mol/L hydrochloric acid in 1,4-dioxane was added. The reaction was carried out at normal temperature for 1 h. The reaction solution was concentrated under reduced pressure, dissolved by adding 100 mL of dichloromethane, and the pH was adjusted to 12 with 3 mol/L sodium hydroxide solution. The organic phase was separated, dried over anhydrous sodium sulfate, and concentrated under reduced pressure, to obtain crude product 1-((3R,4R)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (111b) (2.7 g).

### Step 3:

### 5-(3-((3-((3R,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 111)

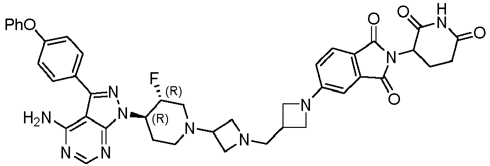

The above crude product 1-((3R,4R)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (1 1 1b) (1.95 g) was dissolved in 20 mL of DMSO, and 2.0 mL of DIPEA and 2-(2,6-dioxopiperidin-3-yl)-5-fluoroisoindoline-1,3-dione (see WO 2017197056 for the synthetic method) (1.6 g, 5.8 mmol) were added, and the reaction was warmed to 85°C and reacted for 3 h. The reaction solution was cooled to room temperature, and diluted with 100 mL of ethyl acetate. The organic phase was washed with 100 mL of water, dried over anhydrous sodium sulfate, and concentrated under reduced pressure. The crude product was separated and purified by silica gel column chromatography (dichloromethane /methanol (v/v) = 15 : 1), to obtain 5-(3-((3-((3R,4R)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 111) (1.4 g, two-step yield from compound 111a: 47%).
¹H NMR (400 MHz, CDCl₃) δ 11.49 (br.s, 1H), 8.41 (s, 1H), 7.80 - 7.52 (m, 3H), 7.47 - 7.25 (m, 2H), 7.24 - 7.00 (m, 5H), 6.98 - 6.84 (m, 1H), 6.57 - 6.37 (m, 1H), 5.98 (br.s, 2H), 5.38 - 5.06 (m, 1H), 5.04 - 4.76 (m, 2H), 4.25 - 3.95 (m, 2H), 3.82 - 3.48 (m, 4H), 3.35 - 2.97 (m, 4H), 2.96 - 2.65 (m, 7H), 2.58 - 2.30 (m, 1H), 2.25 - 1.95 (m, 4H).
LCMS m/z = 785.3 [M+1]⁺.

### Example 112:

### 5-(3-((3-((3S,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 112)

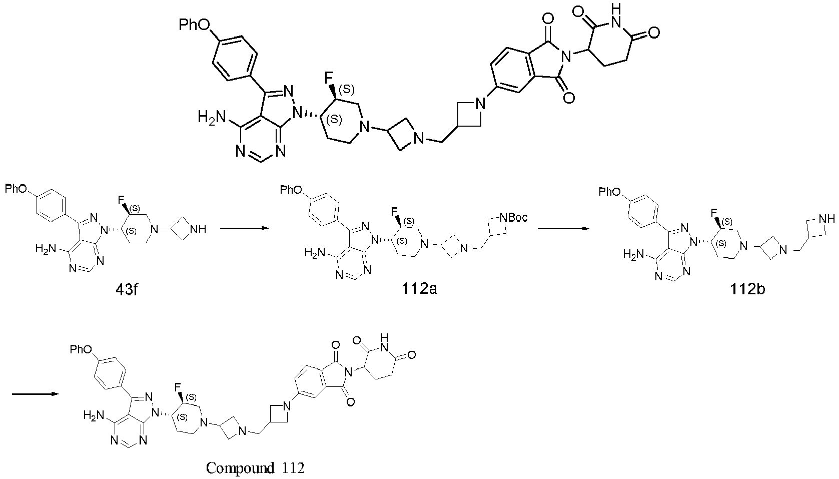

### Step 1:

### tert-butyl 3-((3-((3 S,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidine-1-carboxylate (112a)

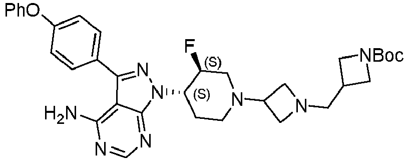

1-[(3S,4S)-1-(azetidin-3-yl)-3-fluoro-4-piperidyl]-3-(4-phenoxyphenyl)pyrazolo[3,4-d]pyrimidin-4-amine (43f) (see WO 2020239103 for the synthetic method) (1.05 g, 2.3 mmol) was added to 50 mL of 1,2-dichloroethane, tert-butyl 3-formylazetidine-1-carboxylate (0.46 g, 2.5 mmol) was added, the mixture was stirred at room temperature for 1 h, and sodium triacetoxyborohydride (0.53 g, 2.5 mmol) was added. The reaction was carried out at room temperature for 12 h. To the reaction solution was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 3-((3-((3S,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidine-1-carboxylate (112a) (1.35 g, yield: 93%).
LCMS m/z = 629.3 [M+1]⁺.

### Step 2:

### 1-((3 S,4S)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (112b)

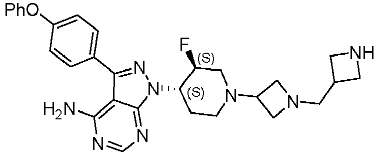

Tert-butyl 3-((3-((3 S,4S)-4-(4-amino-3 -(4-phenoxyphenyl)-1H-pyrazolo[3,4-d]pyrimidin-1-yl)-3-fluoropiperidin-1-yl)azetidin-1-yl)methyl)azetidine-1-carboxylate (112a) (1.35 g, 2.15 mmol) was dissolved in 20 mL of methanol, 20 mL of 4 mol/L hydrochloric acid in 1,4-dioxanewas added. The reaction was carried out at normal temperature for 1 h. The reaction solution was concentrated under reduced pressure, dissolved by adding 100 mL of dichloromethane, and the pH was adjusted to 12 with 3 mol/L sodium hydroxide solution. The organic phase was separated, dried over anhydrous sodium sulfate, and concentrated under reduced pressure, to obtain crude product 1-((3S,4S)-1-(1 -(azetidin-3-ylmethyl)azetidin-3-yl)-3 -fluoropiperidin-4-yl)-3-(4-phenoxyphenyl)-1H-pyrazolo[3,4-d]pyrimidin-4-amine (112b) (1.1 g).

### Step 3:

### 5-(3-((3-((3S,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 112)

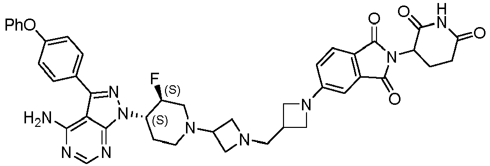

The above crude product 1-((3S,4S)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (112b) (0.7 g) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (0.5 g, 1.8 mmol) were added, and the reaction was warmed to 85°C and reacted for 3 h. The reaction solution was cooled to room temperature, and diluted with 100 mL of ethyl acetate. The organic phase was washed with 100 mL of water, dried over anhydrous sodium sulfate, and concentrated under reduced pressure. The crude product was separated and purified by silica gel column chromatography (dichloromethane /methanol (v/v) = 15 : 1), to obtain 5-(3-((3-((3S,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)isoindoline-1,3-dione (compound 112) (0.33 g, two-step yield from compound 112a: 31%).
¹H NMR (400 MHz, CDCl₃) δ 8.40 (s, 1H), 7.72 - 7.58 (m, 3H), 7.44 - 7.32 (m, 2H), 7.23 - 7.00 (m, 5H), 6.93 - 6.83 (m, 1H), 6.53 - 6.43 (m, 1H), 5.74 (br.s, 2H), 5.34 - 5.08 (m, 1H), 5.02 - 4.80 (m, 2H), 4.17 - 3.97 (m, 2H), 3.80 - 3.55 (m, 4H), 3.30 - 2.97 (m, 4H), 2.97 - 2.66 (m, 7H), 2.52 - 2.34 (m, 1H), 2.24 - 1.98 (m, 4H).
LCMS m/z = 785.3 [M+1]⁺.

### Example 113:

### 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)-3-fluoroazetidin-1-yl)-2-(2,6-dioxopiperidin-3-yl)-6-fluoroisoindoline-1,3-dione (compound 113)

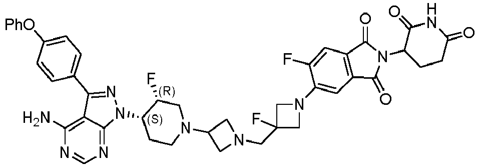

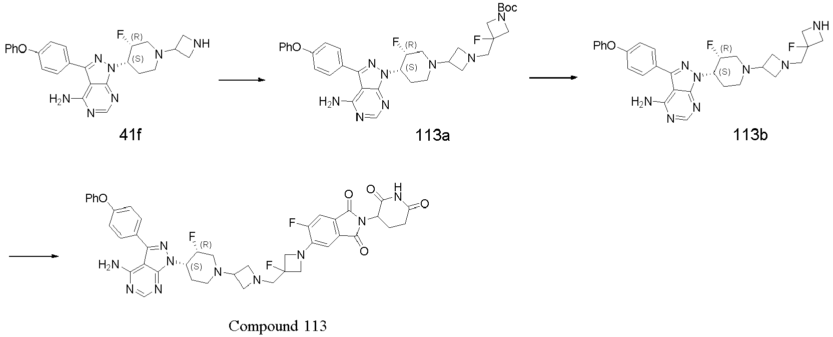

### Step 1:

### tert-butyl 3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)-3-fluoroazetidine-1-carboxylate (113a)

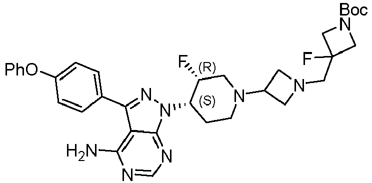

1-((3R,4S)-1-(azetidin-3-yl)-3-fluoropiperidin-4-yl)-3 -(4-phenoxyphenyl)-1H-pyrazolo[3,4-d]pyrimidin-4-amine (41f) (see WO 2020239103 for the synthetic method) (510 mg, 1.11 mmol) was added to 50 mL of 1,2-dichloroethane, tert-butyl 3-fluoro-3-formylazetidine-1-carboxylate (see WO 2018089355 for the synthetic method) (304 mg, 1.5 mmol) was added, the reaction was carried out at room temperature for 1 h, then sodium triacetoxyborohydride (318 mg, 1.5 mmol) was added, and the mixture was reacted at room temperature for another 12 h. To the reaction system was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)-3-fluoroazetidine-1-carboxylate (113a) (600 mg, yield: 84%).
LCMS m/z = 647.5 [M+1]⁺.

### Step 2:

### 1-((3R,4S)-3-fluoro-1-(1-((3-fluoroazetidin-3-yl)methyl)azetidin-3-yl)piperidin-4-yl)-3-(4-phenoxyphenyl)-1H-pyrazolo[3,4-d]pyrimidin-4-amine (113b)

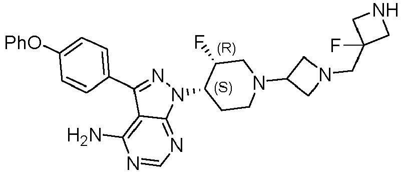

Tert-butyl 3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)-3-fluoroazetidine-1-carboxylate (113a) (600 mg, 0.93 mmol) was dissolved in 20 mL of methanol, 20 mL of 4 mol/L hydrochloric acid in 1,4-dioxanewas added. The reaction was carried out at room temperature for 1 h. The reaction solution was concentrated under reduced pressure, dissolved by adding 100 mL of dichloromethane, and the pH was adjusted to 12.0 with 3 mol/L sodium hydroxide solution. The organic phase was separated, dried over anhydrous sodium sulfate, and concentrated under reduced pressure, to obtain crude product 1-((3R,4S)-3-fluoro-1-(1-((3-fluoroazetidin-3-yl)methyl)azetidin-3-yl)-piperidin-4-yl)-3-(4-phenoxyphenyl)-1H-pyrazolo[3,4-d]pyrimidin-4-amine (113b) (450 mg).
LCMS m/z = 547.3 [M+1]⁺.

### Step 3:

### 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)-3-fluoroazetidin-1-yl)-2-(2,6-dioxopiperidin-3-yl)-6-fluoroisoindoline-1,3-dione (compound 113)

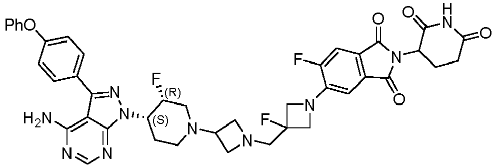

The above crude product 1-((3R,4S)-3-fluoro-1-(1-((3-fluoroazetidin-3-yl)methyl)azetidin-3-yl)-piperidin-4-yl)-3-(4-phenoxyphenyl)-1H-pyrazolo[3,4-d]pyrimidin-4-amine (113b) (450 mg) was dissolved in 10 mL of DMSO, and 1.0 mL of DIPEA and 2-(2,6-dioxopiperidin-3-yl)-5.6-difluoroisoindoline-1,3-dione (see WO 2020239103 for the synthetic method) (300 mg, 1.02 mmol) were added, and the reaction was stirred at 85°C for 3 h. The reaction solution was cooled to room temperature, 200 mL of water was added, and the reaction solution was filtered. The filter cake was dissolved with 100 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)-3-fluoroazetidin-1-yl)-2-(2,6-dioxopiperidin-3-yl)-6-fluoroisoindoline-1,3-dione (compound 113) (300 mg, two-step yield from compound 113a: 39%).
¹H NMR (400 MHz, CDCl₃) δ 8.31 (d, 1H), 7.62 - 7.56 (m, 2H), 7.35 - 7.27 (m, 3H), 7.14 - 7.03 (m, 3H), 7.03 - 6.98 (m, 2H), 6.98 - 6.87 (m, 1H), 5.61 (br.s, 2H), 5.12 - 4.71 (m, 3H), 4.25 - 4.06 (m, 4H), 3.73 - 3.53 (m, 2H), 3.38 - 3.15 (m, 2H), 3.15 - 3.04 (m, 2H), 3.04 - 2.88 (m, 4H), 2.84 - 2.64 (m, 3H), 2.36 - 2.16 (m, 1H), 2.16 - 2.07 (m, 1H), 2.07 - 1.96 (m, 2H).
LCMS m/z = 821.3 [M+1]⁺.

### Example 114:

### 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)-6-fluoroisoindoline-1,3-dione (compound 114)

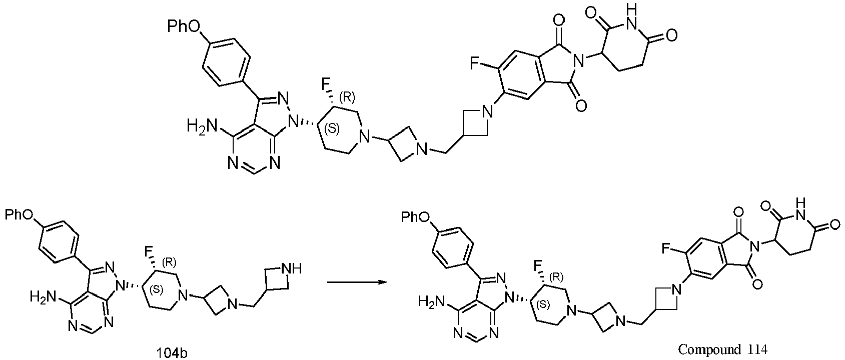

The above crude product 1-((3R,4S)-1-(1-(azetidin-3-ylmethyl)azetidin-3-yl)-3-fluoropiperidin-4-yl)-3-(4-phenoxyphenyl)-1H-pyrazolo[3,4-d]pyrimidin-4-amine (104b) (450 mg) was dissolved in 10 mL of DMSO, and 1.0 mL of DIPEA and 2-(2,6-dioxopiperidin-3-yl)-5.6-difluoroisoindoline-1,3-dione (see WO 2020239103 for the synthetic method) (300 mg, 1.02 mmol) were added, and the reaction was stirred at 85°C for 3 h. The reaction solution was cooled to room temperature, 200 mL of water was added, and the reaction solution was filtered. The filter cake was dissolved with 100 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-((3-((3R,4S)-4-(4-amino-3-(4-phenoxyphenyl)-1H-pyrazolo[3,4-d]pyrimidin-1-yl)-3-fluoropiperidin-1-yl)azetidin-1-yl)methyl)azetidin-1-yl)-2-(2,6-dioxopiperidin-3-yl)-6-fluoroisoindoline-1,3-dione (compound 114) (200 mg, two-step yield from compound 104a: 28%).
¹H NMR (400 MHz, CDCl₃) δ 8.37 (d, 1H), 7.69 - 7.62 (m, 2H), 7.42 - 7.30 (m, 3H), 7.20 - 7.04 (m, 5H), 7.02 - 6.91 (m, 1H), 5.63 (br.s, 2H), 5.20 - 4.78 (m, 3H), 4.30 - 4.17 (m, 2H), 3.94 - 3.77 (m, 2H), 3.77 - 3.52 (m, 2H), 3.32 - 2.97 (m, 6H), 2.97 - 2.65 (m, 6H), 2.43 - 2.14 (m, 2H), 2.14 - 2.02 (m, 2H).
LCMS m/z = 803.3 [M+1]⁺.

### Example 115:

### 5-(3-((3-(4-(4-amino-3-(4-phenoxyphenyl)-1H-pyrazolo[3,4-d]pyrimidin-1-yl)piperidin-1-yl)azetidin-1-yl)methyl)-3-fluoroazetidin-1-yl)-2-(2,6-dioxopiperidin-3-yl)-6-fluoroisoindoline-1,3-dione (compound 115)

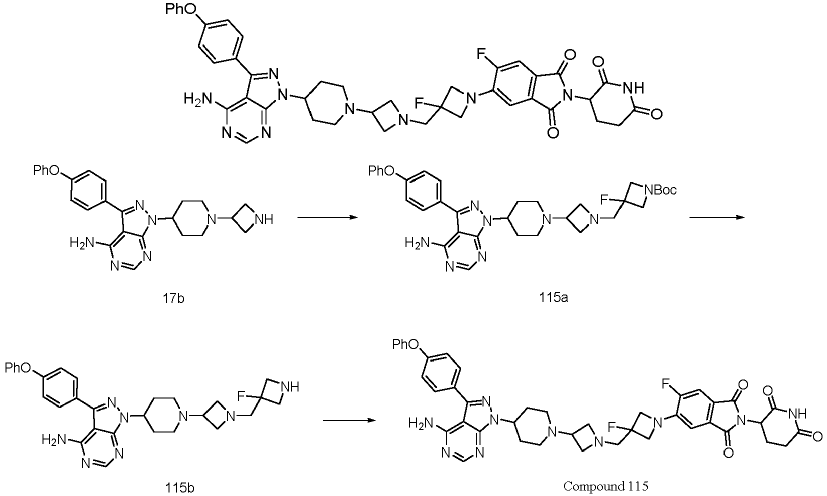

### Step 1:

### tert-butyl 3-((3-(4-(4-amino-3-(4-phenoxyphenyl)-1H-pyrazolo[3,4-d]pyrimidin-1-yl) piperidin-1-yl)azetidin-1-yl)methyl)-3-fluoroazetidine-1-carboxylate (115a)

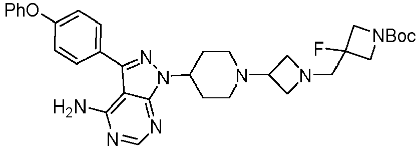

1-[1-(azetidin-3-yl)-4-piperidyl]-3-(4-phenoxyphenyl)pyrazolo[3,4-d]pyrimidin-4-amine (17b) (see WO 2020239103 for the synthetic method) (900 mg, 2.04 mmol) was added to 50 mL of 1,2-dichloroethane, tert-butyl 3-fluoro-3-formylazetidine-1-carboxylate (see WO 2018089355 for the synthetic method) (610 mg, 3.0 mmol) was added, the reaction was carried out at room temperature for 1 h, then sodium triacetoxyborohydride (630 mg, 3.0 mmol) was added, and the mixture was reacted at room temperature for another 12 h. To the reaction system was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 3-((3-(4-(4-amino-3-(4-phenoxyphenyl)-1H-pyrazolo[3,4-d]pyrimidin-1-yl)-piperidin-1-yl)azetidin-1-yl)methyl)-3-fluoroazetidine-1-carboxylate (115a) (1.0 g, yield: 78%).
LCMS m/z = 629.3 [M+1]⁺.

### Step 2:

### 1-(1-(1-((3-fluoroazetidin-3-yl)methyl)azetidin-3-yl)piperidin-4-yl)-3-(4-phenoxyphenyl)-1H-pyrazolo[3,4-d]pyrimidin-4-amine (115b)

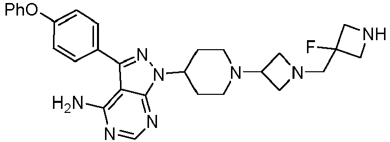

Tert-butyl 3 -((3 -(4-(4-amino-3-(4-phenoxyphenyl)-1H-pyrazolo[3,4-d]pyrimidin-1-yl)-piperidin-1-yl)azetidin-1-yl)methyl)-3-fluoroazetidine-1-carboxylate (115a) (1.0 g, 1.6 mmol) was dissolved in 20 mL of methanol, 20 mL of 4 mol/L hydrochloric acid in 1,4-dioxane was added. The reaction was carried out at room temperature for 1 h. The reaction solution was concentrated under reduced pressure, dissolved by adding 100 mL of dichloromethane, and the pH was adjusted to 12.0 with 3 mol/L sodium hydroxide solution. The organic phase was separated, dried over anhydrous sodium sulfate, and concentrated under reduced pressure, to obtain crude product 1-(1-(1-((3-fluoroazetidin-3-yl)methyl)azetidin-3-yl)-piperidin-4-yl)-3-(4-phenoxyphenyl)-1H-pyrazolo[3,4-d]pyrimidin-4-amine (115b) (830 mg).
LCMS m/z = 529.3 [M+1]⁺.

### Step 3:

### 5-(3-((3-(4-(4-amino-3-(4-phenoxyphenyl)-1H-pyrazolo[3,4-d]pyrimidin-1-yl)piperidin-1-yl)azetidin-1-yl)methyl)-3-fluoroazetidin-1-yl)-2-(2,6-dioxopiperidin-3-yl)-6-fluoroisoindoline-1,3-dione (compound 115)

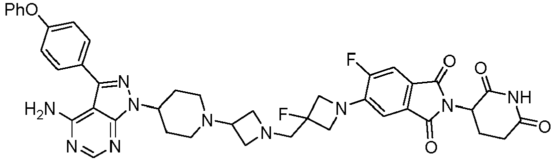

The above crude product 1-(1-(1-((3-fluoroazetidin-3-yl)methyl)azetidin-3-yl)-piperidin-4-yl)-3-(4-phenoxyphenyl)-1H-pyrazolo[3,4-d]pyrimidin-4-amine (115b) (400 mg) was dissolved in 10 mL of DMSO, and 1.0 mL of DIPEA and 2-(2,6-dioxopiperidin-3-yl)-5.6-difluoroisoindoline-1,3-dione (see WO 2020239103 for the synthetic method) (300 mg, 1.02 mmol) were added, and the reaction was stirred at 85°C for 3 h. The reaction solution was cooled to room temperature, 200 mL of water was added, and the reaction solution was filtered. The filter cake was dissolved with 100 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-((3-(4-(4-amino-3-(4-phenoxyphenyl)-1H-pyrazolo[3,4-d]pyrimidin-1-yl)-piperidin-1-yl)azetidin-1-yl)methyl)-3-fluoroazetidin-1-yl)-2-(2,6-dioxopiperidin-3-yl)-6-fluoroisoindoline-1,3-dione (compound 115) (420 mg, two-step yield from compound 115a: 68%).
¹H NMR (400 MHz, CDCl₃) δ 10.35 (br.s, 1H), 8.31 (s, 1H), 7.62 - 7.53 (m, 2H), 7.37 - 7.26 (m, 3H), 7.14 - 6.97 (m, 5H), 6.91 (d, 1H), 5.71 (br.s, 2H), 4.92 - 4.82 (m, 1H), 4.80 - 4.64 (m, 1H), 4.26 - 4.00 (m, 4H), 3.69 - 3.54 (m, 2H), 3.28 - 3.09 (m, 2H), 3.09 - 2.95 (m, 2H), 2.95 - 2.83 (m, 3H), 2.83 - 2.61 (m, 3H), 2.44 - 2.26 (m, 2H), 2.16 - 1.92 (m, 5H).
LCMS m/z = 803.2 [M+1]⁺.

### Example 116:

### 5-(3-((3-(4-(4-amino-3-(4-phenoxyphenyl)-1H-pyrazolo[3,4-d]pyrimidin-1-yl)piperidin-1-yl)azetidin-1-yl)methyl)-3-fluoroazetidin-1-yl)-2-(2,6-dioxopiperidin-3-yl)isoindoline-1,3-dione (compound 116)

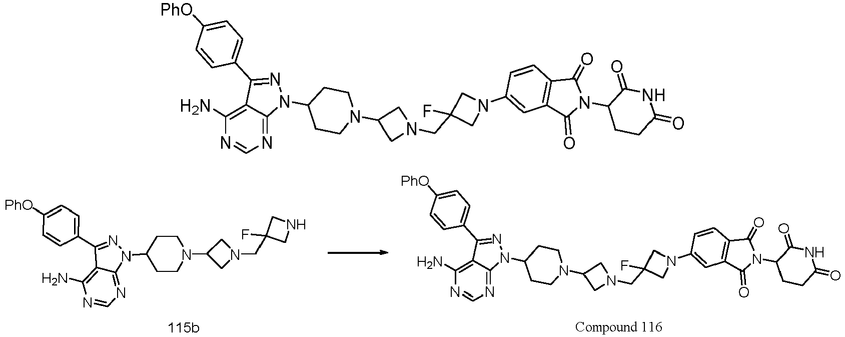

The above crude product 1-(1-(1-((3-fluoroazetidin-3-yl)methyl)azetidin-3-yl)-piperidin-4-yl)-3-(4-phenoxyphenyl)-1H-pyrazolo[3,4-d]pyrimidin-4-amine (115b) (430 mg) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (300 mg, 1.1 mmol) were added, and the reaction was stirred at 85°C for 3 h. The reaction solution was cooled to room temperature, 200 mL of water was added, and the reaction solution was filtered. The filter cake was dissolved with 100 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(3-((3-(4-(4-amino-3-(4-phenoxyphenyl)-1H-pyrazolo[3,4-d]pyrimidin-1-yl)-piperidin-1-yl)azetidin-1-yl)methyl)-3-fluoroazetidin-1-yl)-2-(2,6-dioxopiperidin-3-yl)-isoindoline-1,3-dione (compound 116) (420 mg, two-step yield from compound 115a: 65%).
¹H NMR (400 MHz, CDCl₃) δ 9.43 (br.s, 1H), 8.37 (s, 1H), 7.70 - 7.61 (m, 3H), 7.43 - 7.35 (m, 2H), 7.21 - 7.04 (m, 5H), 6.87 (d, 1H), 6.56 (dd, 1H), 5.72 (br.s, 2H), 5.00 - 4.70 (m, 2H), 4.18 - 4.01 (m, 4H), 3.74 - 3.62 (m, 2H), 3.34 - 3.07 (m, 3H), 3.07 - 2.67 (m, 7H), 2.53 - 2.36 (m, 2H), 2.20 - 1.95 (m, 5H).
LCMS m/z = 785.3 [M+1]⁺.

### Example 117:

### 5-(4-((3-(4-(4-amino-3-(4-phenoxyphenyl)-1H-pyrazolo[3,4-d]pyrimidin-1-yl)piperidin-1-yl)azetidin-1-yl)methyl)-4-fluoropiperidin-1-yl)-2-(2,6-dioxopiperidin-3-yl)-6-fluoroisoindoline-1,3-dione (compound 117)

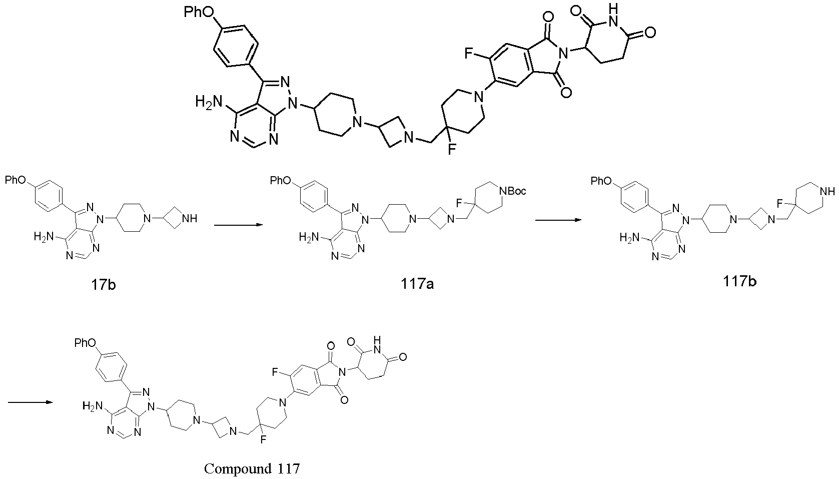

### Step 1:

### tert-butyl 4-((3-(4-(4-amino-3-(4-phenoxyphenyl)-1H-pyrazolo[3,4-d]pyrimidin-1-yl) piperidin-1-yl)azetidin-1-yl)methyl)-4-fluoropiperidine-1-carboxylate (117a)

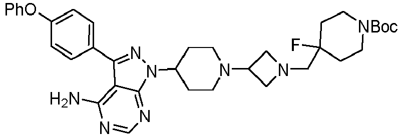

1-[1-(azetidin-3-yl)-4-piperidyl]-3-(4-phenoxyphenyl)pyrazolo[3,4-d]pyrimidin-4-amine (17b) (see WO 2020239103 for the synthetic method) (1.5 g, 3.4 mmol) was added to 50 mL of 1,2-dichloroethane, tert-butyl 4-fluoro-4-formylpiperidine-1-carboxylate (920 mg, 4.0 mmol) was added, the reaction was carried out at room temperature for 1 h, then sodium triacetoxyborohydride (844 mg, 4.0 mmol) was added, and the mixture was reacted at room temperature for another 12 h. To the reaction system was added 50 mL of dichloromethane, and the pH was adjusted to 9.0 with saturated sodium bicarbonate solution. The organic phase was separated, dried over anhydrous sodium sulfate, and concentrated under reduced pressure. The crude product was separated and purified by silica gel column chromatography (dichloromethane/methanol (v/v) = 10 : 1), to obtain tert-butyl 4-((3-(4-(4-amino-3-(4-phenoxyphenyl)-1H-pyrazolo[3,4-d]pyrimidin-1-yl)piperidin-1-yl)azetidin-1-yl)methyl)-4-fluoropiperidine-1-carboxylate (117a) (1.2 g, yield: 54%).
LCMS m/z = 657.3 [M+1]⁺.

### Step 2:

### 1-(1-(1-((4-fluoropiperidin-4-yl)methyl)azetidin-3-yl)piperidin-4-yl)-3-(4-phenoxyphenyl)-1H-pyrazolo[3,4-d]pyrimidin-4-amine (117b)

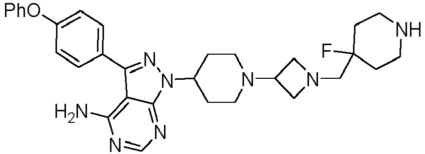

Tert-butyl 4-((3-(4-(4-amino-3-(4-phenoxyphenyl)-1H-pyrazolo[3,4-d]pyrimidin-1-yl)piperidin-1-yl)azetidin-1-yl)methyl)-4-fluoropiperidine-1-carboxylate (117a) (1.2 g, 1.8 mmol) was dissolved in 20 mL of methanol, 20 mL of 4 mol/L hydrochloric acid in 1,4-dioxane was added. The reaction was carried out at room temperature for 1 h. The reaction solution was concentrated under reduced pressure, dissolved by adding 100 mL of dichloromethane, and the pH was adjusted to 12.0 with 3 mol/L sodium hydroxide solution. The organic phase was separated, dried over anhydrous sodium sulfate, and concentrated under reduced pressure, to obtain crude product 1-(1-(1-((4-fluoropiperidin-4-yl)methyl)azetidin-3-yl)piperidin-4-yl)-3-(4-phenoxyphenyl)-1H-pyrazolo[3,4-d]pyrimidin-4-amine (117b) (980 mg).
LCMS m/z = 557.3 [M+1]⁺.

### Step 3:

### 5-(4-((3-(4-(4-amino-3-(4-phenoxyphenyl)-1H-pyrazolo[3,4-d]pyrimidin-1-yl)piperidin-1-yl)azetidin-1-yl)methyl)-4-fluoropiperidin-1-yl)-2-(2,6-dioxopiperidin-3-yl)-6-fluoroisoindoline-1,3-dione (compound 117)

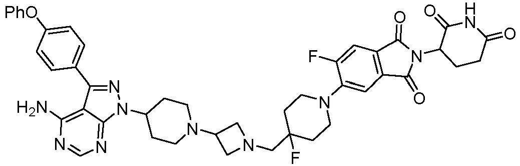

The above crude product 1-(1-(1-((4-fluoropiperidin-4-yl)methyl)azetidin-3-yl)piperidin-4-yl)-3-(4-phenoxyphenyl)-1H-pyrazolo[3,4-d]pyrimidin-4-amine (117b) (450 mg) was dissolved in 10 mL of DMSO, and 1.0 mL of DIPEA and 2-(2,6-dioxopiperidin-3-yl)-5,6-difluoroisoindoline-1,3-dione (see WO 2020239103 for the synthetic method) (300 mg, 1.02 mmol) were added, and the reaction was stirred at 85°C for 3 h. The reaction solution was cooled to room temperature, 200 mL of water was added, and the reaction solution was filtered. The filter cake was dissolved with 100 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(4-((3-(4-(4-amino-3-(4-phenoxyphenyl)-1H-pyrazolo[3,4-d]pyrimidin-1-yl)-piperidin-1-yl)azetidine-1-yl)methyl)-4-fluoropiperidin-1-yl)-2-(2,6-dioxopiperidin-3-yl)-6-fluoroisoindoline-1,3-dione (compound 117) (230 mg, two-step yield from compound 117a: 33%).
¹H NMR (400 MHz, CDCl₃) δ 9.71 (br.s, 1H), 8.31 (s, 1H), 7.62 - 7.52 (m, 2H), 7.44 - 7.26 (m, 4H), 7.14 - 6.96 (m, 5H), 5.69 (br.s, 2H), 4.92 - 4.81 (m, 1H), 4.78 - 4.63 (m, 1H), 3.63 - 3.49 (m, 2H), 3.46 - 3.32 (m, 2H), 3.18 - 3.05 (m, 2H), 3.05 - 2.92 (m, 3H), 2.92 - 2.50 (m, 7H), 2.43 - 2.26 (m, 2H), 2.11 - 1.90 (m, 7H), 1.79 - 1.62 (m, 2H).
LCMS m/z = 831.3 [M+1]⁺.

### Example 118:

### 5-(4-((3-(4-(4-amino-3-(4-phenoxyphenyl)-1H-pyrazolo[3,4-d]pyrimidin-1-yl)piperidin-1-yl)azetidin-1-yl)methyl)-4-fluoropiperidin-1-yl)-2-(2,6-dioxopiperidin-3-yl)isoindoline-1,3-dione (compound 118)

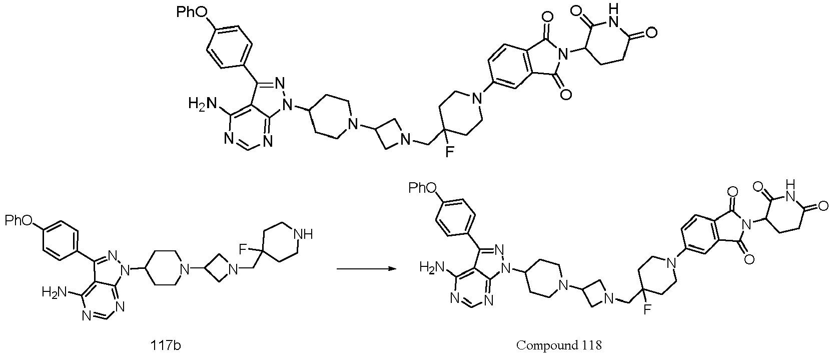

The above crude product 1-(1-(1-((4-fluoropiperidin-4-yl)methyl)azetidin-3-yl)piperidin-4-yl)-3-(4-phenoxyphenyl)-1H-pyrazolo[3,4-d]pyrimidin-4-amine (117b) (500 mg) was dissolved in 10 mL of DMSO, and 1.0 mL of DIPEA and 2-(2,6-dioxopiperidin-3-yl)-5-fluoroisoindoline-1,3-dione (see WO 2017197056 for the synthetic method) (300 mg, 1.1 mmol) were added, and the reaction was stirred at 85°C for 3 h. The reaction solution was cooled to room temperature, 200 mL of water was added, and the reaction solution was filtered. The filter cake was dissolved with 100 mL of dichloromethane, dried over anhydrous sodium sulfate, and concentrated under reduced pressure, and then the crude product was separated and purified by silica gel column chromatography (dichloromethane/methanol (v/v) = 15 : 1), to obtain 5-(4-((3-(4-(4-amino-3-(4-phenoxyphenyl)-1H-pyrazolo[3,4-d]pyrimidin-1-yl)-piperidin-1-yl)azetidin-1-yl)methyl)-4-fluoropiperidin-1-yl)-2-(2,6-dioxopiperidin-3-yl)-isoindoline-1,3-dione (compound 118) (300 mg, two-step yield from compound 117a: 40%).
¹H NMR (400 MHz, CDCl₃) δ 8.97 (br.s, 1H), 8.31 (s, 1H), 7.65 - 7.53 (m, 3H), 7.36 - 7.27 (m, 2H), 7.22 (d, 1H), 7.15 - 6.95 (m, 6H), 5.56 (br.s, 2H), 4.92 - 4.82 (m, 1H), 4.80 - 4.64 (m, 1H), 3.75 - 3.65 (m, 2H), 3.63 - 3.52 (m, 2H), 3.32 - 3.19 (m, 2H), 3.17 - 2.96 (m, 3H), 2.93 - 2.56 (m, 7H), 2.44 - 2.28 (m, 2H), 2.14 - 1.86 (m, 7H), 1.75 - 1.53 (m, 2H).
LCMS m/z = 813.4 [M +1]⁺.

### VII: Effects

### 1. Experiment on inhibiting cell proliferation

SU-DHL-4, Mino, and SU-DHL-6 cells, in the culture medium of RPMI1640 + 10% FBS, were cultured in an incubator at 37°C under 5% CO₂. Cells were plated in a 96-well plate. Specifically, SU-DHL-4 cells were at 20000/well, Mino and SU-DHL-6 cells were at 5000/well, with 90 µL/well. 10 µL of the compounds at different concentrations were added to each well. There were 3 replicate wells for each concentration, and the last column, added with DMSO, was used as a vehicle control group. The cultures were continued for 72 hours at 37°C under 5% CO₂. After 72 hours, 100 µL of detection reagent (Cell Viability Assay, Promega, G7573) was added to each well, and the cultures were uniformly mixed for 2 minutes, and incubated at room temperature for 10 minutes. The fluorescence signal value was measured with a microplate reader (PHERAstar FSX). The IC₅₀ value of the compound on cell proliferation inhibition was calculated using origin9.2 software, and the inhibition rate at the highest concentration of the compound (Max inhi.%) was calculated according to formula (1). $Max inhi .% = \left(1-T{72}_{administration}/T{72}_{vehicle}\right) \times 100$

OCI-LY10 and TMD-8 cells, in the culture medium of RPMI1640 + 10% FBS, were cultured in an incubator at 37°C under 5% CO₂. Cells were plated in a 96-well plate. Specifically, OCI-LY10 cells were at 10000/well, and TMD-8 cells were at 8000/well, with 90 µL/well. These cells were cultured at 37°C under 5% CO₂ overnight. On the next day, 10 µL of the compounds at different concentrations were added to each well. There were 3 replicate wells for each concentration, and the last column, added with DMSO, was used as a vehicle control group. The cultures were continued for 72 hours at 37°C under 5% CO₂. After 72 hours, 100 µL of detection reagent (Cell Viability Assay, Promega, G7573) was added to each well, and the cultures were uniformly mixed for 2 minutes, and incubated at room temperature for 10 minutes. The fluorescence signal value was measured with an Envision2104 plate reader (PerkinElmer). The inhibition rate was calculated using formula (2), wherein RLU _{compound} was the readout of the drug treated group, RLU _{control} was the average value of the vehicle control group, and RLU _{blank} was the average value of the cell-free wells. The IC₅₀ value was calculated using GraphPad Prism software. $IR \left(%\right) = \left(1-\left({RLU}_{compound}-{RLU}_{blank}\right)/\left({RLU}_{control}-{RLU}_{blank}\right)\right) * 100 %$

The results for IC₅₀ values on proliferation inhibition of Mino cells were shown in Table 1.

**Table 1 IC₅₀ value on proliferation inhibition of Mino cells**

| Serial No. | Compound No. | IC50 (nM) |
|---|---|---|
| 1 | P13I* | 844 |
| 2 | Compound 65 | 14 |
| 3 | Compound 66 | 27 |
| 4 | Compound 70 | 101 |
| 5 | Compound 75 | 37 |
| 6 | Compound 76 | 141 |
| 7 | Compound 84 | 32 |
| 8 | Compound 85 | 32 |
| 9 | Compound 86 | 42 |
| 10 | Compound 95 | 100 |
| 11 | Compound 96 | 501 |
| 12 | Trifluoroacetate of compound 98 | 20 |
| 13 | Trifluoroacetate of compound 99 | 21 |
| 14 | Trifluoroacetate of compound 100 | 123 |
| 15 | Trifluoroacetate of compound 101 | 77 |
| 16 | Trifluoroacetate of compound 102 | 42 |
| 17 | Compound 104 | 16 |
| 18 | Compound 105 | 208 |
| 19 | Compound 106 | 119 |
| 20 | Trifluoroacetate of compound 107 | 34 |
| 21 | Trifluoroacetate of compound 109 | 356 |
| 22 | Compound 110 | 35 |
| 23 | Compound 111 | 50 |
| 24 | Compound 112 | 39 |
| 26 | Compound 113 | 61 |
| 27 | Compound 114 | 66 |
| 28 | Compound 115 | 166 |
| 29 | Compound 116 | 29 |
| 30 | Compound 117 | 119 |
| 31 | Compound 118 | 52 |

| | | |
|---|---|---|
| Notes: denoting a trifluoroacetate thereof. | | |

The results for Max inhi.% values on proliferation inhibition of Mino cells were shown in Table 1-1.

**Table 1-1. Max inhi.% value on proliferation inhibition of Mino cells**

| Serial No. | Compound No. | Mino Max inhi.% |
|---|---|---|
| 1 | Compound 64 | 83.3 |
| 2 | Compound 68 | 94.2 |
| 3 | Compound 69 | 99.9 |
| 4 | Trifluoroacetate of compound 71 | 95.2 |
| 5 | Compound 72 | 88.5 |
| 6 | Compound 73 | 99.8 |
| 7 | Compound 74 | 88.3 |
| 8 | Compound 80 | 99.1 |
| 9 | Compound 81 | 99.9 |
| 10 | Compound 82 | 99.9 |
| 11 | Compound 83 | 99.7 |

The results for IC₅₀ values on proliferation inhibition of SU-DHL-4 cells were shown in Table 1-2.

**Table 1-2 IC₅₀ value on inhibition of SU-DHL-4 cells**

| Serial No. | Compound No. | IC₅₀(nM) |
|---|---|---|
| 1 | P13I* | 1184 |
| 2 | Compound 85 | 624 |

| | | |
|---|---|---|
| *Notes: denoting a trifluoroacetate thereof. | | |

The results for IC₅₀ values on proliferation inhibition of SU-DHL-6 cells were shown in Table 1-3.

**Table 1-3 IC₅₀ value on inhibition of SU-DHL-6 cells**

| Serial No. | Compound No. | IC₅₀ (nM) |
|---|---|---|
| 1 | P13I* | 1638 |
| 2 | Compound 85 | 26 |
| 3 | Trifluoroacetate of compound 98 | 12 |
| 4 | Trifluoroacetate of compound 99 | 24 |
| 5 | Trifluoroacetate of compound 100 | 99 |
| 6 | Trifluoroacetate of compound 101 | 77 |
| 7 | Trifluoroacetate of compound 102 | 23 |
| 4 | Compound 104 | 23 |
| 5 | Compound 105 | 331 |
| 6 | Compound 106 | 95 |
| 7 | Trifluoroacetate of compound 107 | 22 |
| 8 | Trifluoroacetate of compound 109 | 145 |
| 9 | Compound 110 | 31 |
| 10 | Compound 111 | 23 |
| 11 | Compound 112 | 36 |
| 12 | Compound 113 | 100 |
| 13 | Compound 114 | 24 |
| 14 | Compound 115 | 133 |
| 15 | Compound 116 | 52 |
| 16 | Compound 117 | 98 |
| 17 | Compound 118 | 50 |

| | | |
|---|---|---|
| *Notes: denoting a trifluoroacetate thereof. | | |

Conclusion: The compounds synthesized by using the technique of the present invention had a significant inhibitory effect on the proliferation of SU-DHL-4 cells and SU-DHL-6 cells (human B lymphoma cells), Mino cells (mantle cell lymphoma cells), OCI-LY10 cells (diffuse large B cell lymphoma cells) and TMD-8 cells (human diffuse large B lymphoma cells). In the embodiment of the invention, the IC₅₀ value of compounds 63-70, 72, 75-79, 84-86, 95-96, trifluoroacetate of 98-102, 104-106, trifluoroacetate of 107, trifluoroacetate of 109, and 110-118 on Mino cells (mantle cell lymphoma cells) is 10-1000 nM, and the maximum inhibition rate of compounds 64, 68, 69, trifluoroacetate of 71, 72-74, and 80-83 on Mino cells is higher than 80%, The IC₅₀ value of compound 85 on DHL-4 cells (human B lymphoma cells) is 10-1000 nM, and the IC₅₀ value of compound 85, trifluoroacetate of 98-102, 104-106, trifluoroacetate of 107, trifluoroacetate of 109, and 110-118 on DHL-6 cells (human B lymphoma cells) is 10-1000 nM.

### 2. Pharmacokinetic experiment in rats

**Experimental objective:** In this experiment, a single dose of each test compound was administered to SD rats intravenously and intragastrically, the concentrations of the test compound in plasma of rats were measured, and the pharmacokinetic characteristics and bioavailability of the test compound in rats were evaluated.

**Experimental animals:** Male SD rats, about 200 g, 6-8 weeks old, 6 rats/compound. Rats were purchased from Hunan SJA Laboratory Animal Co., Ltd, with the laboratory animal production license number of SCXK (Xiang) 2019-004.

**Experimental method:** On the day of the experiment, 6 SD rats were randomly grouped according to their body weight. The animals were fasted with water available for 12 to 14 hours one day before the administration of a test compound, and were fed 4 hours after the administration.

| Group | No. of rats | Administration information | | | | | | |
|---|---|---|---|---|---|---|---|---|
| | Male | Test compound | Administrati on dosage* (mg/kg) | Administration concentration (mg/mL) | Administrat ion volume (mL/kg) | Collected samples | Mode of administra tion | Vehicle |
| G1 | 3 | Compounds | 5 | 1 | 5 | Plasma | Intraveno usly | 10% DMA+10% |
| | | | | | | | | Solutol+80% Saline |
| G2 | 3 | Compounds | 20 or 5 | 2 or 0.5 | 10 | Plasma | Intragastri cally | 5% DMSO+5% |
| | | | | | | | | Solutol+30% |
| | | | | | | | | PEG400+60% (20% SBE-CD) |

| | | | | | | | | |
|---|---|---|---|---|---|---|---|---|
| *Dosage is calculated based on free base. | | | | | | | | |

### Sampling

Before and after the administration, 0.1 mL of blood was taken from the orbit of the rats under isoflurane anesthesia, and placed in an EDTAK2 centrifuge tube. Centrifugation was carried out at 5000 rpm at 4°C for 10 min, and the plasma was collected.

Time points for sample collection in G1&G2 groups comprise
G1: 0, 5 min, 15 min, 30 min, 1, 2, 4, 6, 8 and 24 h;
G2: 0, 15 min, 30 min, 1, 2, 4, 6, 8 and 24 h.

Before analysis and detection, all samples were stored at -80°C. The samples were detected by HPLC-MS/MS.

**Table 2 Pharmacokinetic parameters of compounds in plasma of rats**

| Test compounds | Mode of administration* | AUC₀₋ₜ (ng/mL·h) | F (%) |
|---|---|---|---|
| Compound 84 | i.g.(20 mg/kg) | 176 ± 57 | N/A |
| Compound 85 | i.g.(20 mg/kg) | 3034 ± 982 | 17.4 ± 5.6 |
| Compound 86 | i.g.(20 mg/kg) | 247 ± 73 | N/A |
| Compound 95 | i.g.(20 mg/kg) | 4267 ± 739 | 12.3 ± 2.1 |
| Trifluoroacetate of compound 98 | i.g.(20 mg/kg) | 347 ± 55 | N/A |
| Trifluoroacetate of compound 99 | i.g.(5 mg/kg) | 504 ± 131 | N/A |
| Compound 104 | i.g.(20 mg/kg) | 1323 ± 653 | N/A |
| Compound 106 | i.g.(20 mg/kg) | 3207 ± 728 | 14.4 ± 3.3 |
| Compound 107 | i.g.(20 mg/kg) | 610 ± 131 | N/A |
| Compound 110 | i.g.(20 mg/kg) | 614 ± 125 | N/A |
| Compound 111 | i.g.(20 mg/kg) | 753 ± 261 | N/A |
| Compound 112 | i.g.(20 mg/kg) | 1785 ± 663 | N/A |
| Compound 113 | i.g.(20 mg/kg) | 3819 ± 811 | N/A |
| Compound 114 | i.g.(20 mg/kg) | 2668 ± 738 | N/A |
| Compound 115 | i.g.(20 mg/kg) | 5818 ± 2260 | N/A |
| Compound 116 | i.g.(20 mg/kg) | 4439 ± 544 | N/A |
| Compound 117 | i.g.(20 mg/kg) | 2281 ± 64 | N/A |
| Compound 118 | i.g.(20 mg/kg) | 2083 ± 985 | N/A |

| | | | |
|---|---|---|---|
| *Notes: i.g. (intragastrically) administration of the compounds. N/A represents that no test is performed. Conclusion: The compounds synthesized by using the technique of the present invention had a certain oral absorption in rats. | | | |

### 3. Detection of BTK degradation in Mino cells

Mino human mantle cell lymphoma cell line was purchased from ATCC and cultured under the following conditions: RPMI-1640 + 15% FBS + 1% double antibody in a 37°C, 5% CO₂ incubator. Cells were plated in a 6-well plate, with 5 × 10⁵ cells/well. After plating, the compounds at different concentrations were added and cultured in an incubator at 37°C under 5% CO₂ for 48 hours. After culturing, the cells were collected, and RIPA lysis buffer (Beyotime, Cat. P0013B) was added. The cells were lysed on ice for 15 minutes, and centrifuged at 12000 rpm at 4°C for 10 minutes. The protein sample of the supernatant was collected, and the protein was quantified by using the BCA kit (Beyotime, Cat. P0009), and then the protein was diluted to 0.25 mg/mL. The expressions of BTK (CST, Cat. 8547S) and the internal reference β-actin (CST, Cat. 3700S) were detected using a fully automated western blot quantitative analyzer (Proteinsimple) with a kit (Protein simple, Cat. SM-W004). The expression level of BTK relative to the internal reference was calculated using Compass software, and the DC₅₀ value was calculated using Origin9.2 software according to formula (3). Specifically, the BTK_{administration} denoted the expression level of BTK in administration groups at different doses, and the BTK_{vehicle} denoted the expression level of BTK in the vehicle control group. $BTK % = {BTK}_{administration} / {BTK}_{vehicle} \times 100 %$

**Table 3 DC₅₀ value of BTK degradation in Mino cells**

| Serial No. | Compound No. | DC₅₀ (nM) |
|---|---|---|
| 1 | Compound 85 | 1.6 |

| | | |
|---|---|---|
| Conclusion: The compounds synthesized by using the technique of the present invention had a significant degrading effect on BTK in Mino cells. | | |

### 4. Detection of BTK protein degradation in spleen of mice

Female ICR mice, 6-8 weeks old, were purchased from BEIJING VITAL RIVER LABORATORY ANIMAL TECHNOLOGY CO., LTD, and the experiment was started after 3 days of adaptation. After 3 consecutive days of intragastric administration of different doses of the compound, the spleen of mice was taken, the spleen cells were collected, and RIPA lysis buffer (Beyotime, Cat. P0013B) was added. The cells were lysed on ice for 15 minutes, and centrifuged at 12000 rpm at 4°C for 10 minutes. The protein sample of the supernatant was collected, and the protein was quantified by using the BCA kit (Beyotime, Cat. P0009), and then the protein was diluted to 0.25 mg/mL. The expressions of BTK (CST, Cat. 8547S) and the internal reference β-actin (CST, Cat. 3700S) were detected using a fully automated western blot quantitative analyzer (Proteinsimple). The expression level of BTK relative to the internal reference was calculated using Compass software, and the DD₅₀ value was calculated using Origin9.2 software according to formula (4). Specifically, the BTK_{administration} denoted the expression level of BTK in administration groups at different doses, and the BTK_{vehicie} denoted the expression level of BTK in the vehicle control group. $BTK % = {BTK}_{administration} {/BTK}_{vehicle} \times 100 %$

**Table 4 DD₅₀ value of compounds on BTK protein degradation in spleen of mice**

| Serial No. | Compound No. | DD₅₀ (mg/kg) |
|---|---|---|
| 1 | Compound 85 | 3.3 |

| | | |
|---|---|---|
| Conclusion: The compounds synthesized by using the technique of the present invention had a significant degrading effect on BTK protein in spleen of mice. | | |

### 5. Detection of Aiolos degradation in Mino cells

Mino is a human mantle cell lymphoma cell line purchased from ATCC and cultured under the following conditions: RPMI-1640 + 15% FBS + 1% double antibody in a 37°C, 5% CO₂ incubator. Cells were plated in a 6-well plate, with 5 × 10⁵ cells/well. After plating, the compounds at different concentrations were added and cultured in an incubator at 37°C under 5% CO₂ for 48 hours. After culturing, the cells were collected, and RIPA lysis buffer (Beyotime, Cat. P0013B) was added. The cells were lysed on ice for 15 minutes, and centrifuged at 12000 rpm at 4°C for 10 minutes. The protein sample of the supernatant was collected, and the protein was quantified by using the BCA kit (Beyotime, Cat. P0009), and then the protein was diluted to 0.25 mg/mL. The expressions of Aiolos (CST, Cat. 15103S) and the internal reference GAPDH (CST, Cat. 5174S) were detected using a fully automated western blot quantitative analyzer (Proteinsimple). The expression level of Aiolos relative to the internal reference was calculated using Compass software, and the DC₅₀ value was calculated using Origen9.2 software according to formula (5). Specifically, the Aiolos_{administration} denoted the expression level of Aiolos in administration groups at different doses, and the Aiolos_{vehicle} denoted the expression level of Aiolos in the vehicle control group. $Aiolos % = {Aiolos}_{administration} / {Aiolos}_{vehicle} \times 100 %$

**Table 5 DC₅₀ value of Aiolos degradation in Mino cells**

| Serial No. | Compound No. | DC₅₀ (nM) |
|---|---|---|
| 1 | Compound 85 | ≥ 1000 |

| | | |
|---|---|---|
| Conclusion: The compounds synthesized by using the technique of the present invention had no obvious degrading effect on Aiolos in Mino cells. | | |

### 6. Experimental method and experimental results of the effect of the compound of the present invention on hERG potassium ion channel

Experimental platform: electrophysiological manual patch-clamp system

Cell line: Chinese hamster ovary (CHO) cells stably expressing hERG potassium ion channel

Experimental method: In CHO (Chinese Hamster Ovary) cells stably expressing hERG potassium channel, whole cell patch-clamp technique was used to recorded hERG potassium channel current at room temperature. The glass microelectrode is made of a glass electrode blank (BF150-86-10, Sutter) by a puller. The tip resistance after filling the liquid in the electrode is about 2-5 MΩ. The glass microelectrode can be connected to the patch-clamp amplifier by inserting the glass microelectrode into an amplifier probe. The clamping voltage and data recording are controlled and recorded by the pClamp 10 software through a computer. The sampling frequency is 10 kHz, and the filtering frequency is 2 kHz. After the whole cell records were obtained, the cells were clamped at -80 mV, and the step voltage that induced the hERG potassium current (*I* _{hERG}) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

### Data processing:

Data analysis processing was carried out by using pClamp 10, GraphPad Prism 5 and Excel software. The inhibition degree of hERG potassium current (peak value of hERG tail current induced at -50 mV) at different compound concentrations was calculated by the following formula: $Inhibition% = \left[1-\left(I/{I}_{o}\right)\right] \times 100 %$

Among the formula, Inhibition % represents the percentage of inhibition of hERG potassium current by the compound, and I and *Iₒ* represent the amplitude of hERG potassium current after and before dosing, respectively.

Compound IC₅₀ was calculated using GraphPad Prism 5 software by fitting according to the following equation: $Y = Bottom + \left(Top-Bottom\right) / \left(1+{10}^{∧}\left(\left({LogIC}_{50}-X\right)*HillSlope\right)\right)$

Among the equation, X represents the Log value of the tested concentration of the test sample, Y represents the inhibition percentage at the corresponding concentration, and Bottom and Top represent the minimum and maximum inhibition percentage, respectively.

The IC₅₀ value of the inhibitory effect of the compound of the present invention on hERG potassium channel current is shown in Table 6.

**Table 6 IC₅₀ value of inhibitory effect on hERG potassium channel current**

| Serial No. | Compound No. | IC₅₀ (µM) |
|---|---|---|
| 1 | Compound 113 | > 40 |
| 2 | Compound 115 | > 40 |

| | | |
|---|---|---|
| Conclusion: The compounds synthesized by using the technique of the present invention had no obvious inhibitory effects on hERG potassium channel current. | | |

## Claims

1. A compound represented by general formula (I) or a stereoisomer, a solvate, a prodrug, a metabolite, a pharmaceutically acceptable salt or a co-crystal thereof, wherein
B-L-K (I);
L is selected from -Ak1-Cy1-Ak2-Cy2-Ak3-Cy3-Ak4-Cy4-Ak5- or a bond;
Ak1, Ak2, Ak3, Ak4 and Ak5 are each independently selected from CH₂, O or a bond;
Cy1, Cy2, Cy3 and Cy4 are each independently selected from a 3- to 12-membered heterocyclic ring, 3- to 12-membered cycloalkyl, 6- to 10-membered aryl or a bond, wherein the heterocyclic ring, cycloalkyl or aryl is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy, and the heterocyclic ring contains 1 to 4 heteroatoms selected from O, S or N;
Cy1, Cy2, Cy3 and Cy4 cannot all be a bond;
when Ak1, Ak2, Ak3, Ak4 or Ak5 is O, they cannot be directly connected to B;
when Ak1, Ak2, Ak3, Ak4 or Ak5 is not a bond, they cannot be directly connected to one another;
when 4 or more of Ak1, Cy1, Ak2, Cy2, Ak3, Cy3, Ak4, Cy4 and Ak5 are not a bond, at least one of Cy1, Cy2, Cy3 and Cy4 is not piperidine, piperazine, pyrimidine or pyridine;
B is selected from B1-W1-B2-B3-B4-;
B1 is selected from a 6-membered heteroaryl ring or phenyl, wherein the heteroaryl ring or phenyl is optionally further substituted with 0 to 4 R^{b1}, and the heteroaryl ring contains 1 to 4 heteroatoms selected from O, S or N;
W1 is selected from -O-, -S-, -NH-, -NHCO- or -CONH-;
B2 is selected from a 6-membered heteroaryl ring or phenyl, wherein the heteroaryl ring or phenyl is optionally further optionally substituted with 0 to 4 R^{b2}, and the heteroaryl ring contains 1 to 4 heteroatoms selected from O, S or N;
B3 is selected from an 8- to 10-membered fused heterocyclic ring, wherein the fused heterocyclic ring is optionally further optionally substituted with 0 to 4 R^{b3}, and the fused heterocyclic ring contains 1 to 4 heteroatoms selected from O, S or N;
B4 is selected from a 4- to 10-membered saturated heterocyclic ring, wherein the saturated heterocyclic ring is selected from a monocyclic ring, a fused ring or a spiro ring, the saturated heterocyclic ring, monocyclic ring, fused ring or spiro ring is optionally further optionally substituted with 0 to 4 R^{b4}, and the saturated heterocyclic ring contains 1 to 2 heteroatoms selected from O, S or N;
when 3 of Cy1, Cy2, Cy3 and Cy4 are a bond, B is not 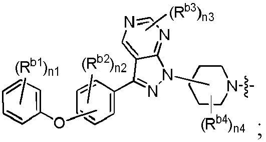
R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
K is selected from 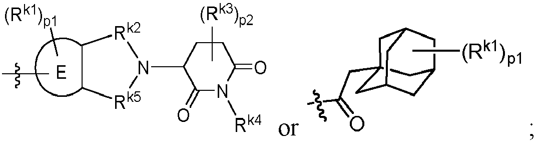
ring E is selected from a benzene ring or a 5- to 6-membered heteroaryl ring, wherein the heteroaryl ring contains 1 to 2 heteroatoms selected from O, S or N;
R^{k2} is each independently selected from CH₂, C=O, S=O or SO₂;
R^{k1}, R^{k3} or R^{k4} is each independently selected from H, F, Cl, Br, I, OH, NH₂, CF₃, CN, COOH, C₁₋₄ alkyl or C₁₋₄ alkoxy;
R^{k5} is selected from C=O or 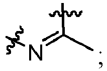 p1 or p2 is each independently selected from 0, 1, 2, 3 or 4;
n1, n2, n3, and n4 are each independently selected from 0, 1, 2, 3 or 4; and
the compound is not a compound shown in Table A.

2. The compound or the stereoisomer, solvate, prodrug, metabolite, pharmaceutically acceptable salt or co-crystal thereof according to claim 1, wherein
Cy1, Cy2, Cy3 and Cy4 are each independently selected from a bond, a 4- to 7-membered mono-heterocyclic ring, a 5- to 10-membered fused heterocyclic ring, a 6- to 12-membered spiro-heterocyclic ring, a 7- to 10-membered bridged-heterocyclic ring, a 4- to 7-membered monocycloalkyl, a 5- to 10-membered fused cycloalkyl, a 6- to 12-membered spiro cycloalkyl, a 7- to 10-membered bridged cycloalkyl or a 6- to 10-membered aryl, wherein the aryl, cycloalkyl, mono-heterocyclic ring, fused heterocyclic ring, spiro-heterocyclic ring or bridged-heterocyclic ring is optionally further substituted with 0 to 4 substituents selected from H, F, Cl, Br, I, OH, NH₂, oxo, CF₃, COOH, CN, C₁₋₄ alkyl, hydroxyl-substituted C₁₋₄alkyl, halogen-substituted C₁₋₄ alkyl or C₁₋₄ alkoxy, and the mono-heterocyclic ring, fused heterocyclic ring, spiro-heterocyclic ring or bridged-heterocyclic ring contains 1 to 4 heteroatoms selected from O, S or N;
B1 is selected from phenyl or pyridyl, wherein the phenyl or pyridyl is optionally further substituted with 0 to 4 R^{b1};
W1 is selected from -O-, -NHCO- or -CONH-;
B2 is selected from phenyl or pyridyl, wherein the phenyl or pyridyl is optionally further substituted with 0 to 4 R^{b2};
B3 is selected from one of the following substituted or unsubstituted groups: imidazopyrimidine, pyrazolopyrimidine, imidazopyrazine, pyrazolopyrazine, imidazotetrahydropyrimidine or pyrazolotetrahydropyrimidine which, when substituted, is optionally further optionally substituted with 0 to 4 R^{b3};
B4 is selected from one of the following substituted or unsubstituted groups: azetidinyl, azacyclopentyl, piperidine, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, cyclobutyl-spiro-piperidine, cyclopentyl-spiro-piperidine, azetidinyl-spiro-piperidine, azacyclopentyl-spiro-piperidine, 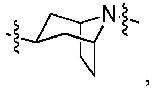 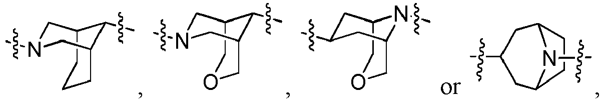 which, when substituted, is optionally further optionally substituted with 0 to 4 R^{b4}; and
ring E is selected from a benzene ring, pyridine, thiophene, furan, pyrrole, thiazole, oxazole, imidazole or pyrazole.

3. The compound or the stereoisomer, solvate, prodrug, metabolite, pharmaceutically acceptable salt or co-crystal thereof according to claim 2, wherein
K is selected from 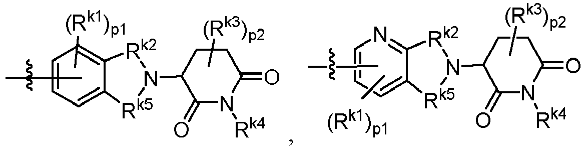 or

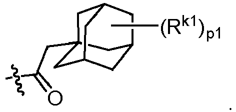

4. The compound or the stereoisomer, solvate, prodrug, metabolite, pharmaceutically acceptable salt or co-crystal thereof according to claim 3, wherein
Cy1, Cy2, Cy3 and Cy4 are each independently selected from a bond or one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 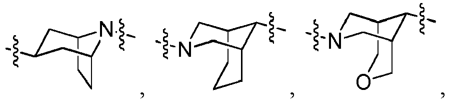 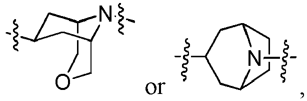 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
B is selected from 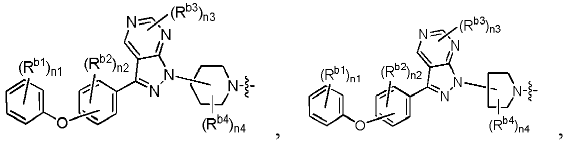 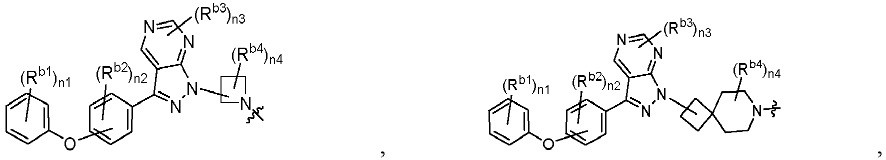 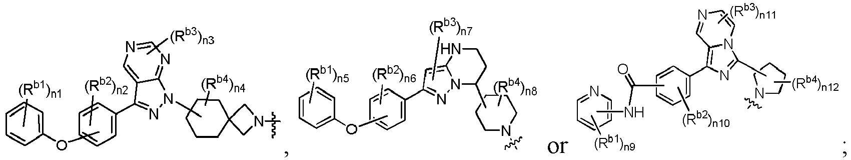
or B is selected from 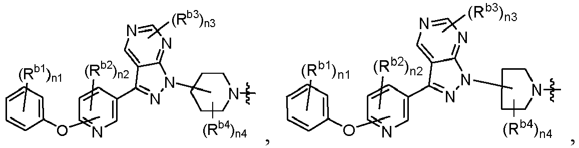 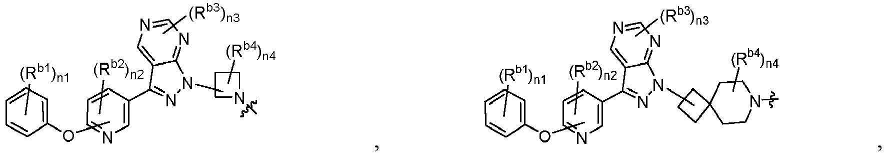 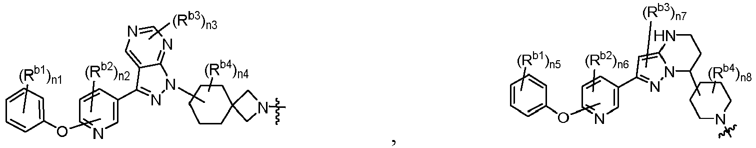 or 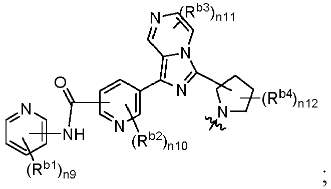
R^{b1}, R^{b2}, R^{b3} or R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I;
n1, n2, n3, n4, n5, n6, n7, n8, n9, n10, n11, and n12 are each independently selected from 0, 1, 2, 3 or 4;
K is selected from 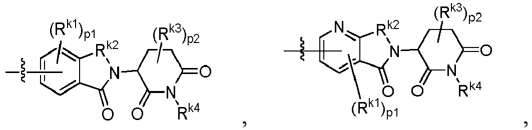 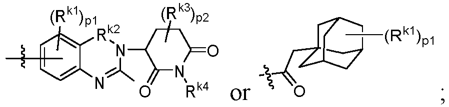
R^{k2} is each independently selected from CH₂ or C=O;
R^{k1}, R^{k3} or R^{k4} is each independently selected from H, F, Cl, Br, I, OH or NH₂; and
p1 or p2 is each independently selected from 0, 1 or 2.

5. The compound or the stereoisomer, solvate, prodrug, metabolite, pharmaceutically acceptable salt or co-crystal thereof according to claim 3, wherein the compound is represented by general formula (Ia), 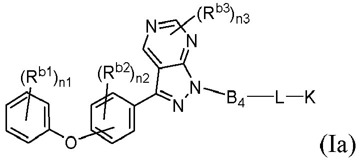
B4 is selected from one of the following substituted or unsubstituted groups: azetidinyl, azacyclopentyl, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, cyclobutyl-spiro-piperidine, cyclopentyl-spiro-piperidine, azetidinyl-spiro-piperidine, azacyclopentyl-spiro-piperidine, 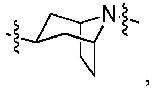 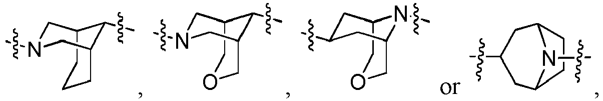 which, when substituted, is optionally further optionally substituted with 0 to 4 R^{b4};
R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
the definitions of L and K are consistent with those in claim 3; and
n1, n2 and n3 are each independently selected from 0, 1, 2, 3 or 4.

6. The compound or the stereoisomer, solvate, prodrug, metabolite, pharmaceutically acceptable salt or co-crystal thereof according to claim 3, wherein the compound is represented by general formula (Ib), 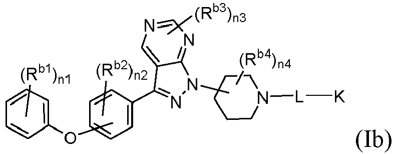
Cy1, Cy2, Cy3 and Cy4 are each independently selected from a bond or one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 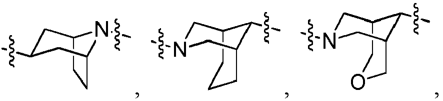 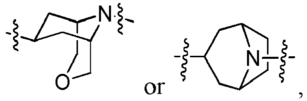 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
provided that at least one of Cy1, Cy2, Cy3 and Cy4 is substituted with 1 to 4 substituents selected from CF₃, COOH, CN or hydroxyl-substituted C₁₋₄ alkyl;
R^{b1}, R^{b2}, R^{b3} or R^{b4} is each independently selected from H, F, Cl, Br, I, OH, NH₂, CN, CF₃, COOH, CONH₂, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy;
the definitions of L and K are consistent with those in claim 3; and
n1, n2, n3, and n4 are each independently selected from 0, 1, 2, 3 or 4.

7. The compound or the stereoisomer, solvate, prodrug, metabolite, pharmaceutically acceptable salt or co-crystal thereof according to claim 4, wherein
L is selected from a bond, 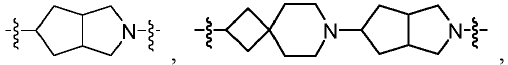 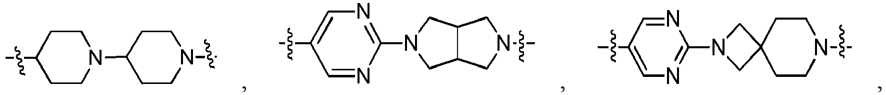 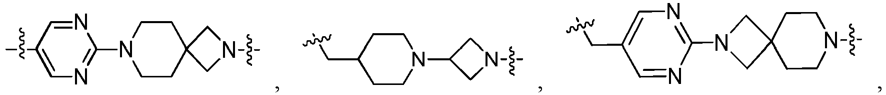 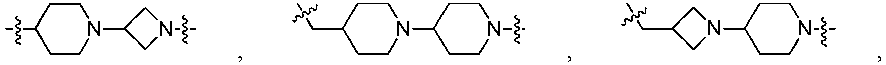 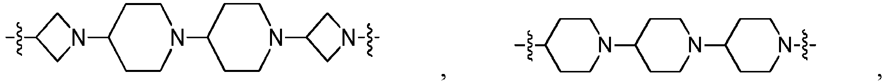 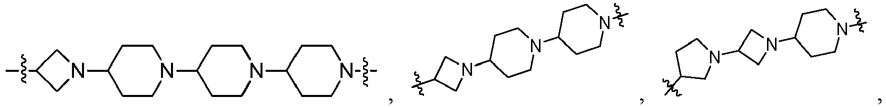 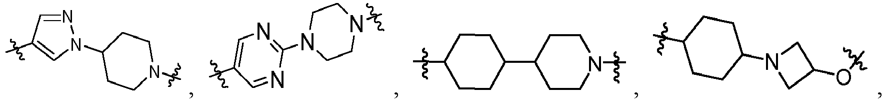 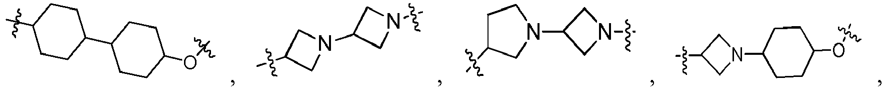 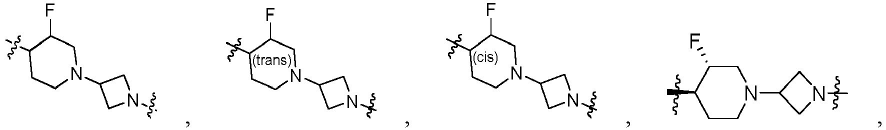 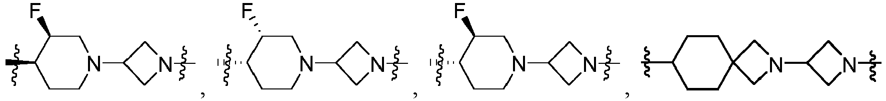 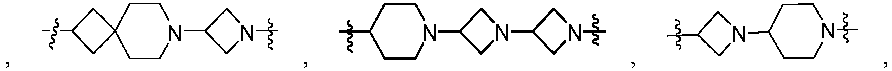 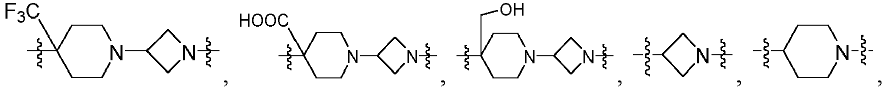 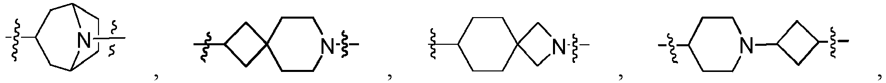 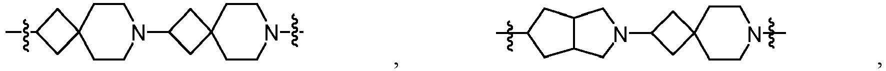 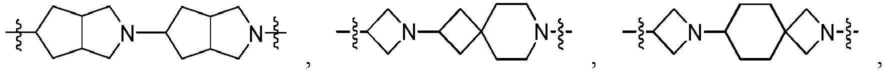 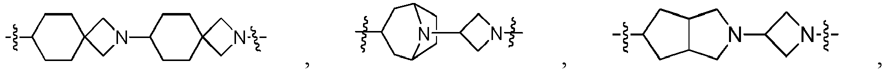 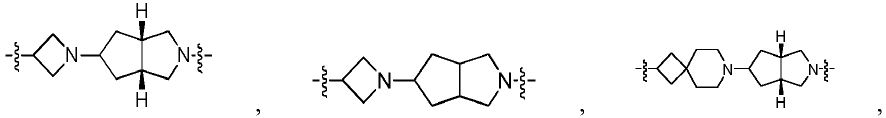 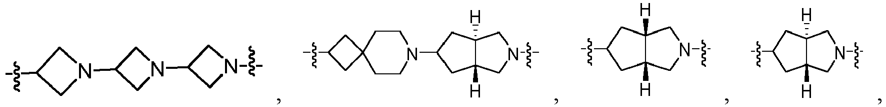 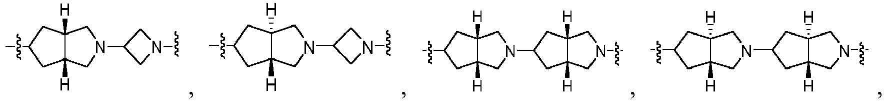 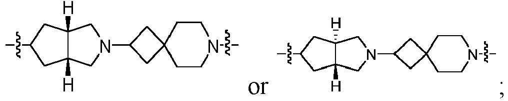
or L is selected from 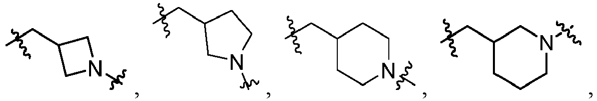 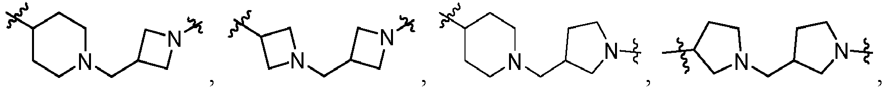 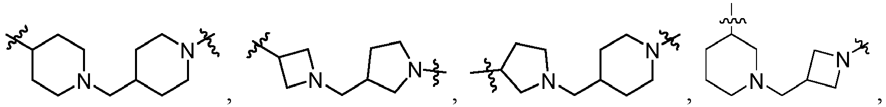 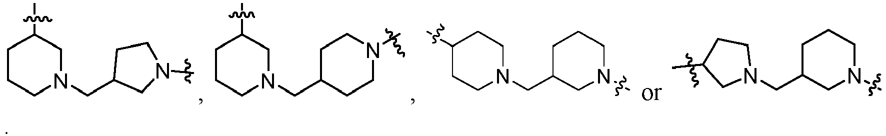
; or L is selected from 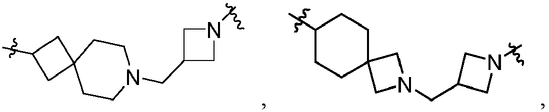 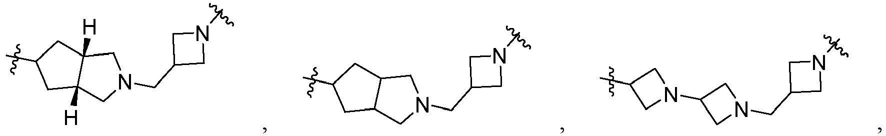 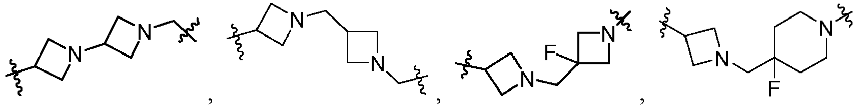 or 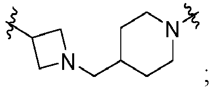
L is connected to B on the left side, and is connected to K on the right side;
B is selected from 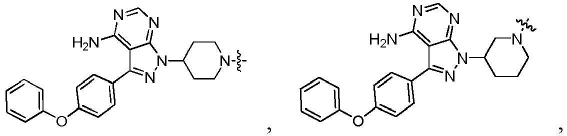 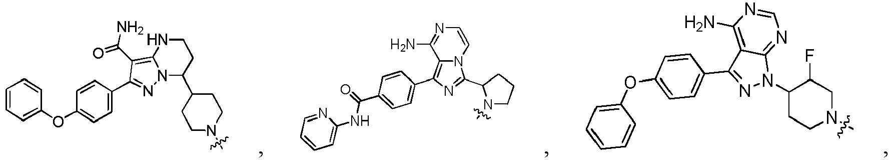 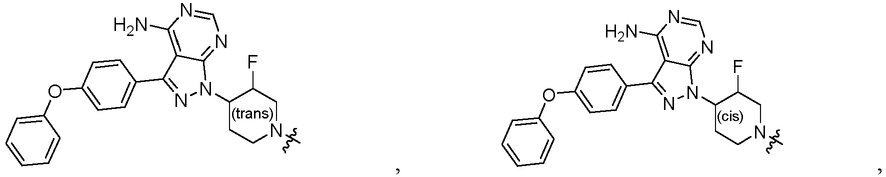 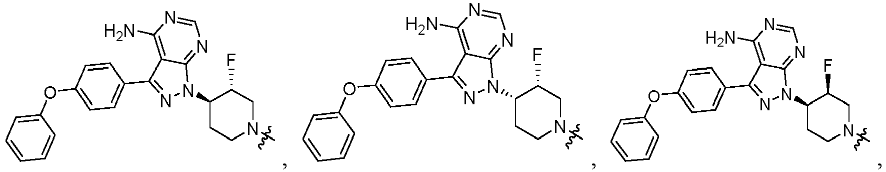 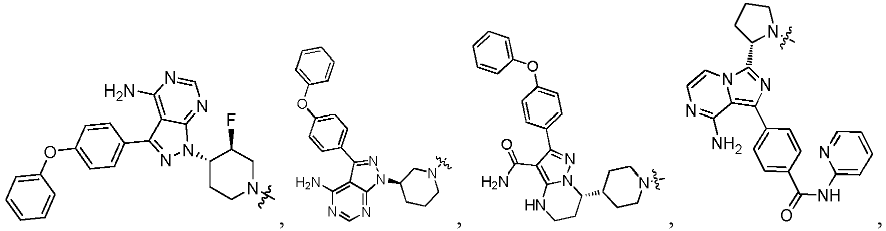 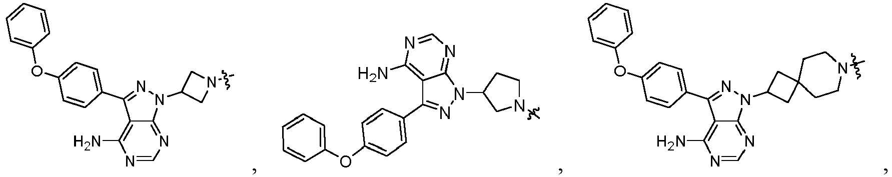 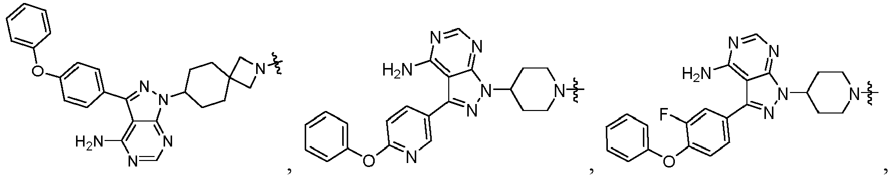 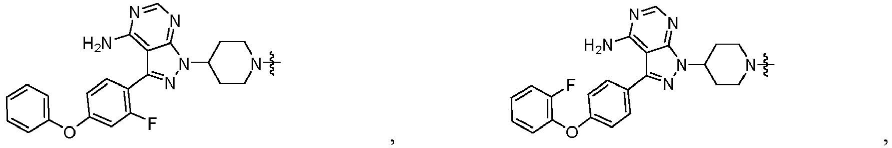 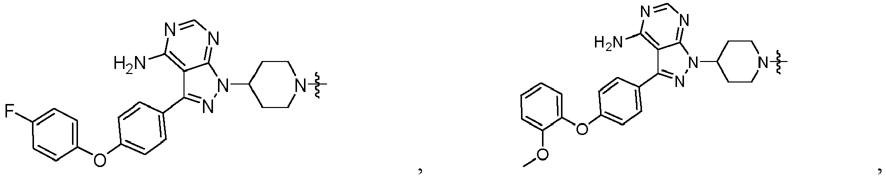 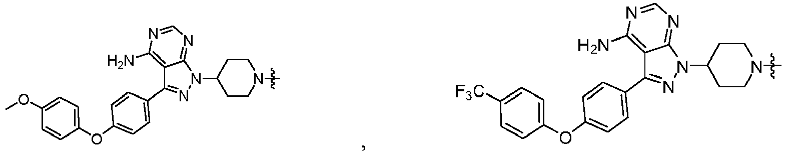 or 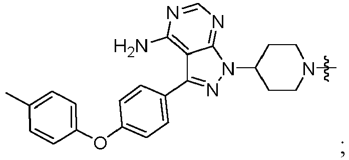 and
K is selected from

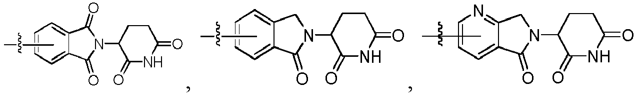

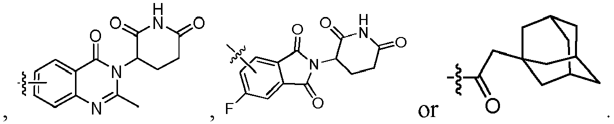

8. The compound or the stereoisomer, solvate, prodrug, metabolite, pharmaceutically acceptable salt or co-crystal thereof according to claim 1, wherein the compound is represented by general formula (Ic), 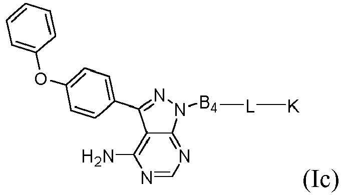 the definitions of L and K are consistent with those in claim 7; and
B4 is selected from one of the following substituted or unsubstituted groups: azetidinyl, azacyclopentyl, 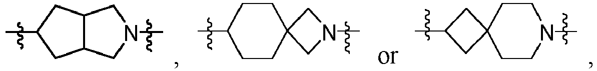 which, when substituted, is optionally further optionally substituted with 0 to 4 R^{b4}; and
R^{b4} is each independently selected from H, F, Cl, Br, I, OH, NH₂, CN, CF₃, CONH₂, COOH, C₁₋₄ alkyl, hydroxyl-substituted C₁₋₄ alkyl, halogen-substituted C₁₋₄ alkyl or C₁₋₄ alkoxy, wherein the alkyl and alkoxy are optionally further substituted with 0 to 4 substituents selected from H, F, Cl, Br, I, OH, NH₂, CN, CONH₂, C₁₋₄ alkyl or C₁₋₄ alkoxy.

9. The compound or the stereoisomer, solvate, prodrug, metabolite, pharmaceutically acceptable salt or co-crystal thereof according to claim 6, wherein the compound is represented by general formula (Ib),
L is selected from 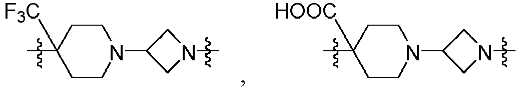 or 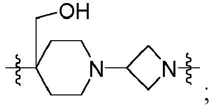
L is connected to B on the left side, and is connected to K on the right side; the definition of K is consistent with that in claim 7.

10. The compound or the stereoisomer, solvate, prodrug, metabolite, pharmaceutically acceptable salt or co-crystal thereof according to claim 8, wherein
B4 is selected from one of the following substituted or unsubstituted groups: 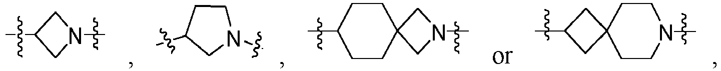 which, when substituted, is optionally further optionally substituted with 0 to 4 substituents selected from R^{b4}; and
R^{b4} is each independently selected from H, F, Cl, Br, I, OH, NH₂, CN, CONH₂, CF₃, COOH, hydroxymethyl, methyl or methoxy, wherein the methyl or methoxy is optionally further substituted with 0 to 4 substituents selected from H, F, Cl, Br or I.

11. The compound or the stereoisomer, solvate, prodrug, metabolite, pharmaceutically acceptable salt or co-crystal thereof according to any one of claims 7 to 10, wherein
K is selected from

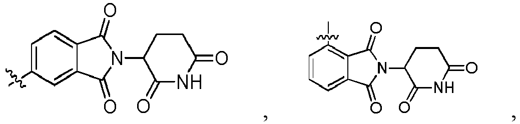

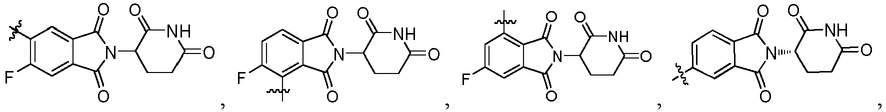

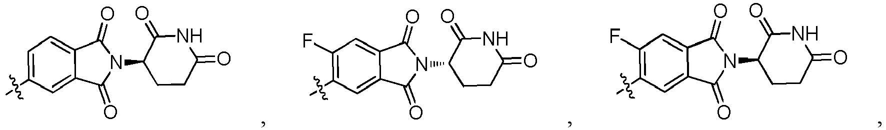

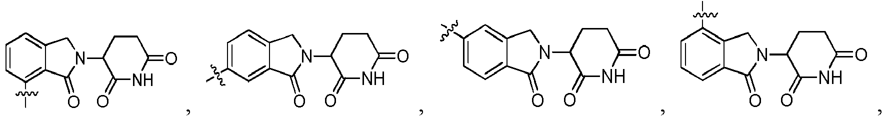

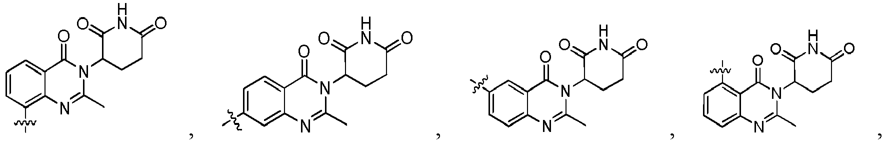

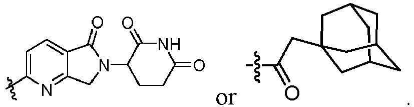

12. The compound or the stereoisomer, solvate, prodrug, metabolite, pharmaceutically acceptable salt or co-crystal thereof according to claim 11, wherein
K is selected from

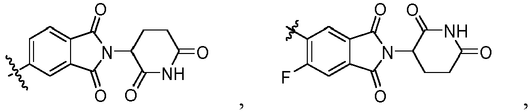

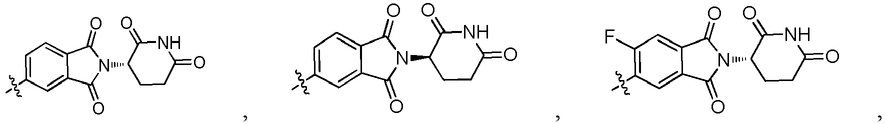

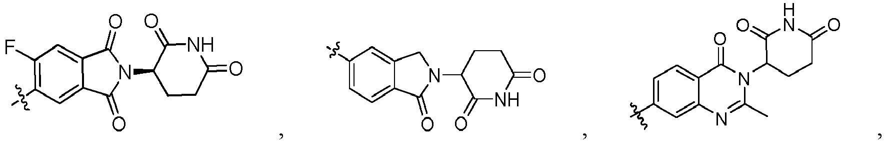

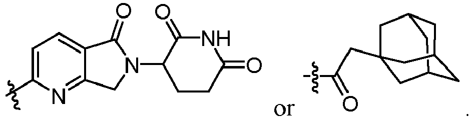

13. The compound or the stereoisomer, solvate, prodrug, metabolite, pharmaceutically acceptable salt or co-crystal thereof according to claim 12, wherein the compound is represented by general formula (I),
B is selected from

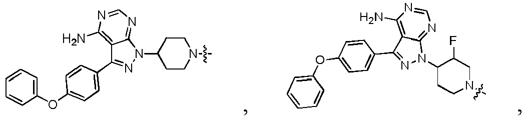

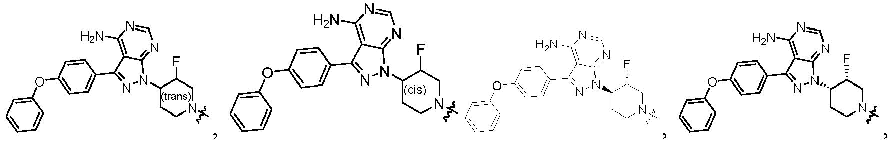

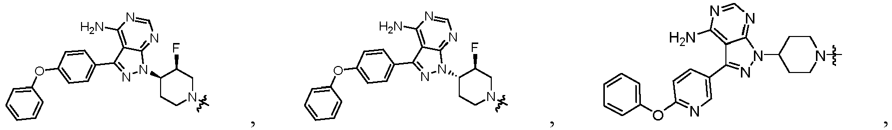

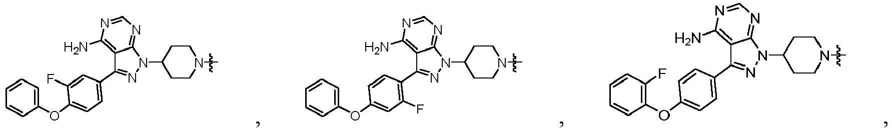

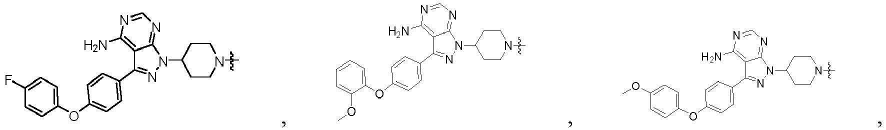

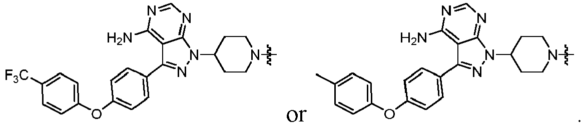

14. The compound or the stereoisomer, solvate, prodrug, metabolite, pharmaceutically acceptable salt or co-crystal thereof according to claim 12, wherein the compound is represented by general formula (I),
B is selected from

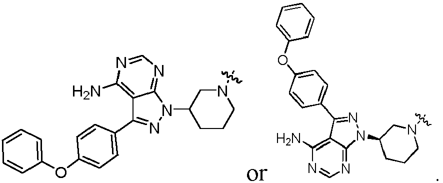

15. The compound or the stereoisomer, solvate, prodrug, metabolite, pharmaceutically acceptable salt or co-crystal thereof according to claim 12, wherein the compound is represented by general formula (I),
B is selected from

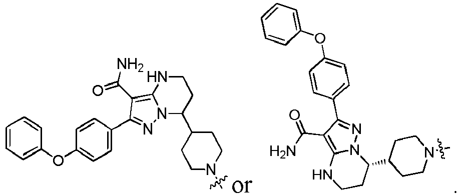

16. The compound or the stereoisomer, solvate, prodrug, metabolite, pharmaceutically acceptable salt or co-crystal thereof according to claim 12, wherein the compound is represented by general formula (I),
B is selected from

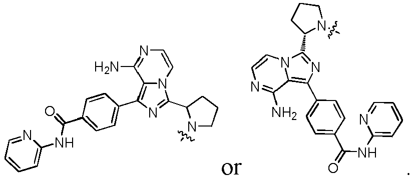

17. The compound or the stereoisomer, solvate, prodrug, metabolite, pharmaceutically acceptable salt or co-crystal thereof according to claim 12, wherein the compound is represented by general formula (I),
B is selected from

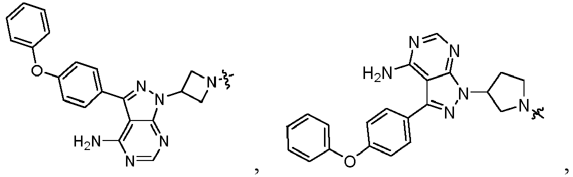

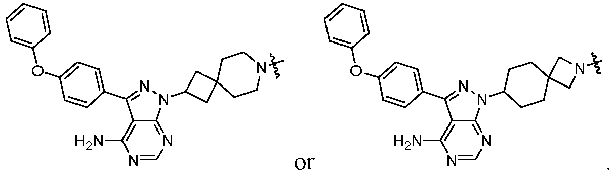

18. A compound represented by general formula (II) or a stereoisomer, a solvate, a prodrug, a metabolite, a pharmaceutically acceptable salt or a co-crystal thereof, wherein 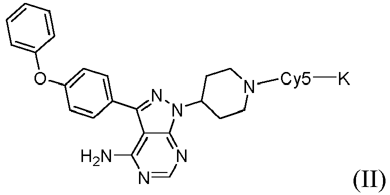
Cy5 is selected from one of the following substituted or unsubstituted groups: phenyl, naphthyl, cyclobutyl, cyclopentyl, cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-fused-cyclobutyl, cyclopropyl-fused-cyclopentyl, cyclopropyl-fused-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-spiro-cyclobutyl, cyclopropyl-spiro-cyclopentyl, cyclopropyl-spiro-cyclohexyl, azetidinyl, azacyclopentyl, piperidine, morpholine, piperazine, pyrrole, pyrazole, imidazole, thiazole, pyridine, pyridazine, pyrimidine, pyrazine, triazole, tetrazole, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine, 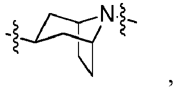 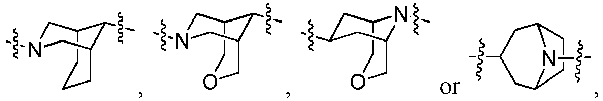 which, when substituted, is optionally further substituted with 0 to 4 substituents selected from H, F, Cl, Br, I, OH, NH₂, oxo, CF₃, COOH, CN, C₁₋₄ alkyl, hydroxyl-substituted C₁₋₄ alkyl, halogen-substituted C₁₋₄ alkyl or C₁₋₄ alkoxy;
the definition of K is consistent with that in claim 2.

19. The compound or the stereoisomer, solvate, prodrug, metabolite, pharmaceutically acceptable salt or co-crystal thereof according to claim 18, wherein
Cy5 is selected from one of the following substituted or unsubstituted groups: 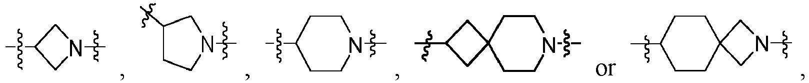 which, when substituted, is optionally further substituted with 0 to 4 substituents selected from H, F, Cl, Br, I, OH, NH₂, oxo, CF₃, COOH, CN, methyl, hydroxymethyl or methoxy; and
the definition of K is consistent with that in claim 11.

20. A compound or a stereoisomer, a solvate, a prodrug, a metabolite, a pharmaceutically acceptable salt or a co-crystal thereof, wherein the compound is of the structure selected from one of:

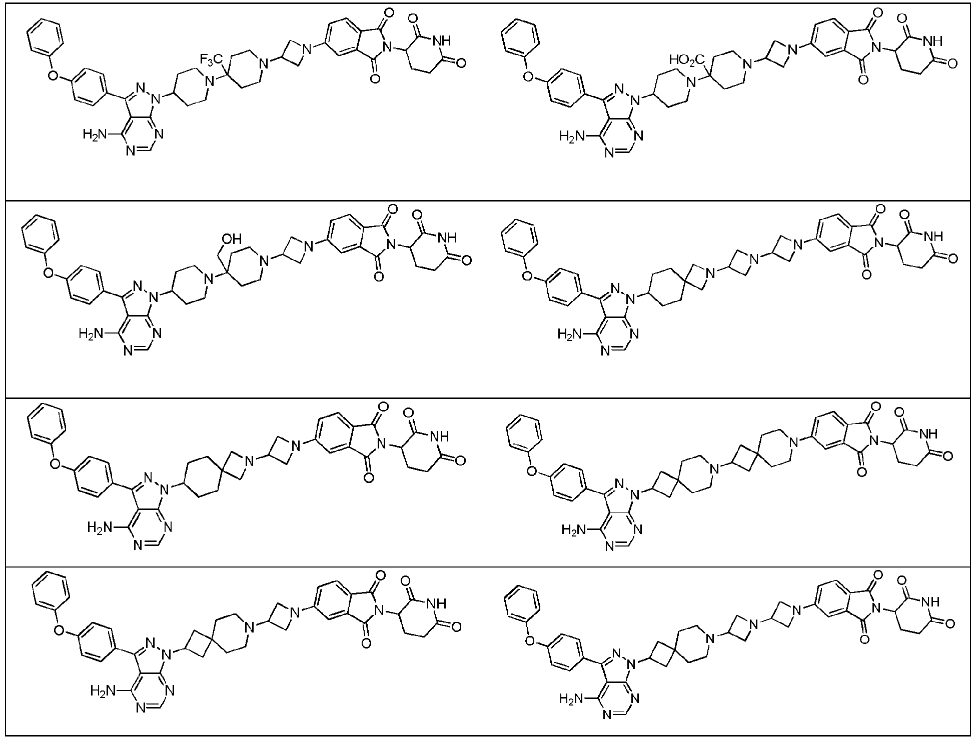

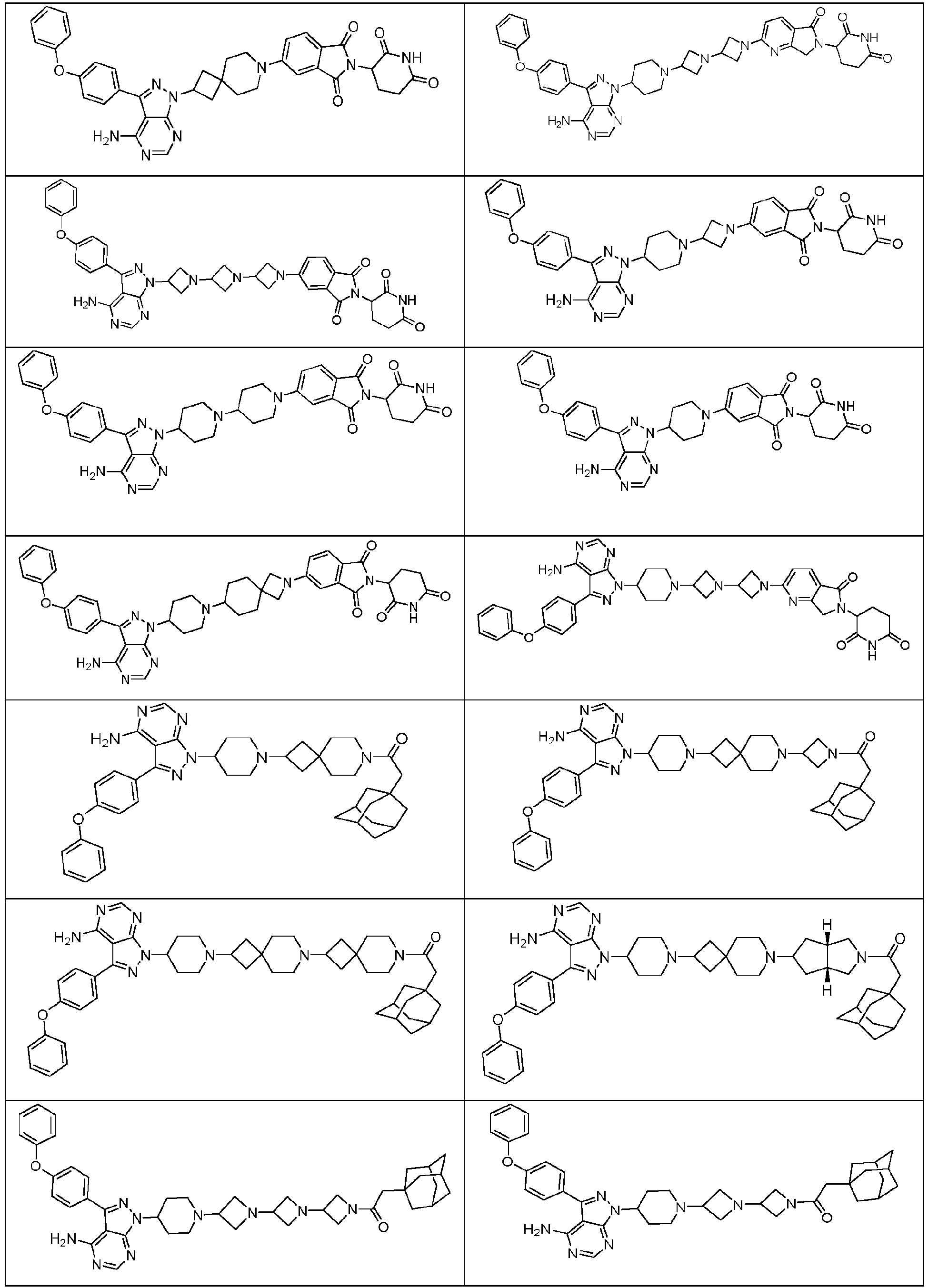

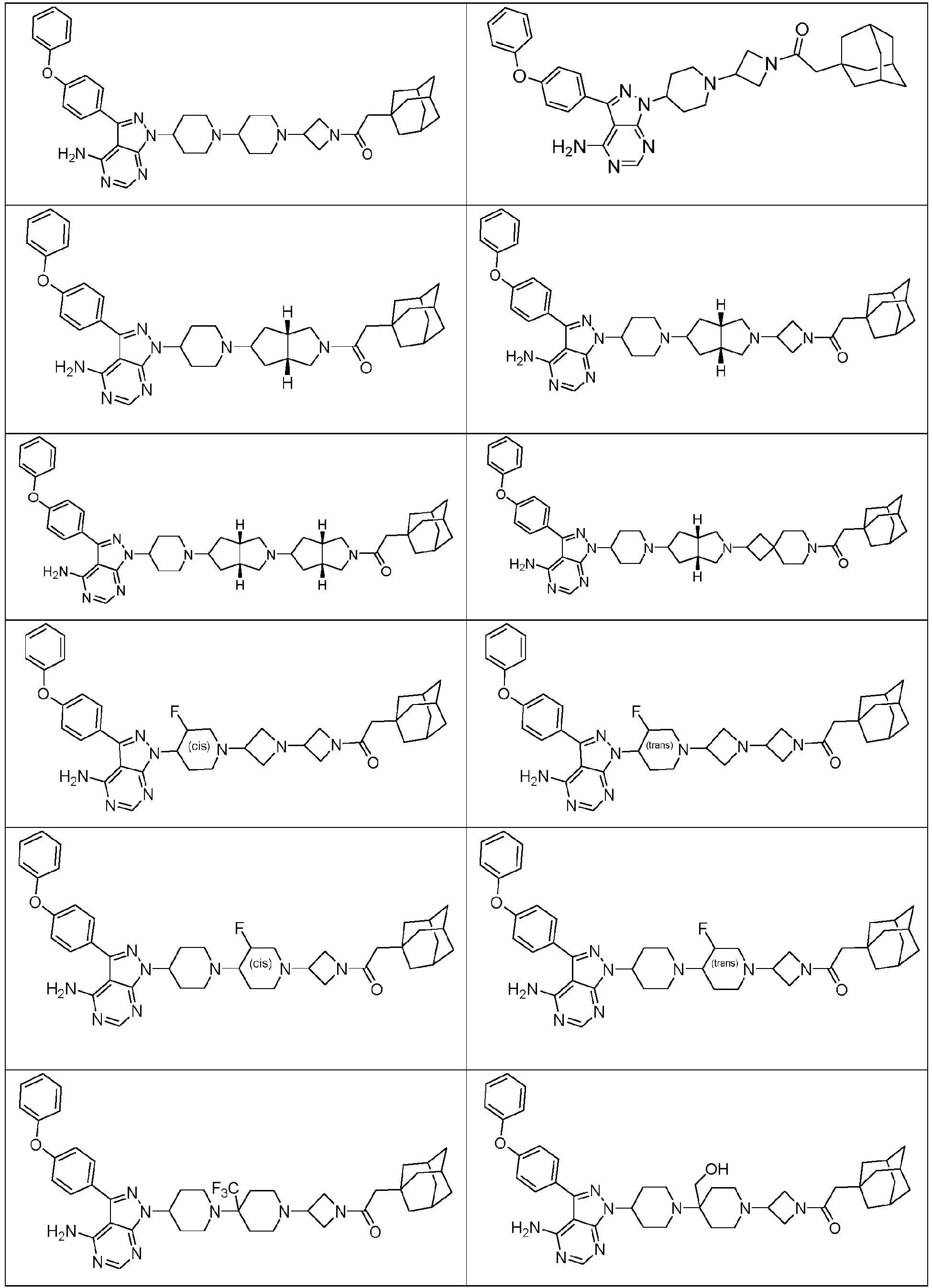

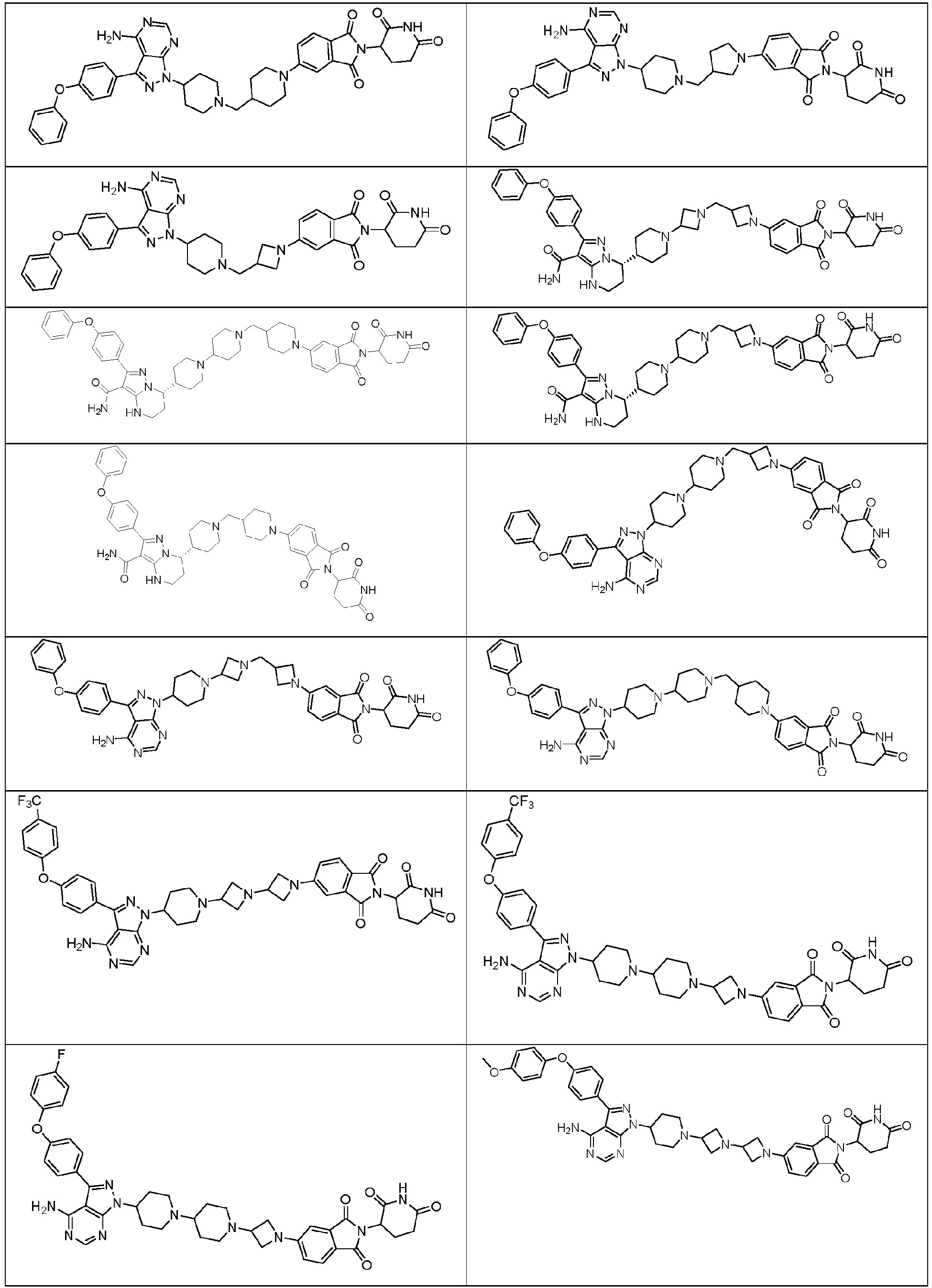

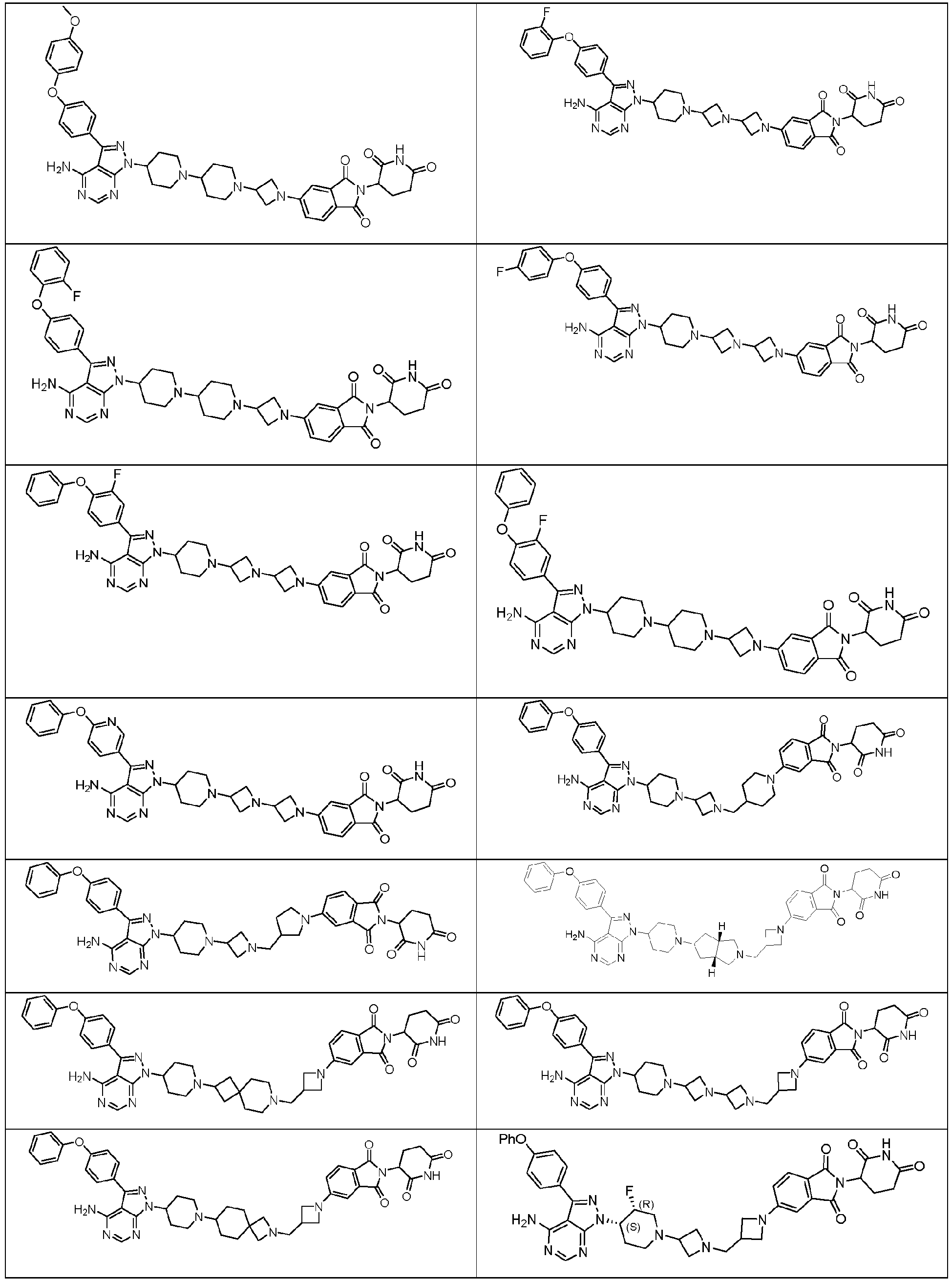

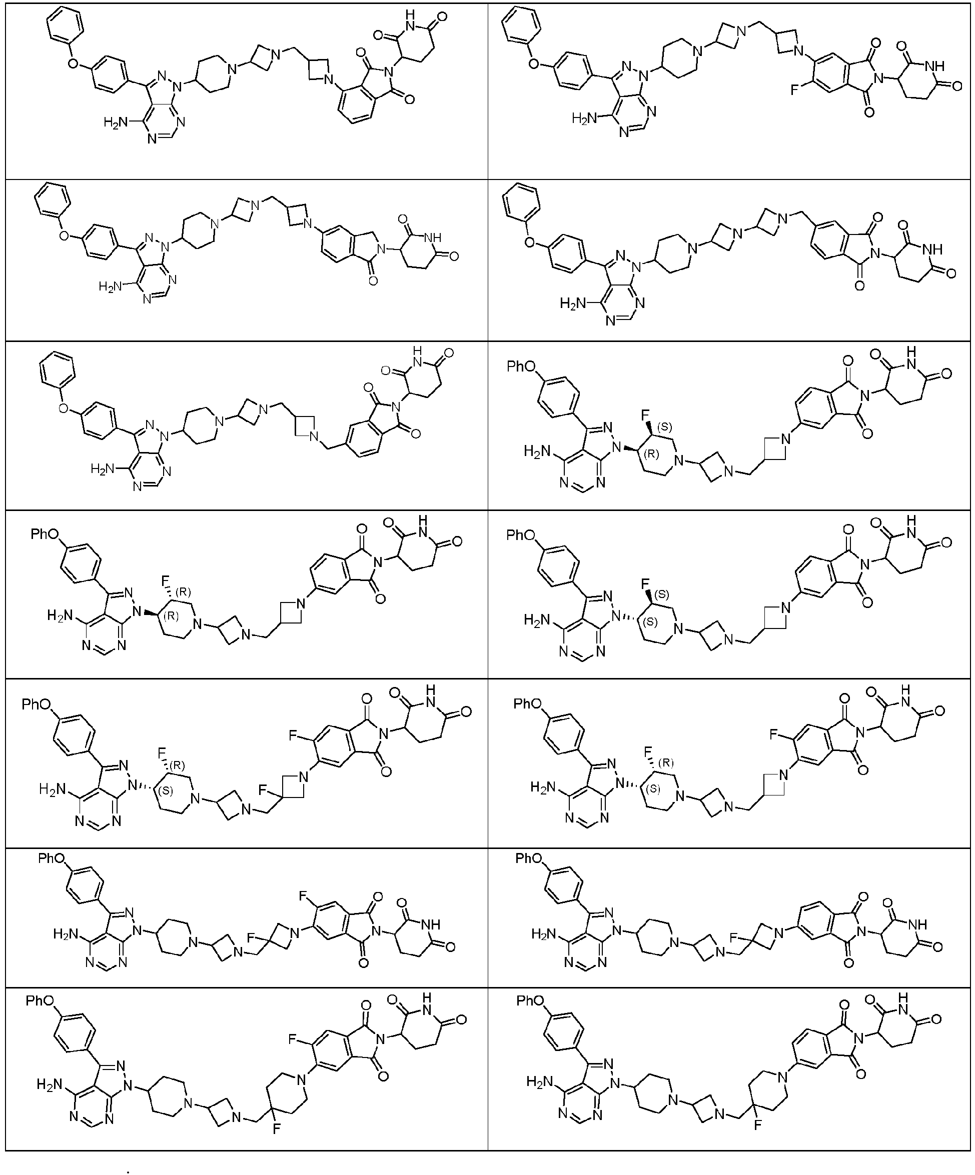

21. The compound or the stereoisomer, solvate, prodrug, metabolite, pharmaceutically acceptable salt or co-crystal thereof according to any one of claims 1 to 20, wherein the salt is selected from trifluoroacetate.

22. A pharmaceutical composition, comprising the compound or the stereoisomer, solvate, prodrug, metabolite, pharmaceutically acceptable salt or co-crystal thereof according to any one of claims 1 to 21, and a pharmaceutically acceptable carrier.

23. Use of the compound or the stereoisomer, solvate, prodrug, metabolite, pharmaceutically acceptable salt or co-crystal thereof according to any one of claims 1-21 in the manufacture of a drug for treating a disease related to BTK activity or expression level.

24. Use of the compound or the stereoisomer, solvate, prodrug, metabolite, pharmaceutically acceptable salt or co-crystal thereof according to any one of claims 1-21 in the manufacture of a drug for treating a disease related to the inhibition or degradation of BTK.

25. Use according to claim 23 or 24, wherein the disease is selected from a tumor or an autoimmune disease.

26. Use according to claim 25, wherein the tumor is selected from non-Hodgkin's lymphoma, chronic lymphocytic leukemia, mantle cell lymphoma or B cell lymphoma, and the autoimmune disease is selected from rheumatoid arthritis or psoriasis.
